(19) Europäisches Patentamt
European Patent Office
Office européen des brevets

(11) EP 1 233 059 A1

# (12) EUROPEAN PATENT APPLICATION

(43) Date of publication:
**21.08.2002 Bulletin 2002/34**

(21) Application number: **01103060.8**

(22) Date of filing: **09.02.2001**

(51) Int Cl.[7]: **C12N 7/00**, C12N 15/86, C07K 14/11, A61K 35/76, A61K 39/145

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **ARTEMIS Pharmaceuticals GmbH**
**51063 Köln (DE)**

(72) Inventors:
- **Hobom, Gert**
  **79106 Freiburg (DE)**
- **Menke, Annette**
  **50670 Köln (DE)**

(74) Representative:
**Helbing, Jörg, Dr. Dipl.-Chem. et al**
**Patentanwälte**
**von Kreisler-Selting-Werner,**
**Postfach 10 22 41**
**50462 Köln (DE)**

## (54) Influenza viruses with enhanced transcriptional and replicational capacities

(57) The present invention provides human influenza viruses comprising an RNA-sequence encoding a modified RNA-polymerase, a process for the preparation thereof, pharmaceutical compositions comprising said human influenza viruses and their use for gene transfer into mammalian cells, for *ex vivo* gene transfer into antigen-presenting cells, such as dendritic cells, for *in vivo* somatic gene therapy, or *in vivo* vaccination purposes. The invention also relates to other non-avian influenza viruses, including equine, porcine (swine) influenza viruses.

EP 1 233 059 A1

## Description

The present invention provides human influenza viruses comprising an RNA-sequence encoding a modified RNA-polymerase, a process for the preparation thereof, pharmaceutical compositions comprising said human influenza viruses and their use for gene transfer into mammalian cells, for *ex vivo* gene transfer into antigen-presenting cells, such as dendritic cells, for *in vivo* somatic gene therapy, or *in vivo* vaccination purposes. The invention also relates to other non-avian influenza viruses, including equine, porcine (swine) influenza viruses.

## Background of the Invention

The RNA-dependent RNA-polymerase of the influenza virus, which is comprised of three viral polymerase (P) subunits, PB1, PB2 and PA, catalyses the synthesis of both viral mRNA (transcription) as well as complementary RNA and progeny viral RNA (replication) in infected cells (Lamb R.A., Krug R.M. Fields Virology 3: pp 1353-1445 (1996)). In the virion the enzyme is found tightly associated at each of the eight different species of viral RNAs (vRNAs) with their 5' and 3' ends, which in combination constitute the promoter structure, while all other parts of the vRNA molecules are covered by a large number of influenza nuclear protein (NP) molecules, one per 27 nucleotides in average (Ortin J. et al., Options for the Control of Influenza IV; Osterhaus A., ed. in print (2000)), altogether described as the viral RNP complexes. Upon infection the vRNPs are released from the virion and transferred into the nucleus of the infected cell, where viral mRNA synthesis is initiated by the promoter-associated enzyme according to the cap-snatching scheme, i.e. employing primer oligonucleotides that are derived from cellular mRNAs or hnRNAs by endonucleolytic cleavage (Krug R.M. et al., The Influenza Viruses, Plenum Press, New York, NY, pp. 1-87 (1989)). While during progression of mRNA synthesis along the vRNA template molecule its 3'end looses contact to viral polymerase, the enzyme maintains its tight association with the 5'vRNA end throughout the entire first and all consecutive rounds of transcription. Synthesis of mRNA molecules is terminated via poly-adenylation at a 5'promoter sequence-adjacent series of 5 or 6 uridine template residues, i.e. the very 5'-terminal sequence covered by the enzyme is not transcribed into viral mRNA.

The conformation of the vRNA promoter sequence in its association with viral polymerase has been demonstrated by reverse genetic analysis to constitute a "corkscrew" structure, with exposed single-stranded tetranucleotide sequences supported by two intra-strand basepairs in both the 5'and 3'branches of the promoter sequence. In the course of that analysis also several promoter-up variants of the terminal vRNA sequence have been described, mainly through base-*pair* exchanges involving positions 3 and 8 from the 3'end, and positions 3 and 8 from the 5'end (Neumann G., Hobom G., J. Gen Virol. 76 (<pt 7):1709-17 (1995); Flick R. et al., RNA, 2(10):1046-57 (1996); Flick R., Hobom G., J. Gen Virol. 80(Pt 10):2565-72 (1999); WO96/10641). With typical increases in reporter gene or other foreign gene expression of up to 20 times the wildtype promoter yields, such influenza virus vectors range also four- to fivefold above the expression level achieved with plasmid DNAs under control of the standard cytomegalovirus early promoter ($p_{CMV}$).

However, there are two limitations to this effect: 1.) That gain of 20 times the wildtype promoter level in expression efficiency is true for inserts up to 1500 nucleotides in size under control of influenza promoter-up variants, while further increases in insert size up to 3000 nucleotides will steadily reduce that gain in promoter efficiency, and only low expression rates have been achieved with inserts 4000 nucleotides in length (M. Azzeh, G. Hobom, unpublished). 2.) Such increased expression rates have only been obtained as long as avian influenza virus (fowl plague viruses, FPV: H7N7) or rather viral polymerases derived from FPVs were used together with promoter-up sequence variants; no such effect has been seen with other influenza viral strains tested : WSN (H1N1); Asia (H2N2) or Victoria (H3N2) (Hoffmann E., Hobom G., unpublished data).

Thus, what is needed for an application of those biotechnologically valuable increased expression rates exerted upon foreign genes in human cells or organs is a transfer of the FPV Bratislava polymerase properties in recognizing such promoter sequence variations into the respective polymerase coding sequences of other influenza viruses, able to replicate efficiently in human tissue. In addition, the use of H1N1 (WSN; PR/8) or H3N2 (Victoria or other) viral variants instead of FPV, if possible would constitute a gain in biological safety. Due to the amino acid sequence of H1 and H3 hemagglutinin-carrying viruses these become activated for infection only through cleavage by a narrow spectrum of proteases, as opposed to hemagglutinin H7 which becomes activated also through cleavage by a number of additional, ubiquitous proteolytic enzymes.

## Summary of the Invention

It was found that specific modifications of the RNA sequence within the respective viruses which code for the RNA-polymerase, in particular for the PB1 subunit thereof - so as to code for a polypeptide chain having a higher similarity with FPV Bratislava RNA-polymerase - provides viruses capable of recognition of vRNA and cRNA promoter sequence variations (the so called promoter-up variants mentioned above) leading to an increase in transcription and/or replication initiation rates. The present invention thus provides

(1) a human influenza virus comprising an RNA-sequence encoding a modified RNA-polymerase which differs from the wild-type RNA-polymerase of said human influenza virus in that at least one of the amino acid residue(s) distinguishing the wild-type RNA-polymerase of said human influenza virus from FPV Bratislava RNA-polymerase has been replaced with the respective amino acid residues of FPV Bratislava RNA-polymerase ("FPV Bratislava" and "FPV Bratislava RNA-polymerase" are hereinafter also shortly referred to as "FPV" and "FPV RNA-polymerase", respectively);

(2) in a preferred embodiment of the influenza virus defined in (1) above, the modified RNA-polymerase is capable of recognition of segments with modified vRNA promoter sequences resulting in an enhanced rate of transcription and/or replication, relative to said wild-type influenza virus RNA-polymerase;

(3) in a preferred embodiment of the influenza virus defined in (2) above, the influenza virus is suitable for high yielding expression of one or more foreign recombinant or altered viral proteins, preferably said influenza virus contains

(i) one or more segment(s) with a foreign recombinant or altered viral gene sequence in addition to the RNA segments of the normal viral genome (additional segment) or partially replacing them (hereinafter replacing segment ), whereby the additional segment(s) and replacing segment(s) comprise the foreign or altered gene encoding the protein to be expressed in monocistronic arrangement and have modified vRNA promoter sequences as defined in (2) above; and/or

(ii) one or more bicistronic vRNA segment(s), preferably in ambisense or in tandem arrangement, whereby the bicistronic vRNA segment(s) has/have foreign gene(s) encoding the protein(s) to be expressed and being in covalent linkage with one of the authentic viral genes, preferably the neuraminidase gene, and has/have modified vRNA promoter sequences as defined in (2) above;

(4) in a preferred embodiment of the influenza virus defined in (3) above, the influenza virus has at least one segment coding for one or more foreign (or altered proper) genes in monocistronic arrangement;

(5) in a preferred embodiment of the influenza virus defined in (3) above, the influenza virus is genetically stable in the absence of any helper virus and comprises at least one viral RNA segment being an ambisense RNA molecule (hereinafter "ambisense RNA segment") and containing one of the standard viral genes in sense orientation and a foreign, recombinant gene in anti-sense orientation, or *vice versa*, in overall convergent arrangement;

(6) in a preferred embodiment of the influenza virus defined in (3) above, the influenza virus is genetically stable in the absence of any helper virus and comprises at least one viral RNA segment being a bicistronic RNA molecule coding for two genes in tandem arrangement (hereinafter "tandem RNA segment"), in said tandem RNA segment one of the standard viral genes being in covalent junction with a foreign, recombinant gene and said tandem RNA segment having an upstream splice donor and a downstream splice acceptor signal surrounding the proximal coding region;

(7) a non-avian, non-human influenza virus, preferably an equine or a porcine influenza virus, comprising an RNA-sequence encoding a modified RNA-polymerase which differs from the wild-type RNA-polymerase of said non-avian, non-human influenza virus in that at least one of the amino acid residue(s) distinguishing the wild-type RNA-polymerase of said non-avian, non-human influenza virus from FPV Bratislava RNA-polymerase has been replaced with the corresponding amino acid residue(s) as present in FPV Bratislava RNA-polymerase, preferably said influenza virus is as defined in (2) to (6) above;

(8) a process for preparing the influenza virus as defined in (1) to (7) above, which comprises replacing the RNA-sequence encoding the wild-type RNA-polymerase of said influenza virus with an RNA-sequence encoding the modified RNA-polymerase;

(9) in a preferred embodiment of the process defined in (8) above, the process is suitable for preparing PB1-chimeric viruses as defined in (1) and (2) above as well as recombinant viruses as defined in (1) to (7) above said viruses being generated via cotransfection of up to eight cDNA plasmids containing the viral cDNAs, or chimeric (segment 2: PB1) and bicistronic recombinant (segment 6: NA/foreign gene) cDNA sequences instead, in such a way that they are transcribed *in vivo* by both RNA-polymerase I and RNA-polymerase II and jointly give rise to progeny viruses according to the plasmid insert design;

(10) a pharmaceutical composition comprising the influenza virus as defined in (1) to (7) above;

(11) the use of the influenza virus as defined in (1) to (7) above for preparing an agent

(i) for gene transfer into cells, preferably into mammalian cells, more preferably into human cells, by viral infection;

(ii) for gene transfer into antigen-presenting cells and the use of the obtained product for *ex vivo* immunotherapy;
(iii) for *in vivo* somatic gene therapy;
(iv) for *in vivo* vaccination, including therapeutic and prophylactic vaccination;
(v) for eliciting an immune response, including the induction of T-cell response;
(vi) for treating a growing tumor or a chronic infectious disease;

(12) a method for

(i) gene transfer into cells, preferably into mammalian cells, more preferably into human cells, by viral infection;
(ii) gene transfer into antigen-presenting cells and the use of the obtained product for *ex vivo* immunotherapy;
(iii) *in vivo* somatic gene therapy;
(iv) *in vivo* vaccination, including therapeutic and prophylactic vaccination;
(v) eliciting an immune response, including the induction of a T-cell response, preferably a CD4+ T-cell response, a CD8 T-cell response or both, or the induction of an antibody response;
(vi) treating a growing tumor or a chronic infectious disease;
(vii) preparing a vaccine;
(viii) preventing and/or treating influenza;

which comprises contacting the cells (including human or mammalian cells), the antigen-presenting cells, the person or the patient in need for vaccination, influenza treatment or for somatic gene therapy, or cell cultures with the influenza virus as defined in (1) to (7) above;
(13) a method for the production of proteins or glycoproteins which comprises utilizing the influenza virus as defined in (1) to (7) above as expression vector;
(14) the use of the influenza virus as defined in (1) to (7) above for preparing agents

(i) for transfer and expression of foreign genes into cells infected by such viruses, or
(ii) for transfer and expression of RNA molecules into cells infected by such viruses, preferably the RNA molecules to be expressed are antisense sequences or double-strand sequences relative to the target cell cellular mRNA molecules, and/or the agent is suitable for sequence-specific gene silencing, preferably by antisense RNA or RNA interference mechanisms such as ribozyme cleavage of target RNAs;

(15) a method for transfer and expression of foreign genes into cells, and for transfer and expression of RNA molecules into cells, which method comprises infecting the cells with the influenza virus as defined in (1) to (7) above;
(16) the use of the influenza virus as defined in (1) to (7) above for preparing agents for immunotherapy, preferably for autologous immunotherapy;
(17) a method for an immunotherapy which comprises *ex vivo* infection of immune cells, preferably dendritic cells, with the influenza virus as defined in (1) to (7) above, and introduction of the transduced cells into the patient;
(18) a method to elicit an immune response directed against an antigen, comprising the steps of introducing the influenza virus as defined in (1) to (7) above, preferably the human influenza virus as defined in (1) to (6) above, into a cell or administering it to a mammal, wherein said influenza virus contains at least one foreign gene encoding the antigen;
(19) a vaccine for therapeutic or prophylactic purposes which is

(a) a human influenza virus vaccine comprising a human influenza virus as defined in (1) to (6) above or in (18) above, preferably said human influenza virus encodes the antigen for a membrane protein and in addition contains the membrane protein in the viral envelope; or
(b) a non-human influenza virus vaccine, preferably an equine or porcine influenza virus vaccine, comprising a virus as defined in (7) above;

(20) transduced cells, preferably antigen-presenting cells, obtainable by the method described in (12), option (i) or (ii) above;
(21) a vaccine comprising transduced cells as defined in (20) above, preferably comprising transduced antigen-presenting cells, more preferably transduced dendritic cells, and most preferably mature dendritic cells, wherein said antigen-presenting cells are transduced *in vitro;* and
(22) a method to identify a polynucleotide sequence encoding at least one HLA-restricted epitope comprising the steps of

(a) preparing a gene bank or a cDNA bank from the cell or the microorganism to be tested;
(b) incorporating the cDNA or the DNA of the gene bank into the genome of the influenza virus as defined (1) to (7) above to yield recombinant virus particles,
(c) infecting immortalized autologous cells, which are capable of expression of HLA-class I molecules and/or HLA-class II molecules on their surface, with the recombinant virus particles obtained in step (b),
(d) expressing the proteins encoded by said cDNA or said DNA of the gene bank in the autologous cells and presenting the fragments of the proteins produced by the autologous cells or the cell surface in connection with HLA molecules;
(e) co-cultivating T-cells with the autologous cells; and
(f) stimulating the T-cells by such autologous cells which present antigens on their surface, whereby said antigens are recognized by the T-cells.

**Short Description of the Figures**

Figure 1: shows a comparison of variant amino acid positions in the PB1 segment of influenza A viruses, such as FPV, WSN and others, the numbering being relative to WSN. The underlined amino acid residues representing substitutions present exclusively in WSN, while amino acid residues in bold print point out those substitutions observed only in FPV Bratislava. The complete RNA sequence of the PB1 segment of WSN is shown in SEQ ID NO: 24 (nucleotides 191 to 2461) and the corresponding polypeptide is shown in SEQ ID NO:25, while the complete sequence of FPV-PB1 is shown in SEQ ID NO:22, and the corresponding polypeptide constitutes SEQ ID NO:23.

Figure 2: Chimeric structure and determination of promoter-recognition proficiency of a first set of WSN/FPV-PB1 constructs; Sections of FPV sequence within otherwise WSN-derived PB1 are indicated in heavy lining; WSN (pPolI-WSN-PB1) and FPV (pHL3115= WF1; pHL1844) are included for comparison.

Figure 3: Chimeric structure and determination of promoter-recognition proficiency of a second, more detailed set of WSN/FPV constructs.

Figure 4: shows FPV-Bratislava-PB1, the exact 5169 bp nucleotide sequence thereof is shown in SEQ ID NO:22 (nucleotides 191 to 2461 thereof encoding the PB1 segment of FPV Bratislava wild-type RNA-polymerase shown in SEQ ID NO:23).

Figure 5: shows WSN-PB1, the exact 5169 bp nucleotide sequence is shown in SEQ ID NO:24 (nucleotides 191 to 2461 thereof encoding the PB1 segment of WSN wild-type RNA-polymerase shown in SEQ ID NO:25).

Figure 6: shows plasmid pHL3102, the exact 5169 bp nucleotide sequence is shown in SEQ ID NO:26 (nucleotides 191 to 2461 thereof encoding the modified WSN RNA-polymerase PB1 segment shown in SEQ ID NO:27, with the following "major" modifications: L628M, V644A, and T741A and the following "minor" modifications: I576L, H584R, N633S, D636E, I645V, N654S).

Figure 7: shows vector pHL3103, the exact 5169 bp nucleotide sequence is shown in SEQ ID NO:28 (nucleotides 191 to 2461 thereof encoding the modified WSN RNA-polymerase PB1 segment shown in SEQ ID NO:29, with the following "major" modifications: S384P and L396I, and the "minor" modifications as pointed out in Fig. 1, positions 52 to 473).

Figure 8: shows vector pHL3130, the exact 5169 bp nucleotide sequence is shown in SEQ ID NO:30 (nucleotides 191 to 2461 thereof encoding the modified WSN RNA-polymerase PB1 segment shown in SEQ ID NO:31, with the following "major" modifications: S384P, L396I, L628M, V644A and T741A, and the "minor" modifications according to Fig. 1, positions 298-654).

Figure 9: shows vector pHL3131, the exact 5169 bp nucleotide sequence is shown in SEQ ID NO:32 (nucleotides 191 to 2461 thereof encoding the modified WSN RNA-polymerase PB1 segment shown in SEQ ID NO:33, with the following "major" modifications: S384P and L396I, and the "minor" modifications according to Fig. 1, positions 298-473).

Figure 10: shows vector pHL3203, the exact 5169 bp nucleotide sequence is shown in SEQ ID NO:34 (nucleotides 191 to 2461 thereof encoding the modified WSN RNA-polymerase PB1 segment shown in SEQ ID NO:35, with the following "major" modifications: L628M, V644A and T741A, and the "minor" modifications according to Fig. 1, positions 633-654).

Figure 11: shows vector pHL3204, the exact 5169 bp nucleotide sequence is shown in SEQ ID NO:36 (nucleotides 191 to 2461 thereof encoding the modified WSN RNA-polymerase PB1 segment shown in SEQ ID NO:37, with no "major" modifications and the following "minor" modifications: I576L and H584R).

Figure 12: shows vector pHL3246, the exact 5169 bp nucleotide sequence is shown in SEQ ID NO:38 (nucleotides 191 to 2461 thereof encoding the modified WSN RNA-polymerase PB1 segment shown in SEQ ID NO:39, with no "major" modifications and the following "minor" modifications: I298L and I364L).

Figure 13: shows vector pHL3247, the exact 5169 bp nucleotide sequence is shown in SEQ ID NO:40 (nucleotides 191 to 2461 thereof encoding the modified WSN RNA-polymerase PB1 segment shown in SEQ ID NO:41, with "major" modifications S384P and L396I, and the following "minor" modifications: D383E, H431T, N464D and L473V).

Figure 14: shows vector pHL3258, the exact 5169 bp nucleotide sequence is shown in SEQ ID NO:42 (nucleotides 191 to 2461 thereof encoding the modified WSN RNA-polymerase PB1 segment shown in SEQ ID NO:43, with "major" modification S384P and "minor" modification D383E).

Figure 15: shows vector pHL3259, the exact 5169 bp nucleotide sequence is shown in SEQ ID NO:44 (nucleotides 191 to 2461 thereof encoding the modified WSN RNA-polymerase PB1 segment shown in SEQ ID NO:45, with "major" modification S384P and the following "minor" modifications: D383E, I576L and H584R ).

Figure 16: shows vector pHL3268, the exact 5169 bp nucleotide sequence is shown in SEQ ID NO:46 (nucleotides 191 to 2461 thereof encoding the modified WSN RNA-polymerase PB1 segment shown in SEQ ID NO:47, with the following "major" modifications: S384P, L628M, V644A and T741A, and the following "minor" modifications: D383E, N633S, D636E, I645V, N654S).

**Detailed Description of the Invention**

In the present application "human influenza virus" includes all types of non-avian influenza viruses, including human, equine and porcine influenza viruses and the like, with human influenza viruses being the preferred ones. In the present application the influenza virus is also referred to as "vector", "expression vector" or "virus vector".
"Organism" embraces prokaryotic and eukaryotic systems as well as multicellular systems such as vertebrates (including mammals) and invertebrates, plants, etc. The term "cell" includes all types of cells of the "organism" defined above. A "mammal" according to the present invention includes humans and animals, "mammalian cells" include human cells and animal cells.
"Infected cells" and "infecting cells" according to the present invention also include "abortively infected cells" and "abortively infecting cells", respectively.
"Monocistronic" and "monocistronic arrangement" according to the present invention refers to a viral RNA segment, vRNA, cRNA or mRNA having one independent gene in "regular" (or "native") arrangement, while "bicistronic" according to the present invention refers to a viral RNA segment, vRNA, cRNA or mRNA that includes two independent genes in covalent junction (in a preferred embodiment of the present invention one of these genes is of viral origin, while the other one codes for a foreign, recombinant gene product).

In embodiment (1) of the invention as defined above, the influenza virus is preferably selected from influenza A including strains of type H1N1, H2N2 and H3N2, influenza B and influenza C, and more preferably is an influenza A type H1N1, including WSN/33, PR8/34 or the like, an influenza A type H2N2, including Asia/57 or the like, or an influenza A type H3N2, including Victoria/68 or the like.

It is moreover preferred that the at least one distinguishing amino acid residue is located within the PB1 segment of the virus. Possible substitutions within the PB1 segment can be derived from Fig. 1. Among those, and specifically for WSN, it is preferred to replace specifically one or more of the amino acid residues at positions 384, 396, 628, 644 and 741 (based on the numbering of WSN-PB1 shown in SEQ ID NO:25) which are also designated "major modifications" or "major replacements" with the respective FPV Bratislava amino acid residues (see Fig. 1). Preferably, the influenza virus strain used is WSN-K68 carrying five distinguishing amino acids. As an alternative to or in addition to those major modifications the PB1 may have the "minor" modifications as set forth in Fig. 1, namely at positions 52, 54, 105, 175, 208, 298, 364, 383, 431, 464, 473, 576, 584, 633, 636, 645 and 654. Most preferred PB1 segments are those coding for the amino acid sequences shown in SEQ ID NO: 27, 35, 43, 45 or 47.

The RNA-polymerase catalytic subunit PB1 of influenza virus WSN has been adapted by mutagenization to recognize and respond to the various nucleotide exchanges introduced in the vRNA promoter sequence that constitute promoter-up mutations in both transcription and replication. This result has been achieved through exchange of vRNA or indeed of plasmid cDNA sections within the coding sequence for polymerase subunit PB1 (segment 2) of influenza virus WSN (H1N1) using the corresponding sections derived from FPV Bratislava (H7N7) segment 2. Because of the large number of identical amino acids within both homologous segments and the rather small fragments being switched in that construction of chimeric PB1 the genetic transfer is equivalent to an introduction of a small number of amino acid substitutions within the PB1 polypeptide chain of WSN viral RNA-polymerase, while subunits PB2 and PA remain unchanged. In principle those amino-acids required for recognition of the rather minute promoter-up variations in the template molecules should be involved in direct interactions with the respective parts of the promoter structure, in particular with nucleotides 3 and 8 from the 3'-end, which constitute one of the base-pairs in the "cork-screw" proximal promoter element. The amino acid substitutions required for that purpose are distributed over two regions that broadly are known to be involved in vRNA and cRNA binding, respectively, in two separate sections in the PB1 polypeptide chain to the left and right of the enzymatic reaction centre. No difference has been observed in virus stability, viral yields or other properties in the PB1 variants as compared to standard PB1 containing WSN virus, as long as only wildtype promoter sequences are present in all of the influenza virus RNA segments, but segments carrying promoter-up variant sequences are transcribed and replicated at elevated rates, resulting in an up to 14 times the wildtype promoted expression level.

The generation of recombinant influenza viruses was hampered for a long time by the fact that the virus has a segmented RNA genome. The development of the RNA-polymerase I technique allows the generation of recombinant viruses with additional genomic segments capable of expressing complete heterologous genes (G. Neumann et al., Virology 202, 477-479 (1994)), which was built around the *in vivo* synthesis of recombinant vRNA molecules by cellular RNA-polymerase I transcription of the respective template cDNA constructs. Modified terminal viral RNA sequences (hereinafter "promoter-up mutations" or promoter-up variants") have been designed by nucleotide substitutions (Neumann and Hobom, Mutational analysis of influenza virus promoter elements *in vivo,* J. Gen. Virol. 76, 1709-1717 (1995); WO 96/10641). The above promoter-up variants carry up to five nucleotide substitutions (in promoter-up variant 1920; see Flick and Hobom, J. Gen. Virol. 80, 2565-2572 (1999)). When these promoter-up variants are attached to a recombinant ninth vRNA segment its increased transcription and amplification rates will not only compensate for the losses suffered spontaneously, but even cause accumulation of the foreign vRNA segment during simple viral passaging, in the absence of any selection.

As set forth in embodiment (2) above, a preferred method of the invention is where the recombinant virus contains terminal viral RNA sequences, which are active as promoter signal, have been modified by nucleotide substitution in up to 5 positions, resulting in improved transcription rates (of both the vRNA promoter and the cRNA promoter as present in the complementary sequence) as well as enhanced replication and/or expression rates relative to the wild-type sequence. Said modified terminal viral RNA sequences differ from the wild-type sequence in that in said vRNA segment the 12 nucleotide conserved influenza 3' terminal sequence has been modified by replacement of one to three nucleotides occurring in said sequence at positions 3, 5 and 8 relative to the 3' end by other nucleotides provided that the nucleotides introduced in positions 3 and 8 are forming a base pair (i.e., if the nucleotide position 3 is G, than that in position 8 is C; if the nucleotide in position 3 is C, than that in position 8 is G; etc.).

The 3' conserved regions of the wild-type influenza virus have the following sequences:

Influenza A: (5')-CCUGCUUUUGCU-3'

Influenza B: (5')-NN(C/U)GCUUCUGCU-3'

Influenza C: (5')-CCUGCUUCUGCU-3'.

Moreover, the 13 nucleotide conserved influenza 5'-terminal sequence may be modified by replacement of one or two nucleotides occurring in said sequence at positions 3 and 8 by other nucleotides, again provided that the introduced nucleotides are forming a base pair. The 5' conserved regions of the wild-type influenza virus have the following sequences:

Influenza A: 5'-AGUAGAAACAAGG

Influenza B: 5'-AGUAG(A/U)AACA(A/G)NN

Influenza C: 5'-AGCAGUAGCAAG(G/A):

Preferred influenza viruses of the invention are those wherein in the 3' conserved region the replacements G3A and C8U have been performed, more preferred are those where also the replacement U5C has been performed (the above mutations are annotated relative to the 3' end; such counting from the 3' end is also indicated by a line on top of the digit, e.g., G $\bar{3}$A). Another preferred influenza virus mutant comprises the 3'-terminal nucleotide sequence G3C, U5C and C8G (relative to the 3' end) resulting in the following 3' terminal nucleotide sequence (5')-CCUGGUUCUCCU-3'. Among the influenza viruses defined hereinbefore those having a 3'-terminal nucleotide sequence of (5') -CCUGUUUCUACU-3' are most preferred. In case of an influenza A virus the segment may further have the modifications U3A and A8U in its 5' terminal sequence, in case of influenza C it may have the modifications C3U and G8A in its 5' terminal sequence. The most preferred influenza viruses of the present invention comprise the following general structures:

Influenza A (mutant pHL1102):

5'-AGUAGAAACAAGGNNNU$_{5-6}$..(860-2310 ntds)..N'N'N'CCUGUUUUUACU-3'

Influenza A (mutant pHL1104):

5'-AGUAGAAACAAGGNNNU$_{5-6}$..(860-2310 ntds)..N'N'N'CCUGUUUCUACU-3'

Influenza A (mutant pHL1920):

5'-AGAAGAAUCAAGGNNNU$_{5-6}$..(860-2310 ntds)..N'N'N'CCUGUUUCUACU-3'

Influenza A (mutant pHL1948):

5'-AGUAGAAACAAGGNNNU$_{5-6}$..(860-2310 ntds)..N'N'N'CCUGGUUCUCCU-3'

Influenza B:

5'-AGUAG(A/U)AACA(A/G)NNNNNU$_{5-6}$..(860-2310 ntds)..N'N'N'N'N'(C/U)GUUUCUACU-3'

Influenza C:

5'-AGUAGUAACAAG(G/A)GU$_{5-6}$..(860-2310 ntds)..CCCCUGUUUCUACU-3'

In the above structures the variables are defined as follows:

(1) Underlined letters show the required mutations relative to the wild-type sequence for preparing a promoter mutant with enhanced properties;
(2) enlarged A in position 10 in the 5'-part of the sequence: unpaired A residue, bulge-forming;
(3) (A/G) in one position: different isolates or single segments with alternative sequence at the respective position, which are functionally interchangeable;
(4) N and N': positions undefined, but base-paired relative to each other because of complementarity between the 5' and 3' termini, different among the 8 segments, but constant for each segment throughout all viral isolates;
(5) (860-2310 ntds): the lengths of the authentic viral RNA segments, in case of segments with foreign genes increased up to 4,000 nucleotides.

Introduction of promoter-up recognition properties into standard human viruses of types H1N1 (WSN, PR/8), H2N2 (Asia), H3N2 (Victoria, Aichi, etc.) or other through PB1 mutagenization solves the problem of constructing influenza virus vectors other than FPV-derived bearing the property of expressing foreign proteins at very high rates that are suitable for expression of foreign genes in human cells or tissues including somatic gene therapy and therapeutic or prophylactic immunization.

The influenza A virus genome consists of eight segments of negative-strand viral RNA, i.e. their polypeptide coding frames are present in those vRNAs only in antisense orientation. They range in size from 890 nucleotides (segment 8) to 2341 nucleotides (segments 1 and 2). Among these the three largest segments code for three polypeptide chains that together constitute the viral RNA-polymerase: PB1, PB2 and PA, jointly comparable with RNA-polymerases as present in non-segmented negative-strand RNA viruses, which often are encoded in around 5000 nucleotides of vRNA.

Out of the three polymerase subunits that stay attached to each other in constituting the viral enzyme as present in the RNP complexes both in the virion and in the nucleus of the infected cell, the PB1 subunit can be regarded as the central catalytic subunit, since it carries all the enzymatic functions known to date: NTP binding, RNA chain elongation, and endonucleolytic cleavage (of the cellular RNA that thereafter is used as a primer for mRNA synthesis). In addition, PB1 also includes template binding sites for both the vRNA 5' and 3'-terminal sequences as well as the cRNA 5' and 3'-terminal regions (Li M.L. et al., EMBO J. Oct 1; 17(19):5844-52 (1998); Gonzales S., Ortin J., EMBO J. Jul 1;18(13): 3767-75 (1999)), i.e. for the various promoter elements, and finally is known to have attachment regions for both PB2 (at its C-terminus) and PA (at its N-terminus), which in turn are not directly attached to each other (Toyoda T. et al., J. Gen. Virol. Sep;77(Pt 9):2149-57 (1996); Gonzales S. et al., Nucleic Acids Res., Nov. 15;24(22):4456-63 (1996)). Subunit PB2 is known to specifically bind to the 5'-cap structure of cellular mRNA and hnRNA molecules, and thereby initiate the cap-snatching mode of viral mRNA transcription (Ulmanen I. et al., Proc. Natl. Acad. Sci. USA Dec.; 78(12): 7355-9 (1981); Shi L. et al., Virus Res. Jun.;42(1-2):1-9 (1996)). Subunit PA which is known to act as a phosphoprotein appears to have a role in cRNA and vRNA synthesis, i.e. in replication, and possibly also in the corresponding switch from mRNA to cRNA synthesis (Mahy B.W.J., Genetics of Influenza Viruses, Springer Verlag, Wien, pp. 192-253 (1983); Sanz-Esquerro J.J. et al., J. Gen Virol. Mar;79(Pt 3):471-8 (1998)). Somewhat aberrantly PA if expressed in the absence of PB1 and PB2 appears to lead to proteolytic degradation of co-expressed proteins (Sanz-Esquerro J.J. et al., J. Gen Virol. Mar;79(Pt 3):471-8 (1998)).

Nuclear translocation signals have been determined within all three polypeptides, in accordance with the nuclear localization of viral polymerase in infected cells (Nieto A. et al., J. Gen. Virol. Jan;75(Pt 1):29-36 (1994)).

Interaction of viral polymerase with the 5' and 3' ends of vRNA or cRNA has been detected in one set of experiments through binding of $^{32}$P-5'-oligonucleotides, 16-18 residues in size, which carried a single *thio*-uridine in 5' position 15 or in 3' position 10, respectively, followed by UV cross-linking and determination of $^{32}$P-carrying peptides. In this way the 5' vRNA sequence was observed to bind primarily to the PB1 polypeptide chain at the region centred around arginine residues 571 and 572, while the 3'vRNA model sequence attaches to the region PB1: 249-256, which includes two phenylalanine residues at positions 251 and 254 (Li M.L. et al., EMBO J. Oct 1; 17(19):5844-52 (1998)). In addition to primary vRNA binding sites as determined by UV cross-linking, in another approach secondary binding regions have also been observed for vRNA in the N-terminal region (1-139) and in the C-terminal region (493-757) using PB1 deletion variants. And while one of the cRNA binding sites determined in the same way overlaps in the N-terminal region (1-139) with vRNA binding, a second cRNA binding region is located in the central section (267-493) rather than in the C-terminal region (493-757) as detected for vRNA binding (Gonzales S., Ortin J., EMBO J. Jul 1;18 (13):3767-75 (1999)).

A sequence comparison between the PB1 polypeptide chains of FPV Bratislava (H7N7) and WSN (H1N1), proficient and deficient in recognizing the promoter-up variant sequences, revealed a divergence in 22 out of 757 amino acid positions. Upon extending that comparison to include PB1 sequences also from other influenza viruses such as PR/8 (H1N1), Asia (H2N2) and Victoria (H3N2) that number of divergent positions could tentatively be reduced by 11 amino acids, which are present only in WSN and not in any other PB1 sequence in a collection of over 150 viruses representing a large variety of influenza strains. Whereas the same residue is invariably present in the respective positions both in FPV Bratislava leading to proficiency and throughout all (or most, positions 584 and 741) other isolates leading to deficiency in recognizing the promoter sequence variants. Among the remaining 11 divergent positions seven of the FPV specific residues appear in several, but not in all other viral isolates, while four amino acid residues are specific for FPV Bratislava, and do not appear anywhere else: S384P, L396I, L628M, V644A. While the divergent positions in this category are most attractive in the search for specific properties of FPV, at this stage it cannot be ruled out that others among the altogether nine inconsistently variable amino acid residues may at least assist structurally or functionally in recognition of the variant promoter structures, in particular true for D383E because of its adjacent position relative to S384P. The apparently most important five divergent positions cluster in two groups, in the central region (383-396) not very far from the centre of polymerization activity, and in a near C-terminal region (628-741), i.e. within the brackets of the C-terminal vRNA binding region (see Fig. 1).

In a first step of analysis we have created WSN-FPV reassortant viruses carrying either a single one of the FPV subunit vRNAs in an otherwise all WSN background, or including the three FPV polymerase subunit vRNAs simultaneously. This was achieved via direct generation of influenza viruses from a set of cloned cDNAs (Neumann G. et al., Proc. Natl. Acad. Sci. USA, Aug. 3;96(16):9345-50 (1999)) designed to be transcribed *in vivo* by cellular RNA-polymerase I into eight individual viral RNA molecules (Neumann G. & Hobom G., J. Gen. Virol. Jul;76 (Pt 7):1709-17 (1995)), which were co-transfected together with four expression plasmids for early influenza virus proteins (PB1, PB2, PA, NP; Pleschka S. et al., J. Virol. Jun;70(6):4188-92 (1997)). All four viral reassortants turned out to be viable even if not yielding a full titer as compared to parental WSN or FPV. They were used to determine which of the three FPV polymerase subunits was responsible for recognition of the promoter-up variant sequences and caused increased expression rates of reporter genes controlled by them. Both reassortant viruses carrying either all three polymerase subunits originating from FPV, or carrying only the PB1 subunit derived from that avian influenza virus showed increased chloramphenicol acetyltransferase activity in the transfected cells as well as during consecutive steps of viral propagation with promoter-up variant 1104-CAT vRNAs, while the two reassortants containing either FPV-PB2 or FPV-PA in an otherwise WSN background of vRNA segments did not. From these data we conclude that it is indeed the FPV-PB1 subunit, already known from the above to interact with the vRNA and cRNA terminal sequences, i.e. the promoter structures, which is also recognizing the basepair and nucleotide substitutions present in the promoter-up variants. Another result derived from these initial experiments is the observed potential for free exchange of viral polymerase P-subunits between FPV and WSN viruses without major reduction in activity rates due to incompatibility.

Starting out from the FPV/WSN sequence comparison of PB1 and the present knowledge about the location of its functional domains a first round of chimeric PB1 clones was designed, which carried sections of both FPV-PB1 and WSN-PB1 at approximately one third and two thirds of the either polypeptide chain, see Figure 2. The results confirm that the N-terminal section even though it is known to include one of the binding sites for the vRNA and the cRNA terminal sequences is not involved in promoter variant recognition, as was suggested already by the small number of amino acid exchanges, which also might be regarded to be conservative, and because all of them show up (individually) in the majority of non-proficient viruses other than WSN: R52K, R54K, T105N, N175D and R208K. Instead, the chimeric PB1 protein carrying the C-terminal FPV section (492-757) attached to the N-terminal part of the WSN polypeptide (in pHL3102), and the PB1 chimera pHL3131 carrying a central FPV region (241-492) surrounded on either side by WSN sequence resulted in high or moderately high recognition of variant promoter sequences, in accordance with being divergent by 8 or 9 amino acid exchanges, respectively. In the extended comparison of proficient versus deficient viral PB1 sequences 2 and 2 substitutions thereof might be regarded to be "major" exchanges, i.e. being present only in FPV Bratislava. Because of the experimental data described below substitution T741A may also be regarded to be in the "major" (or main assisting) category, even though the alanine residue in this position is also present in several other, non-proficient viruses.

In a second round of PB1 chimera constructions the previously used central and C-terminal FPV sections of 34 % the entire length in both pHL3131 and pHL3102 have been divided further into halves, i.e. with regard to the number of amino acid divergencies remaining in pHL3131 and pHL3102 relative to full-size WSN-PB1. In this way pHL3204 constitutes the FPV:492-599 hybrid PB1 clone, pHL3203 the FPV: 599-757 containing polypeptide chain, pHL3256 carries an FPV section extending from position 241 to position 374, and pHL3257 contains FPV sequence from position 374 to 492, see Figure 2. In addition, a selected small section of FPV-PB1 sequence (374-394) covering a most tightly clustered group of two amino acid exchanges relative to WSN-PB1: D383E, and S384P has also been inserted into WSN-PB1 both on its own: pHL3258, and in combination with a second section of FPV-PB1: 492-599 (pHL3259) or 599-757 (pHL3268), i.e. the same regions as present individually in pHL3204 or pHL3203. As documented in Figure 3, it is the latter combination of two short, separate sections of FPV carrying in one section one "major" and one "minor" substitutions, and in the other three plus four amino acids in these categories exchanged, which gave the best results in recognition of promoter-up variant 1104, with rates above each of the individual constituents, pHL3203 or pHL3258, and at 70% the level of FPV polymerase itself (see pHL1844).

The difference remaining may be due to a negative effect caused by one or more of the amino acid substitutions present in pHL3268 resulting from sterical interactions with other parts of the WSN-PB1 molecule, while that respective amino acid residue may or may not be involved directly in promoter sequence recognition, too. In the latter case the promoter activity might be further increased upon determination and elimination of that disturbing residue among the few amino acid substitutions remaining at present. In the other case, i.e. with both effects caused simultaneously at least in part by the same residue, an improvement over the present level would not be possible. In any case the expression level for foreign proteins achieved so far for a WSN influenza virus carrying a set of only five amino acid substitutions (plus four most likely irrelevant changes) in its PB1 sequence, which in response to the standard pHL1104 mutant results in an expression rate 14 times above the wildtype promoter level (instead of 20 times for FPV), appears to be sufficient for its use in H1N1 influenza virus expression vectors.

The WSN-K68 virus carrying five constitutive amino acid substitutions in its PB1 polymerase subunit, which are modelled according to the FPV Bratislava sequence is a plaque-forming, stable virus strain indistinguishable from influenza virus WSN in its cell specificity and virus yields as long as it consists only of influenza vRNA segments carrying wildtype promoter sequences. In the presence of an (additional) influenza vRNA segment carrying a promoter-up terminal sequence the corresponding viral mRNA will be synthesized at high rates and that vRNA will be amplified disproportionately causing production of defective particles due to over-abundance of that single (foreign) viral segment over all the others. As described earlier (WO 00/53789 and EP 00115626.4) this can be brought back into a balanced, stable situation via construction of bicistronic segments, carrying the foreign gene in covalent junction with one of the viral genes, either according to the ambisense or to the tandem design. In addition, a replicational balance has to be achieved between that bicistronic segment and the set of seven regular segments through variation of the overall length of the bicistronic segment and the variant promoter sequence attached to it. With regard to transcription and consequent protein expression the lower-level yield of the viral gene product has to be brought into approximate balance with other viral gene products, while the higher yielding foreign gene product expression is maintained in imbalance with regard to the viral genes.

Alternatively, influenza virus strain WSN-K68 may be used directly as a helper virus for production of unstable recombinant viral progeny, with inherent suicide properties equivalent to attenuation. A disadvantage of that scheme is the presence of progeny helper viruses besides recombinant viruses in the supernatant of plasmid DNA transfected and helpervirus infected cells.

Construction of influenza virus strain WSN-K68 (H1N1) and expectedly of similar K68 variants of H2N2 or H3N2 viruses solves the problem of biological safety; it helps to avoid the use of H7-type viral hemagglutinin-containing viral vectors and their sensitivity to ubiquitous proteases.

The respective "major" amino acid positions in the PB1 subunit of the various parental H2N2 and H3N2 viruses mentioned earlier are largely identical to WSN, and always different from the unique sequence of FPV Bratislava (see Fig. 1), and therefore, these other viral strains are expected to become similarly converted from enhanced transcription-deficient into enhanced transcription-proficient viruses by that same operation.

The location of the two groups of apparently crucial amino acid substitutions within the PB1 sequence overlaps in one group: L628M, V644A and T741A with one of the known binding regions for the vRNA promoter sequence, and also is in the neighbourhood of the 5'vRNA cross-link site at R571/R572. The other group of exchanges in WSN-K68: D383E, S385P, and I396L is located within the region of primary (S445/D446/D447) and in particular secondary consensus sequence elements predicted to be involved in nucleotide polymerization enzymatic activity (Poch O. et al, EMBO J. Dec. 1;8(12):3867-74 (1990); Biswas S.K., Nayak D.P., J. Virol. Mar;68(3):1819-25 (1994)). Whereas only cRNA and not vRNA terminal sequence binding has been observed in that region (Gonzales S., Ortin J., EMBO J. Jul 1;18(13):3767-75 (1999)), the enzymatic reaction centre would have to be expected to get into close contact with both of its substrates, cRNA and vRNA.

No amino acid exchanges and hence no influence on PB1 or on viral polymerase promoter recognition properties is observed here for the N-terminal region of the PB1 polypeptide chain, which is known to interact with both the vRNA and cRNA promoter (1-143), and the same is true for the region of the 3'vRNA cross-link site (249-256). That result may be regarded to be disappointing, since the major effect on promoter-up variation originates from base-pair exchanges at positions 3 and 8 from the 3'-end. While it is obvious from the various results obtained previously with different methods and also including our own data, that widely separated parts of the PB1 polypeptide chain do interact, simultaneously or consecutively, with individual structural elements of the template molecules, and more specifically with both parts of the two RNA promoter structures, the 3D structure(s) of the entire enzyme or its PB1 subunit are not yet known.

The binding studies of Gonzales S., Ortin J., EMBO J. Jul 1;18(13):3767-75 (1999) have been done *in vitro* using large deletion variants of the PB1 polypeptide chain, which might cause severe structural deformations in the protein fragments remaining, and hence yield artifactual results. The cross-linking studies by Li M.L. et al., EMBO J. Oct. 1;17(19):5844-52 (1998) used rather short 5' *or* 3' oligonucleotides with a single thio-uridine residue in 5' position 15 or in 3' position 10. Both positions are located in the distal promoter element which is known to be double-stranded, while only single-stranded oligonucleotides have been used in these experiments. Certainly for the distal promoter element with its double-stranded RNA structure, but most likely also for the proximal promoter element, simultaneous binding of the 5' and 3' terminal sequence sections would have been more appropriate and might have given different results. Also, in a consecutive binding reaction of first the 5' vRNA terminal sequence, and thereafter the 3' vRNA end a major conformational shift of the entire molecule has been observed (Klumpp et al., EMBO J; 16:1248-1257 (1997)). Finally, it is known that the proximal and the distal promoter elements are independent conformational units in the vRNA promoter structure, and it is the proximal element that has to be recognized in detail by polymerase, since it is the one that carries the promoter-up mutations (Flick R. et al., RNA, Oct;2(10):1046-57 (1996)). Therefore, the UV cross-linking results obtained for two nucleotide positions only in the distal element might be misleading here also for that reason. On the other hand, our *in vivo* determination of a set of amino acid positions involved in enhanced transcription proficiency versus deficiency, i.e. in recognition of single nucleotide and/or base-pair exchanges within the vRNA promoter structure (and located in full-size functional polymerase molecules) is certainly a much more gentle and more reliable way of determining polymerase functional elements than any of the *in vitro* methods used so far. The PB1 amino acids involved in recognizing that 3'-3:8 basepair and sensing a single base-pair substitution at that location, are likely to be either in direct contact with those nucleotides or at most might be located in the subsequent chain of domain interactions within the protein leading to conformational changes and the observed response, i.e. enhanced transcription initiation rates.

According to embodiment (3) defined above and the explanations given hereinbefore, the influenza virus of the invention is suitable for high yield expression of one or more foreign or altered proteins. The foreign recombinant or altered viral gene may be present within an additional RNA segment or in a replacing segment, which comprise the foreign or altered gene encoding the protein to be expressed in monocistronic arrangement and have a modified vRNA promoter sequence as defined above (embodiment (4)), and/or within a bicistronic vRNA segment, preferably in ambisense or in tandem arrangement (embodiments (5) and (6), respectively), which includes the foreign gene encoding the protein to be expressed and has a modified vRNA promoter sequence as defined above.

Concerning embodiment (4), which relates to the expression of foreign glycoprotein genes and incorporation of those glycoproteins in the viral envelopes, it is referred to DE 197 09 512 (the disclosure thereof is herewith incorporated by reference). According to said embodiment the genes of different foreign glycoproteins of general type I, i.e., with a hydrophobic membrane anchoring sequence close to the C terminus, can be used for corresponding cDNA constructions, as designed for the expression of other genes, so that corresponding artificial vRNA molecules can be formed, i.e., in minus strand orientation. The foreign genes are inserted instead of the coding sequence of an influenza gene, flanked by authentic or slightly modified non-coding regions, as present in the influenza vRNA molecule. In addition to its own signal peptide sequence or the one borrowed from hemagglutinin (HA), the recombinant glycoprotein sequence then comprises its complete own, i.e., HA-foreign, ectodomain, followed by either its own transmembrane domain or, more frequently, that of hemagglutinin including its C-terminal "cytoplasmic" tail sequence (for HA: 26 + 11 amino acids). The same principle of construction applies to non-glycoproteins which may be converted into artificial surface proteins by being connected with the two flanking signal peptide and membrane anchor elements derived from viral hemagglutinin.

Concerning embodiment (5) it is referred to WO 00/53789 (the disclosure thereof is herewith incorporated by reference). In the influenza virus of said embodiment preferably at least one of the regular viral RNA segments is replaced by an ambisense RNA segment which contains one of the standard viral genes in sense orientation and a foreign, recombinant gene in anti-sense orientation or vice versa in overall convergent arrangement. It is moreover preferred that in the ambisense RNA molecule said foreign recombinant gene is covalently bound to one of the viral genes while the original vRNA segment coding for the same gene is deleted from the recombinant virus by a specific ribozyme cleavage, or left out from the set of RNA-polymerase I viral cDNA clones and substituted by the corresponding ambisense RNA expressing cDNA clone in the process of direct generation of recombinant influenza viruses (see below).

The foreign gene(s) in ambisense covalent junction with the viral gene(s) preferably code for proteins and/or glycoproteins which are secreted from cells infected with the recombinant virus, such as lymphokines or extracellular enzymes, or code for glycoproteins that are incorporated into the viral envelope as well as the plasma membrane of the infected cell. In another preferred embodiment the foreign gene(s) in ambisense covalent junction with the viral gene(s) code for proteins or artificial polypeptides designed to support an efficient HLA-restricted presentation of inherent epitopes at the surface of infected cells, for stimulation of B cell and/or T cell response. Such proteins or artificial polypeptides constitute for instance a tumor antigen or an artificial oligomeric series of T cell epitopes. Finally, the foreign genetic insert(s) may be suitable for transfer and expression of RNA molecules, including antisense RNAs and ribozymes within the infected cells. Such recombinant influenza viruses are suitable for sequence specific gene silencing, for example by RNA antisense or ribozyme RNA interference mechanisms.

A preferred virus of embodiment (5) of the invention is where in the regular viral RNA segments one or both of the standard glycoproteins hemagglutinin and neuraminidase have been exchanged into foreign glycoprotein(s), or preferably into fusion glycoproteins consisting of an anchor segment derived from hemagglutinin and an ectodomain obtained from the foreign source, viral or cellular, or in which such recombinant glycoprotein has been inserted as a third molecular species in addition to the remaining standard components.

Concerning embodiment (6) of the invention it is referred to EP 00115626.4 (the disclosure thereof is herewith incorporated by reference). In the tandem RNA segment of said embodiment, one of the standard viral genes is preferably in covalent junction with a foreign, recombinant gene and said tandem RNA segment has an upstream splice donor and a downstream splice acceptor signal surrounding the proximal coding region. "Proximal" and "proximal position" according to the present invention refers to the 5' position of one of the genes in the bicistronic viral mRNA, i.e., ahead (upstream) of the second gene in "distal position".

Expression of both gene products in the tandem constructions is made possible by way of an upstream splice donor and a downstream splice acceptor signal surrounding the proximal coding region of such a quality that splicing does occur in part of the mRNA molecules only, i.e., both mRNAs spliced and unspliced are present in the infected cell. For compensation with regard to the vRNA length the bicistronic segment is connected to a promoter variant of enhanced replication and transcription rates as defined hereinbefore.

The splice donor and the splice acceptor signals are selected from authentic sequences as present in influenza segments 7 and 8 or other partially effective splice reaction substrates, preferably those of influenza virus WSN segment 7, i.e., 5'-AG↓GTACGTTC-3' (donor) and 5'-GCTGAAAAATGATCTTCTTGAAAATTGCAG↓GC-3' (acceptor).

In a preferred influenza virus according to embodiment (6) at least one of the regular viral RNA segments is replaced by a tandem RNA segment which contains one of the standard viral genes in distal position, and a foreign, recombinant gene in proximal position, both in anti-sense orientation, or vice-versa. It is moreover preferred that the same viral gene as present in the bicistronic RNA segment is deleted from the recombinant virus by specific ribozyme cleavage or is left out from the set of RNA-polymerase I cDNA clones and substituted by the corresponding tandem bicistronic RNA expressing cDNA clone in the direct generation of recombinant influenza viruses from plasmid DNAs (see below).

The foreign gene(s) in tandem covalent junction with the viral gene(s) preferably code for proteins and/or glycoproteins which are secreted from cells infected with the recombinant virus, such as lymphokines oe extracellular enzymes, or code for glycoproteins that are incorporated into the viral envelope as well as the plasma membrane of the (abortively) infected cell. In another preferred embodiment the foreign gene(s) in tandem covalent junction with the viral gene(s) code for proteins or artificial polypeptides designed to support an efficient HLA-restricted presentation of inherent epitopes at the surface of infected cells, for stimulation of B cell and/or T cell responses. Such proteins or artificial polypeptides constitute for instance a tumor antigen or an artificial oligomeric series of T cell epitopes that have been identified within a polypeptide chain. Finally, the foreign genetic insert(s) may be suitable for expression of RNA molecules, including antisense RNAs and ribozymes, within the infected cells. Such recombinant influenza viruses are suitable for sequence specific gene silencing, for example by RNA antisense or ribozyme interference mechanisms.

A preferred recombinant virus of embodiment (6) of the invention is where in the regular viral RNA segments one or both of the standard glycoproteins hemagglutinin and neuraminidase have been exchanged into foreign glycoproteins, or preferably into fusion glycoproteins consisting of an anchor segment derived from hemagglutinin and an ectodomain obtained from the foreign source, viral or cellular, or in which such recombinant glycoprotein has been inserted as a third molecular species in addition to the remaining standard components.

According to embodiments (5) and (6) the invention provides

- a stable recombinant influenza virus containing (up to) seven regular vRNA segments plus one (or more) additional bicistronic segment(s) coding for a foreign gene in covalent conjunction with one of the influenza genes, in ambisense or in tandem arrangement, and
- a method for the construction of stable recombinant influenza viruses through (a) bicistronic vRNA segment(s) in ambisense or in tandem arrangement, that is also applicable as a method for attenuation and for prevention of reassortment between co-infecting influenza viruses.

In a particular application of embodiment (6) the tandem bicistronic mRNA codes for one of the viral genes, such as hemagglutinin, in conjunction with (all or) part of the viral neuraminidase coding sequence or the viral NS1 coding sequence, in inverted (antisense) orientation, while the authentic neuraminidase vRNA segment or NS1 coding sequence is otherwise missing entirely in these recombinant viruses. In another variation of these constructs an anti-neuraminidase or anti-NS1 ribozyme sequence is also provided together with the (partial) neuraminidase or NS1 antisense sequence, in the proximal or in the distal position of these bicistronic recombinant segments. Recombinant viruses of this character are propagated in culture media with addition of exogenous neuraminidase or in tissue culture cells with inactivated interferon genes, e.g., Vero cells.

The absence of a functional neuraminidase gene serves as a strong attenuation mechanism resulting in single-step infections of such recombinant viruses only. While a functional neuraminidase gene could be provided in case of another (wildtype) influenza virus superinfecting the same cell, expression of that gene is very much reduced through antisense RNA interaction and/or destruction of the corresponding vRNA through ribozyme cleavage, designed to interfere with production of infectious progeny even from co-infected cells; as a barrier against reassortment in double infected cells. The same argument applies for the NS1-deleted viruses which in addition carry an anti-NS1 vRNA antisense or ribozyme sequence, as a second feature besides carrying a foreign recombinant gene in ambisense or tandem bicistronic design.

Recombinant viral RNAs coding simultaneously for two genes in tandem organization within a construct, in which one of the viral genes is connected in covalent junction with a foreign coding sequence, are constructed via E. coli plasmid vector DNAs designed for an *in vivo* transcription of minus-strand vRNAs by cellular RNA-polymerase I. In these constructs the gene in plus-strand proximal (upstream) position is surrounded by splice signals of limited activity such that both mRNAs, spliced and unspliced are present in the infected cell. Either the foreign gene or the viral gene may be in that upstream position. In the majority of applications the higher rate of expression will be reserved for the foreign coding sequence, while the lower expression rate of the viral gene is adapted to be approximately in balance with expression of the other viral genes encoded by the regular viral segments.

To achieve such a balanced rate of expression, the splice signals and the promoter have to be chosen properly (Flick and Hobom, Interaction of influenza virus polymerase with viral RNA in the 'corkscrew' conformation, J. Gen. Virol. 80, 2565-2572 (1999)). At an increased overall transcription rate, the resulting mRNAs shall be spliced inefficiently if the viral gene is in the distal (downstream) position. Vice-versa, if the foreign gene is in the distal position, splicing to obtain the foreign mRNA shall be achieved efficiently. Both designs serve to reach an over-expression of the foreign gene relative to its comparison viral gene, of which the expression shall be in balance with the expression of the other viral genes. Further, the promoter variant attached to the bicistronic segment has the function to compensate for the increased gene length by way of an increased replication rate.

The influenza vRNA segments preferably used for construction of bicistronic segments include the neuraminidase (No. 6), hemagglutinin (No. 4) and NS segment (No. 8). In the NS segment the foreign gene may also substitute for the NS1 gene, leaving the viral NS2 gene in its place. These recombinant viruses can, as an example, be made by the following procedure: A recombinant virus population can be selected by repeated ribozyme-mediated cleavage of a helper-virus segment carrying inserted ribozyme cleavage sites in flanking positions of the same viral gene in the monocistronic segment as has been included in the bicistronic construct (PCT/EP00/01903). By serial viral passaging and relying on the ouptut of reporter genes in equivalently constructed bicistronic segments, a balanced mode of expression can be achieved in choosing the right set of elements: promoter variant, splice signals, plus a limited variation in segment length. The construct that gives rise to the balanced, stable expression may then be used for designing a multiple cDNA transfection procedure of helper-free generation of influenza viruses according to Hoffmann et al., Proc. Natl. Acad. Sci. USA, Vol 97, 6108-6114 (Mai 2000). The resulting recombinant influenza virus, obtained via single plaque in pure helper-free state, is subjected to another series of propagation steps to finally evaluate its properties.

In a particular application this design is used for establishing a controlled mode of viral attenuation. Attenuation of influenza viruses so far has been achieved in cold-sensitive mutants (Edwards et al., J. Infect. Dis. 169, 68-76(1994)), by deletion of the NS1 gene (partial attenuation, Egorov et al., J. Virol. 72, 6437-6441 (August 1998) and Palese et al., Proc. Natl. Acad Sci USA, 4309-4314 (April 2000)), or through deletion of the neuraminidase gene (full attenuation, Kawaoka et al., J. Virol. 74, 5206-5212 (June 2000)). The latter approach is adapted here, together with a novel technique for viral attenuation, which for the first time is also able to interfere with (chance) superinfection by wild-type viruses.

In this embodiment of the invention a second bicistronic cDNA construct is designed, which instead of carrying another foreign gene is coding for part of the viral neuraminidase gene in antisense orientation, with or without being surrounded both by splice donor and acceptor elements. In another version of that design a 2 x 50 nucleotide antisense segment complementary to a region of the neuraminidase sequence has been cloned in flanking positions relative to a ribozyme construct according to the hammerhead design and oriented against a common GUC triplett within the neuraminidase sequence in a majority of current post-viral isolates. In a preferred design this antisense expression construct has been attached to the hemagglutinin vRNA segment, while another gene or reporter gene is encoded in a second bicistronic vRNA, in conjunction with NS2. The same design applies for an anti-NS1 virus, which itself does not carry an NS1 gene, and has a foreign recombinant gene in conjunction with neuraminidase vRNA or NS2 vRNA.

Propagation of recombinant viruses deleted for the neuraminidase (NA) gene requires an addition of external neuraminidase to the medium. In the absence of neuraminidase, infection by the NA deletion viruses is abortive: no infectious progeny is produced. Upon co-infection (3 + 3 per cell) of recombinant viruses together with wildtype viruses no progeny virus or plaque is observed, which is attributed to antisense-blocked expression and/or (partial) ribozyme destruction of the neuraminidase segment originating from the wild-type virus. According to this design, the recombinant viruses described are not only attenuated in single infections, but simultaneously interfere with wildtype virus superinfection, and therefore, no re-assortment between the two viruses will occur.

Concerning the process of embodiment (8) it is referred to the disclosure of WO 96/10641, PCT/EP00/01903 and EP 00115626 referred to above (the disclosures of which are herewith incorporated by reference) and the detailed discussion of the production method set forth above.

Concerning the embodiment (9) a final step in the generation of WSN/FPV-PB1 chimeric viruses such as influenza WSN-K68 consists in performing an eight cDNA plasmid-cotransfection into 293T cells designed according to Hoffmann et al (Hoffmann E. et al., Proc. Natl. Acad. Sci. USA 97 (11), 6108-6113 (2000)), i.e., carrying inserts cloned into vector plasmid pHW2000 or an equivalently organized plasmid vector containing flanking RNA-polymerase I as well as RNA-polymerase II transcription units, in opposite direction to each other and extending across the central cDNA insert. The inserts used in a set of eight dual expression plasmids constructed accordingly consist of wildtype WSN cDNAs derived from the 7 segments except for segment 2 (PB1), which was constructed using PB1 chimeric plasmid pHL3268 as a starting material. Cotransfection with all eight plasmids after 72 hours resulted in virus containing supernatants, which can be used for plaque purification and further propagation on MDCK cells, and for characterization of the viral isolate by RT-PCR followed by restriction analysis or DNA sequencing.

Generation of recombinant influenza viruses followed the same outline, but in this case one of the WSN segments, preferably segment 6 (NA) has been exchanged for a bicistronic construct containing the NA gene in covalent junction with the foreign gene, in ambisense or in tandem design. In addition, the bicistronic segment carries promoter-up mutations in its flanking 5' and 3' terminal sequences. Other recombinant viruses have been created using a bicistronic segment 8 (NS2/foreign) with or without an anti-NS1 ribozyme sequence inserted in a flanking position in segment 6, and still other of the segment 8 bicistronic viruses (NS2/foreign) have their segment 6 deleted entirely, with or without an anti-NA ribozyme sequence inserted in a flanking position of segment 4(HA).

The pharmaceutical composition according to embodiment (10) above and the agent of embodiments (11), (14) and (16) above (hereinafter also referred to as "medicament") contain the recombinant influenza virus in a pharmaceutically effective amount. Besides said recombinant influenza virus, the pharmaceutical composition and the medicament may contain further pharmaceutically acceptable carrier substances well-known to a person skilled in the art, such as binders, desintegrants, diluents, buffers, preservatives, etc. The pharmaceutical compositions and medicaments are solid or liquid preparations and are suitable to be administered orally, intravenously or subcutaneously. For treatment of humans a human influenza virus according to embodiments (1) to (6) is preferably used.

The pharmaceutical composition of embodiment (10) is, among others, suitable

(i) for gene transfer into cells, preferably into mammalian cells, more preferably into human cells, by recombinant viral infection (namely via standard viral infection or employing in addition a specific attenuation mechanism);
(ii) for gene transfer into antigen-presenting cells, preferably into dendritic cells, and the use of the obtained product for *ex vivo* immunotherapy (whereby *ex vivo* therapy is therapeutic application involving the modification of antigen-presenting cells, such as dendritic cells, wherein the modified antigen presenting cells are injected directly to the patient);
(iii) for *in vivo* somatic gene therapy;
(iv) for *in vivo* vaccination, including therapeutic and prophylactic vaccination;
(v) for eliciting an immune response, including the induction of a T-cell response;
(vi) for treating a growing tumor or a chronic infectious disease.

The pharmaceutical composition of embodiment (10) and the medicament according to embodiment (11) above is preferably suitable as a medicament against influenza and/or against other infections. The recombinant influenza virus may be present in form of inactivated preparations or may be present in form of live recombinant viruses, preferably as attenuated viruses.

Live recombinant viral vaccines, live but attenuated recombinant viral vaccines or inactivated recombinant viral vaccine can be formulated. Inactivated vaccines are "dead" in the sense that their infectivity has been destroyed. Ideally, the infectivity is destroyed without affecting its immunogenicity. For preparation of inactivated vaccines, the recombinant virus may be grown in cell cultures or in embryonated chicken eggs, purified, and inactivated by formaldehyde or β-propiolactone. The resulting vaccine is usually administered intramuscularly.

Inactivated viruses may be formulated with suitable adjuvants to enhance the immunological response. Such adjuvants include, but are not limited to, mineral gels, e.g., aluminum hydroxide, surface-active substances such as pluronic polyols, lysolecithin, peptides, oil emulsions, and potentially useful human adjuvants such as BCG.

Many methods may be used to introduce the vaccine formulations above, for example the oral, intradermal, intramuscular, intraperitoneal, subcutaneous, or intranasal routes. Where a live recombinant virus vaccine is used, it is preferred to introduce the formulation via the natural route of infection for influenza virus.

The agent according to embodiments (11), (14) and (16) above is preferably suitable for prophylactic or therapeutic vaccination, or both, against influenza and other infections. For example, recombinant viruses can be made for use in vaccines against HIV, hepatitis B virus, hepatitis C virus, herpes viruses, papilloma viruses, to name but a few. In one embodiment the recombinant virus contains the genes for surface proteins of the viruses, in another the genes for non-structural or regulatory genes. The recombinant viruses may be present in form of inactivated preparations or may be present in form of live recombinant viruses, or as live, but attenuated viruses. In an attenuated virus the recombinant virus would go through a single or at most very few propagation cycle(s) and induce a sufficient level of immune response, but would not cause disease. Such viruses lack one of the essential influenza genes or contain mutations to introduce temperature sensitivity.

The agents of embodiments (11), (14) and (16) above of the invention are applicable in *ex vivo* and *in vivo* application schemes. The RNA molecule to be expressed by means of the agent of the embodiment (14) is of an antisense sequence or double strand sequence (in ambisense bidirectional transcription) relative to a target cellular mRNA molecule. In embodiment (14) the agent is preferably suitable for sequence-specific gene silencing, preferably by RNA antisense or ribozyme interference mechanisms.

The method for the production of proteins or glycoproteins of embodiment (13) is preferably performed in tissue culture cells or in fertilized chicken eggs, in accordance with standard techniques within the general knowledge of a person skilled in the art. The proteins or glycoproteins to be expressed are those incorporated into the virus, in monocistronic or bicistronic arrangement as defined hereinbefore.

The methods according to embodiments (12), (17) and (18) of the invention include the administration of an effective amount to the mammal (i.e. the patient in need for vaccination, for influenza treatment or for somatic gene therapy) or the administration of a sufficient infective dose of the recombinant virus to the cell system (including antigen-presenting cells, cell cultures, etc.) that is used for *ex vivo* therapy or for *in vitro* investigations, whereby the amount and dose will be determined by a person skilled in the respective arts or knowledgeable of the desired treatments. For treatment of human patients the use of human influenza viruses as defined hereinbefore is preferred.

The method of embodiment (18) is disclosed in EP 00123687.6 (which is hereby incorporated by reference). In particular, said method comprises expression of one or more tumour-associated antigens (TAA) or virus-associated antigens (VAA) by dendritic cells by

(a) preparing a recombinant influenza virus containing a nucleotide sequence coding for the TAA or VAA, and
(b) infecting dendritic cells with the recombinant influenza virus obtained in step (a).

The nucleotide sequence coding for the TAA or VAA may be present in the recombinant influenza virus in one of the regular segments in bicistronic arrangement or as an additional segment as explained in detail hereinbefore.

Vaccination with dendritic cells presenting tumor antigens will induce a potent primary immune response or amplify existing cytotoxic antitumor T cell responses. Therefore, tumor antigens most suitable for immunotherapy are, ideally, strictly tumor specific, or at least the immune response should have tumoral specificity. Many tumor antigens, however, are shared by tumors with normal cells and are only overexpressed in the tumor. This implies that an immune response could potentially be harmful, if an immune response to self-antigens occurs causing autoimmunity. The technology for DC vaccines shall thus result in an immune response of sufficient quality and magnitude of tumor-specific T cell responses.

Tumor-specific antigens are rare. However, a growing family of testicular antigens has been identified that are aberrantly expressed in a significant proportion of tumors of various histological types and -in addition- only in testis cells. These antigens, called cancer/testis antigens, discovered by scientists of the Ludwig Institute for Cancer Research (LICR) in Brussels and New York, and their collaborators, should ensure strict tumoral specifcity of the immune responses as the germ line cells do not express MHC-I molecules.

These antigens, for example the MAGE-, GAGE- and BAGE-families, NY-ESO-I or HOM-MEL-40 (aka SSX-2) are thus prime candidates for the DC-based antitumor vaccines, when part of a potent dendritic cell vaccine based on influenza virus-mediated gene transfer. These antigens have the required selectivity for a flu-vector based DC vaccine, can most likely be readily incorporated into the recombinant virus, and are able to induce cellular immune responses. Epitope peptides derived from MAGE-A3 have been HLA-attached ("loaded") to the dendritic cells by Schuler and coworkers and the vaccine was found to induce specific CTL in patients and *in vitro.*

Furthermore, the number of tumor antigens suitable for potent therapeutic vaccines is still limited and a search for novel tumor antigens, as well as viral antigens, seems warranted. The influenza virus vector system is suitable for antigen discovery (see above). Co-expression of said antigens with LICR antigens is an important option for widening the vaccine spectrum.

In experiments it was shown that genetically modified DC, which express tumor-associated antigens can efficiently induce anti-tumour immunity and thus have a high potential as tools in cancer therapy. The gene delivery is most efficiently achieved by viral vectors. Genes encoding a melanoma derived TAA, such as MAGE-3, or the green fluorescence protein (GFP) were introduced into a high-expression avian influenza virus vector. Monocyte-derived mature DC infected by these recombinants efficiently produced GFP or MAGE-3. More than 90 % of the infected DC can express a transduced gene. Importantly, these transduced DC retained their characteristic phenotype, their potent allogeneic T cell stimulatory capacity and were able to stimulate MAGE-3 specific $CD8^+$ cytotoxic T cells. Thus influenza virus vectors provide a highly efficient gene delivery system in order to transduce human DC with TAA, which consequently stimulate TAA specific T cells.

The agent of embodiment (17) of the invention is preferably utilized to infect, transfect or transduce patient-derived immune cells. The agent is suitable for treatment of cancer or chronic viral infections. For this purpose, patient derived immune cells, preferably dendritic cells, are *ex vivo* infected with recombinant influenza viruses expressing, e. g., tumor antigens or viral antigens. The transduced cells are then reintroduced into the patient.

The preferred method for immunotherapy of embodiment (18) of the invention is an autologous immunotherapy, wherein the cells which are *ex vivo* infected are patient-derived and the transduced cells are reintroduced into the patient. The diseases to be treated by this method include cancer and chronic viral infections. For details regarding preparation of the treatment with dendritic cells see discussion of embodiment (12) above.

The method for inducing an immune response against an antigen according to embodiment (18) of the invention is suitable for inducing antibodies to foreign proteins including glycoproteins, following the administration of protein or glycoprotein antigens as part of a recombinant influenza virus in an authentic conformation, whereby the virus is purified by gentle procedures based on hemagglutination, and the gene is expressed at high rates in the infected cells. Suitable foreign genes encoding one of these antigens are polynucleotide sequences associated with a disease, preferably an infectious disease or tumor disease, preferably the antigen is exemplified by, but not limited to,

(i) virus-associated antigens such as the HIV antigens gp160, gp 120, rev, tat, NC, the HBV e-antigen or core antigen, the HPV E6 or E7 antigen, the herpes simplex virus glycoproteins or core proteins, other herpesvirus antigens and further viral and microbial antigens known to those skilled in the art,
(ii) tumor associated antigens, especially the so-called cancer testis-antigens exemplified by the MAGE, BAGE and GAGE family of antigens, the NY-ESO-1 antigen, the SSX antigens, exemplified by the HOM-MEL-40.

The above polynucleotide sequences

(i) are derivable from cDNA libraries isolated from tumor cells, or testis cells, or virus-infected cells, or micriobially infected cells, or cell-lines,
(ii) are fusion proteins with the hemagglutinin membrane anchor sequence, or polypeptides consisting of epitopes derived from one or more T-cell specific epitope sequences as present in viral or other pathogens, or in tumor associated antigens.

As influenza viruses have a wide host range, recombinant influenza viruses can be used to obtain strong immune responses in, and isolate antibodies from, a wide range of animals, including, but not limited to, fowl, pigs, horses, seals and mice. Further, influenza viruses adapted to the mouse can be used for the infection of mice by several routes including the intranasal route. This results in infection of the pharyngeal mucosal cells and results in an additional type of B cell response (e.g., as recognized in the ratio of IgG to IgA). Mice are of particular utility in the induction of immune responses in transgenic mice that have been engineered to express human antibodies. As gentle procedures based on hemadsorption are used to purify influenza viruses, antibodies to antigens in native conformation can be isolated from the infected mammals.
The vaccines according to embodiments (19) and (21) of the invention may contain further ingredients, e.g., those set forth with regard to the pharmaceutical composition or agents set forth above.
Concerning embodiment (22) of the invention it is referred to PCT/EP00/09217, which is herewith incorporated by reference. The method of embodiment (22) is also suitable to study gene function in antigen presenting cells.
The present invention is hereinafter described in more detail by way of the following description of the figures and examples, which are, however, not to be construed so as to limit the invention.

**Detailed Description of the Tables and Figures**

Table 1

Table 1.

| Reassorted viral strains used as helper viruses in infection of plasmid pHL1844 transfected 293T cells, followed by propagation of resulting recombinant progeny virus in MDCK cells. | | | | | | | |
|---|---|---|---|---|---|---|---|
| Viral strains | Origin of segments | | | | | pHL1844-CAT-activity | |
| | PB1 | PB2 | PA | NP | other | 293T | MDCK (1) |
| WSN | WSN | WSN | WSN | WSN | WSN | 10 | <2 |
| WF1 | FPV | WSN | WSN | WSN | WSN | 21 | 30 |
| WF2 | WSN | FPV | WSN | WSN | WSN | 6 | <2 |
| WF3 | WSN | WSN | FPV | WSN | WSN | 8 | <2 |
| WF4 | FPV | FPV | FPV | WSN | WSN | 26 | 35 |
| FPV | FPV | FPV | FPV | FPV | FPV | 41 | **100** |
| Chloramphenicol acetyl-transferase enzymatic activities in 293T and MDCK (first viral passage) cell lysates, relative to standard FPV (=100). | | | | | | | |

Figure 1: Comparison of relevant PB1 amino acid positions in a selected set of influenza virus strains. The 22 amino acid positions divergent between FPV Bratislava (line 1) and WSN/33 (line 3) have been compared in the context of a large, representative group of viral isolates. Amino acid residues present only in WSN and in no other PB1 sequence have been underlined, while those four amino acid residues characterizing FPV Bratislava as approved to all other strains are given in bold print.
Figure 2: Chimeric structure and determination of promoter-recognition proficiency of a first set of WSN/FPV-PB1 constructs; Sections of FPV sequence within otherwise WSN-derived PB1 are indicated in heavy lining; WSN (pPolI-WSN-PB1) and FPV (pHL3115; pHL1844) are included for comparison. Upon generation of chimeric viruses in 293T cells stocks have been prepared on MDCK cells. 293T cells transfected by plasmid pHL1844 which carries the standard promoter-up variation as present originally in pHL1104 have been infected by the various viral constructs as helper viruses at m.o.i. 1 (with viral strains derived from pHL3130 and pHL3115 only at m.o.i. 0.2 and 0.5, respectively). The resulting supernatants containing the viral progeny were used for viral passage onto MDCK cells. Cell lysates from both transfected and/or infected cells were used for CAT activity measurements relative to pHL1844 recombinant FPV viruses in MDCK cells (=100), known to recognize promoter-up variant pHL1844.

A selection of functional elements within the PB1 protein sequence together with the position of six "major" amino acid substitutions have been indicated in the first line of the figure. Proposed binding regions for the vRNA (v1, v2) or the cRNA (c1, c2) promoter, and known cross-linking sites for the 5'(x5) and 3'(x3) vRNA termini are marked. P refers to the active center of polymerase activity (PB1: 445-447). Amino acid exchanges are indicated by the WSN residue on top of the FPV residue, e.g. S/P refers to amino acid substitution S384P (WSN/FPV).

Figure 3: Chimeric structure and determination of promoter-recognition proficiency of a second, more detailed set of WSN/FPV constructs. For descriptions of experimental details, see Fig. 2.

## Examples

Plasmid pHL1844: The CAT-gene containing expression plasmid pHL1844 has been designed for pseudo-vRNA synthesis by human RNA-polymerase I and carries the human rDNA core promoter region (-411 to -1) plus the murine rDNA terminator region (+ 566 to + 785 relative to the mature 28S rRNA 3'-end), both in flanking positions relative to the cDNA insert. The cDNA construct inserted in between consists of the 5'and 3'noncoding sequences as present in FPV vRNA segment 5, with the NP coding sequence itself being exchanged for the coding sequence of chloramphenicol acetyltransferase (CAT) of bacterial origin, in antisense orientation relative to RNA-polymerase I transcription. pHL1844 carries three point mutations in the 3' viral RNA promoter sequence ( 3'-G $\bar{3}$ A, U $\bar{5}$ C, C $\bar{8}$ U : 3'-UCAUCUUUGUCCCCAU), as originally introduced into plasmid pHL1104 (Neumann G., Hobom G, J. Gen. Virol. Jul.;76(Pt 7):1709-17 (1995)).

Plasmid construction of WSN/FPV-PB1 chimera: Original plasmids WSN-PB1 (pPolI-WSN-PB1; Neumann G. et al., Proc. Natl. Acad. Sci. USA, Aug. 3;96(16):9345-50 (1999)), and FPV-PB1 as obtained by RT-PCR from FPV vRNA using PB1 terminal primer oligonucleotides that were designed for insertion into pHH21 (Hoffmann E., Justus Liebig Universität Giessen (1997)) via *Bsm*BI cleavage (Menke A. and Hobom G., unpublished) were used as starting points. Fragments have been exchanged either through restriction cleavage at homologous unique restriction sites or following PCR reactions designed to insert a "missing" restriction site in homologous position and included within one of the primer oligonucleotides. All PCR-derived constructs have been confirmed by sequencing across the region inserted in one way or another.

Cells and viruses: Influenza viral strains A/$FPV_{Bratislava}$ (H7/N7) and A/WSN/33 (H1/N1) as well as all viral reassortants or PB1-chimeric viral constructs were grown in Madin-Darby canine kidney (MDCK) cells. Human 293T cells were used for DNA transfection and consecutive superinfection with FPV or with the various other strains used as helper virus. The resulting recombinant influenza viruses were propagated in MDCK cells. All cell lines were grown in Dulbecco's modified Eagle's Medium (DMEM; GIBCO/BRL), supplemented with 10% fetal calf serum and antibiotics.

Generation of infectious WSN, FPV and FPV/WSN-PB1 chimeric influenza viruses: Subconfluent 293 T cells were transfected with a set of eight RNA-polymerase I plasmids yielding the eight different species of influenza vRNA molecules by *in vivo* transcription, according to Neumann et al., Proc Natl. Acad. Sci. 96, 9345-9350 (1999), together with a second set of four to five RNA-polymerase II expression plasmids for synthesis of early viral mRNA and proteins: PB1, PB2, PA, NP and (inconsistently) NS2. The latter plasmids for expression of early FPV viral proteins (pCMV-PB1, pCMV-PB2, pCMV-PA, pCMV-NP, pCMV-NS2 (A. Menke and G. Hobom, unpublished) were used in amounts of: 1 µg/1 µg/ 0.1 µg/ 1µg/ 0.3µg, respectively, while 1 µg each was used for the set of eight RNA-polymerase I vRNA plasmids. While the entire set of FPV plasmids in this category has been obtained by RT-PCR cloning into pHH21 (Menke A. and Hobom G., unpublished), the corresponding set of WSN has been obtained from G. Neumann, Madison: pPolI-WSN-PB1, pPolI-WSN-PB2, pPolI-WSN-PA, pPolI-WSN-HA, pPolI-WSN-NP, pPolI-WSN-NA, pPolI-WSN-M, pPolI-WSN-NS. Mixtures between the two series have been used for generation of WSN-FPV viral reassortants, and for generation of WSN/FPV-PB1 chimeric viruses pPolI-WSN-PB1 has been substituted by the corresponding plasmid constructs. The combined sets of plasmids have been mixed in preparation for DNA transfection with Lipofectamin plus in a ratio of 3 µg of plasmid DNAs together with 6 µl of Lipofectamin and 8µl of Lipofectamin plus, and treated as described below. Alternatively, a set of only eight plasmids according to Hoffmann et al., Proc. Natl. Acad. Sci. 97, 6108-6113 (2000), has been used instead, again with chimeric constructs of the PB1 segment in exchange for a regular WSN-PB1 coding sequence. After 48 to 72 h the supernatant of the DNA transfected 293 T cells was used for passage on MDCK cells, and directly or thereafter also for plaque-purification and determination of the yields achieved in the generation of viral strains. Upon preparation of viral stocks the constitution in particular of PB1 vRNA as well as others was confirmed via RT-PCR analysis and diagnostic restriction cleavages of the PCR bands obtained.

pHL1844 plasmid DNA transfection and influenza virus infection: For pHL1844 DNA transfection we used ~$3.6 \times 10^6$ subconfluent 293T cells. Briefly: 3µg of plasmid DNA in 186µl serum-free DMEM were gently mixed with 6µl Lipofectamine and 8µl of Lipofectamine Plus (GIBCO/BRL) and incubated for 30 min at room temperature. In the meantime cells were washed with serum-free medium, and the transfection mix filled up to 3 ml with medium was carefully dispersed over the cells. After 6 h of incubation the medium was changed to DMEM containing 10% FCS and further incubated for 15 h. The transfected cells were washed very carefully with PBS+ (2.5 mM $MgCl_2$; 3.4 mM $CaCl_2$ added) and superinfected with FPV helper virus at an m.o.i. of 2-3 in 1 ml of PBS+. After 1 h the cells were washed and finally resupplied with DMEM containing 10% FCS for another 8 h of incubation. A complete replication cycle of the virus takes place in this period.

Serial passage of virus containing supernatants: After 8 h of viral propagation the supernatant containing the progeny virus was collected, and after a brief centrifugation step (10 000 rpm, 5 min) for cell debris removal it was used for passaging the recombinant virus mixture onto confluent MDCK cells as described previously (Flick R. et al. RNA, Oct.;2(10):1046-57 (1996)).

CAT assay: Cell extracts of 110µl were prepared as described by Gorman *et al.* (1982). In an initial series 50µl of each cell lysate, and depending on the data obtained, also serially diluted amounts of the various cell lysates (always in parallel including material from one or more reference reactions) were mixed with 10µl of 4 mM acetyl-CoA and 8µl of fluorescent-labeled chloramphenicol (borondipyrromethane difluoride fluorophore: BODIPY CAM substrate, FLASH CAT kit, Stratagene). Samples were incubated at 37°C for 3 h. For extracting the reaction products 0.5 ml of ethyl acetate were added, and after a centrifugation step for 3 min at 13000 rpm the upper phase containing the acetylated products was transferred into new Eppendorf tubes and vacuum dried.

The resulting pellet was resuspended in 20µl ethylacetate and the reaction products were separated by thin layer chromatography (TLC plates 20/20 cm, Silica gel 60) using a solvent mixture (mobile phase) of chloroform and methanol (87:13). Finally, the reaction products were visualized by UV illumination, documented by photography and evaluated using the WinCam system (Cybertech, Berlin). Ratios of activity have been calculated relative to reference construct pHL1844 based on three independent sets of serial dilutions of cell lysates down to yielding below 30-50% of product formation in each case. The results are summarized in Table 1 and Figures 2 and 3, right hand columns.

## Claims

1. A human influenza virus comprising an RNA-sequence encoding a modified RNA-polymerase which differs from the wild-type RNA-polymerase of said human influenza virus in that at least one of the amino acid residue(s) distinguishing the wild-type RNA-polymerase of said human influenza virus from FPV Bratislava RNA-polymerase has been replaced with the corresponding amino acid residue(s) as present in FPV Bratislava RNA-polymerase.

2. The influenza virus of claim 1, which is selected from influenza A including strains of type H1N1, H2N2 and H3N2, influenza B and influenza C, and preferably is an influenza A type H1N1, including WSN/33, PR8/34 or the like, an influenza A type H2N2, including Asia/57 or the like, or an influenza A type H3N2, including Victoria/68 or the like.

3. The influenza virus of claim 1 or 2, wherein the at least one distinguishing amino acid residue to be replaced is located within the PB1 segment of the virus.

4. The influenza virus of claim 3, wherein at least one or all of the following PB1 amino acid substitutions S384P, L396I, L628M, V644A, T741A, relative to the wild-type WSN-PB1 polypeptide shown in SEQ ID NO:25 have been effected, preferably the influenza virus strain used is WSN-K68 carrying five distinguishing amino acids.

5. The influenza virus of claim 3, which encodes the PB1 segment shown in SEQ ID NO:27, 29, 31, 33, 35, 37, 39, 41, 43, 45 or 47, preferably said influenza virus comprises a PB1 (segment2) RNA sequence corresponding to the nucleotide sequence shown in SEQ ID NO:26, 28, 30, 32, 34, 36, 38, 40, 42, 44 or 46.

6. The influenza virus according to any one of claims 1 to 5, wherein the modified RNA-polymerase is capable of recognition of segments with modified vRNA promoter sequences resulting in an enhanced rate of transcription and/or replication relative to said wild-type human influenza virus RNA-polymerase.

7. The influenza virus of claim 6, wherein the segments with modified vRNA promoter sequences contain terminal viral RNA sequences which have been modified by nucleotide substitutions in up to five positions, resulting in improved transcription rates of both the vRNA promoter as well as the cRNA promoter as present in the complementary sequences.

8. The influenza virus of claim 7, wherein the 12 nucleotide conserved influenza 3' terminal sequence has been modified by replacement of one to three nucleotides occurring in said sequence at positions 3, 5 and 8 relative to the 3' end by other nucleotides, and/or wherein the 13 nucleotide conserved influenza 5' terminal sequence has been modified by replacement of one or two nucleotides occurring in said sequence at positions 3 and 8 by other nucleotides.

9. The influenza virus of claim 8, wherein the replacements in the 3' terminal nucleotide sequence comprises the modifications G3A and C8U, or G3C and C8G, preferably the replacements in the 3' terminal nucleotide sequence comprises the modifications G3A, U5C and C8U, or G3C, U5C and C8G.

10. The influenza virus of claim 9, which comprises a 3' terminal nucleotide sequence of (5')-CCUGUUUCUACU-3' or (5')-CCUGUUUUUACU-3'.

11. The influenza virus according to any one of claims 7 to 10, wherein the 5' terminal nucleotide sequence comprises the modifications U3A and A8U resulting in a 5'-terminal sequence of 5'-AGAAGAAUCAAGG.

12. The influenza virus according to any one of claims 1 to 11 which is suitable for high yielding expression of one or more foreign recombinant or altered viral proteins, preferably said influenza virus contains

    (i) one or more segment(s) with a foreign recombinant or altered viral gene sequence in addition to the RNA segments of the normal viral genome (additional segment) or partially replacing them (replacing segment), whereby the additional segment(s) and replacing segment(s) comprise the foreign or altered gene encoding the protein to be expressed in monocistronic arrangement and have modified vRNA promoter sequences as defined in claims 7 to 11; and/or
    (ii) one or more bicistronic vRNA segment(s), preferably in ambisense or in tandem arrangement, whereby the bicistronic vRNA segment(s) has/have foreign gene(s) encoding the protein(s) to be expressed and being in covalent linkage with one of the authentic viral genes, preferably the neuraminidase gene, and has/have modified vRNA promoter sequences as defined in claims 7 to 11.

**13.** The influenza viruses according to claim 12 having at least one additional segment coding for one or more foreign genes or one or more altered viral genes in monocistronic arrangement.

**14.** The influenza virus according to claim 13 in which the at least one additional segment codes for a glycoprotein of foreign-viral, animal, human or other origin, with or without in-frame fusion linkage to influenza coding sequences, in which the glycoprotein is at the same time incorporated itself in the virion envelopes, preferably the foreign glycoprotein sequence incorporated as vRNA and as a protein is derived from the genome of the contagious swine fever virus (CSFV), the bovine viral diarrhea virus (BVDV), the vesicular stomatitis virus (VSV), the Borna virus (BDV), the Marburg virus, the Ebola virus, the hepatitis C virus, the tick-borne meningo-encephalitis virus (TBE), the Western Nile virus or the human immunodeficiency virus (HIV).

**15.** The influenza virus according to claim 13 in which one or more of the incorporated foreign genes code

(a) for a lymphokine of human or animal origin which is secreted by the influenza vector infected cell, or
(b) for the expression of an apoptosis-inducing gene or a toxin gene, effective in the primarily infected cell or, after secretion, in neighboring cells.

**16.** The influenza virus of claim 12, which is genetically stable in the absence of any helper virus and which comprises at least one viral RNA segment being an ambisense RNA molecule (ambisense RNA segment) and containing one of the standard viral genes in sense orientation and a foreign, recombinant gene in anti-sense orientation, or *vice versa*, in overall convergent arrangement.

**17.** The influenza virus of claim 16, wherein at least one of the regular viral RNA segments is replaced by an ambisense RNA segment which contains one of the standard viral genes in sense orientation and a foreign, recombinant gene in anti-sense orientation, or *vice versa,* in overall convergent arrangement.

**18.** The influenza virus of claim 16 or 17, wherein in the ambisense RNA molecule said foreign recombinant gene is covalently bound to one of the viral genes, while the original vRNA segment coding for the same gene is deleted from the recombinant virus by way of specific ribozyme cleavage or is left out from the set of RNA-polymerase I promoted vRNA synthesizing plasmids, able to result in infectious viruses.

**19.** The influenza virus according to any one of claims 16 to 18, wherein one or more of the standard viral RNA segments, differing from said at least one ambisense RNA segment, comprises a vRNA encoding a foreign gene, preferably one or more of the regular viral RNA segments has (have) been exchanged for a vRNA encoding a foreign gene, preferably one or both of the standard glycoproteins hemagglutinin and neuraminidase have been exchanged into foreign glycoprotein(s) or into fusion glycoproteins consisting of an anchor segment derived from hemagglutinin and an ectodomain obtained from the foreign source, viral or cellular, or in which such recombinant glycoprotein has been inserted as a third molecular species in addition to the remaining standard components.

**20.** The influenza virus of claim 12, which is genetically stable in the absence of any helper virus and which comprises at least one viral RNA segment being a bicistronic RNA molecule coding for two genes in tandem arrangement (tandem RNA segment), in said tandem RNA segment one of the standard viral genes being in covalent junction with a foreign, recombinant gene and said tandem RNA segment having an upstream splice donor and a downstream splice acceptor signal surrounding the proximal coding region.

**21.** The influenza virus of claim 20, wherein the tandem RNA segment contains one of the standard viral genes in distal mRNA position behind a foreign, recombinant gene in proximal position, or vice versa, both in antisense orientation with regard to the viral RNA as present within the virus.

**22.** The influenza virus of claim 20 or 21, wherein at least one of the regular viral RNA segments is replaced by a tandem RNA segment, preferably the replaced regular viral RNA segment is selected from the neuraminidase segment, hemagglutinin segment and NS segment.

**23.** The influenza virus according to any one of claims 20 to 22, wherein the splice donor and splice acceptor signals are selected from sequences as present in influenza WSN segment 7 and 8 or other partially effective splice reacting substrates, preferably the splice donor and splice acceptor signals are selected from sequences as present in influenza WSN segment 7.

**24.** The influenza virus according to any one of claims 20 to 23, wherein one or more of the regular viral RNA segments, differing from said at least one tandem RNA segment, comprises a vRNA encoding a foreign gene which may or may not be in covalent connection to one of the viral genes, and preferably one or more of the regular viral RNA segments has (have) been deleted and replaced by a tandem vRNA encoding in addition a foreign gene.

**25.** The influenza virus according to any one of claims 20 to 24, in which the foreign gene(s) in the tandem RNA segment

(i) code for proteins and/or glycoproteins which are secreted from cells infected with the recombinant virus;
(ii) code for proteins or artificial polypeptides designed to support an efficient HLA-restricted presentation of inherent epitopes at the surface of infected cells, for stimulation of a B cell and/or T cell response;
(iii) is a nucleotide sequence causing viral attenuation, preferably the foreign gene is coding for part of the viral neuraminidase gene in inverted, i.e. sense orientation, with or without an inserted ribozyme sequence,

preferably the tandem segment part of the neuraminidase gene in sense orientation is attached to the hemagglutinin vRNA segment, and optionally another gene or reporter gene is encoded in a second tandem vRNA, preferably in conjunction with NS2.

**26.** The influenza virus according to any one of claims 16 to 25 which is suitable for the expression of non-influenza genes or synthetic genes, or gene-inhibitory sequences such as, but not limited to, antisense genes or ribozymes, whereby

(i) the non-influenza genes are covalently linked to one of the viral genes,
(ii) the non-influenza gene constitutes a membrane glycoprotein consisting of a fusion of the viral HA transmembrane and cytoplasmic regions with the foreign ectodomain sequence.

**27.** A non-avian, non-human influenza virus, preferably an equine or a porcine influenza virus comprising an RNA-sequence encoding a modified RNA-polymerase which differs from the wild-type RNA-polymerase of said non-avian, non-human influenza virus in that at least one of the amino acid residue(s) distinguishing the wild-type RNA-polymerase of said non-avian, non-human influenza virus from FPV Bratislava RNA-polymerase has been replaced with the corresponding amino acid residue(s) as present in FPV Bratislava RNA-polymerase, preferably said influenza virus is as defined in any one of claims 2 to 26.

**28.** A process for preparing the influenza virus of claims 1 to 27 which comprises replacing the RNA-sequence encoding the wild-type RNA-polymerase of said influenza virus with an RNA-sequence encoding the modified RNA-polymerase.

**29.** The process of claim 28, which is suitable for preparing PB1-chimeric viruses as defined in claims 1 to 11 and 27 as well as recombinant viruses as defined in claims 12 to 27, said viruses being generated via cotransfection of up to eight cDNA plasmids containing the viral cDNAs, or chimeric (segment 2: PB1) and bicistronic recombinant (segment 6: NA/foreign gene) cDNA sequences instead, in such a way that they are transcribed *in vivo* by both RNA-polymerase I and RNA-polymerase II and jointly give rise to progeny viruses according to the plasmid insert design.

**30.** A pharmaceutical composition comprising the influenza virus according to any one of claims 1 to 27.

**31.** The pharmaceutical composition of claim 30 which is suitable

(i) for gene transfer into cells, preferably into mammalian cells, more preferably into human cells, by recombinant viral infection;
(ii) for gene transfer into antigen-presenting cells, preferably into dendritic cells, and the use of the obtained product for *ex vivo* immunotherapy;
(iii) for *in vivo* somatic gene therapy;
(iv) for *in vivo* vaccination, including therapeutic and prophylactic vaccination;
(v) for eliciting an immune response, including the induction of a T-cell response;
(vi) for treating a growing tumor or a chronic infectious disease.

**32.** Use of the influenza virus according to any one of claims 1 to 27 for preparing an agent

(i) for gene transfer into cells, preferably into mammalian cells, more preferably into human cells, by viral infection;
(ii) for gene transfer into antigen-presenting cells and the use of the obtained product for *ex vivo* immunotherapy;
(iii) for *in vivo* somatic gene therapy;
(iv) for *in vivo* vaccination, including therapeutic and prophylactic vaccination;
(v) for eliciting an immune response, including the induction of a T-cell response;
(vi) for treating a growing tumor or a chronic infectious disease.

**33.** A method for

(i) gene transfer into cells, preferably into mammalian cells, more preferably into human cells, by viral infection;
(ii) gene transfer into antigen-presenting cells, and the use of the obtained product for *ex vivo* immunotherapy;
(iii) *in vivo* somatic gene therapy;
(iv) *in vivo* vaccination, including therapeutic and prophylactic vaccination;
(v) eliciting an immune response, including the induction of a T-cell response, preferably a CD4+ T-cell response, a CD8 T-cell response or both, or the induction of an antibody response;
(vi) treating a growing tumor or a chronic infectious disease;
(vii) preparing a vaccine;
(viii) preventing and/or treating influenza;

which comprises contacting the cells, the antigen-presenting cells, the person or the patient in need for vaccination, for influenza treatment or for somatic gene therapy, or cell cultures with the influenza virus according to any one of claims 1 to 27.

**34.** A method for the production of proteins or glycoproteins which comprises utilizing the influenza virus according to claims 1 to 27 as expression vector, preferably the production method is performed in cell culture cells or in fertilized chicken eggs.

**35.** Use of the influenza virus according to claims 1 to 27 for preparing agents

(i) for transfer and expression of foreign genes into cells infected by such viruses, or
(ii) for transfer and expression of RNA molecules into cells infected by such viruses, preferably the RNA molecules to be expressed are antisense sequences or double-strand sequences relative to the target cell cellular mRNA molecules, and/or the agent is suitable for sequence-specific gene silencing, preferably by antisense RNA or RNA interference mechanisms such as ribozyme cleavages of target RNAs.

**36.** A method for transfer and expression of foreign genes into cells, and for transfer and expression of RNA molecules into cells, which method comprises infecting the cells with the influenza virus according to claims 1 to 27.

**37.** Use of the influenza virus according to claims 1 to 27 for preparing agents for immunotherapy, preferably for autologous immunotherapy.

**38.** A method for an immunotherapy which comprises *ex vivo* infection of immune cells, preferably dendritic cells, with the influenza virus according to claims 1 to 27, and introduction of the transduced cells into the patient.

**39.** A method to elicit an immune response directed against an antigen, comprising the steps of introducing the influenza virus as defined in claims 1 to 27, preferably the human influenza virus as defined in claims 1 to 26, into a cell or administering it to a mammal, wherein said influenza virus contains at least one foreign gene encoding the antigen.

**40.** The method of claim 39, wherein said foreign gene encoding the antigen is a polynucleotide sequence associated with a disease, preferably an infectious diseases, or a tumor disease, preferably the antigen is exemplified by, but not limited to,

(i) virus-associated antigens such as the HIV antigens gp160, gp 120, rev, tat, NC, the HBV e-antigen or core antigen, the HPV E6 or E7 antigen, the herpes simplex virus glycoproteins or core proteins, other herpesvirus antigens and further viral and microbial antigens known to those skilled in the art, or
(ii) tumor associated antigens, especially the so-called cancer testis-antigens exemplified by the MAGE, BAGE and GAGE family of antigens, the NY-ESO-1 antigen, the SSX antigens, exemplified by the HOM-MEL-40.

**41.** The method of claim 39 or 40, wherein the polynucleotide sequence

(i) is derivable from a cDNA library isolated from tumor cells, or testis cells, or virus-infected cells, or micriobially infected cells, or cell-lines,
(ii) is a fusion protein consisting of epitopes derived from one or more T-cell specific epitope sequences as present in viral or other pathogens, or in tumor associated antigens.

**42.** A vaccine for therapeutic or prophylactic purposes which is

(a) a human influenza virus vaccine comprising a human influenza virus as defined in claims 1 to 26 or in claims 39 to 41, preferably said human influenza virus encodes the antigen for a membrane protein and in addition contains the membrane protein in the viral envelope; or
(b) a non-human influenza virus vaccine, preferably an equine or porcine influenza virus vaccine, comprising a virus as defined in claim 27.

**43.** The vaccine according to claim 42, wherein the virus

(i) is capable of being attenuated according to the tandem attenuation mechanism;
(ii) is only capable of limited replication; or
(iii) is an inactivated virus.

**44.** Transduced cells, preferably antigen-presenting cells, obtainable by the method of claim 33, option (i) or (ii).

**45.** A vaccine comprising transduced cells as defined in claim 44, preferably comprising transduced antigen-presenting cells, more preferably transduced dendritic cells, and most preferably mature dendritic cells, wherein said antigen-presenting cells are transduced *in vitro*.

**46.** A method to identify a polynucleotide sequence encoding at least one HLA-restricted epitope comprising the steps of

(a) preparing a gene bank or a cDNA bank from the cell or the microorganism to be tested;
(b) incorporating the cDNA or the DNA of the gene bank into the genome of the influenza virus as defined in claims 1 to 27 to yield recombinant virus particles,
(c) infecting immortalized autologous cells, which are capable of expression of HLA-class I molecules and/or HLA-class II molecules on their surface, with the recombinant virus particles obtained in step (b),
(d) expressing the proteins encoded by said cDNA or said DNA of the gene bank in the autologous cells and presenting the fragments of the proteins produced by the autologous cells or the cell surface in connection with HLA molecules;
(e) co-cultivating T-cells with the autologous cells; and
(f) stimulating the T-cells by such autologous cells which present antigens on their surface, whereby said antigens are recognized by the T-cells.

**47.** A method to study gene function in antigen presenting cells comprising steps (a) to (f) of claim 46.

**FIG. 1**

| Type | Origin | PB1: Positions 52 | 54 | 105 | 175 | 208 | 298 | 364 | 383 | 384 | 396 | 431 | 464 |
|---|---|---|---|---|---|---|---|---|---|---|---|---|---|
| H7N7 | Bratislava 82 chicken | K | K | N | D | K | L | L | E | **P** | **I** | Y | D |
| H7N7 | Rostock34 chicken | K | K | N | D | K | L | L | E | S | L | Y | D |
| H1N1 | WSN33 human | R | R | T | N | R | I | I | D | S | L | H | N |
| H1N1 | Wisconsin88 human | R | K | N | E | K | L | L | D | S | L | Y | D |
| H2N2 | Singapore57 human | K | K | N | D | K | L | L | E | S | L | Y | D |
| H2N2 | Ann Arbor60 human | K | K | N | D | K | L | L | E | S | L | Y | D |
| H3N2 | Honkong68 human | K | K | N | D | K | L | L | E | S | L | Y | D |
| H3N2 | Shiga97 human | K | K | N | D | K | L | L | E | S | L | Y | D |
| H3N2 | Hongkong82 swine | K | K | N | D | K | L | L | E | S | L | Y | D |
| H3N2 | Katakyushu93 human | R | K | N | D | K | L | L | E | S | L | Y | D |
| H3N8 | Tennessee86 equine | K | K | N | N | K | L | L | E | S | L | Y | D |
| H4N2 | Minnesota80 turkey | K | K | N | D | K | L | L | E | S | L | Y | D |
| H4N6 | Ontario99 swine | K | K | N | D | K | L | L | E | S | L | Y | D |
| H5N1 | Hongkong97 human | K | R | N | D | K | L | L | E | S | L | Y | D |
| H6N1 | Taiwan99 chicken | K | K | N | D | K | L | L | E | S | L | Y | D |
| H7N7 | London73 equine | K | K | N | D | K | L | L | D | S | L | Y | D |
| H9N2 | Pakistan99 chicken | K | K | N | D | K | L | L | E | S | L | Y | D |

**FIG 1** (continued)

| Type | Origin | PB1: Positions 473 | 576 | 584 | 628 | 633 | 636 | 644 | 645 | 654 | 741 |
|---|---|---|---|---|---|---|---|---|---|---|---|
| H7N7 | Bratislava 82 chicken | V | L | R | **M** | S | E | **A** | V | S | A |
| H7N7 | Rostock34 chicken | V | L | R | L | S | E | V | V | S | A |
| H1N1 | WSN33 human | L | I | H | L | N | D | V | I | N | T |
| H1N1 | Wisconsin88 human | L | I | R | L | S | E | V | V | T | A |
| H2N2 | Singapore57 human | V | L | R | L | S | E | V | V | S | A |
| H2N2 | Ann Arbor60 human | V | L | R | L | S | E | V | V | S | A |
| H3N2 | Honkong68 human | V | L | Q | L | S | E | V | V | S | S |
| H3N2 | Shiga97 human | V | L | Q | L | S | E | V | V | S | S |
| H3N2 | Hongkong82 swine | V | L | R | L | S | E | V | V | S | A |
| H3N2 | Katakyushu93 human | V | L | Q | L | S | E | V | V | S | S |
| H3N8 | Tennessee86 equine | V | L | R | L | S | E | V | V | S | A |
| H4N2 | Minnesota80 turkey | V | L | R | L | S | E | V | V | N | A |
| H4N6 | Ontario99 swine | V | L | R | L | S | E | V | V | S | A |
| H5N1 | Hongkong97 human | V | L | R | L | S | E | V | V | S | A |
| H6N1 | Taiwan99 chicken | V | L | R | L | S | E | V | V | S | A |
| H7N7 | London73 equine | V | L | R | L | S | E | V | V | S | A |
| H9N2 | Pakistan99 chicken | V | L | R | L | S | E | V | V | S | A |

Fig.2

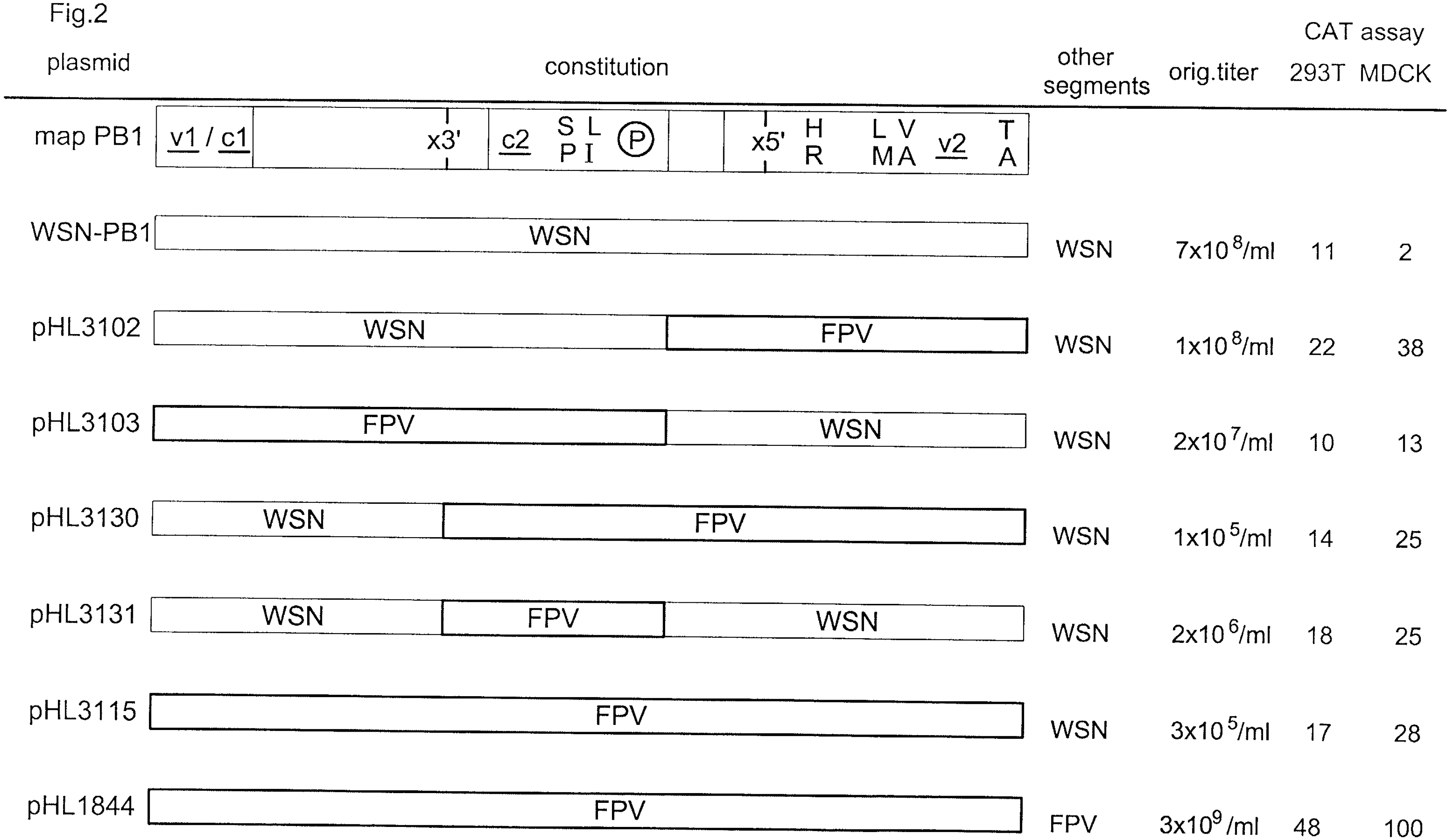
plasmid
constitution
other segments
orig.titer
CAT assay
293T MDCK
map PB1
v1 / c1
x3'
c2
S L
P I
P
x5'
H
R
L V
M A
v2
T
A
WSN-PB1
WSN
WSN
7x10^8/ml
11
2
pHL3102
WSN
FPV
WSN
1x10^8/ml
22
38
pHL3103
FPV
WSN
WSN
2x10^7/ml
10
13
pHL3130
WSN
FPV
WSN
1x10^5/ml
14
25
pHL3131
WSN
FPV
WSN
WSN
2x10^6/ml
18
25
pHL3115
FPV
WSN
3x10^5/ml
17
28
pHL1844
FPV
FPV
3x10^9/ml
48
100

Fig.3

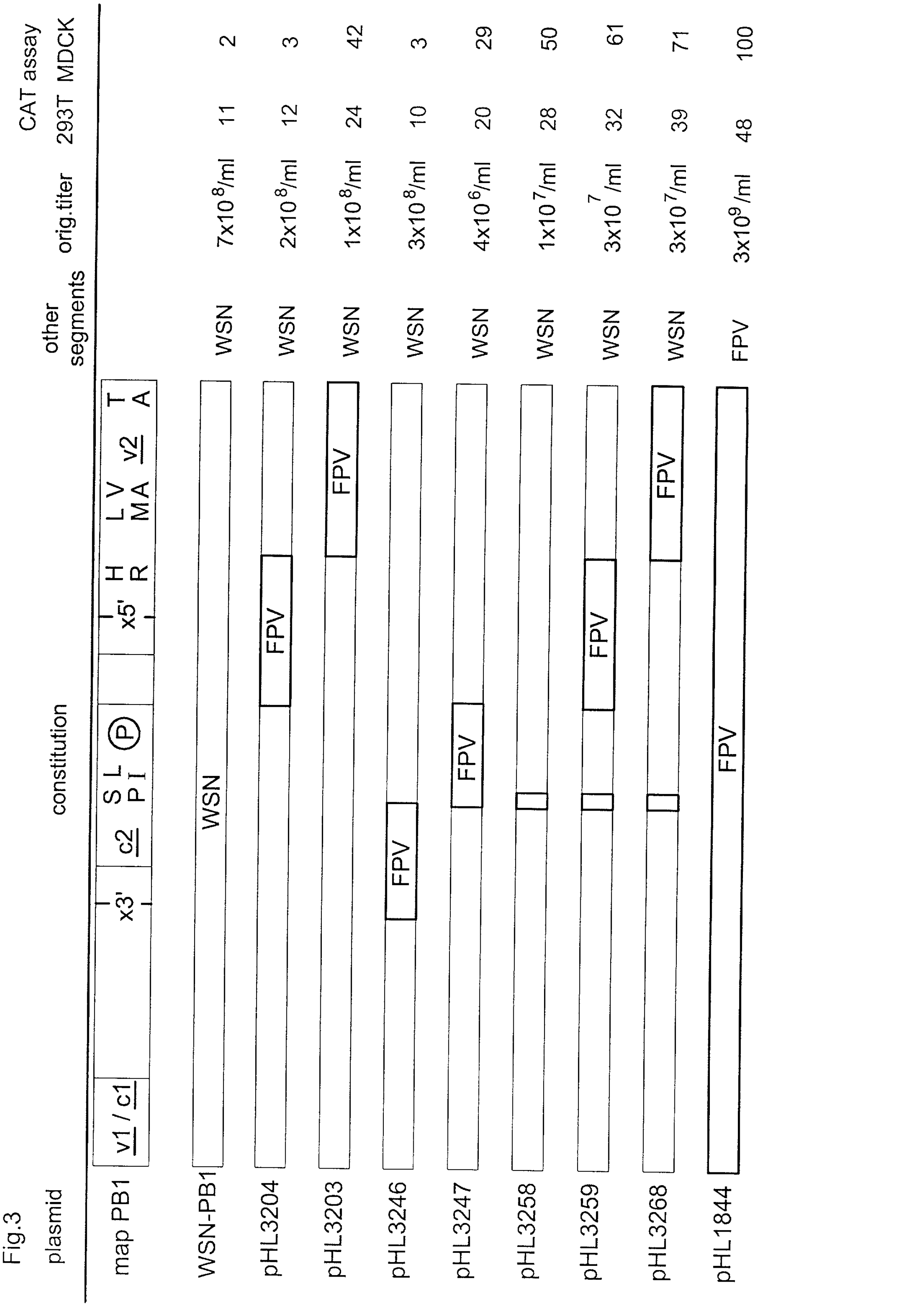
plasmid
constitution
other segments
orig.titer
CAT assay
293T MDCK
map PB1
v1 / c1
x3'
c2
S L P I
P
x5'
H R
L V MA
v2
T A
WSN-PB1
WSN
WSN
7x10⁸/ml 11 2
pHL3204
FPV
WSN
2x10⁸/ml 12 3
pHL3203
FPV
WSN
1x10⁸/ml 24 42
pHL3246
FPV
WSN
3x10⁸/ml 10 3
pHL3247
FPV
WSN
4x10⁶/ml 20 29
pHL3258
WSN
1x10⁷/ml 28 50
pHL3259
FPV
WSN
3x10⁷/ml 32 61
pHL3268
FPV
WSN
3x10⁷/ml 39 71
pHL1844
FPV
FPV
3x10⁹/ml 48 100

Fig. 4

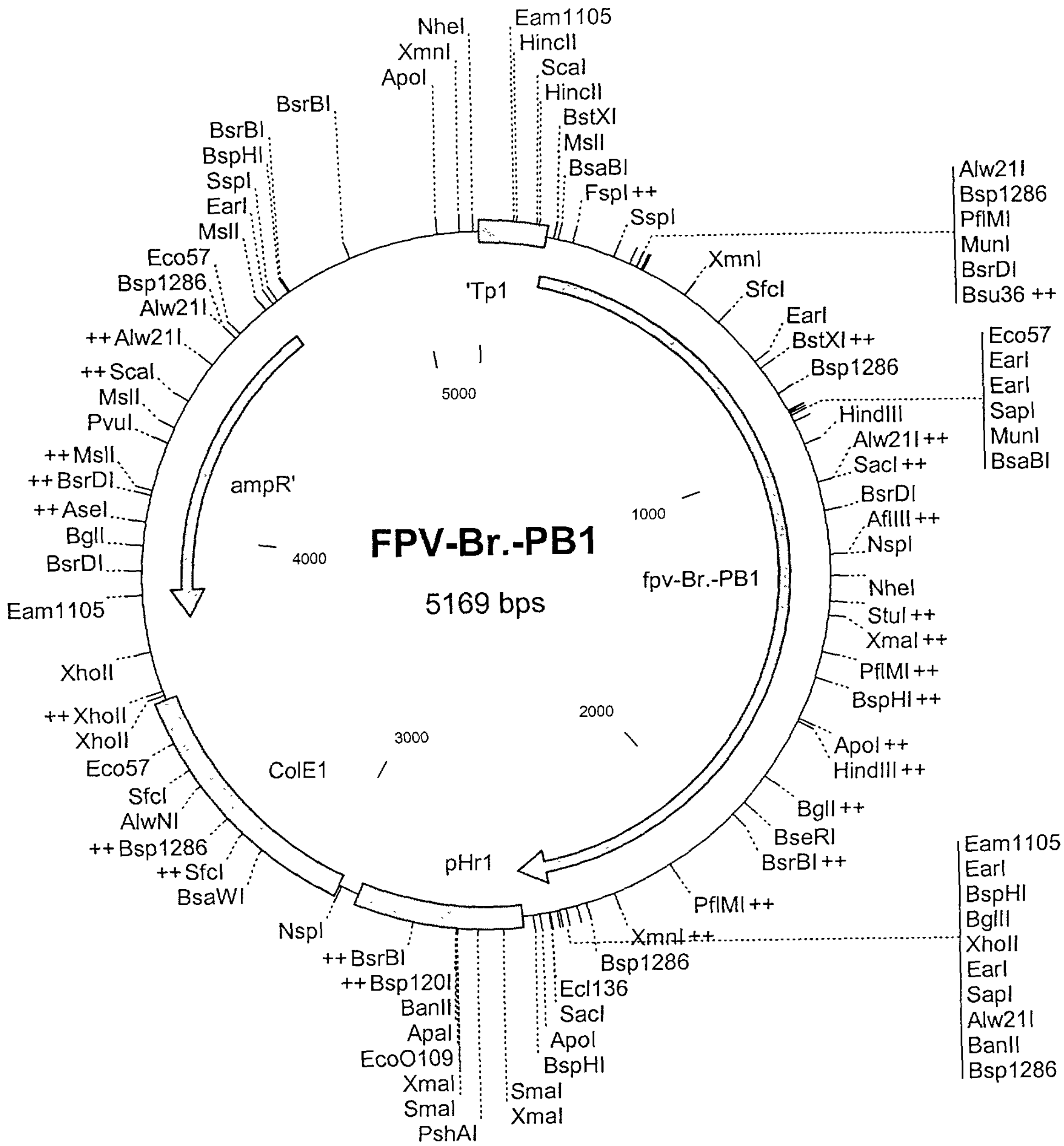
FPV-Br.-PB1
5169 bps
'Tp1
fpv-Br.-PB1
ampR'
ColE1
pHr1
5000
1000
2000
3000
4000
Eam1105
HincII
ScaI
HincII
BstXI
MslI
BsaBI
FspI ++
SspI
NheI
XmnI
ApoI
BsrBI
BsrBI
BspHI
SspI
EarI
MslI
Eco57
Bsp1286
Alw21I
++ Alw21I
++ ScaI
MslI
PvuI
++ MslI
++ BsrDI
++ AseI
BglI
BsrDI
Eam1105
XhoII
++ XhoII
XhoII
Eco57
SfcI
AlwNI
++ Bsp1286
++ SfcI
BsaWI
NspI
++ BsrBI
++ Bsp120I
BanII
ApaI
EcoO109
XmaI
SmaI
PshAI
SmaI
XmaI
BspHI
ApoI
SacI
Ecl136
Bsp1286
XmnI ++
PflMI ++
BsrBI ++
BseRI
BglI ++
HindIII ++
ApoI ++
BspHI ++
PflMI ++
XmaI ++
StuI ++
NheI
NspI
AflIII ++
BsrDI
SacI ++
Alw21I ++
HindIII
Bsp1286
BstXI ++
EarI
SfcI
XmnI
Alw21I
Bsp1286
PflMI
MunI
BsrDI
Bsu36 ++
Eco57
EarI
EarI
SapI
MunI
BsaBI
Eam1105
EarI
BspHI
BglII
XhoII
EarI
SapI
Alw21I
BanII
Bsp1286

Fig. 5

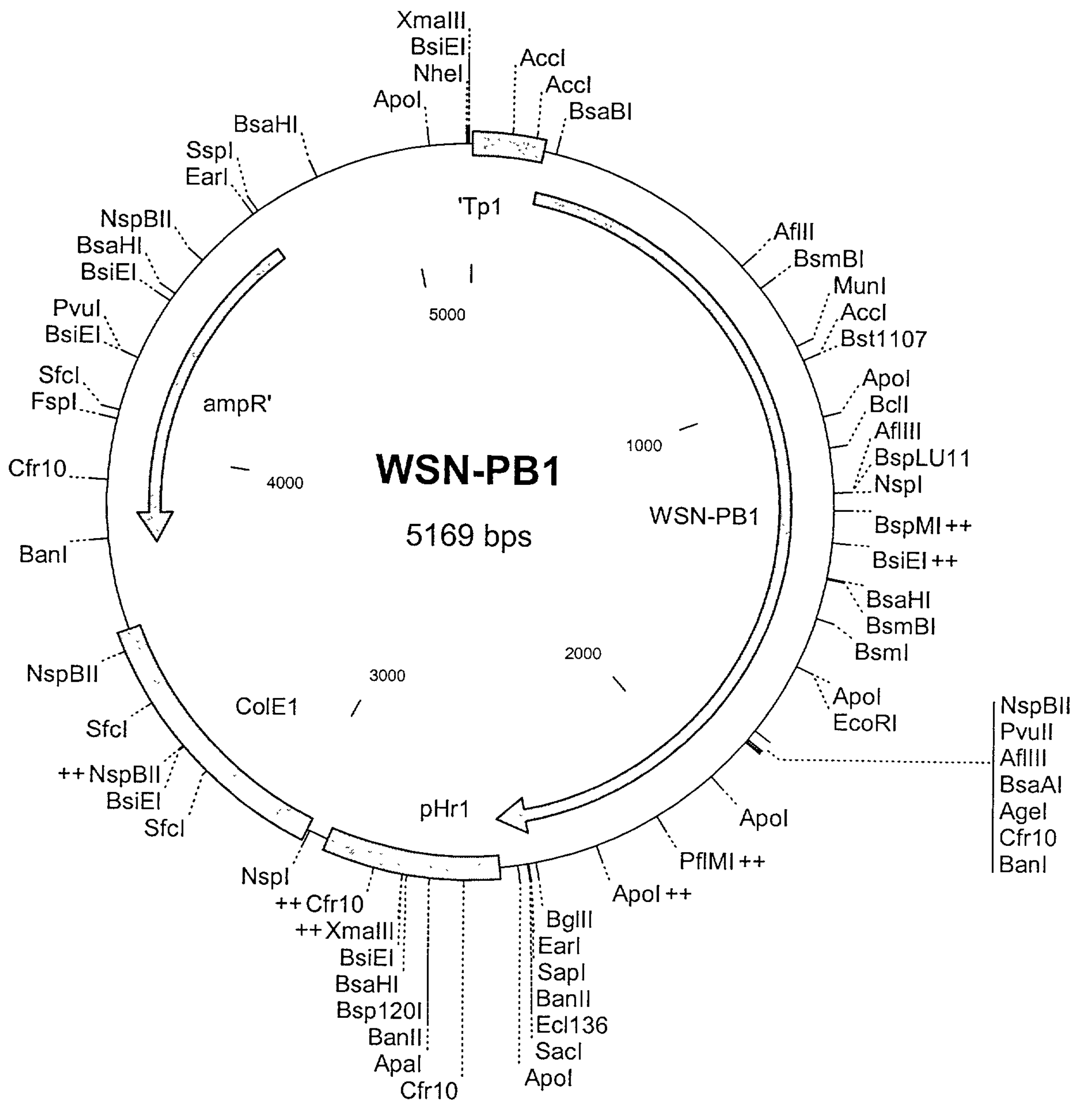
WSN-PB1
5169 bps
'Tp1
WSN-PB1
pHr1
ColE1
ampR'
5000
1000
2000
3000
4000
XmaIII
BsiEI
NheI
ApoI
AccI
AccI
BsaBI
BsaHI
SspI
EarI
NspBII
BsaHI
BsiEI
PvuI
BsiEI
SfcI
FspI
Cfr10
BanI
AflII
BsmBI
MunI
AccI
Bst1107
ApoI
BclI
AflIII
BspLU11
NspI
BspMI ++
BsiEI ++
BsaHI
BsmBI
BsmI
ApoI
EcoRI
NspBII
PvuII
AflIII
BsaAI
AgeI
Cfr10
BanI
ApoI
PflMI ++
ApoI ++
BglII
EarI
SapI
BanII
Ecl136
SacI
ApoI
NspBII
SfcI
++NspBII
BsiEI
SfcI
NspI
++Cfr10
++XmaIII
BsiEI
BsaHI
Bsp120I
BanII
ApaI
Cfr10

Fig. 6

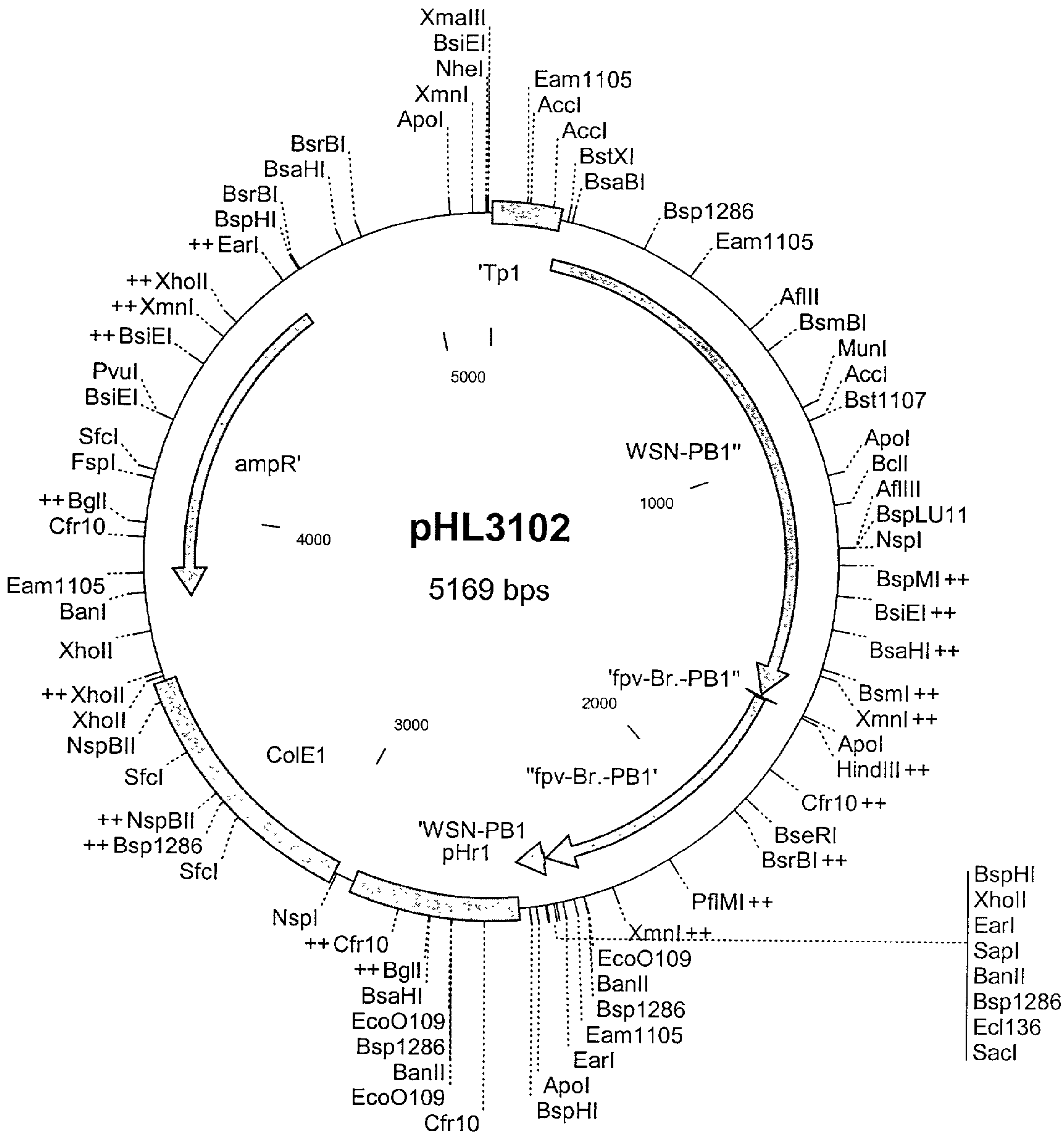
pHL3102
5169 bps
'Tp1
WSN-PB1"
'fpv-Br.-PB1"
"fpv-Br.-PB1'
'WSN-PB1
pHr1
ColE1
ampR'
1000
2000
3000
4000
5000
XmaIII
BsiEI
NheI
XmnI
ApoI
Eam1105
AccI
AccI
BstXI
BsaBI
Bsp1286
Eam1105
AflII
BsmBI
MunI
AccI
Bst1107
ApoI
BclI
AflIII
BspLU11
NspI
BspMI ++
BsiEI ++
BsaHI ++
BsmI ++
XmnI ++
ApoI
HindIII ++
Cfr10 ++
BseRI
BsrBI ++
PflMI ++
XmnI ++
EcoO109
BanII
Bsp1286
Eam1105
EarI
ApoI
BspHI
BspHI
XhoII
EarI
SapI
BanII
Bsp1286
Ecl136
SacI
Cfr10
EcoO109
BanII
Bsp1286
EcoO109
BsaHI
++ BglI
++ Cfr10
NspI
SfcI
++ Bsp1286
++ NspBII
SfcI
NspBII
XhoII
++ XhoII
XhoII
BanI
Eam1105
Cfr10
++ BglI
FspI
SfcI
BsiEI
PvuI
++ BsiEI
++ XmnI
++ XhoII
++ EarI
BspHI
BsrBI
BsaHI
BsrBI

Fig. 7

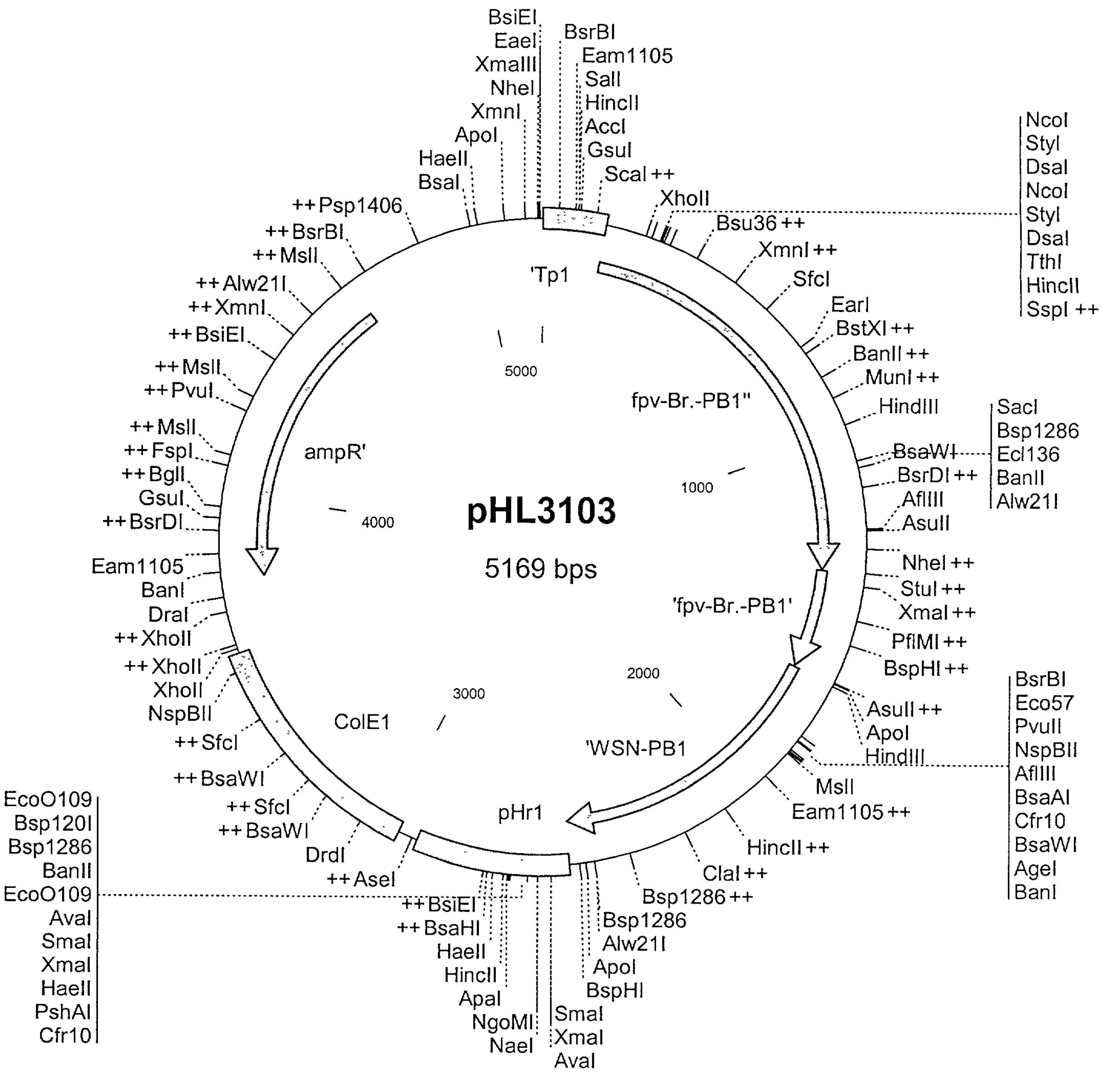
pHL3103
5169 bps
'Tp1
fpv-Br.-PB1"
'fpv-Br.-PB1'
'WSN-PB1
pHr1
ColE1
ampR'
1000
2000
3000
4000
5000
BsiEI
EaeI
XmaIII
NheI
XmnI
ApoI
HaeII
BsaI
BsrBI
Eam1105
SalI
HincII
AccI
GsuI
ScaI ++
XhoII
NcoI
StyI
DsaI
NcoI
StyI
DsaI
TthI
HincII
SspI ++
Bsu36 ++
XmnI ++
SfcI
EarI
BstXI ++
BanII ++
MunI ++
HindIII
SacI
Bsp1286
Ecl136
BanII
Alw21I
BsaWI
BsrDI ++
AflIII
AsuII
NheI ++
StuI ++
XmaI ++
PflMI ++
BspHI ++
BsrBI
Eco57
PvuII
NspBII
AflIII
BsaAI
Cfr10
BsaWI
AgeI
BanI
AsuII ++
ApoI
HindIII
MslI
Eam1105 ++
HincII ++
ClaI ++
Bsp1286 ++
Bsp1286
Alw21I
ApoI
BspHI
SmaI
XmaI
AvaI
NaeI
NgoMI
ApaI
HincII
HaeII
++ BsaHI
++ BsiEI
EcoO109
Bsp120I
Bsp1286
BanII
EcoO109
AvaI
SmaI
XmaI
HaeII
PshAI
Cfr10
++ AseI
DrdI
++ BsaWI
++ SfcI
++ BsaWI
++ SfcI
NspBII
XhoII
++ XhoII
++ XhoII
DraI
BanI
Eam1105
++ BsrDI
GsuI
++ BglI
++ FspI
++ MslI
++ PvuI
++ MslI
++ BsiEI
++ XmnI
++ Alw21I
++ MslI
++ BsrBI
++ Psp1406

Fig. 8

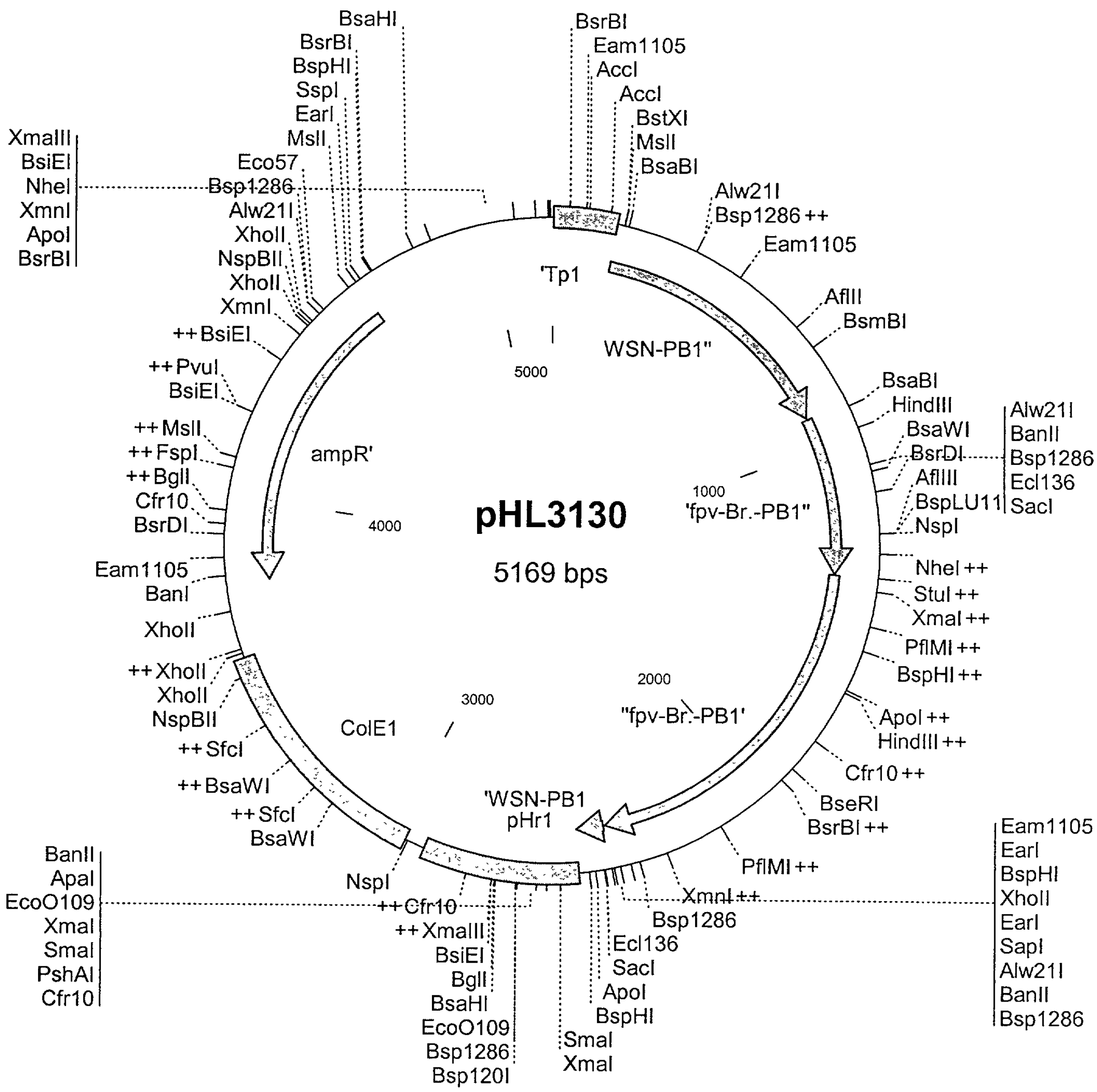
pHL3130
5169 bps
'Tp1
WSN-PB1"
'fpv-Br.-PB1"
"fpv-Br.-PB1'
'WSN-PB1
pHr1
ColE1
ampR'
1000
2000
3000
4000
5000
BsrBI
Eam1105
AccI
AccI
BstXI
MslI
BsaBI
Alw21I
Bsp1286 ++
Eam1105
AflII
BsmBI
BsaBI
HindIII
BsaWI
BsrDI
AflIII
BspLU11I
NspI
Alw21I
BanII
Bsp1286
Ecl136
SacI
NheI ++
StuI ++
XmaI ++
PflMI ++
BspHI ++
ApoI ++
HindIII ++
Cfr10 ++
BseRI
BsrBI ++
PflMI ++
XmnI ++
Bsp1286
Ecl136
SacI
ApoI
BspHI
SmaI
XmaI
Eam1105
EarI
BspHI
XhoII
EarI
SapI
Alw21I
BanII
Bsp1286
++ Cfr10
++ XmaIII
BsiEI
BglI
BsaHI
EcoO109
Bsp1286
Bsp120I
BanII
ApaI
EcoO109
XmaI
SmaI
PshAI
Cfr10
NspI
BsaWI
++ SfcI
++ BsaWI
++ SfcI
NspBII
XhoII
++ XhoII
XhoII
BanI
Eam1105
BsrDI
Cfr10
++ BglI
++ FspI
++ MslI
BsiEI
++ PvuI
++ BsiEI
XmnI
XhoII
NspBII
XhoII
Alw21I
Bsp1286
Eco57
MslI
EarI
SspI
BspHI
BsrBI
BsaHI
XmaIII
BsiEI
NheI
XmnI
ApoI
BsrBI

Fig. 9

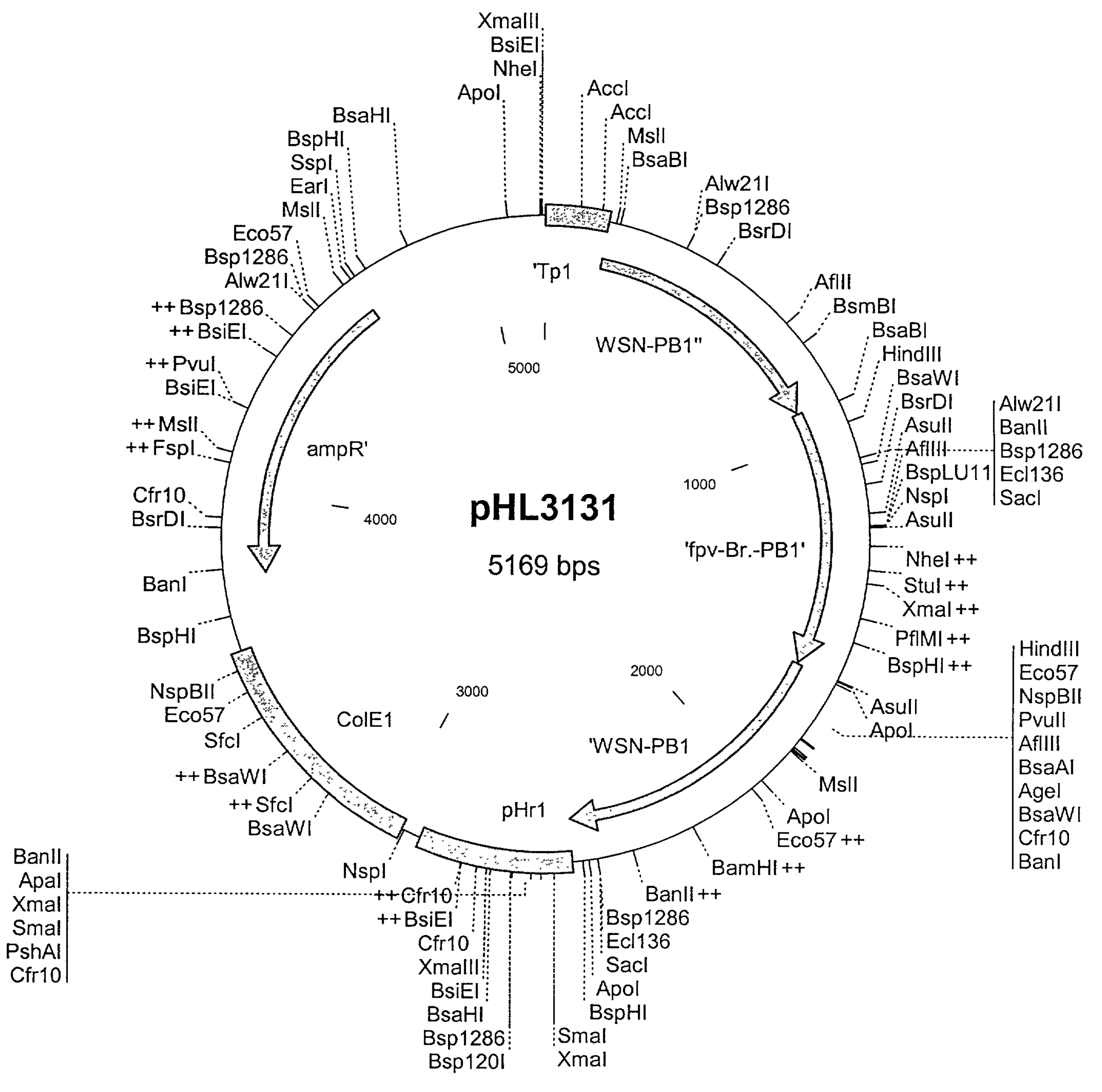
pHL3131
5169 bps
'Tp1
WSN-PB1"
'fpv-Br.-PB1'
'WSN-PB1
pHr1
ColE1
ampR'
1000
2000
3000
4000
5000
XmaIII
BsiEI
NheI
ApoI
AccI
AccI
MslI
BsaBI
Alw21I
Bsp1286
BsrDI
AflII
BsmBI
BsaBI
HindIII
BsaWI
BsrDI
AsuII
AflIII
BspLU11
NspI
AsuII
Alw21I
BanII
Bsp1286
Ecl136
SacI
NheI ++
StuI ++
XmaI ++
PflMI ++
BspHI ++
AsuII
ApoI
HindIII
Eco57
NspBII
PvuII
AflIII
BsaAI
AgeI
BsaWI
Cfr10
BanI
MslI
ApoI
Eco57 ++
BamHI ++
BanII ++
Bsp1286
Ecl136
SacI
ApoI
BspHI
SmaI
XmaI
Bsp120I
Bsp1286
BsaHI
BsiEI
XmaIII
Cfr10
++ BsiEI
++ Cfr10
NspI
BanII
ApaI
XmaI
SmaI
PshAI
Cfr10
BsaWI
++ SfcI
++ BsaWI
SfcI
Eco57
NspBII
BspHI
BanI
BsrDI
Cfr10
++ FspI
++ MslI
BsiEI
++ PvuI
++ BsiEI
++ Bsp1286
Alw21I
Bsp1286
Eco57
MslI
EarI
SspI
BspHI
BsaHI

Fig. 10

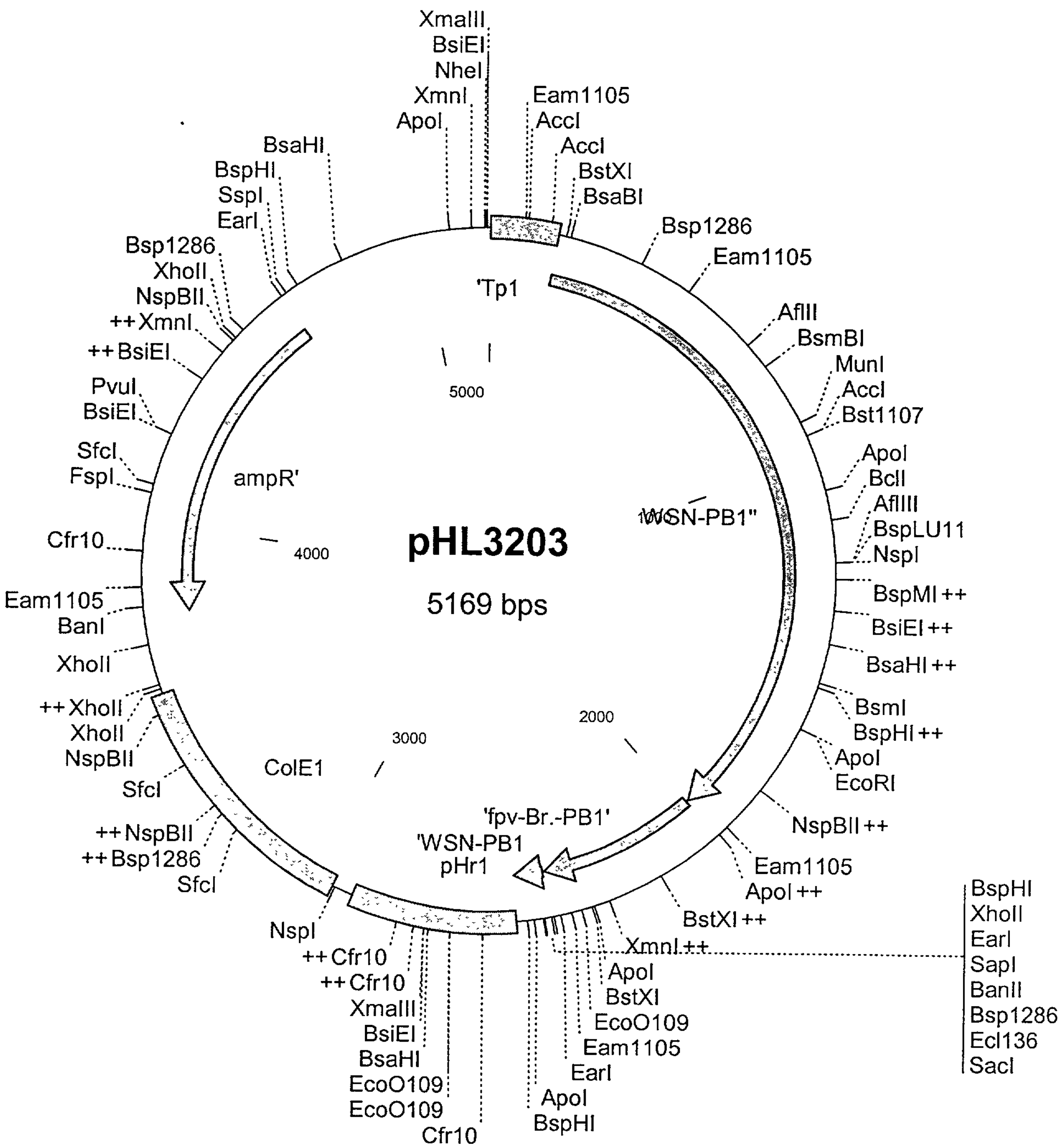
pHL3203
5169 bps
'Tp1
WSN-PB1"
'fpv-Br.-PB1'
'WSN-PB1
pHr1
ColE1
ampR'
1000
2000
3000
4000
5000
XmaIII
BsiEI
NheI
XmnI
ApoI
Eam1105
AccI
AccI
BstXI
BsaBI
Bsp1286
Eam1105
AflII
BsmBI
MunI
AccI
Bst1107
ApoI
BclI
AflIII
BspLU11
NspI
BspMI ++
BsiEI ++
BsaHI ++
BsmI
BspHI ++
ApoI
EcoRI
NspBII ++
Eam1105
ApoI ++
BstXI ++
XmnI ++
BspHI
XhoII
EarI
SapI
BanII
Bsp1286
Ecl136
SacI
ApoI
BstXI
EcoO109
Eam1105
EarI
ApoI
BspHI
Cfr10
EcoO109
EcoO109
BsaHI
BsiEI
XmaIII
++Cfr10
++Cfr10
NspI
SfcI
++Bsp1286
++NspBII
SfcI
NspBII
XhoII
++XhoII
XhoII
BanI
Eam1105
Cfr10
FspI
SfcI
BsiEI
PvuI
++BsiEI
++XmnI
NspBII
XhoII
Bsp1286
EarI
SspI
BspHI
BsaHI

Fig. 11

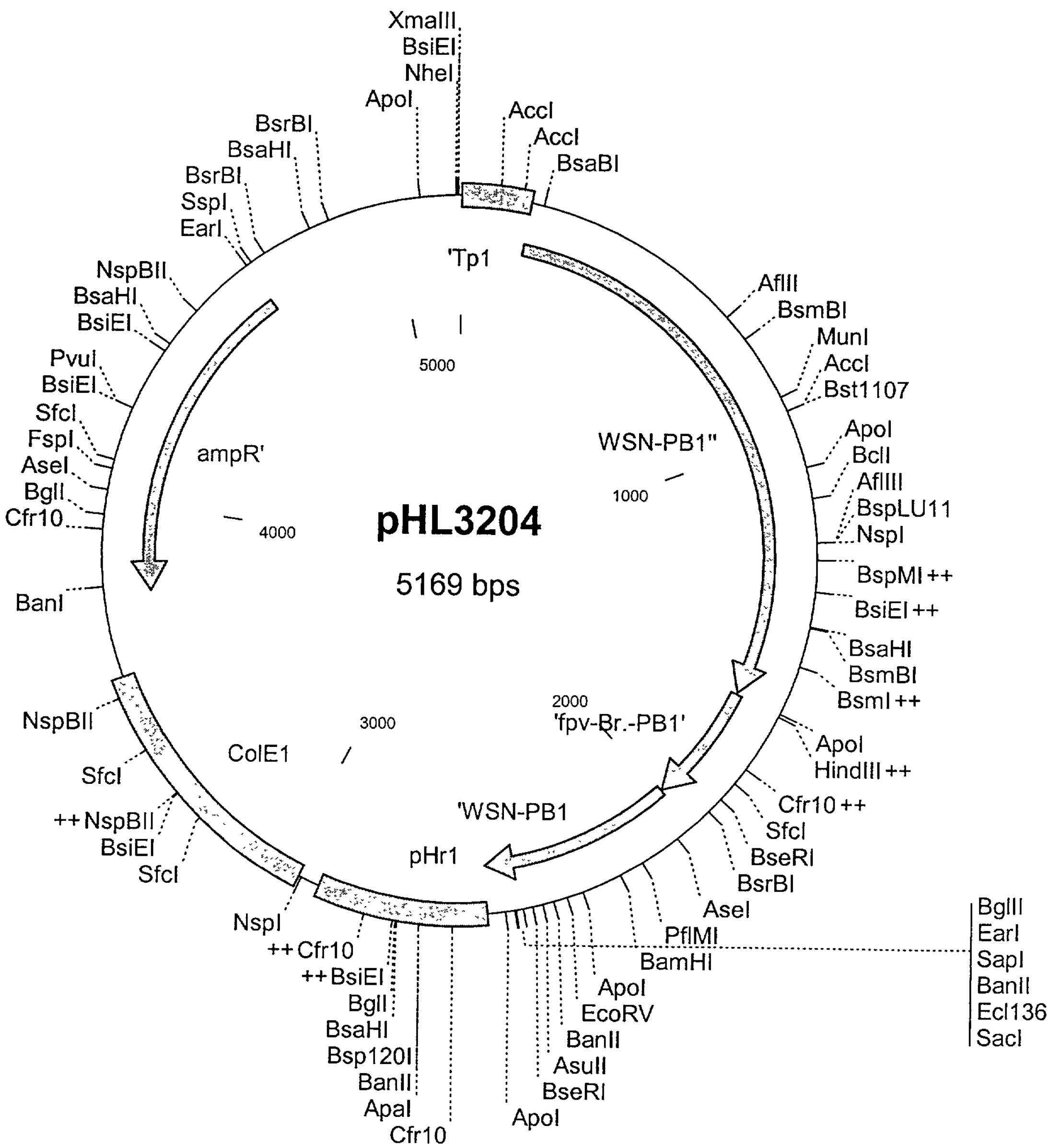
pHL3204
5169 bps
XmaIII
BsiEI
NheI
ApoI
AccI
AccI
BsaBI
BsrBI
BsaHI
BsrBI
SspI
EarI
NspBII
BsaHI
BsiEI
PvuI
BsiEI
SfcI
FspI
AseI
BglI
Cfr10
BanI
'Tp1
5000
ampR'
4000
WSN-PB1"
1000
AflII
BsmBI
MunI
AccI
Bst1107
ApoI
BclI
AflIII
BspLU11
NspI
BspMI ++
BsiEI ++
BsaHI
BsmBI
BsmI ++
2000
'fpv-Br.-PB1'
ApoI
HindIII ++
Cfr10 ++
SfcI
BseRI
BsrBI
AseI
PflMI
BamHI
ApoI
EcoRV
BanII
AsuII
BseRI
ApoI
BglII
EarI
SapI
BanII
Ecl136
SacI
3000
ColE1
'WSN-PB1
pHr1
NspBII
SfcI
++ NspBII
BsiEI
SfcI
NspI
++ Cfr10
++ BsiEI
BglI
BsaHI
Bsp120I
BanII
ApaI
Cfr10

Fig. 12

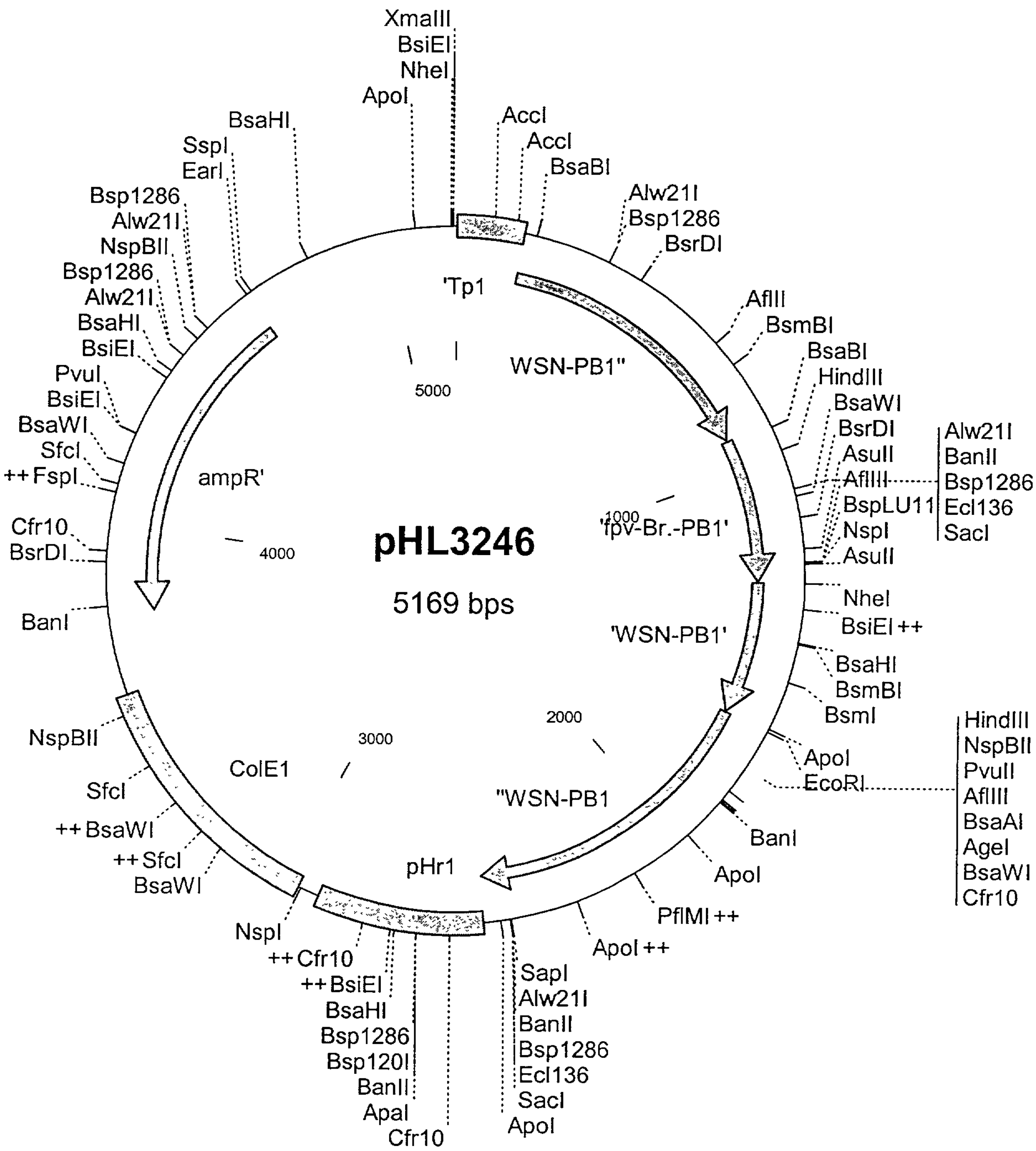
pHL3246
5169 bps
'Tp1
WSN-PB1"
'fpv-Br.-PB1'
'WSN-PB1'
"WSN-PB1
pHr1
ColE1
ampR'
1000
2000
3000
4000
5000
XmaIII
BsiEI
NheI
ApoI
AccI
AccI
BsaBI
Alw21I
Bsp1286
BsrDI
AflII
BsmBI
BsaBI
HindIII
BsaWI
BsrDI
AsuII
AflIII
BspLU11
NspI
AsuII
Alw21I
BanII
Bsp1286
Ecl136
SacI
NheI
BsiEI ++
BsaHI
BsmBI
BsmI
ApoI
EcoRI
HindIII
NspBII
PvuII
AflIII
BsaAI
AgeI
BsaWI
Cfr10
BanI
ApoI
PflMI ++
ApoI ++
SapI
Alw21I
BanII
Bsp1286
Ecl136
SacI
ApoI
Cfr10
ApaI
BanII
Bsp120I
Bsp1286
BsaHI
++ BsiEI
++ Cfr10
NspI
BsaWI
++ SfcI
++ BsaWI
SfcI
NspBII
BanI
BsrDI
Cfr10
++ FspI
SfcI
BsaWI
BsiEI
PvuI
BsiEI
BsaHI
Alw21I
Bsp1286
NspBII
Alw21I
Bsp1286
EarI
SspI
BsaHI

Fig. 13

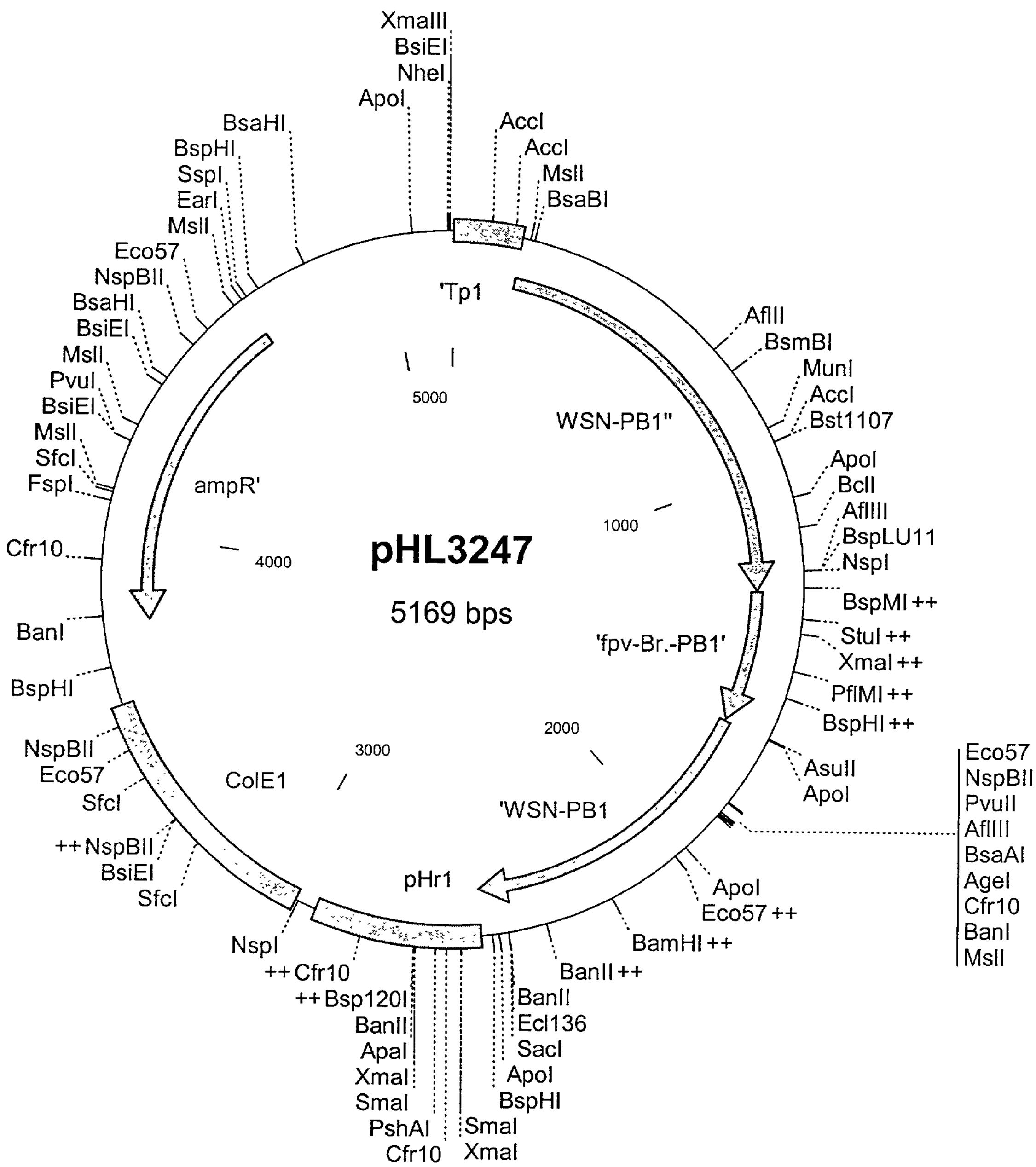
pHL3247
5169 bps
'Tp1
WSN-PB1"
'fpv-Br.-PB1'
'WSN-PB1
pHr1
ColE1
ampR'
5000
1000
2000
3000
4000
XmaIII
BsiEI
NheI
ApoI
AccI
AccI
MslI
BsaBI
BsaHI
BspHI
SspI
EarI
MslI
Eco57
NspBII
BsaHI
BsiEI
MslI
PvuI
BsiEI
MslI
SfcI
FspI
Cfr10
BanI
BspHI
NspBII
Eco57
SfcI
++NspBII
BsiEI
SfcI
NspI
++Cfr10
++Bsp120I
BanII
ApaI
XmaI
SmaI
PshAI
Cfr10
SmaI
XmaI
BanII
Ecl136
SacI
ApoI
BspHI
BanII ++
BamHI ++
ApoI
Eco57 ++
AsuII
ApoI
Eco57
NspBII
PvuII
AflIII
BsaAI
AgeI
Cfr10
BanI
MslI
BspHI ++
PflMI ++
XmaI ++
StuI ++
BspMI ++
NspI
BspLU11
AflIII
BclI
ApoI
Bst1107
AccI
MunI
BsmBI
AflII

Fig. 14

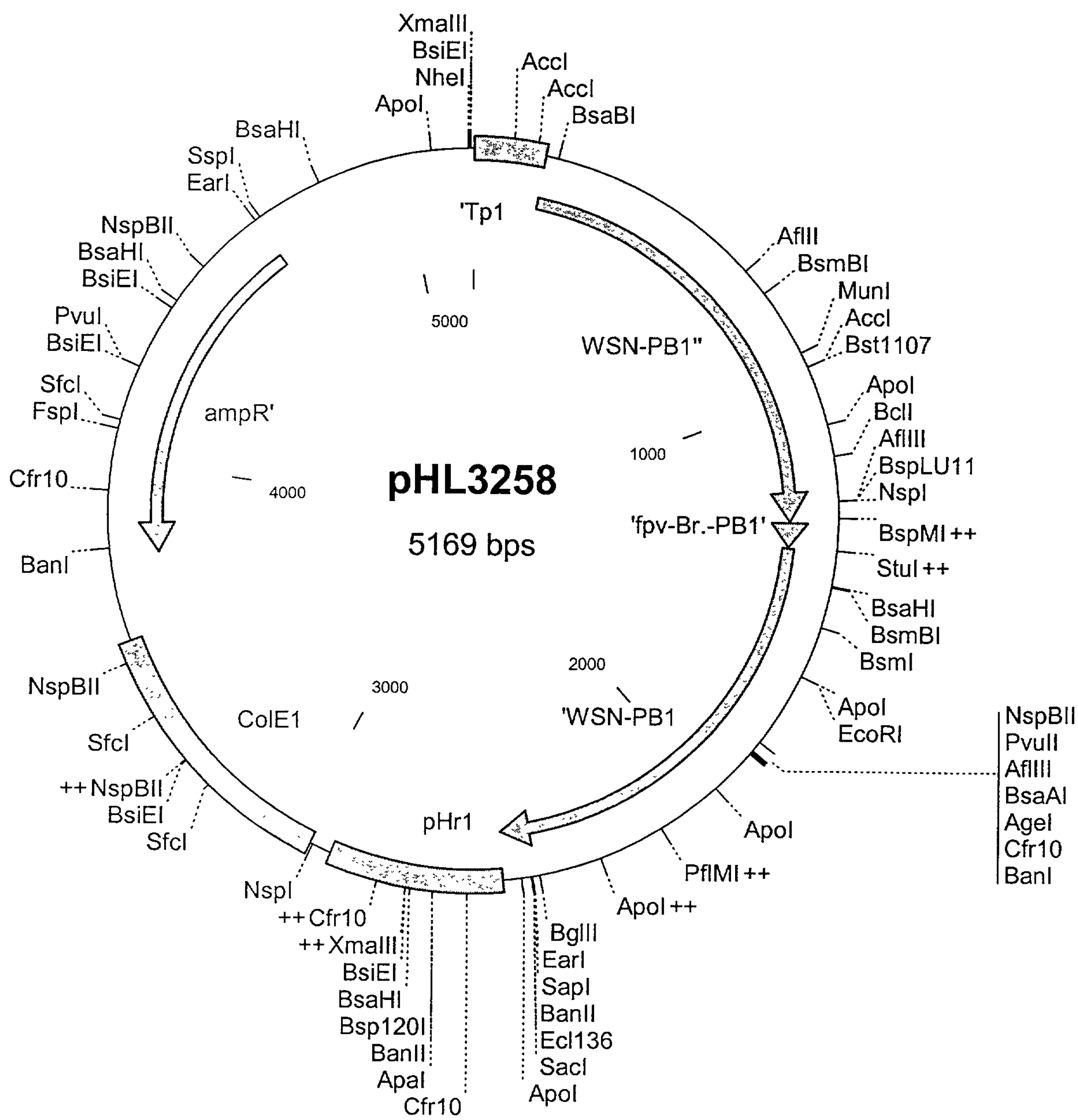
XmaIII
BsiEI
NheI
ApoI
AccI
AccI
BsaBI
BsaHI
SspI
EarI
NspBII
BsaHI
BsiEI
PvuI
BsiEI
SfcI
FspI
Cfr10
BanI
NspBII
SfcI
++NspBII
BsiEI
SfcI
NspI
++Cfr10
++XmaIII
BsiEI
BsaHI
Bsp120I
BanII
ApaI
Cfr10
BglII
EarI
SapI
BanII
Ecl136
SacI
ApoI
ApoI ++
PflMI ++
ApoI
NspBII
PvuII
AflIII
BsaAI
AgeI
Cfr10
BanI
ApoI
EcoRI
BsmI
BsmBI
BsaHI
StuI ++
BspMI ++
NspI
BspLU11
AflIII
BclI
ApoI
Bst1107
AccI
MunI
BsmBI
AflII
'Tp1
WSN-PB1"
'fpv-Br.-PB1'
'WSN-PB1
pHr1
ColE1
ampR'
pHL3258
5169 bps
5000
1000
2000
3000
4000

Fig. 15

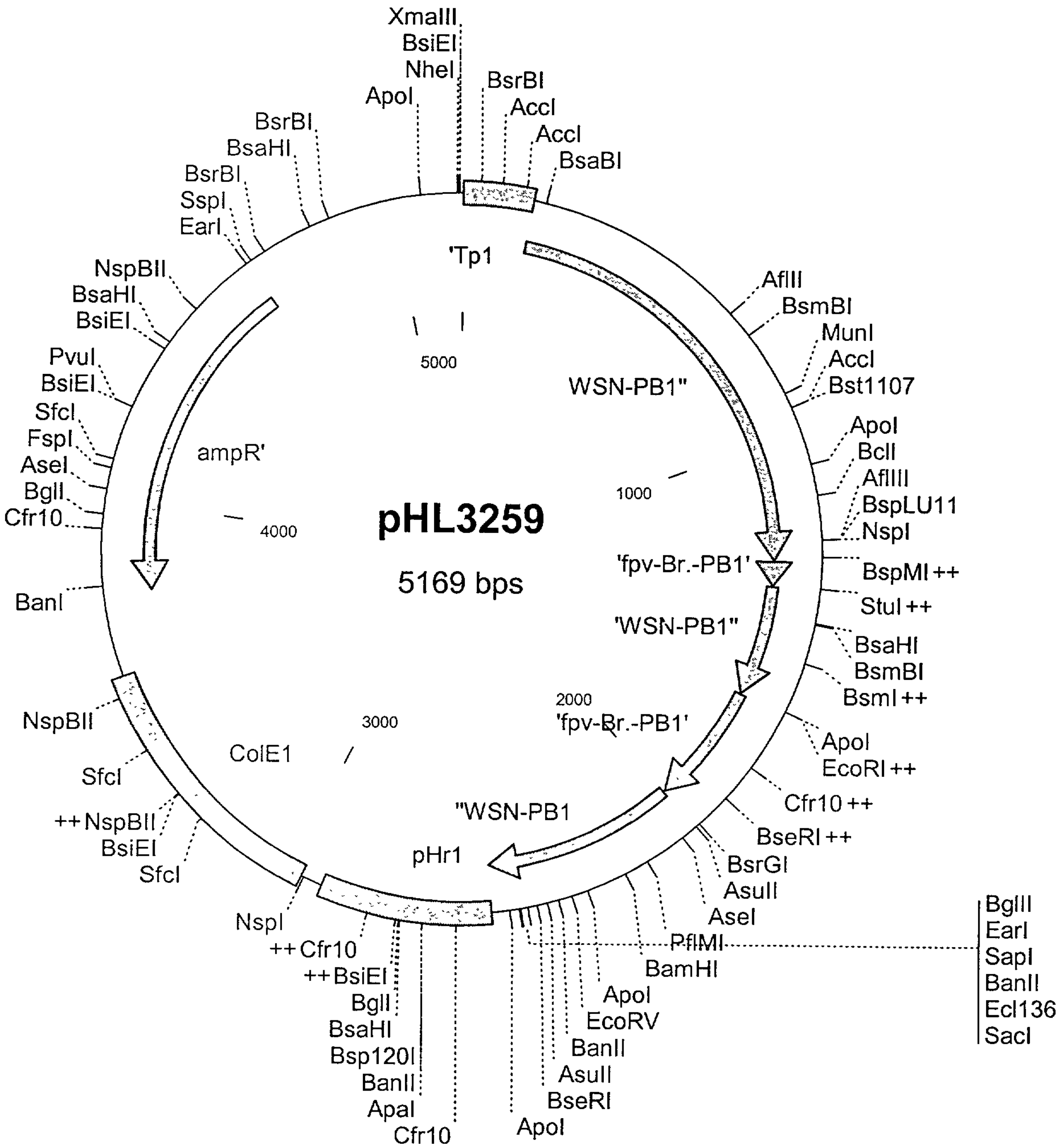
pHL3259
5169 bps
'Tp1
WSN-PB1"
'fpv-Br.-PB1'
'WSN-PB1"
'fpv-Br.-PB1'
"WSN-PB1
pHr1
ColE1
ampR'
1000
2000
3000
4000
5000
XmaIII
BsiEI
NheI
ApoI
BsrBI
AccI
AccI
BsaBI
BsrBI
BsaHI
BsrBI
SspI
EarI
NspBII
BsaHI
BsiEI
PvuI
BsiEI
SfcI
FspI
AseI
BglI
Cfr10
BanI
AflII
BsmBI
MunI
AccI
Bst1107
ApoI
BclI
AflIII
BspLU11
NspI
BspMI ++
StuI ++
BsaHI
BsmBI
BsmI ++
ApoI
EcoRI ++
Cfr10 ++
BseRI ++
BsrGI
AsuII
AseI
PflMI
BamHI
ApoI
EcoRV
BanII
AsuII
BseRI
ApoI
BglII
EarI
SapI
BanII
Ecl136
SacI
NspBII
SfcI
++NspBII
BsiEI
SfcI
NspI
++Cfr10
++BsiEI
BglI
BsaHI
Bsp120I
BanII
ApaI
Cfr10

Fig. 16

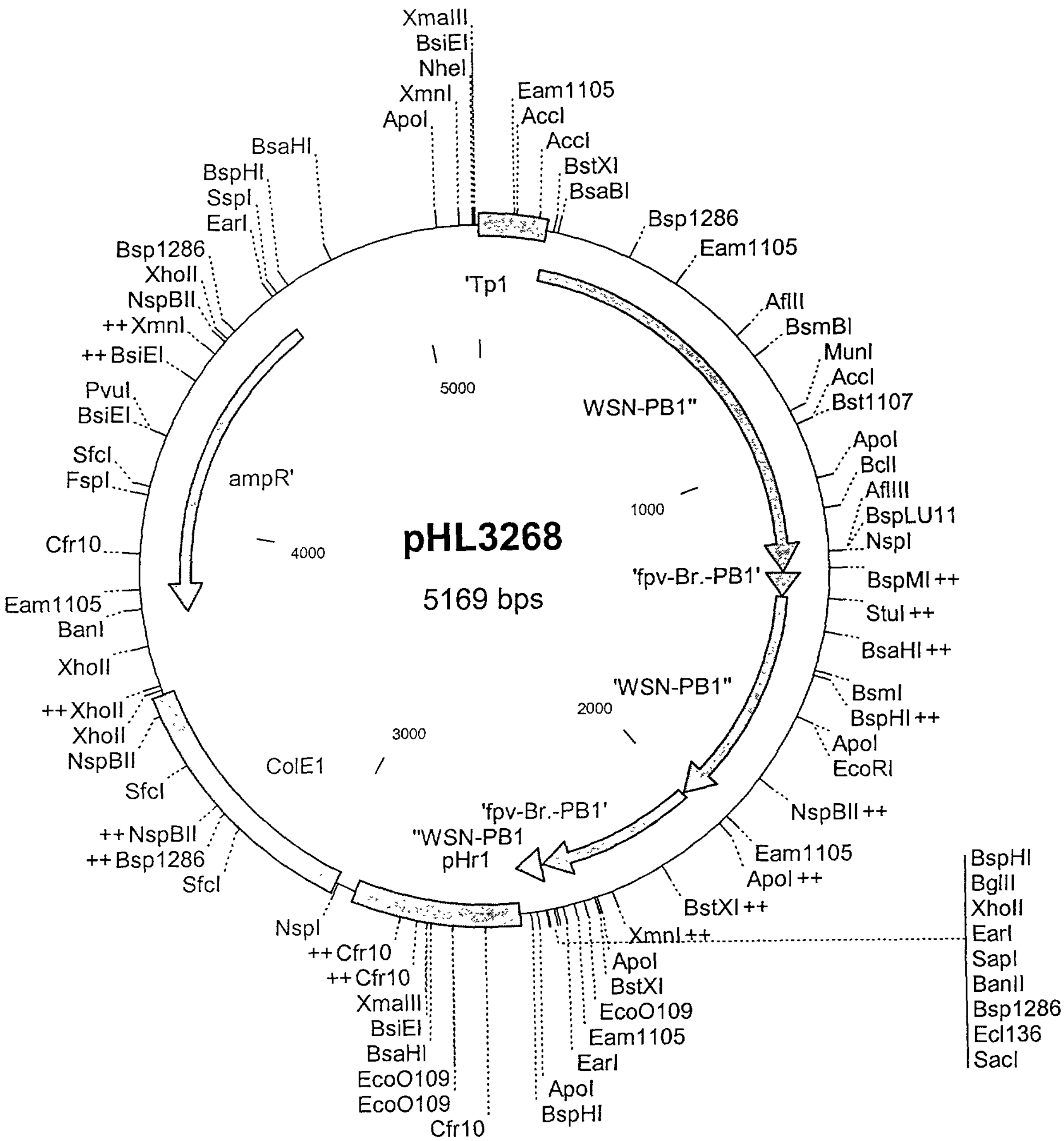
pHL3268
5169 bps
'Tp1
WSN-PB1"
'fpv-Br.-PB1'
'WSN-PB1"
'fpv-Br.-PB1'
"WSN-PB1
pHr1
ColE1
ampR'
1000
2000
3000
4000
5000
XmaIII
BsiEI
NheI
XmnI
ApoI
Eam1105
AccI
AccI
BstXI
BsaBI
Bsp1286
Eam1105
AflII
BsmBI
MunI
AccI
Bst1107
ApoI
BclI
AflIII
BspLU11
NspI
BspMI ++
StuI ++
BsaHI ++
BsmI
BspHI ++
ApoI
EcoRI
NspBII ++
Eam1105
ApoI ++
BstXI ++
XmnI ++
ApoI
BstXI
EcoO109
Eam1105
EarI
ApoI
BspHI
BspHI
BglII
XhoII
EarI
SapI
BanII
Bsp1286
Ecl136
SacI
Cfr10
EcoO109
EcoO109
BsaHI
BsiEI
XmaIII
++ Cfr10
++ Cfr10
NspI
SfcI
++ Bsp1286
++ NspBII
SfcI
NspBII
XhoII
++ XhoII
XhoII
BanI
Eam1105
Cfr10
FspI
SfcI
BsiEI
PvuI
++ BsiEI
++ XmnI
NspBII
XhoII
Bsp1286
EarI
SspI
BspHI
BsaHI

European Patent Office

# EUROPEAN SEARCH REPORT

Application Number
EP 01 10 3060

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.7) |
| A,D | FLICK R AND HOBOM G: "Interaction of influenza virus polymerase with viral RNA in the 'corkscrew' conformation" JOURNAL OF GENERAL VIROLOGY, SOCIETY FOR GENERAL MICROBIOLOGY, READING, GB, vol. 80, no. 10, October 1999 (1999-10), pages 2565-2572, XP002140120 ISSN: 0022-1317 * the whole document * | 1-12,27 | C12N7/00 C12N15/86 C07K14/11 A61K35/76 A61K39/145 |
| A,D | NEUMANN G AND HOBOM G: "Mutational analysis of influenza virus promoter elements in vivo" JOURNAL OF GENERAL VIROLOGY, SOCIETY FOR GENERAL MICROBIOLOGY, READING, GB, vol. 76, no. 7, 1995, pages 1709-1717, XP002140118 ISSN: 0022-1317 * the whole document * | 1-12,27 | |
| | | | TECHNICAL FIELDS SEARCHED (Int.Cl.7) |
| A | EP 1 035 209 A (ARTEMIS PHARMACEUTICALS GMBH) 13 September 2000 (2000-09-13) * the whole document * | 12 | C12N C07K A61K |

~~The present search report has been drawn up for all claims~~

| Place of search | Date of completion of the search | Examiner |
|---|---|---|
| MUNICH | 27 September 2001 | Nichogiannopoulou, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

Application Number
EP 01 10 3060

## CLAIMS INCURRING FEES

The present European patent application comprised at the time of filing more than ten claims.

- [X] Only part of the claims have been paid within the prescribed time limit. The present European search report has been drawn up for the first ten claims and for those claims for which claims fees have been paid, namely claim(s):

  11, 12, 27

- [ ] No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [ ] None of the further search fees have been paid within the fixed time limit. The present European search report has been drawn up for those parts of the European patent application which relate to the invention first mentioned in the claims, namely claims:

## ANNEX TO THE EUROPEAN SEARCH REPORT ON EUROPEAN PATENT APPLICATION NO. EP 01 10 3060

This annex lists the patent family members relating to the patent documents cited in the above-mentioned European search report.
The members are as contained in the European Patent Office EDP file on
The European Patent Office is in no way liable for these particulars which are merely given for the purpose of information.

27-09-2001

| Patent document cited in search report | Publication date | Patent family member(s) | Publication date |
|---|---|---|---|
| EP 1035209 A | 13-09-2000 | EP 1035209 A1 | 13-09-2000 |
| | | AU 3427100 A | 28-09-2000 |
| | | WO 0053786 A1 | 14-09-2000 |

EPO FORM P0459

For more details about this annex : see Official Journal of the European Patent Office, No. 12/82

SEQUENCE LISTING

<110> Methesys GmbH

<120> Influenza Viruses with Enhanced Transcriptional and Replicative Capacities

<130> 003143ep/JH/ml

<140>
<141>

<160> 47

<170> PatentIn Ver. 2.1

<210> 1
<211> 12
<212> RNA
<213> Influenza A virus

<400> 1
ccugcuuuug cu 12

<210> 2
<211> 12
<212> RNA
<213> Influenza B virus

<400> 2
nnygcuucug cu 12

<210> 3
<211> 12
<212> RNA
<213> Influenza C virus

<400> 3
ccugcuucug cu 12

<210> 4
<211> 12
<212> RNA
<213> Artificial Sequence

<220>
<223> Description of Artificial Sequence: Modified influenza A 3'-sequence

<400> 4
ccuguuuuua cu 12

<210> 5
<211> 12
<212> RNA
<213> Artificial Sequence

<220>

<223> Description of Artificial Sequence: Modified influenza A 3'-sequence

<400> 5
ccuguuucua cu 12

<210> 6
<211> 12
<212> RNA
<213> Artificial Sequence

<220>
<223> Description of Artificial Sequence: Modified influenza A 3'-sequence

<400> 6
ccugguucuc cu 12

<210> 7
<211> 13
<212> RNA
<213> Influenza A virus

<400> 7
aguagaaaca agg 13

<210> 8
<211> 13
<212> RNA
<213> Influenza B virus

<400> 8
aguagwaaca rnn 13

<210> 9
<211> 13
<212> RNA
<213> Influenza C virus

<400> 9
agcaguagca agr 13

<210> 10
<211> 13
<212> RNA
<213> Artificial Sequence

<220>
<223> Description of Artificial Sequence: Modified influenza A 5'-sequence

<400> 10
agaagaauca agg 13

<210> 11
<211> 21
<212> RNA
<213> Influenza A virus

<400> 11
aguagaaaca aggnnnuuuu u 21

```
<210> 12
<211> 21
<212> RNA
<213> Artificial Sequence

<220>
<223> Description of Artificial Sequence: Modified
      influenza A 5'-sequence (pHL1920)

<400> 12
agaagaauca aggnnnuuuu u                                                21


<210> 13
<211> 21
<212> RNA
<213> Influenza B virus

<400> 13
aguagwaaca rnnnnnuuuu u                                                21


<210> 14
<211> 19
<212> RNA
<213> Artificial Sequence

<220>
<223> Description of Artificial Sequence: Modified
      influenza C 5'-sequence

<400> 14
aguaguaaca agrguuuuu                                                   19


<210> 15
<211> 15
<212> RNA
<213> Artificial Sequence

<220>
<223> Description of Artificial Sequence:  Modified
      influenza A 3'-sequence (pHL1102)

<400> 15
nnnccuguuu uuacu                                                       15


<210> 16
<211> 15
<212> RNA
<213> Artificial Sequence

<220>
<223> Description of Artificial Sequence: Modified
      influenza A 3'-sequence (pHL1104 and pHL1920)

<400> 16
nnnccuguuu cuacu                                                       15


<210> 17
<211> 15
<212> RNA
<213> Artificial Sequence
```

<220>
<223> Description of Artificial Sequence: Modified influenza A 3'-sequence (pHL1948)

<400> 17
nnnccugguu cuccu 15

<210> 18
<211> 15
<212> RNA
<213> Artificial Sequence

<220>
<223> Description of Artificial Sequence: Modified influenza B 3' sequence

<400> 18
nnnnnyguuu cuacu 15

<210> 19
<211> 14
<212> RNA
<213> Artificial Sequence

<220>
<223> Description of Artificial Sequence: Modified influenza C 3'-sequence

<400> 19
ccccuguuuc uacu 14

<210> 20
<211> 10
<212> DNA
<213> Influenza A virus

<400> 20
aggtacgttc 10

<210> 21
<211> 32
<212> DNA
<213> Influenza A virus

<400> 21
gctgaaaaat gatcttcttg aaaattgcag gc 32

<210> 22
<211> 5169
<212> DNA
<213> Artificial Sequence

<220>
<223> Description of Artificial Sequence: FPV-Bratislava-PB1

<220>
<221> CDS
<222> (191)..(2461)

<400> 22
cccaaaaaaa aaaaaaaaaa aagagtccag agtggccccg ccgctccgcg ccgggggggg 60

```
gggggggggg ggacactttc ggacatctgg tcgacctcca gcatcggggg aaaaaaaaaa 120

acaaagtgtc gcccggagta ctggtcgacc tccgaagttg gggggagcg aaagcaggca 180

aaccatttga atg gat gtc aat ccg act tta ctg ttc ttg aaa gtt cct    229
           Met Asp Val Asn Pro Thr Leu Leu Phe Leu Lys Val Pro
            1               5                   10

gcg caa aat gca ata agt act acg ttc cct tac act gga gat cct cca    277
Ala Gln Asn Ala Ile Ser Thr Thr Phe Pro Tyr Thr Gly Asp Pro Pro
    15                  20                  25

tac agc cat gga aca ggg aca gga tac acc atg gac aca gtc aac agg    325
Tyr Ser His Gly Thr Gly Thr Gly Tyr Thr Met Asp Thr Val Asn Arg
30                  35                  40                  45

aca cac caa tat tcg gaa aag ggg aaa tgg aca aca aac act gag act    373
Thr His Gln Tyr Ser Glu Lys Gly Lys Trp Thr Thr Asn Thr Glu Thr
                50                  55                  60

gga gca ccc caa ctt aat cca att gat ggc cca ttg cct gag gac aat    421
Gly Ala Pro Gln Leu Asn Pro Ile Asp Gly Pro Leu Pro Glu Asp Asn
            65                  70                  75

gaa cca agt gga tat gca caa aca gac tgc gtc ctg gaa gca atg gct    469
Glu Pro Ser Gly Tyr Ala Gln Thr Asp Cys Val Leu Glu Ala Met Ala
        80                  85                  90

ttc ctt gaa gaa tcc cat cca gga atc ttt gaa aac tcg tgt ctt gag    517
Phe Leu Glu Glu Ser His Pro Gly Ile Phe Glu Asn Ser Cys Leu Glu
    95                  100                 105

acg atg gaa gtt gtt caa caa aca aga gtg gac aaa ctg acc caa ggc    565
Thr Met Glu Val Val Gln Gln Thr Arg Val Asp Lys Leu Thr Gln Gly
110                 115                 120                 125

cgt cag act tat gat tgg act ttg aat aga aac cag cct gct gca act    613
Arg Gln Thr Tyr Asp Trp Thr Leu Asn Arg Asn Gln Pro Ala Ala Thr
                130                 135                 140

gca tta gca aac act ata gag gtc ttt aga tcg aat ggt cta aca gct    661
Ala Leu Ala Asn Thr Ile Glu Val Phe Arg Ser Asn Gly Leu Thr Ala
            145                 150                 155

aat gaa tca ggg agg ctc ata gat ttc ctc aag gat gtg atg gaa tca    709
Asn Glu Ser Gly Arg Leu Ile Asp Phe Leu Lys Asp Val Met Glu Ser
        160                 165                 170

atg gat aag gag gaa atg gag ata aca aca cat ttc caa cga aag aga    757
Met Asp Lys Glu Glu Met Glu Ile Thr Thr His Phe Gln Arg Lys Arg
    175                 180                 185

aga gta aga gac aac atg acc aag aaa atg gtc aca caa aga aca ata    805
Arg Val Arg Asp Asn Met Thr Lys Lys Met Val Thr Gln Arg Thr Ile
190                 195                 200                 205

ggg aag aaa aag cag aga ctt aac aaa agg agc tac cta ata agg gct    853
Gly Lys Lys Lys Gln Arg Leu Asn Lys Arg Ser Tyr Leu Ile Arg Ala
                210                 215                 220

cta aca ttg aac aca atg acg aaa gat gca gaa aga ggt aaa ctg aag    901
Leu Thr Leu Asn Thr Met Thr Lys Asp Ala Glu Arg Gly Lys Leu Lys
            225                 230                 235
```

```
aga aga gca att gca aca cca ggg atg cag atc aga ggg ttt gtg tac    949
Arg Arg Ala Ile Ala Thr Pro Gly Met Gln Ile Arg Gly Phe Val Tyr
        240                 245                 250

ttt gtt gag aca ctg gcg aga agc att tgc gag aag ctt gaa cag tct    997
Phe Val Glu Thr Leu Ala Arg Ser Ile Cys Glu Lys Leu Glu Gln Ser
    255                 260                 265

ggg cta cca gtt gga ggg aat gag aag aaa gct aaa ttg gca aat gtc    1045
Gly Leu Pro Val Gly Gly Asn Glu Lys Lys Ala Lys Leu Ala Asn Val
270                 275                 280                 285

gtg agg aag atg atg acg aac tca caa gac act gag ctc tct ttc aca    1093
Val Arg Lys Met Met Thr Asn Ser Gln Asp Thr Glu Leu Ser Phe Thr
                290                 295                 300

atc acc gga gac aat acc aaa tgg aat gag aac caa aac ccc cga atg    1141
Ile Thr Gly Asp Asn Thr Lys Trp Asn Glu Asn Gln Asn Pro Arg Met
            305                 310                 315

ttc ctg gca atg ata aca tac atc aca aga aac caa cct gag tgg ttt    1189
Phe Leu Ala Met Ile Thr Tyr Ile Thr Arg Asn Gln Pro Glu Trp Phe
        320                 325                 330

aga aat gtc ttg agc atc gcg ccg ata atg ttt tcg aac aaa atg gcg    1237
Arg Asn Val Leu Ser Ile Ala Pro Ile Met Phe Ser Asn Lys Met Ala
    335                 340                 345

agg cta ggg aaa ggg tac atg ttc gaa agc aaa agc atg aag ctc cga    1285
Arg Leu Gly Lys Gly Tyr Met Phe Glu Ser Lys Ser Met Lys Leu Arg
350                 355                 360                 365

aca caa ata cca gca gaa atg cta gca agt att gat cta aaa tat ttc    1333
Thr Gln Ile Pro Ala Glu Met Leu Ala Ser Ile Asp Leu Lys Tyr Phe
                370                 375                 380

aat gaa cca aca agg aag aaa atc gag aaa ata agg cct ctc ata ata    1381
Asn Glu Pro Thr Arg Lys Lys Ile Glu Lys Ile Arg Pro Leu Ile Ile
            385                 390                 395

gac ggc aca gcc tca tta agc ccg gga atg atg atg ggt atg ttc aac    1429
Asp Gly Thr Ala Ser Leu Ser Pro Gly Met Met Met Gly Met Phe Asn
        400                 405                 410

atg ctg agt aca gtg ttg gga gtc tca atc ctg aat ctt ggg caa aag    1477
Met Leu Ser Thr Val Leu Gly Val Ser Ile Leu Asn Leu Gly Gln Lys
    415                 420                 425

aga tac acc aaa acc aca tac tgg tgg gat gga ctt cag tcc tct gat    1525
Arg Tyr Thr Lys Thr Thr Tyr Trp Trp Asp Gly Leu Gln Ser Ser Asp
430                 435                 440                 445

gat ttt gct ctc atc gtg aat gca cca aat cat gag gga ata caa gcg    1573
Asp Phe Ala Leu Ile Val Asn Ala Pro Asn His Glu Gly Ile Gln Ala
                450                 455                 460

gga gtg gat aga ttc tac aga acc tgc aag cta gtt ggg atc aat atg    1621
Gly Val Asp Arg Phe Tyr Arg Thr Cys Lys Leu Val Gly Ile Asn Met
            465                 470                 475

agc aag aaa aag tcc tat ata aat agg aca gga aca ttc gaa ttc aca    1669
Ser Lys Lys Lys Ser Tyr Ile Asn Arg Thr Gly Thr Phe Glu Phe Thr
        480                 485                 490

agc ttt ttc tac cgc tat gga ttt gta gcc aat ttt agt atg gag ttg    1717
Ser Phe Phe Tyr Arg Tyr Gly Phe Val Ala Asn Phe Ser Met Glu Leu
    495                 500                 505
```

```
ccc agc ttt gga gta tca gga att aat gaa tcg gct gat atg agc att   1765
Pro Ser Phe Gly Val Ser Gly Ile Asn Glu Ser Ala Asp Met Ser Ile
510                 515                 520                 525

gga gta aca gtg ata aag aat aac atg ata aac aat gat ctt gga ccg   1813
Gly Val Thr Val Ile Lys Asn Asn Met Ile Asn Asn Asp Leu Gly Pro
                530                 535                 540

gca aca gcc caa atg gct ctc caa tta ttc atc aag gac tac aga tat   1861
Ala Thr Ala Gln Met Ala Leu Gln Leu Phe Ile Lys Asp Tyr Arg Tyr
            545                 550                 555

aca tac agg tgt cac agg gga gac aca caa atc caa acg agg agg tca   1909
Thr Tyr Arg Cys His Arg Gly Asp Thr Gln Ile Gln Thr Arg Arg Ser
        560                 565                 570

ttc gag cta aag aag ctg tgg gag cag acc cgc tca aag gca gga ctg   1957
Phe Glu Leu Lys Lys Leu Trp Glu Gln Thr Arg Ser Lys Ala Gly Leu
    575                 580                 585

ttg gtt tca gat ggc gga cca aac ctg tac aac att cgg aat ctc cac   2005
Leu Val Ser Asp Gly Gly Pro Asn Leu Tyr Asn Ile Arg Asn Leu His
590                 595                 600                 605

atc ccg gaa gtt tgc ctg aaa tgg gaa cta atg gat gaa gac tat cag   2053
Ile Pro Glu Val Cys Leu Lys Trp Glu Leu Met Asp Glu Asp Tyr Gln
                610                 615                 620

gga aga ctt tgt aat ccc atg aac ccg ttt gtc agt cat aag gaa att   2101
Gly Arg Leu Cys Asn Pro Met Asn Pro Phe Val Ser His Lys Glu Ile
            625                 630                 635

gaa tct gta aac aat gct gcg gta atg cca gcc cat ggt cca gcc aaa   2149
Glu Ser Val Asn Asn Ala Ala Val Met Pro Ala His Gly Pro Ala Lys
        640                 645                 650

agc atg gaa tat gat gct gtg gca act aca cac tct tgg atc cct aag   2197
Ser Met Glu Tyr Asp Ala Val Ala Thr Thr His Ser Trp Ile Pro Lys
    655                 660                 665

aga aac cgt tcc att ctc aat acg agt caa agg gga atc ctt gag gat   2245
Arg Asn Arg Ser Ile Leu Asn Thr Ser Gln Arg Gly Ile Leu Glu Asp
670                 675                 680                 685

gaa caa atg tac cag aag tgt tgc aac cta ttc gag aaa ttc ttc cct   2293
Glu Gln Met Tyr Gln Lys Cys Cys Asn Leu Phe Glu Lys Phe Phe Pro
                690                 695                 700

agc agc tca tac aga aga cca gtt gga att tcc agt atg gtg gag gcc   2341
Ser Ser Ser Tyr Arg Arg Pro Val Gly Ile Ser Ser Met Val Glu Ala
            705                 710                 715

atg gtg tct agg gcc cgg att gat gca cga att gac ttc gag tct gga   2389
Met Val Ser Arg Ala Arg Ile Asp Ala Arg Ile Asp Phe Glu Ser Gly
        720                 725                 730

agg att aag aag gaa gag ttt gct gag atc atg aag atc tgt tcc acc   2437
Arg Ile Lys Lys Glu Glu Phe Ala Glu Ile Met Lys Ile Cys Ser Thr
    735                 740                 745

att gaa gag ctc aga cgg cag aaa tagtgaattt agcttgtcct tcatgaaaaa  2491
Ile Glu Glu Leu Arg Arg Gln Lys
750                 755

atgccttgtt tctactaata acccggcggc ccaaaatgcc gactcggagc gaaagatata 2551
```

cctccccgg ggccgggagg tcgcgtcacc gaccacgccg ccggcccagg cgacgcgcga 2611
cacggacacc tgtccccaaa aacgccacca tcgcagccac acacggagcg cccggggccc 2671
tctggtcaac cccaggacac acgcgggagc agcgccgggc cggggacgcc ctcccggccg 2731
cccgtgccac acgcaggggg ccggcccgtg tctccagagc gggagccgga agcattttcg 2791
gccggcccct cctacgaccg ggacacacga gggaccgaag gccggccagg cgcgacctct 2851
cgggccgcac gcgcgctcag ggagcgctct ccgactccgc acggggactc gccagaaagg 2911
atcgtgacct gcattaatga atcaggggat aacgcaggaa agaacatgtg agcaaaaggc 2971
cagcaaaagg ccaggaaccg taaaaaggcc gcgttgctgg cgtttttcca taggctccgc 3031
cccccctgacg agcatcacaa aaatcgacgc tcaagtcaga ggtggcgaaa cccgacagga 3091
ctataaagat accaggcgtt tccccctgga agctccctcg tgcgctctcc tgttccgacc 3151
ctgccgctta ccggatacct gtccgccttt ctcccttcgg gaagcgtggc gctttctcat 3211
agctcacgct gtaggtatct cagttcggtg taggtcgttc gctccaagct gggctgtgtg 3271
cacgaacccc ccgttcagcc cgaccgctgc gccttatccg gtaactatcg tcttgagtcc 3331
aacccggtaa gacacgactt atcgccactg gcagcagcca ctggtaacag gattagcaga 3391
gcgaggtatg taggcggtgc tacagagttc ttgaagtggt ggcctaacta cggctacact 3451
agaaggacag tatttggtat ctgcgctctg ctgaagccag ttaccttcgg aaaaagagtt 3511
ggtagctctt gatccggcaa acaaaccacc gctggtagcg gtggtttttt tgtttgcaag 3571
cagcagatta cgcgcagaaa aaaaggatct caagaagatc ctttgatctt ttctacgggg 3631
tctgacgctc agtggaacga aaactcacgt taagggattt tggtcatgag attatcaaaa 3691
aggatcttca cctagatcct tttaaattaa aaatgaagtt ttaaatcaat ctaaagtata 3751
tatgagtaaa cttggtctga cagttaccaa tgcttaatca gtgaggcacc tatctcagcg 3811
atctgtctat ttcgttcatc catagttgcc tgactccccg tcgtgtagat aactacgata 3871
cgggagggct taccatctgg ccccagtgct gcaatgatac cgcgagaccc acgctcaccg 3931
gctccagatt tatcagcaat aaaccagcca gccggaaggg ccgagcgcag aagtggtcct 3991
gcaactttat ccgcctccat ccagtctatt aattgttgcc gggaagctag agtaagtagt 4051
tcgccagtta atagtttgcg caacgttgtt gccattgcta caggcatcgt ggtgtcacgc 4111
tcgtcgtttg gtatggcttc attcagctcc ggttcccaac gatcaaggcg agttacatga 4171
tcccccatgt tgtgcaaaaa agcggttagc tccttcggtc ctccgatcgt tgtcagaagt 4231
aagttggccg cagtgttatc actcatggtt atggcagcac tgcataattc tcttactgtc 4291
atgccatccg taagatgctt ttctgtgact ggtgagtact caaccaagtc attctgagaa 4351
tagtgtatgc ggcgaccgag ttgctcttgc ccggcgtcaa cacgggataa taccgcgcca 4411
catagcagaa ctttaaaagt gctcatcatt ggaaaacgtt cttcggggcg aaaactctca 4471
aggatcttac cgctgttgag atccagttcg atgtaaccca ctcgtgcacc caactgatct 4531
tcagcatctt ttactttcac cagcgtttct gggtgagcaa aaacaggaag gcaaaatgcc 4591

gcaaaaaagg gaataagggc gacacggaaa tgttgaatac tcatactctt cctttttcaa 4651

tattattgaa gcatttatca gggttattgt ctcatgagcg gatacatatt tgaatgtatt 4711

tagaaaaata aacaaaagag tttgtagaaa cgcaaaaagg ccatccgtca ggatggcctt 4771

ctgcttaatt tgatgcctgg cagtttatgg cgggcgtcct gcccgccacc ctccgggccg 4831

ttgcttcgca acgttcaaat ccgctcccgg cggatttgtc ctactcagga gagcgttcac 4891

cgacaaacaa cagataaaac gaaaggccca gtctttcgac tgagcctttc gttttatttg 4951

atgcctggca gttccctact ctcgcatggg gagaccccac actaccatcg gcgctacggc 5011

gtttcacttc tgagttcggc atggggtcag gtgggaccac cgcgctactg ccgccaggca 5071

aattctgttt tatcagaccg cttctgcgtt ctgatttaat ctgtatcagg ctgaaaatct 5131

tctctcatcc gccaaaacag ccaagctagc ggccgatc 5169

<210> 23
<211> 757
<212> PRT
<213> Artificial Sequence
<223> Description of Artificial Sequence: FPV-Bratislava-PB1

<400> 23
Met Asp Val Asn Pro Thr Leu Leu Phe Leu Lys Val Pro Ala Gln Asn
1 5 10 15

Ala Ile Ser Thr Thr Phe Pro Tyr Thr Gly Asp Pro Pro Tyr Ser His
20 25 30

Gly Thr Gly Thr Gly Tyr Thr Met Asp Thr Val Asn Arg Thr His Gln
35 40 45

Tyr Ser Glu Lys Gly Lys Trp Thr Thr Asn Thr Glu Thr Gly Ala Pro
50 55 60

Gln Leu Asn Pro Ile Asp Gly Pro Leu Pro Glu Asp Asn Glu Pro Ser
65 70 75 80

Gly Tyr Ala Gln Thr Asp Cys Val Leu Glu Ala Met Ala Phe Leu Glu
85 90 95

Glu Ser His Pro Gly Ile Phe Glu Asn Ser Cys Leu Glu Thr Met Glu
100 105 110

Val Val Gln Gln Thr Arg Val Asp Lys Leu Thr Gln Gly Arg Gln Thr
115 120 125

Tyr Asp Trp Thr Leu Asn Arg Asn Gln Pro Ala Ala Thr Ala Leu Ala
130 135 140

Asn Thr Ile Glu Val Phe Arg Ser Asn Gly Leu Thr Ala Asn Glu Ser
145 150 155 160

Gly Arg Leu Ile Asp Phe Leu Lys Asp Val Met Glu Ser Met Asp Lys
165 170 175

Glu Glu Met Glu Ile Thr Thr His Phe Gln Arg Lys Arg Arg Val Arg
180 185 190

Asp Asn Met Thr Lys Lys Met Val Thr Gln Arg Thr Ile Gly Lys Lys
195 200 205

```
Lys Gln Arg Leu Asn Lys Arg Ser Tyr Leu Ile Arg Ala Leu Thr Leu
    210                 215                 220

Asn Thr Met Thr Lys Asp Ala Glu Arg Gly Lys Leu Lys Arg Arg Ala
225                 230                 235                 240

Ile Ala Thr Pro Gly Met Gln Ile Arg Gly Phe Val Tyr Phe Val Glu
                245                 250                 255

Thr Leu Ala Arg Ser Ile Cys Glu Lys Leu Glu Gln Ser Gly Leu Pro
            260                 265                 270

Val Gly Gly Asn Glu Lys Lys Ala Lys Leu Ala Asn Val Val Arg Lys
        275                 280                 285

Met Met Thr Asn Ser Gln Asp Thr Glu Leu Ser Phe Thr Ile Thr Gly
    290                 295                 300

Asp Asn Thr Lys Trp Asn Glu Asn Gln Asn Pro Arg Met Phe Leu Ala
305                 310                 315                 320

Met Ile Thr Tyr Ile Thr Arg Asn Gln Pro Glu Trp Phe Arg Asn Val
                325                 330                 335

Leu Ser Ile Ala Pro Ile Met Phe Ser Asn Lys Met Ala Arg Leu Gly
            340                 345                 350

Lys Gly Tyr Met Phe Glu Ser Lys Ser Met Lys Leu Arg Thr Gln Ile
        355                 360                 365

Pro Ala Glu Met Leu Ala Ser Ile Asp Leu Lys Tyr Phe Asn Glu Pro
    370                 375                 380

Thr Arg Lys Lys Ile Glu Lys Ile Arg Pro Leu Ile Ile Asp Gly Thr
385                 390                 395                 400

Ala Ser Leu Ser Pro Gly Met Met Met Gly Met Phe Asn Met Leu Ser
                405                 410                 415

Thr Val Leu Gly Val Ser Ile Leu Asn Leu Gly Gln Lys Arg Tyr Thr
            420                 425                 430

Lys Thr Thr Tyr Trp Trp Asp Gly Leu Gln Ser Ser Asp Asp Phe Ala
        435                 440                 445

Leu Ile Val Asn Ala Pro Asn His Glu Gly Ile Gln Ala Gly Val Asp
    450                 455                 460

Arg Phe Tyr Arg Thr Cys Lys Leu Val Gly Ile Asn Met Ser Lys Lys
465                 470                 475                 480

Lys Ser Tyr Ile Asn Arg Thr Gly Thr Phe Glu Phe Thr Ser Phe Phe
                485                 490                 495

Tyr Arg Tyr Gly Phe Val Ala Asn Phe Ser Met Glu Leu Pro Ser Phe
            500                 505                 510

Gly Val Ser Gly Ile Asn Glu Ser Ala Asp Met Ser Ile Gly Val Thr
        515                 520                 525

Val Ile Lys Asn Asn Met Ile Asn Asn Asp Leu Gly Pro Ala Thr Ala
    530                 535                 540

Gln Met Ala Leu Gln Leu Phe Ile Lys Asp Tyr Arg Tyr Thr Tyr Arg
545                 550                 555                 560
```

```
Cys His Arg Gly Asp Thr Gln Ile Gln Thr Arg Arg Ser Phe Glu Leu
                565                 570                 575

Lys Lys Leu Trp Glu Gln Thr Arg Ser Lys Ala Gly Leu Leu Val Ser
            580                 585                 590

Asp Gly Gly Pro Asn Leu Tyr Asn Ile Arg Asn Leu His Ile Pro Glu
        595                 600                 605

Val Cys Leu Lys Trp Glu Leu Met Asp Glu Asp Tyr Gln Gly Arg Leu
    610                 615                 620

Cys Asn Pro Met Asn Pro Phe Val Ser His Lys Glu Ile Glu Ser Val
625                 630                 635                 640

Asn Asn Ala Ala Val Met Pro Ala His Gly Pro Ala Lys Ser Met Glu
                645                 650                 655

Tyr Asp Ala Val Ala Thr Thr His Ser Trp Ile Pro Lys Arg Asn Arg
            660                 665                 670

Ser Ile Leu Asn Thr Ser Gln Arg Gly Ile Leu Glu Asp Glu Gln Met
        675                 680                 685

Tyr Gln Lys Cys Cys Asn Leu Phe Glu Lys Phe Phe Pro Ser Ser Ser
    690                 695                 700

Tyr Arg Arg Pro Val Gly Ile Ser Ser Met Val Glu Ala Met Val Ser
705                 710                 715                 720

Arg Ala Arg Ile Asp Ala Arg Ile Asp Phe Glu Ser Gly Arg Ile Lys
                725                 730                 735

Lys Glu Glu Phe Ala Glu Ile Met Lys Ile Cys Ser Thr Ile Glu Glu
            740                 745                 750

Leu Arg Arg Gln Lys
        755
```

<210> 24
<211> 5169
<212> DNA
<213> Artificial Sequence

<220>
<223> Description of Artificial Sequence: WSN-PB1

<220>
<221> CDS
<222> (191)..(2461)

<400> 24

```
cccaaaaaaa aaaaaaaaaa aagagtccag agtggccccg ccgctccgcg ccgggggggg 60

gggggggggg ggacactttc ggacatctgg tcgacctcca gcatcggggg aaaaaaaaaa 120

acaaagtgtc gcccggagta ctggtcgacc tccgaagttg ggggggagcg aaagcaggca 180

aaccatttga atg gat gtc aat ccg act tta ctt ttc tta aaa gtg cca    229
           Met Asp Val Asn Pro Thr Leu Leu Phe Leu Lys Val Pro
            1               5                  10

gca caa aat gct ata agc aca act ttc cct tat act gga gac cct cct    277
Ala Gln Asn Ala Ile Ser Thr Thr Phe Pro Tyr Thr Gly Asp Pro Pro
    15                  20                  25
```

```
tac agc cat ggg aca gga aca gga tac acc atg gat act gtc aac agg    325
Tyr Ser His Gly Thr Gly Thr Gly Tyr Thr Met Asp Thr Val Asn Arg
 30                 35                      40                  45

aca cat cag tac tca gaa agg gga aga tgg aca aca aac acc gaa act    373
Thr His Gln Tyr Ser Glu Arg Gly Arg Trp Thr Thr Asn Thr Glu Thr
                50                  55                  60

gga gca ccg caa ctc aac ccg att gat ggg cca ctg cca gaa gac aat    421
Gly Ala Pro Gln Leu Asn Pro Ile Asp Gly Pro Leu Pro Glu Asp Asn
            65                  70                  75

gaa cca agt ggt tat gcc caa aca gat tgt gta ttg gaa gca atg gcc    469
Glu Pro Ser Gly Tyr Ala Gln Thr Asp Cys Val Leu Glu Ala Met Ala
        80                  85                  90

ttc ctt gag gaa tcc cat cct ggt atc ttt gag acc tcg tgt ctt gaa    517
Phe Leu Glu Glu Ser His Pro Gly Ile Phe Glu Thr Ser Cys Leu Glu
    95                  100                 105

acg atg gag gtt gtt cag caa aca cga gtg gac aag ctg aca caa ggc    565
Thr Met Glu Val Val Gln Gln Thr Arg Val Asp Lys Leu Thr Gln Gly
110                 115                 120                 125

cga cag acc tat gac tgg act cta aat agg aac cag cct gct gca aca    613
Arg Gln Thr Tyr Asp Trp Thr Leu Asn Arg Asn Gln Pro Ala Ala Thr
                130                 135                 140

gca ttg gcc aac aca ata gaa gtg ttc aga tca aat ggc ctc acg gcc    661
Ala Leu Ala Asn Thr Ile Glu Val Phe Arg Ser Asn Gly Leu Thr Ala
            145                 150                 155

aat gaa tct gga agg ctc ata gac ttc ctt aag gat gta atg gag tca    709
Asn Glu Ser Gly Arg Leu Ile Asp Phe Leu Lys Asp Val Met Glu Ser
        160                 165                 170

atg aac aaa gaa gaa atg gag atc aca act cat ttt cag aga aag aga    757
Met Asn Lys Glu Glu Met Glu Ile Thr Thr His Phe Gln Arg Lys Arg
    175                 180                 185

cga gtg aga gac aat atg act aag aaa atg gtg aca cag aga aca ata    805
Arg Val Arg Asp Asn Met Thr Lys Lys Met Val Thr Gln Arg Thr Ile
190                 195                 200                 205

ggt aaa agg aag cag aga ttg aac aaa agg agt tat cta att agg gca    853
Gly Lys Arg Lys Gln Arg Leu Asn Lys Arg Ser Tyr Leu Ile Arg Ala
                210                 215                 220

tta acc ctg aac aca atg acc aaa gat gct gag aga ggg aag cta aaa    901
Leu Thr Leu Asn Thr Met Thr Lys Asp Ala Glu Arg Gly Lys Leu Lys
            225                 230                 235

cgg aga gca att gca acc cca ggg atg caa ata agg ggg ttt gta tac    949
Arg Arg Ala Ile Ala Thr Pro Gly Met Gln Ile Arg Gly Phe Val Tyr
        240                 245                 250

ttt gtt gag aca cta gca agg agt ata tgt gag aaa ctt gaa caa tca    997
Phe Val Glu Thr Leu Ala Arg Ser Ile Cys Glu Lys Leu Glu Gln Ser
    255                 260                 265

gga ttg cca gtt gga ggc aat gag aag aaa gca aag ttg gca aat gtt    1045
Gly Leu Pro Val Gly Gly Asn Glu Lys Lys Ala Lys Leu Ala Asn Val
270                 275                 280                 285
```

```
gta agg aag atg atg acc aat tct cag gac act gaa att tct ttc acc     1093
Val Arg Lys Met Met Thr Asn Ser Gln Asp Thr Glu Ile Ser Phe Thr
                290                 295                 300

atc act gga gat aac acc aaa tgg aac gaa aat cag aac cct cgg atg     1141
Ile Thr Gly Asp Asn Thr Lys Trp Asn Glu Asn Gln Asn Pro Arg Met
            305                 310                 315

ttt ttg gcc atg atc aca tat ata acc aga aat cag ccc gaa tgg ttc     1189
Phe Leu Ala Met Ile Thr Tyr Ile Thr Arg Asn Gln Pro Glu Trp Phe
        320                 325                 330

aga aat gtt cta agt att gct cca ata atg ttc tca aac aaa atg gcg     1237
Arg Asn Val Leu Ser Ile Ala Pro Ile Met Phe Ser Asn Lys Met Ala
    335                 340                 345

aga ctg gga aag ggg tac atg ttt gag agc aag agt atg aaa att aga     1285
Arg Leu Gly Lys Gly Tyr Met Phe Glu Ser Lys Ser Met Lys Ile Arg
350                 355                 360                 365

act caa ata cct gca gaa atg cta gca agc atc gat ttg aaa tac ttc     1333
Thr Gln Ile Pro Ala Glu Met Leu Ala Ser Ile Asp Leu Lys Tyr Phe
                370                 375                 380

aat gat tca act aga aag aag att gaa aaa atc cgg ccg ctc tta ata     1381
Asn Asp Ser Thr Arg Lys Lys Ile Glu Lys Ile Arg Pro Leu Leu Ile
            385                 390                 395

gat ggg act gca tca ttg agc cct gga atg atg atg ggc atg ttc aat     1429
Asp Gly Thr Ala Ser Leu Ser Pro Gly Met Met Met Gly Met Phe Asn
        400                 405                 410

atg tta agt act gta tta ggc gtc tcc atc ctg aat ctt gga caa aag     1477
Met Leu Ser Thr Val Leu Gly Val Ser Ile Leu Asn Leu Gly Gln Lys
    415                 420                 425

aga cac acc aag act act tac tgg tgg gat ggt ctt caa tct tct gat     1525
Arg His Thr Lys Thr Thr Tyr Trp Trp Asp Gly Leu Gln Ser Ser Asp
430                 435                 440                 445

gat ttt gct ctg att gtg aat gca ccc aat cat gaa ggg att caa gcc     1573
Asp Phe Ala Leu Ile Val Asn Ala Pro Asn His Glu Gly Ile Gln Ala
                450                 455                 460

gga gtc aac agg ttt tat cga acc tgt aag cta ctt gga att aat atg     1621
Gly Val Asn Arg Phe Tyr Arg Thr Cys Lys Leu Leu Gly Ile Asn Met
            465                 470                 475

agc aag aaa aag tct tac ata aac aga aca ggt aca ttt gaa ttc aca     1669
Ser Lys Lys Lys Ser Tyr Ile Asn Arg Thr Gly Thr Phe Glu Phe Thr
        480                 485                 490

agc ttt ttc tat cgt tat ggg ttt gtt gcc aat ttc agc atg gag ctt     1717
Ser Phe Phe Tyr Arg Tyr Gly Phe Val Ala Asn Phe Ser Met Glu Leu
    495                 500                 505

ccc agc ttt ggg gtg tct ggg atc aac gag tct gcg gac atg agt att     1765
Pro Ser Phe Gly Val Ser Gly Ile Asn Glu Ser Ala Asp Met Ser Ile
510                 515                 520                 525

gga gtt act gtc atc aaa aac aat atg ata aac aat gat ctt ggt cca     1813
Gly Val Thr Val Ile Lys Asn Asn Met Ile Asn Asn Asp Leu Gly Pro
                530                 535                 540

gca acc gct caa atg gcc ctt cag ctg ttc atc aaa gat tac agg tac     1861
Ala Thr Ala Gln Met Ala Leu Gln Leu Phe Ile Lys Asp Tyr Arg Tyr
            545                 550                 555
```

```
acg tac cgg tgc cat aga ggt gac aca caa ata caa acc cga aga tca   1909
Thr Tyr Arg Cys His Arg Gly Asp Thr Gln Ile Gln Thr Arg Arg Ser
        560                 565                 570

ttt gaa ata aag aaa ctg tgg gag caa acc cat tcc aaa gct gga ctg   1957
Phe Glu Ile Lys Lys Leu Trp Glu Gln Thr His Ser Lys Ala Gly Leu
    575                 580                 585

ctg gtc tcc gac gga ggc cca aat tta tac aac att aga aat ctc cac   2005
Leu Val Ser Asp Gly Gly Pro Asn Leu Tyr Asn Ile Arg Asn Leu His
590                 595                 600                 605

att cct gaa gtc tgc ttg aaa tgg gaa tta atg gat gag gat tac cag   2053
Ile Pro Glu Val Cys Leu Lys Trp Glu Leu Met Asp Glu Asp Tyr Gln
                610                 615                 620

ggg cgt tta tgc aac cca ctg aac cca ttt gtc aac cat aaa gac att   2101
Gly Arg Leu Cys Asn Pro Leu Asn Pro Phe Val Asn His Lys Asp Ile
            625                 630                 635

gaa tca gtg aac aat gca gtg ata atg cca gca cat ggt cca gcc aaa   2149
Glu Ser Val Asn Asn Ala Val Ile Met Pro Ala His Gly Pro Ala Lys
        640                 645                 650

aac atg gag tat gat gct gtt gca aca aca cac tcc tgg atc ccc aaa   2197
Asn Met Glu Tyr Asp Ala Val Ala Thr Thr His Ser Trp Ile Pro Lys
    655                 660                 665

aga aat cga tcc atc ttg aat aca agc caa aga gga ata ctt gaa gat   2245
Arg Asn Arg Ser Ile Leu Asn Thr Ser Gln Arg Gly Ile Leu Glu Asp
670                 675                 680                 685

gaa caa atg tac caa aag tgc tgc aac tta ttt gaa aaa ttc ttc ccc   2293
Glu Gln Met Tyr Gln Lys Cys Cys Asn Leu Phe Glu Lys Phe Phe Pro
                690                 695                 700

agc agt tca tac aga aga cca gtc ggg ata tcc agt atg gtg gag gct   2341
Ser Ser Ser Tyr Arg Arg Pro Val Gly Ile Ser Ser Met Val Glu Ala
            705                 710                 715

atg gtt tcc aga gcc cga att gat gca cga att gat ttc gaa tct gga   2389
Met Val Ser Arg Ala Arg Ile Asp Ala Arg Ile Asp Phe Glu Ser Gly
        720                 725                 730

agg ata aag aaa gag gag ttc act gag atc atg aag atc tgt tcc acc   2437
Arg Ile Lys Lys Glu Glu Phe Thr Glu Ile Met Lys Ile Cys Ser Thr
    735                 740                 745

att gaa gag ctc aga cgg caa aaa tagtgaattt agcttgtcct tcatgaaaaa  2491
Ile Glu Glu Leu Arg Arg Gln Lys
750                 755

atgccttgtt tctactaata acccggcggc ccaaaatgcc gactcggagc gaaagatata 2551

cctcccccgg ggccgggagg tcgcgtcacc gaccacgccg ccggcccagg cgacgcgcga 2611

cacggacacc tgtccccaaa aacgccacca tcgcagccac acacggagcg cccggggccc 2671

tctggtcaac cccaggacac acgcgggagc agcgccgggc cggggacgcc ctcccggccg 2731

cccgtgccac acgcaggggg ccggcccgtg tctccagagc gggagccgga agcattttcg 2791

gccggcccct cctacgaccg ggacacacga gggaccgaag gccggccagg cgcgacctct 2851

cgggccgcac gcgcgctcag ggagcgctct ccgactccgc acggggactc gccagaaagg 2911
```

atcgtgacct gcattaatga atcaggggat aacgcaggaa agaacatgtg agcaaaaggc 2971
cagcaaaagg ccaggaaccg taaaaaggcc gcgttgctgg cgtttttcca taggctccgc 3031
cccccctgacg agcatcacaa aaatcgacgc tcaagtcaga ggtggcgaaa cccgacagga 3091
ctataaagat accaggcgtt tccccctgga agctccctcg tgcgctctcc tgttccgacc 3151
ctgccgctta ccggatacct gtccgccttt ctcccttcgg gaagcgtggc gctttctcat 3211
agctcacgct gtaggtatct cagttcggtg taggtcgttc gctccaagct gggctgtgtg 3271
cacgaacccc ccgttcagcc cgaccgctgc gccttatccg gtaactatcg tcttgagtcc 3331
aacccggtaa gacacgactt atcgccactg gcagcagcca ctggtaacag gattagcaga 3391
gcgaggtatg taggcggtgc tacagagttc ttgaagtggt ggcctaacta cggctacact 3451
agaaggacag tatttggtat ctgcgctctg ctgaagccag ttaccttcgg aaaaagagtt 3511
ggtagctctt gatccggcaa acaaaccacc gctggtagcg gtggtttttt tgtttgcaag 3571
cagcagatta cgcgcagaaa aaaaggatct caagaagatc ctttgatctt ttctacgggg 3631
tctgacgctc agtggaacga aaactcacgt taagggattt tggtcatgag attatcaaaa 3691
aggatcttca cctagatcct tttaaattaa aaatgaagtt ttaaatcaat ctaaagtata 3751
tatgagtaaa cttggtctga cagttaccaa tgcttaatca gtgaggcacc tatctcagcg 3811
atctgtctat ttcgttcatc catagttgcc tgactccccg tcgtgtagat aactacgata 3871
cgggagggct taccatctgg ccccagtgct gcaatgatac cgcgagaccc acgctcaccg 3931
gctccagatt tatcagcaat aaaccagcca gccggaaggg ccgagcgcag aagtggtcct 3991
gcaactttat ccgcctccat ccagtctatt aattgttgcc gggaagctag agtaagtagt 4051
tcgccagtta atagtttgcg caacgttgtt gccattgcta caggcatcgt ggtgtcacgc 4111
tcgtcgtttg gtatggcttc attcagctcc ggttcccaac gatcaaggcg agttacatga 4171
tcccccatgt tgtgcaaaaa agcggttagc tccttcggtc ctccgatcgt tgtcagaagt 4231
aagttggccg cagtgttatc actcatggtt atggcagcac tgcataattc tcttactgtc 4291
atgccatccg taagatgctt ttctgtgact ggtgagtact caaccaagtc attctgagaa 4351
tagtgtatgc ggcgaccgag ttgctcttgc ccggcgtcaa cacgggataa taccgcgcca 4411
catagcagaa ctttaaaagt gctcatcatt ggaaaacgtt cttcggggcg aaaactctca 4471
aggatcttac cgctgttgag atccagttcg atgtaaccca ctcgtgcacc caactgatct 4531
tcagcatctt ttactttcac cagcgtttct gggtgagcaa aaacaggaag gcaaaatgcc 4591
gcaaaaaagg gaataagggc gacacggaaa tgttgaatac tcatactctt cctttttcaa 4651
tattattgaa gcatttatca gggttattgt ctcatgagcg gatacatatt tgaatgtatt 4711
tagaaaaata aacaaaagag tttgtagaaa cgcaaaaagg ccatccgtca ggatggcctt 4771
ctgcttaatt tgatgcctgg cagtttatgg cgggcgtcct gcccgccacc ctccgggccg 4831
ttgcttcgca acgttcaaat ccgctcccgg cggatttgtc ctactcagga gagcgttcac 4891
cgacaaacaa cagataaaac gaaaggccca gtctttcgac tgagcctttc gttttatttg 4951

atgcctggca gttccctact ctcgcatggg gagaccccac actaccatcg gcgctacggc 5011

gtttcacttc tgagttcggc atggggtcag gtgggaccac cgcgctactg ccgccaggca 5071

aattctgttt tatcagaccg cttctgcgtt ctgatttaat ctgtatcagg ctgaaaatct 5131

tctctcatcc gccaaaacag ccaagctagc ggccgatc 5169

<210> 25
<211> 757
<212> PRT
<213> Artificial Sequence
<223> Description of Artificial Sequence: WSN-PB1

<400> 25
Met Asp Val Asn Pro Thr Leu Leu Phe Leu Lys Val Pro Ala Gln Asn
1 5 10 15

Ala Ile Ser Thr Thr Phe Pro Tyr Thr Gly Asp Pro Pro Tyr Ser His
20 25 30

Gly Thr Gly Thr Gly Tyr Thr Met Asp Thr Val Asn Arg Thr His Gln
35 40 45

Tyr Ser Glu Arg Gly Arg Trp Thr Thr Asn Thr Glu Thr Gly Ala Pro
50 55 60

Gln Leu Asn Pro Ile Asp Gly Pro Leu Pro Glu Asp Asn Glu Pro Ser
65 70 75 80

Gly Tyr Ala Gln Thr Asp Cys Val Leu Glu Ala Met Ala Phe Leu Glu
85 90 95

Glu Ser His Pro Gly Ile Phe Glu Thr Ser Cys Leu Glu Thr Met Glu
100 105 110

Val Val Gln Gln Thr Arg Val Asp Lys Leu Thr Gln Gly Arg Gln Thr
115 120 125

Tyr Asp Trp Thr Leu Asn Arg Asn Gln Pro Ala Ala Thr Ala Leu Ala
130 135 140

Asn Thr Ile Glu Val Phe Arg Ser Asn Gly Leu Thr Ala Asn Glu Ser
145 150 155 160

Gly Arg Leu Ile Asp Phe Leu Lys Asp Val Met Glu Ser Met Asn Lys
165 170 175

Glu Glu Met Glu Ile Thr Thr His Phe Gln Arg Lys Arg Arg Val Arg
180 185 190

Asp Asn Met Thr Lys Lys Met Val Thr Gln Arg Thr Ile Gly Lys Arg
195 200 205

Lys Gln Arg Leu Asn Lys Arg Ser Tyr Leu Ile Arg Ala Leu Thr Leu
210 215 220

Asn Thr Met Thr Lys Asp Ala Glu Arg Gly Lys Leu Lys Arg Arg Ala
225 230 235 240

Ile Ala Thr Pro Gly Met Gln Ile Arg Gly Phe Val Tyr Phe Val Glu
245 250 255

Thr Leu Ala Arg Ser Ile Cys Glu Lys Leu Glu Gln Ser Gly Leu Pro
260 265 270

```
Val Gly Gly Asn Glu Lys Lys Ala Lys Leu Ala Asn Val Val Arg Lys
        275                 280                 285

Met Met Thr Asn Ser Gln Asp Thr Glu Ile Ser Phe Thr Ile Thr Gly
    290                 295                 300

Asp Asn Thr Lys Trp Asn Glu Asn Gln Asn Pro Arg Met Phe Leu Ala
305                 310                 315                 320

Met Ile Thr Tyr Ile Thr Arg Asn Gln Pro Glu Trp Phe Arg Asn Val
                325                 330                 335

Leu Ser Ile Ala Pro Ile Met Phe Ser Asn Lys Met Ala Arg Leu Gly
            340                 345                 350

Lys Gly Tyr Met Phe Glu Ser Lys Ser Met Lys Ile Arg Thr Gln Ile
        355                 360                 365

Pro Ala Glu Met Leu Ala Ser Ile Asp Leu Lys Tyr Phe Asn Asp Ser
    370                 375                 380

Thr Arg Lys Lys Ile Glu Lys Ile Arg Pro Leu Leu Ile Asp Gly Thr
385                 390                 395                 400

Ala Ser Leu Ser Pro Gly Met Met Met Gly Met Phe Asn Met Leu Ser
                405                 410                 415

Thr Val Leu Gly Val Ser Ile Leu Asn Leu Gly Gln Lys Arg His Thr
            420                 425                 430

Lys Thr Thr Tyr Trp Trp Asp Gly Leu Gln Ser Ser Asp Asp Phe Ala
        435                 440                 445

Leu Ile Val Asn Ala Pro Asn His Glu Gly Ile Gln Ala Gly Val Asn
    450                 455                 460

Arg Phe Tyr Arg Thr Cys Lys Leu Leu Gly Ile Asn Met Ser Lys Lys
465                 470                 475                 480

Lys Ser Tyr Ile Asn Arg Thr Gly Thr Phe Glu Phe Thr Ser Phe Phe
                485                 490                 495

Tyr Arg Tyr Gly Phe Val Ala Asn Phe Ser Met Glu Leu Pro Ser Phe
            500                 505                 510

Gly Val Ser Gly Ile Asn Glu Ser Ala Asp Met Ser Ile Gly Val Thr
        515                 520                 525

Val Ile Lys Asn Asn Met Ile Asn Asn Asp Leu Gly Pro Ala Thr Ala
    530                 535                 540

Gln Met Ala Leu Gln Leu Phe Ile Lys Asp Tyr Arg Tyr Thr Tyr Arg
545                 550                 555                 560

Cys His Arg Gly Asp Thr Gln Ile Gln Thr Arg Arg Ser Phe Glu Ile
                565                 570                 575

Lys Lys Leu Trp Glu Gln Thr His Ser Lys Ala Gly Leu Leu Val Ser
            580                 585                 590

Asp Gly Gly Pro Asn Leu Tyr Asn Ile Arg Asn Leu His Ile Pro Glu
        595                 600                 605

Val Cys Leu Lys Trp Glu Leu Met Asp Glu Asp Tyr Gln Gly Arg Leu
    610                 615                 620
```

```
Cys Asn Pro Leu Asn Pro Phe Val Asn His Lys Asp Ile Glu Ser Val
625                 630                 635                 640

Asn Asn Ala Val Ile Met Pro Ala His Gly Pro Ala Lys Asn Met Glu
                645                 650                 655

Tyr Asp Ala Val Ala Thr Thr His Ser Trp Ile Pro Lys Arg Asn Arg
            660                 665                 670

Ser Ile Leu Asn Thr Ser Gln Arg Gly Ile Leu Glu Asp Glu Gln Met
        675                 680                 685

Tyr Gln Lys Cys Cys Asn Leu Phe Glu Lys Phe Phe Pro Ser Ser Ser
    690                 695                 700

Tyr Arg Arg Pro Val Gly Ile Ser Ser Met Val Glu Ala Met Val Ser
705                 710                 715                 720

Arg Ala Arg Ile Asp Ala Arg Ile Asp Phe Glu Ser Gly Arg Ile Lys
                725                 730                 735

Lys Glu Glu Phe Thr Glu Ile Met Lys Ile Cys Ser Thr Ile Glu Glu
            740                 745                 750

Leu Arg Arg Gln Lys
        755


<210> 26
<211> 5169
<212> DNA
<213> Artificial Sequence

<220>
<223> Description of Artificial Sequence: pHL3102

<220>
<221> CDS
<222> (191)..(2461)

<400> 26
cccaaaaaaa aaaaaaaaaa aagagtccag agtggccccg ccgttccgcg ccgggggggg 60

gggggggggg ggacactttc ggacatctgg tcgacctcca gcatcggggg aaaaaaaaaa 120

acaaagtgtc gcccggagta ctggtcgacc tccgaagttg ggggggagcg aaagcaggca 180

aaccatttga atg gat gtc aat ccg act tta ctt ttc tta aaa gtg cca    229
           Met Asp Val Asn Pro Thr Leu Leu Phe Leu Lys Val Pro
           1               5                   10

gca caa aat gct ata agc aca act ttc cct tat act gga gac cct cct   277
Ala Gln Asn Ala Ile Ser Thr Thr Phe Pro Tyr Thr Gly Asp Pro Pro
    15                  20                  25

tac agc cat ggg aca gga aca gga tac acc atg gat act gtc aac agg   325
Tyr Ser His Gly Thr Gly Thr Gly Tyr Thr Met Asp Thr Val Asn Arg
30                  35                  40                  45

aca cat cag tac tca gaa agg gga aga tgg aca aca aac acc gaa act   373
Thr His Gln Tyr Ser Glu Arg Gly Arg Trp Thr Thr Asn Thr Glu Thr
                50                  55                  60

gga gca ccg caa ctc aac ccg att gat ggg cca ctg cca gaa gac aat   421
Gly Ala Pro Gln Leu Asn Pro Ile Asp Gly Pro Leu Pro Glu Asp Asn
            65                  70                  75
```

```
gaa cca agt ggt tat gcc caa aca gat tgt gta ttg gaa gca atg gcc     469
Glu Pro Ser Gly Tyr Ala Gln Thr Asp Cys Val Leu Glu Ala Met Ala
        80                  85                  90

ttc ctt gag gaa tcc cat cct ggt atc ttt gag acc tcg tgt ctt gaa     517
Phe Leu Glu Glu Ser His Pro Gly Ile Phe Glu Thr Ser Cys Leu Glu
    95                  100                 105

acg atg gag gtt gtt cag caa aca cga gtg gac aag ctg aca caa ggc     565
Thr Met Glu Val Val Gln Gln Thr Arg Val Asp Lys Leu Thr Gln Gly
110                 115                 120                 125

cga cag acc tat gac tgg act cta aat agg aac cag cct gct gca aca     613
Arg Gln Thr Tyr Asp Trp Thr Leu Asn Arg Asn Gln Pro Ala Ala Thr
                130                 135                 140

gca ttg gcc aac aca ata gaa gtg ttc aga tca aat ggc ctc acg gcc     661
Ala Leu Ala Asn Thr Ile Glu Val Phe Arg Ser Asn Gly Leu Thr Ala
            145                 150                 155

aat gaa tct gga agg ctc ata gac ttc ctt aag gat gta atg gag tca     709
Asn Glu Ser Gly Arg Leu Ile Asp Phe Leu Lys Asp Val Met Glu Ser
        160                 165                 170

atg aac aaa gaa gaa atg gag atc aca act cat ttt cag aga aag aga     757
Met Asn Lys Glu Glu Met Glu Ile Thr Thr His Phe Gln Arg Lys Arg
    175                 180                 185

cga gtg aga gac aat atg act aag aaa atg gtg aca cag aga aca ata     805
Arg Val Arg Asp Asn Met Thr Lys Lys Met Val Thr Gln Arg Thr Ile
190                 195                 200                 205

ggt aaa agg aag cag aga ttg aac aaa agg agt tat cta att agg gca     853
Gly Lys Arg Lys Gln Arg Leu Asn Lys Arg Ser Tyr Leu Ile Arg Ala
                210                 215                 220

tta acc ctg aac aca atg acc aaa gat gct gag aga ggg aag cta aaa     901
Leu Thr Leu Asn Thr Met Thr Lys Asp Ala Glu Arg Gly Lys Leu Lys
            225                 230                 235

cgg aga gca att gca acc cca ggg atg caa ata agg ggg ttt gta tac     949
Arg Arg Ala Ile Ala Thr Pro Gly Met Gln Ile Arg Gly Phe Val Tyr
        240                 245                 250

ttt gtt gag aca cta gca agg agt ata tgt gag aaa ctt gaa caa tca     997
Phe Val Glu Thr Leu Ala Arg Ser Ile Cys Glu Lys Leu Glu Gln Ser
    255                 260                 265

gga ttg cca gtt gga ggc aat gag aag aaa gca aag ttg gca aat gtt     1045
Gly Leu Pro Val Gly Gly Asn Glu Lys Lys Ala Lys Leu Ala Asn Val
270                 275                 280                 285

gta agg aag atg atg acc aat tct cag gac act gaa att tct ttc acc     1093
Val Arg Lys Met Met Thr Asn Ser Gln Asp Thr Glu Ile Ser Phe Thr
                290                 295                 300

atc act gga gat aac acc aaa tgg aac gaa aat cag aac cct cgg atg     1141
Ile Thr Gly Asp Asn Thr Lys Trp Asn Glu Asn Gln Asn Pro Arg Met
            305                 310                 315

ttt ttg gcc atg atc aca tat ata acc aga aat cag ccc gaa tgg ttc     1189
Phe Leu Ala Met Ile Thr Tyr Ile Thr Arg Asn Gln Pro Glu Trp Phe
        320                 325                 330

aga aat gtt cta agt att gct cca ata atg ttc tca aac aaa atg gcg     1237
Arg Asn Val Leu Ser Ile Ala Pro Ile Met Phe Ser Asn Lys Met Ala
    335                 340                 345
```

```
aga ctg gga aag ggg tac atg ttt gag agc aag agt atg aaa att aga     1285
Arg Leu Gly Lys Gly Tyr Met Phe Glu Ser Lys Ser Met Lys Ile Arg
350                 355                 360                 365

act caa ata cct gca gaa atg cta gca agc atc gat ttg aaa tac ttc     1333
Thr Gln Ile Pro Ala Glu Met Leu Ala Ser Ile Asp Leu Lys Tyr Phe
                370                 375                 380

aat gat tca act aga aag aag att gaa aaa atc cgg ccg ctc tta ata     1381
Asn Asp Ser Thr Arg Lys Lys Ile Glu Lys Ile Arg Pro Leu Leu Ile
            385                 390                 395

gat ggg act gca tca ttg agc cct gga atg atg atg ggc atg ttc aat     1429
Asp Gly Thr Ala Ser Leu Ser Pro Gly Met Met Met Gly Met Phe Asn
        400                 405                 410

atg tta agt act gta tta ggc gtc tcc atc ctg aat ctt gga caa aag     1477
Met Leu Ser Thr Val Leu Gly Val Ser Ile Leu Asn Leu Gly Gln Lys
    415                 420                 425

aga cac acc aag act act tac tgg tgg gat ggt ctt caa tct tct gat     1525
Arg His Thr Lys Thr Thr Tyr Trp Trp Asp Gly Leu Gln Ser Ser Asp
430                 435                 440                 445

gat ttt gct ctg att gtg aat gca ccc aat cat gaa ggg att caa gcc     1573
Asp Phe Ala Leu Ile Val Asn Ala Pro Asn His Glu Gly Ile Gln Ala
                450                 455                 460

gga gtc aac agg ttt tat cga acc tgt aag cta ctt gga att aat atg     1621
Gly Val Asn Arg Phe Tyr Arg Thr Cys Lys Leu Leu Gly Ile Asn Met
            465                 470                 475

agc aag aaa aag tct tac ata aac aga aca ggt aca ttt gaa ttc aca     1669
Ser Lys Lys Lys Ser Tyr Ile Asn Arg Thr Gly Thr Phe Glu Phe Thr
        480                 485                 490

agc ttt ttc tac cgc tat gga ttt gta gcc aat ttt agt atg gag ttg     1717
Ser Phe Phe Tyr Arg Tyr Gly Phe Val Ala Asn Phe Ser Met Glu Leu
    495                 500                 505

ccc agc ttt gga gta tca gga att aat gaa tcg gct gat atg agc att     1765
Pro Ser Phe Gly Val Ser Gly Ile Asn Glu Ser Ala Asp Met Ser Ile
510                 515                 520                 525

gga gta aca gtg ata aag aat aac atg ata aac aat gat ctt gga ccg     1813
Gly Val Thr Val Ile Lys Asn Asn Met Ile Asn Asn Asp Leu Gly Pro
                530                 535                 540

gca aca gcc caa atg gct ctc caa tta ttc atc aag gac tac aga tat     1861
Ala Thr Ala Gln Met Ala Leu Gln Leu Phe Ile Lys Asp Tyr Arg Tyr
            545                 550                 555

aca tac agg tgt cac agg gga gac aca caa atc caa acg agg agg tca     1909
Thr Tyr Arg Cys His Arg Gly Asp Thr Gln Ile Gln Thr Arg Arg Ser
        560                 565                 570

ttc gag cta aag aag ctg tgg gag cag acc cgc tca aag gca gga ctg     1957
Phe Glu Leu Lys Lys Leu Trp Glu Gln Thr Arg Ser Lys Ala Gly Leu
    575                 580                 585

ttg gtt tca gat ggc gga cca aac ctg tac aac att cgg aat ctc cac     2005
Leu Val Ser Asp Gly Gly Pro Asn Leu Tyr Asn Ile Arg Asn Leu His
590                 595                 600                 605
```

```
atc ccg gaa gtt tgc ctg aaa tgg gaa cta atg gat gaa gac tat cag   2053
Ile Pro Glu Val Cys Leu Lys Trp Glu Leu Met Asp Glu Asp Tyr Gln
                610                 615                 620

gga aga ctt tgt aat ccc atg aac ccg ttt gtc agt cat aag gaa att   2101
Gly Arg Leu Cys Asn Pro Met Asn Pro Phe Val Ser His Lys Glu Ile
            625                 630                 635

gaa tct gta aac aat gct gcg gta atg cca gcc cat ggt cca gcc aaa   2149
Glu Ser Val Asn Asn Ala Ala Val Met Pro Ala His Gly Pro Ala Lys
        640                 645                 650

agc atg gaa tat gat gct gtg gca act aca cac tct tgg atc cct aag   2197
Ser Met Glu Tyr Asp Ala Val Ala Thr Thr His Ser Trp Ile Pro Lys
    655                 660                 665

aga aac cgt tcc att ctc aat acg agt caa agg gga atc ctt gag gat   2245
Arg Asn Arg Ser Ile Leu Asn Thr Ser Gln Arg Gly Ile Leu Glu Asp
670                 675                 680                 685

gaa caa atg tac cag aag tgt tgc aac cta ttc gag aaa ttc ttc cct   2293
Glu Gln Met Tyr Gln Lys Cys Cys Asn Leu Phe Glu Lys Phe Phe Pro
                690                 695                 700

agc agc tca tac aga aga cca gtt gga att tcc agt atg gtg gag gcc   2341
Ser Ser Ser Tyr Arg Arg Pro Val Gly Ile Ser Ser Met Val Glu Ala
            705                 710                 715

atg gtg tct agg gcc cgg att gat gca cga att gac ttc gag tct gga   2389
Met Val Ser Arg Ala Arg Ile Asp Ala Arg Ile Asp Phe Glu Ser Gly
        720                 725                 730

agg att aag aag gaa gag ttt gct gag atc atg aag atc tgt tcc acc   2437
Arg Ile Lys Lys Glu Glu Phe Ala Glu Ile Met Lys Ile Cys Ser Thr
    735                 740                 745

att gaa gag ctc aga cgg caa aaa tagtgaattt agcttgtcct tcatgaaaaa  2491
Ile Glu Glu Leu Arg Arg Gln Lys
750                 755

atgccttgtt tctactaata acccggcggc ccaaaatgcc gactcggagc gaaagatata 2551

cctcccccgg ggccgggagg tcgcgtcacc gaccacgccg ccggcccagg cgacgcgcga 2611

cacggacacc tgtccccaaa aacgccacca tcgcagccac acacggagcg cccggggccc 2671

tctggtcaac cccaggacac acgcgggagc agcgccgggc cggggacgcc ctcccggccg 2731

cccgtgccac acgcaggggg ccggcccgtg tctccagagc gggagccgga agcattttcg 2791

gccggcccct cctacgaccg ggacacacga gggaccgaag gccggccagg cgcgacctct 2851

cgggccgcac gcgcgctcag ggagcgctct ccgactccgc acggggactc gccagaaagg 2911

atcgtgacct gcattaatga atcaggggat aacgcaggaa agaacatgtg agcaaaaggc 2971

cagcaaaagg ccaggaaccg taaaaaggcc gcgttgctgg cgtttttcca taggctccgc 3031

cccccctgacg agcatcacaa aaatcgacgc tcaagtcaga ggtggcgaaa cccgacagga 3091

ctataaagat accaggcgtt tccccctgga agctccctcg tgcgctctcc tgttccgacc 3151

ctgccgctta ccggatacct gtccgccttt ctcccttcgg gaagcgtggc gctttctcat 3211

agctcacgct gtaggtatct cagttcggtg taggtcgttc gctccaagct gggctgtgtg 3271

cacgaacccc ccgttcagcc cgaccgctgc gccttatccg gtaactatcg tcttgagtcc 3331
```

aacccggtaa gacacgactt atcgccactg gcagcagcca ctggtaacag gattagcaga 3391
gcgaggtatg taggcggtgc tacagagttc ttgaagtggt ggcctaacta cggctacact 3451
agaaggacag tatttggtat ctgcgctctg ctgaagccag ttaccttcgg aaaaagagtt 3511
ggtagctctt gatccggcaa acaaaccacc gctggtagcg gtggtttttt tgtttgcaag 3571
cagcagatta cgcgcagaaa aaaaggatct caagaagatc ctttgatctt ttctacgggg 3631
tctgacgctc agtggaacga aaactcacgt taagggattt tggtcatgag attatcaaaa 3691
aggatcttca cctagatcct tttaaattaa aaatgaagtt ttaaatcaat ctaaagtata 3751
tatgagtaaa cttggtctga cagttaccaa tgcttaatca gtgaggcacc tatctcagcg 3811
atctgtctat ttcgttcatc catagttgcc tgactccccg tcgtgtagat aactacgata 3871
cgggagggct taccatctgg ccccagtgct gcaatgatac cgcgagaccc acgctcaccg 3931
gctccagatt tatcagcaat aaaccagcca gccggaaggg ccgagcgcag aagtggtcct 3991
gcaactttat ccgcctccat ccagtctatt aattgttgcc gggaagctag agtaagtagt 4051
tcgccagtta atagtttgcg caacgttgtt gccattgcta caggcatcgt ggtgtcacgc 4111
tcgtcgtttg gtatggcttc attcagctcc ggttcccaac gatcaaggcg agttacatga 4171
tcccccatgt tgtgcaaaaa agcggttagc tccttcggtc ctccgatcgt tgtcagaagt 4231
aagttggccg cagtgttatc actcatggtt atggcagcac tgcataattc tcttactgtc 4291
atgccatccg taagatgctt ttctgtgact ggtgagtact caaccaagtc attctgagaa 4351
tagtgtatgc ggcgaccgag ttgctcttgc ccggcgtcaa cacgggataa taccgcgcca 4411
catagcagaa ctttaaaagt gctcatcatt ggaaaacgtt cttcggggcg aaaactctca 4471
aggatcttac cgctgttgag atccagttcg atgtaaccca ctcgtgcacc caactgatct 4531
tcagcatctt ttactttcac cagcgtttct gggtgagcaa aaacaggaag gcaaaatgcc 4591
gcaaaaaagg gaataagggc gacacggaaa tgttgaatac tcatactctt cctttttcaa 4651
tattattgaa gcatttatca gggttattgt ctcatgagcg gatacatatt tgaatgtatt 4711
tagaaaaata aacaaaagag tttgtagaaa cgcaaaaagg ccatccgtca ggatggcctt 4771
ctgcttaatt tgatgcctgg cagtttatgg cgggcgtcct gcccgccacc ctccgggccg 4831
ttgcttcgca acgttcaaat ccgctcccgg cggatttgtc ctactcagga gagcgttcac 4891
cgacaaacaa cagataaaac gaaaggccca gtctttcgac tgagcctttc gttttatttg 4951
atgcctggca gttccctact ctcgcatggg gagaccccac actaccatcg gcgctacggc 5011
gtttcacttc tgagttcggc atggggtcag gtgggaccac cgcgctactg ccgccaggca 5071
aattctgttt tatcagaccg cttctgcgtt ctgatttaat ctgtatcagg ctgaaaatct 5131
tctctcatcc gccaaaacag ccaagctagc ggccgatc 5169

<210> 27
<211> 757
<212> PRT

<213> Artificial Sequence
<223> Description of Artificial Sequence: pHL3102

<400> 27

```
Met Asp Val Asn Pro Thr Leu Leu Phe Leu Lys Val Pro Ala Gln Asn
  1               5                  10                  15

Ala Ile Ser Thr Thr Phe Pro Tyr Thr Gly Asp Pro Pro Tyr Ser His
             20                  25                  30

Gly Thr Gly Thr Gly Tyr Thr Met Asp Thr Val Asn Arg Thr His Gln
         35                  40                  45

Tyr Ser Glu Arg Gly Arg Trp Thr Thr Asn Thr Glu Thr Gly Ala Pro
     50                  55                  60

Gln Leu Asn Pro Ile Asp Gly Pro Leu Pro Glu Asp Asn Glu Pro Ser
 65                  70                  75                  80

Gly Tyr Ala Gln Thr Asp Cys Val Leu Glu Ala Met Ala Phe Leu Glu
                 85                  90                  95

Glu Ser His Pro Gly Ile Phe Glu Thr Ser Cys Leu Glu Thr Met Glu
            100                 105                 110

Val Val Gln Gln Thr Arg Val Asp Lys Leu Thr Gln Gly Arg Gln Thr
        115                 120                 125

Tyr Asp Trp Thr Leu Asn Arg Asn Gln Pro Ala Ala Thr Ala Leu Ala
    130                 135                 140

Asn Thr Ile Glu Val Phe Arg Ser Asn Gly Leu Thr Ala Asn Glu Ser
145                 150                 155                 160

Gly Arg Leu Ile Asp Phe Leu Lys Asp Val Met Glu Ser Met Asn Lys
                165                 170                 175

Glu Glu Met Glu Ile Thr Thr His Phe Gln Arg Lys Arg Arg Val Arg
            180                 185                 190

Asp Asn Met Thr Lys Lys Met Val Thr Gln Arg Thr Ile Gly Lys Arg
        195                 200                 205

Lys Gln Arg Leu Asn Lys Arg Ser Tyr Leu Ile Arg Ala Leu Thr Leu
    210                 215                 220

Asn Thr Met Thr Lys Asp Ala Glu Arg Gly Lys Leu Lys Arg Arg Ala
225                 230                 235                 240

Ile Ala Thr Pro Gly Met Gln Ile Arg Gly Phe Val Tyr Phe Val Glu
                245                 250                 255

Thr Leu Ala Arg Ser Ile Cys Glu Lys Leu Glu Gln Ser Gly Leu Pro
            260                 265                 270

Val Gly Gly Asn Glu Lys Lys Ala Lys Leu Ala Asn Val Val Arg Lys
        275                 280                 285

Met Met Thr Asn Ser Gln Asp Thr Glu Ile Ser Phe Thr Ile Thr Gly
    290                 295                 300

Asp Asn Thr Lys Trp Asn Glu Asn Gln Asn Pro Arg Met Phe Leu Ala
305                 310                 315                 320

Met Ile Thr Tyr Ile Thr Arg Asn Gln Pro Glu Trp Phe Arg Asn Val
                325                 330                 335
```

```
Leu Ser Ile Ala Pro Ile Met Phe Ser Asn Lys Met Ala Arg Leu Gly
            340                 345                 350

Lys Gly Tyr Met Phe Glu Ser Lys Ser Met Lys Ile Arg Thr Gln Ile
        355                 360                 365

Pro Ala Glu Met Leu Ala Ser Ile Asp Leu Lys Tyr Phe Asn Asp Ser
    370                 375                 380

Thr Arg Lys Lys Ile Glu Lys Ile Arg Pro Leu Leu Ile Asp Gly Thr
385                 390                 395                 400

Ala Ser Leu Ser Pro Gly Met Met Met Gly Met Phe Asn Met Leu Ser
                405                 410                 415

Thr Val Leu Gly Val Ser Ile Leu Asn Leu Gly Gln Lys Arg His Thr
            420                 425                 430

Lys Thr Thr Tyr Trp Trp Asp Gly Leu Gln Ser Ser Asp Asp Phe Ala
        435                 440                 445

Leu Ile Val Asn Ala Pro Asn His Glu Gly Ile Gln Ala Gly Val Asn
    450                 455                 460

Arg Phe Tyr Arg Thr Cys Lys Leu Leu Gly Ile Asn Met Ser Lys Lys
465                 470                 475                 480

Lys Ser Tyr Ile Asn Arg Thr Gly Thr Phe Glu Phe Thr Ser Phe Phe
                485                 490                 495

Tyr Arg Tyr Gly Phe Val Ala Asn Phe Ser Met Glu Leu Pro Ser Phe
            500                 505                 510

Gly Val Ser Gly Ile Asn Glu Ser Ala Asp Met Ser Ile Gly Val Thr
        515                 520                 525

Val Ile Lys Asn Asn Met Ile Asn Asn Asp Leu Gly Pro Ala Thr Ala
    530                 535                 540

Gln Met Ala Leu Gln Leu Phe Ile Lys Asp Tyr Arg Tyr Thr Tyr Arg
545                 550                 555                 560

Cys His Arg Gly Asp Thr Gln Ile Gln Thr Arg Arg Ser Phe Glu Leu
                565                 570                 575

Lys Lys Leu Trp Glu Gln Thr Arg Ser Lys Ala Gly Leu Leu Val Ser
            580                 585                 590

Asp Gly Gly Pro Asn Leu Tyr Asn Ile Arg Asn Leu His Ile Pro Glu
        595                 600                 605

Val Cys Leu Lys Trp Glu Leu Met Asp Glu Asp Tyr Gln Gly Arg Leu
    610                 615                 620

Cys Asn Pro Met Asn Pro Phe Val Ser His Lys Glu Ile Glu Ser Val
625                 630                 635                 640

Asn Asn Ala Ala Val Met Pro Ala His Gly Pro Ala Lys Ser Met Glu
                645                 650                 655

Tyr Asp Ala Val Ala Thr Thr His Ser Trp Ile Pro Lys Arg Asn Arg
            660                 665                 670

Ser Ile Leu Asn Thr Ser Gln Arg Gly Ile Leu Glu Asp Glu Gln Met
        675                 680                 685
```

```
Tyr Gln Lys Cys Cys Asn Leu Phe Glu Lys Phe Phe Pro Ser Ser Ser
    690                 695                 700

Tyr Arg Arg Pro Val Gly Ile Ser Ser Met Val Glu Ala Met Val Ser
705                 710                 715                 720

Arg Ala Arg Ile Asp Ala Arg Ile Asp Phe Glu Ser Gly Arg Ile Lys
                725                 730                 735

Lys Glu Glu Phe Ala Glu Ile Met Lys Ile Cys Ser Thr Ile Glu Glu
            740                 745                 750

Leu Arg Arg Gln Lys
        755


<210> 28
<211> 5169
<212> DNA
<213> Artificial Sequence

<220>
<223> Description of Artificial Sequence: pHL3103

<220>
<221> CDS
<222> (191)..(2461)

<400> 28
cccaaaaaaa aaaaaaaaaa aagagtccag agtggccccg ccgctccgcg ccgggggggg 60

gggggggggg ggacactttc ggacatctgg tcgacctcca gcatcggggg aaaaaaaaaa 120

acaaagtgtc gcccggagta ctggtcgacc tccgaagttg ggggggagcg aaagcaggca 180

aaccatttga atg gat gtc aat ccg act tta ctg ttc ttg aaa gtt cct    229
           Met Asp Val Asn Pro Thr Leu Leu Phe Leu Lys Val Pro
           1               5                   10

gcg caa aat gca ata agt act acg ttc cct tac act gga gat cct cca    277
Ala Gln Asn Ala Ile Ser Thr Thr Phe Pro Tyr Thr Gly Asp Pro Pro
    15                  20                  25

tac agc cat gga aca ggg aca gga tac acc atg gac aca gtc aac agg    325
Tyr Ser His Gly Thr Gly Thr Gly Tyr Thr Met Asp Thr Val Asn Arg
30                  35                  40                  45

aca cac caa tat tcg gaa aag ggg aaa tgg aca aca aac act gag act    373
Thr His Gln Tyr Ser Glu Lys Gly Lys Trp Thr Thr Asn Thr Glu Thr
                50                  55                  60

gga gca ccc caa ctt aat cca att gat ggc cca ttg cct gag gac aat    421
Gly Ala Pro Gln Leu Asn Pro Ile Asp Gly Pro Leu Pro Glu Asp Asn
            65                  70                  75

gaa cca agt gga tat gca caa aca gac tgc gtc ctg gaa gca atg gct    469
Glu Pro Ser Gly Tyr Ala Gln Thr Asp Cys Val Leu Glu Ala Met Ala
        80                  85                  90

ttc ctt gaa gaa tcc cat cca gga atc ttt gaa aac tcg tgt ctt gag    517
Phe Leu Glu Glu Ser His Pro Gly Ile Phe Glu Asn Ser Cys Leu Glu
    95                  100                 105

acg atg gaa gtt gtt caa caa aca aga gtg gac aaa ctg acc caa ggc    565
Thr Met Glu Val Val Gln Gln Thr Arg Val Asp Lys Leu Thr Gln Gly
110                 115                 120                 125
```

```
cgt cag act tat gat tgg act ttg aat aga aac cag cct gct gca act     613
Arg Gln Thr Tyr Asp Trp Thr Leu Asn Arg Asn Gln Pro Ala Ala Thr
                130                 135                 140

gca tta gca aac act ata gag gtc ttt aga tcg aat ggt cta aca gct     661
Ala Leu Ala Asn Thr Ile Glu Val Phe Arg Ser Asn Gly Leu Thr Ala
            145                 150                 155

aat gaa tca ggg agg ctc ata gat ttc ctc aag gat gtg atg gaa tca     709
Asn Glu Ser Gly Arg Leu Ile Asp Phe Leu Lys Asp Val Met Glu Ser
        160                 165                 170

atg gat aag gag gaa atg gag ata aca aca cat ttc caa cga aag aga     757
Met Asp Lys Glu Glu Met Glu Ile Thr Thr His Phe Gln Arg Lys Arg
    175                 180                 185

aga gta aga gac aac atg acc aag aaa atg gtc aca caa aga aca ata     805
Arg Val Arg Asp Asn Met Thr Lys Lys Met Val Thr Gln Arg Thr Ile
190                 195                 200                 205

ggg aag aaa aag cag aga ctt aac aaa agg agc tac cta ata agg gct     853
Gly Lys Lys Lys Gln Arg Leu Asn Lys Arg Ser Tyr Leu Ile Arg Ala
                210                 215                 220

cta aca ttg aac aca atg acg aaa gat gca gaa aga ggt aaa ctg aag     901
Leu Thr Leu Asn Thr Met Thr Lys Asp Ala Glu Arg Gly Lys Leu Lys
            225                 230                 235

aga aga gca att gca aca cca ggg atg cag atc aga ggg ttt gtg tac     949
Arg Arg Ala Ile Ala Thr Pro Gly Met Gln Ile Arg Gly Phe Val Tyr
        240                 245                 250

ttt gtt gag aca ctg gcg aga agc att tgc gag aag ctt gaa cag tct     997
Phe Val Glu Thr Leu Ala Arg Ser Ile Cys Glu Lys Leu Glu Gln Ser
    255                 260                 265

ggg cta cca gtt gga ggg aat gag aag aaa gct aaa ttg gca aat gtc    1045
Gly Leu Pro Val Gly Gly Asn Glu Lys Lys Ala Lys Leu Ala Asn Val
270                 275                 280                 285

gtg agg aag atg atg acg aac tca caa gac act gag ctc tct ttc aca    1093
Val Arg Lys Met Met Thr Asn Ser Gln Asp Thr Glu Leu Ser Phe Thr
                290                 295                 300

atc acc gga gac aat acc aaa tgg aat gag aac caa aac ccc cga atg    1141
Ile Thr Gly Asp Asn Thr Lys Trp Asn Glu Asn Gln Asn Pro Arg Met
            305                 310                 315

ttc ctg gca atg ata aca tac atc aca aga aac caa cct gag tgg ttt    1189
Phe Leu Ala Met Ile Thr Tyr Ile Thr Arg Asn Gln Pro Glu Trp Phe
        320                 325                 330

aga aat gtc ttg agc atc gcg ccg ata atg ttt tcg aac aaa atg gcg    1237
Arg Asn Val Leu Ser Ile Ala Pro Ile Met Phe Ser Asn Lys Met Ala
    335                 340                 345

agg cta ggg aaa ggg tac atg ttc gaa agc aaa agc atg aag ctc cga    1285
Arg Leu Gly Lys Gly Tyr Met Phe Glu Ser Lys Ser Met Lys Leu Arg
350                 355                 360                 365

aca caa ata cca gca gaa atg cta gca agt att gat cta aaa tat ttc    1333
Thr Gln Ile Pro Ala Glu Met Leu Ala Ser Ile Asp Leu Lys Tyr Phe
                370                 375                 380

aat gaa cca aca agg aag aaa atc gag aaa ata agg cct ctc ata ata    1381
Asn Glu Pro Thr Arg Lys Lys Ile Glu Lys Ile Arg Pro Leu Ile Ile
            385                 390                 395
```

```
gac ggc aca gcc tca tta agc ccg gga atg atg atg ggt atg ttc aac   1429
Asp Gly Thr Ala Ser Leu Ser Pro Gly Met Met Met Gly Met Phe Asn
        400                 405                 410

atg ctg agt aca gtg ttg gga gtc tca atc ctg aat ctt ggg caa aag   1477
Met Leu Ser Thr Val Leu Gly Val Ser Ile Leu Asn Leu Gly Gln Lys
    415                 420                 425

aga tac acc aaa acc aca tac tgg tgg gat gga ctt cag tcc tct gat   1525
Arg Tyr Thr Lys Thr Thr Tyr Trp Trp Asp Gly Leu Gln Ser Ser Asp
430                 435                 440                 445

gat ttt gct ctc atc gtg aat gca cca aat cat gag gga ata caa gcg   1573
Asp Phe Ala Leu Ile Val Asn Ala Pro Asn His Glu Gly Ile Gln Ala
                450                 455                 460

gga gtg gat aga ttc tac aga acc tgc aag cta gtt ggg atc aat atg   1621
Gly Val Asp Arg Phe Tyr Arg Thr Cys Lys Leu Val Gly Ile Asn Met
            465                 470                 475

agc aag aaa aag tcc tat ata aat agg aca gga aca ttc gaa ttc aca   1669
Ser Lys Lys Lys Ser Tyr Ile Asn Arg Thr Gly Thr Phe Glu Phe Thr
        480                 485                 490

agc ttt ttc tat cgt tat ggg ttt gtt gcc aat ttc agc atg gag ctt   1717
Ser Phe Phe Tyr Arg Tyr Gly Phe Val Ala Asn Phe Ser Met Glu Leu
    495                 500                 505

ccc agc ttt ggg gtg tct ggg atc aac gag tct gcg gac atg agt att   1765
Pro Ser Phe Gly Val Ser Gly Ile Asn Glu Ser Ala Asp Met Ser Ile
510                 515                 520                 525

gga gtt act gtc atc aaa aac aat atg ata aac aat gat ctt ggt cca   1813
Gly Val Thr Val Ile Lys Asn Asn Met Ile Asn Asn Asp Leu Gly Pro
                530                 535                 540

gca acc gct caa atg gcc ctt cag ctg ttc atc aaa gat tac agg tac   1861
Ala Thr Ala Gln Met Ala Leu Gln Leu Phe Ile Lys Asp Tyr Arg Tyr
            545                 550                 555

acg tac cgg tgc cat aga ggt gac aca caa ata caa acc cga aga tca   1909
Thr Tyr Arg Cys His Arg Gly Asp Thr Gln Ile Gln Thr Arg Arg Ser
        560                 565                 570

ttt gaa ata aag aaa ctg tgg gag caa acc cat tcc aaa gct gga ctg   1957
Phe Glu Ile Lys Lys Leu Trp Glu Gln Thr His Ser Lys Ala Gly Leu
    575                 580                 585

ctg gtc tcc gac gga ggc cca aat tta tac aac att aga aat ctc cac   2005
Leu Val Ser Asp Gly Gly Pro Asn Leu Tyr Asn Ile Arg Asn Leu His
590                 595                 600                 605

att cct gaa gtc tgc ttg aaa tgg gaa tta atg gat gag gat tac cag   2053
Ile Pro Glu Val Cys Leu Lys Trp Glu Leu Met Asp Glu Asp Tyr Gln
                610                 615                 620

ggg cgt tta tgc aac cca ctg aac cca ttt gtc aac cat aaa gac att   2101
Gly Arg Leu Cys Asn Pro Leu Asn Pro Phe Val Asn His Lys Asp Ile
            625                 630                 635

gaa tca gtg aac aat gca gtg ata atg cca gca cat ggt cca gcc aaa   2149
Glu Ser Val Asn Asn Ala Val Ile Met Pro Ala His Gly Pro Ala Lys
        640                 645                 650
```

```
aac atg gag tat gat gct gtt gca aca aca cac tcc tgg atc ccc aaa   2197
Asn Met Glu Tyr Asp Ala Val Ala Thr Thr His Ser Trp Ile Pro Lys
    655                 660                 665

aga aat cga tcc atc ttg aat aca agc caa aga gga ata ctt gaa gat   2245
Arg Asn Arg Ser Ile Leu Asn Thr Ser Gln Arg Gly Ile Leu Glu Asp
670                 675                 680                 685

gaa caa atg tac caa aag tgc tgc aac tta ttt gaa aaa ttc ttc ccc   2293
Glu Gln Met Tyr Gln Lys Cys Cys Asn Leu Phe Glu Lys Phe Phe Pro
                690                 695                 700

agc agt tca tac aga aga cca gtc ggg ata tcc agt atg gtg gag gct   2341
Ser Ser Ser Tyr Arg Arg Pro Val Gly Ile Ser Ser Met Val Glu Ala
            705                 710                 715

atg gtt tcc aga gcc cga att gat gca cga att gat ttc gaa tct gga   2389
Met Val Ser Arg Ala Arg Ile Asp Ala Arg Ile Asp Phe Glu Ser Gly
        720                 725                 730

agg ata aag aaa gag gag ttc act gag atc atg aag atc tgt tcc acc   2437
Arg Ile Lys Lys Glu Glu Phe Thr Glu Ile Met Lys Ile Cys Ser Thr
    735                 740                 745

att gaa gag ctc aga cgg caa aaa tagtgaattt agcttgtcct tcatgaaaaa  2491
Ile Glu Glu Leu Arg Arg Gln Lys
750                 755

atgccttgtt tctactaata acccggcggc ccaaaatgcc gactcggagc gaaagatata 2551

cctcccccgg ggccgggagg tcgcgtcacc gaccacgccg ccggcccagg cgacgcgcga 2611

cacggacacc tgtccccaaa aacgccacca tcgcagccac acacggagcg cccggggccc 2671

tctggtcaac cccaggacac acgcgggagc agcgccgggc cggggacgcc ctcccggccg 2731

cccgtgccac acgcaggggg ccggcccgtg tctccagagc gggagccgga agcattttcg 2791

gccggcccct cctacgaccg ggacacacga gggaccgaag gccggccagg cgcgacctct 2851

cgggccgcac gcgcgctcag ggagcgctct ccgactccgc acggggactc gccagaaagg 2911

atcgtgacct gcattaatga atcaggggat aacgcaggaa agaacatgtg agcaaaaggc 2971

cagcaaaagg ccaggaaccg taaaaaggcc gcgttgctgg cgtttttcca taggctccgc 3031

cccccctgacg agcatcacaa aaatcgacgc tcaagtcaga ggtggcgaaa cccgacagga 3091

ctataaagat accaggcgtt tccccctgga agctccctcg tgcgctctcc tgttccgacc 3151

ctgccgctta ccggatacct gtccgccttt ctcccttcgg gaagcgtggc gctttctcat 3211

agctcacgct gtaggtatct cagttcggtg taggtcgttc gctccaagct gggctgtgtg 3271

cacgaacccc ccgttcagcc cgaccgctgc gccttatccg gtaactatcg tcttgagtcc 3331

aacccggtaa gacacgactt atcgccactg gcagcagcca ctggtaacag gattagcaga 3391

gcgaggtatg taggcggtgc tacagagttc ttgaagtggt ggcctaacta cggctacact 3451

agaaggacag tatttggtat ctgcgctctg ctgaagccag ttaccttcgg aaaaagagtt 3511

ggtagctctt gatccggcaa acaaaccacc gctggtagcg gtggtttttt tgtttgcaag 3571

cagcagatta cgcgcagaaa aaaaggatct caagaagatc ctttgatctt ttctacgggg 3631

tctgacgctc agtggaacga aaactcacgt taagggattt tggtcatgag attatcaaaa 3691
```

aggatcttca cctagatcct tttaaattaa aaatgaagtt ttaaatcaat ctaaagtata 3751
tatgagtaaa cttggtctga cagttaccaa tgcttaatca gtgaggcacc tatctcagcg 3811
atctgtctat ttcgttcatc catagttgcc tgactccccg tcgtgtagat aactacgata 3871
cgggagggct taccatctgg ccccagtgct gcaatgatac cgcgagaccc acgctcaccg 3931
gctccagatt tatcagcaat aaaccagcca gccggaaggg ccgagcgcag aagtggtcct 3991
gcaactttat ccgcctccat ccagtctatt aattgttgcc gggaagctag agtaagtagt 4051
tcgccagtta atagtttgcg caacgttgtt gccattgcta caggcatcgt ggtgtcacgc 4111
tcgtcgtttg gtatggcttc attcagctcc ggttcccaac gatcaaggcg agttacatga 4171
tcccccatgt tgtgcaaaaa agcggttagc tccttcggtc ctccgatcgt tgtcagaagt 4231
aagttggccg cagtgttatc actcatggtt atggcagcac tgcataattc tcttactgtc 4291
atgccatccg taagatgctt ttctgtgact ggtgagtact caaccaagtc attctgagaa 4351
tagtgtatgc ggcgaccgag ttgctcttgc ccggcgtcaa cacgggataa taccgcgcca 4411
catagcagaa ctttaaaagt gctcatcatt ggaaaacgtt cttcggggcg aaaactctca 4471
aggatcttac cgctgttgag atccagttcg atgtaaccca ctcgtgcacc caactgatct 4531
tcagcatctt ttactttcac cagcgtttct gggtgagcaa aaacaggaag gcaaaatgcc 4591
gcaaaaaagg gaataagggc gacacggaaa tgttgaatac tcatactctt cctttttcaa 4651
tattattgaa gcatttatca gggttattgt ctcatgagcg gatacatatt tgaatgtatt 4711
tagaaaaata aacaaaagag tttgtagaaa cgcaaaaagg ccatccgtca ggatggcctt 4771
ctgcttaatt tgatgcctgg cagtttatgg cgggcgtcct gcccgccacc ctccgggccg 4831
ttgcttcgca acgttcaaat ccgctcccgg cggatttgtc ctactcagga gagcgttcac 4891
cgacaaacaa cagataaaac gaaaggccca gtctttcgac tgagcctttc gttttatttg 4951
atgcctggca gttccctact ctcgcatggg gagaccccac actaccatcg gcgctacggc 5011
gtttcacttc tgagttcggc atggggtcag gtgggaccac cgcgctactg ccgccaggca 5071
aattctgttt tatcagaccg cttctgcgtt ctgatttaat ctgtatcagg ctgaaaatct 5131
tctctcatcc gccaaaacag ccaagctagc ggccgatc 5169

<210> 29
<211> 757
<212> PRT
<213> Artificial Sequence
<223> Description of Artificial Sequence: pHL3103

<400> 29
Met Asp Val Asn Pro Thr Leu Leu Phe Leu Lys Val Pro Ala Gln Asn
1 5 10 15

Ala Ile Ser Thr Thr Phe Pro Tyr Thr Gly Asp Pro Pro Tyr Ser His
20 25 30

Gly Thr Gly Thr Gly Tyr Thr Met Asp Thr Val Asn Arg Thr His Gln
35 40 45

Tyr Ser Glu Lys Gly Lys Trp Thr Thr Asn Thr Glu Thr Gly Ala Pro
50 55 60

Gln Leu Asn Pro Ile Asp Gly Pro Leu Pro Glu Asp Asn Glu Pro Ser
65 70 75 80

Gly Tyr Ala Gln Thr Asp Cys Val Leu Glu Ala Met Ala Phe Leu Glu
85 90 95

Glu Ser His Pro Gly Ile Phe Glu Asn Ser Cys Leu Glu Thr Met Glu
100 105 110

Val Val Gln Gln Thr Arg Val Asp Lys Leu Thr Gln Gly Arg Gln Thr
115 120 125

Tyr Asp Trp Thr Leu Asn Arg Asn Gln Pro Ala Ala Thr Ala Leu Ala
130 135 140

Asn Thr Ile Glu Val Phe Arg Ser Asn Gly Leu Thr Ala Asn Glu Ser
145 150 155 160

Gly Arg Leu Ile Asp Phe Leu Lys Asp Val Met Glu Ser Met Asp Lys
165 170 175

Glu Glu Met Glu Ile Thr Thr His Phe Gln Arg Lys Arg Arg Val Arg
180 185 190

Asp Asn Met Thr Lys Lys Met Val Thr Gln Arg Thr Ile Gly Lys Lys
195 200 205

Lys Gln Arg Leu Asn Lys Arg Ser Tyr Leu Ile Arg Ala Leu Thr Leu
210 215 220

Asn Thr Met Thr Lys Asp Ala Glu Arg Gly Lys Leu Lys Arg Arg Ala
225 230 235 240

Ile Ala Thr Pro Gly Met Gln Ile Arg Gly Phe Val Tyr Phe Val Glu
245 250 255

Thr Leu Ala Arg Ser Ile Cys Glu Lys Leu Glu Gln Ser Gly Leu Pro
260 265 270

Val Gly Gly Asn Glu Lys Lys Ala Lys Leu Ala Asn Val Val Arg Lys
275 280 285

Met Met Thr Asn Ser Gln Asp Thr Glu Leu Ser Phe Thr Ile Thr Gly
290 295 300

Asp Asn Thr Lys Trp Asn Glu Asn Gln Asn Pro Arg Met Phe Leu Ala
305 310 315 320

Met Ile Thr Tyr Ile Thr Arg Asn Gln Pro Glu Trp Phe Arg Asn Val
325 330 335

Leu Ser Ile Ala Pro Ile Met Phe Ser Asn Lys Met Ala Arg Leu Gly
340 345 350

Lys Gly Tyr Met Phe Glu Ser Lys Ser Met Lys Leu Arg Thr Gln Ile
355 360 365

Pro Ala Glu Met Leu Ala Ser Ile Asp Leu Lys Tyr Phe Asn Glu Pro
370 375 380

Thr Arg Lys Lys Ile Glu Lys Ile Arg Pro Leu Ile Ile Asp Gly Thr
385 390 395 400

Ala Ser Leu Ser Pro Gly Met Met Met Gly Met Phe Asn Met Leu Ser
405 410 415

Thr Val Leu Gly Val Ser Ile Leu Asn Leu Gly Gln Lys Arg Tyr Thr
420 425 430

Lys Thr Thr Tyr Trp Trp Asp Gly Leu Gln Ser Ser Asp Asp Phe Ala
435 440 445

Leu Ile Val Asn Ala Pro Asn His Glu Gly Ile Gln Ala Gly Val Asp
450 455 460

Arg Phe Tyr Arg Thr Cys Lys Leu Val Gly Ile Asn Met Ser Lys Lys
465 470 475 480

Lys Ser Tyr Ile Asn Arg Thr Gly Thr Phe Glu Phe Thr Ser Phe Phe
485 490 495

Tyr Arg Tyr Gly Phe Val Ala Asn Phe Ser Met Glu Leu Pro Ser Phe
500 505 510

Gly Val Ser Gly Ile Asn Glu Ser Ala Asp Met Ser Ile Gly Val Thr
515 520 525

Val Ile Lys Asn Asn Met Ile Asn Asn Asp Leu Gly Pro Ala Thr Ala
530 535 540

Gln Met Ala Leu Gln Leu Phe Ile Lys Asp Tyr Arg Tyr Thr Tyr Arg
545 550 555 560

Cys His Arg Gly Asp Thr Gln Ile Gln Thr Arg Arg Ser Phe Glu Ile
565 570 575

Lys Lys Leu Trp Glu Gln Thr His Ser Lys Ala Gly Leu Leu Val Ser
580 585 590

Asp Gly Gly Pro Asn Leu Tyr Asn Ile Arg Asn Leu His Ile Pro Glu
595 600 605

Val Cys Leu Lys Trp Glu Leu Met Asp Glu Asp Tyr Gln Gly Arg Leu
610 615 620

Cys Asn Pro Leu Asn Pro Phe Val Asn His Lys Asp Ile Glu Ser Val
625 630 635 640

Asn Asn Ala Val Ile Met Pro Ala His Gly Pro Ala Lys Asn Met Glu
645 650 655

Tyr Asp Ala Val Ala Thr Thr His Ser Trp Ile Pro Lys Arg Asn Arg
660 665 670

Ser Ile Leu Asn Thr Ser Gln Arg Gly Ile Leu Glu Asp Glu Gln Met
675 680 685

Tyr Gln Lys Cys Cys Asn Leu Phe Glu Lys Phe Phe Pro Ser Ser Ser
690 695 700

Tyr Arg Arg Pro Val Gly Ile Ser Ser Met Val Glu Ala Met Val Ser
705 710 715 720

Arg Ala Arg Ile Asp Ala Arg Ile Asp Phe Glu Ser Gly Arg Ile Lys
725 730 735

Lys Glu Glu Phe Thr Glu Ile Met Lys Ile Cys Ser Thr Ile Glu Glu
740 745 750

```
Leu Arg Arg Gln Lys
        755


<210> 30
<211> 5169
<212> DNA
<213> Artificial Sequence

<220>
<223> Description of Artificial Sequence: pHL3130

<220>
<221> CDS
<222> (191)..(2461)

<400> 30
cccaaaaaaa aaaaaaaaaa aagagtccag agtggccccg ccgctccgcg ccgggggggg 60

gggggggggg ggacactttc ggacatctgg tcgacctcca gcatcggggg aaaaaaaaaa 120

acaaagtgtc gcccggagta ctggtcgacc tccgaagttg ggggggagcg aaagcaggca 180

aaccatttga atg gat gtc aat ccg act tta ctt ttc tta aaa gtg cca    229
           Met Asp Val Asn Pro Thr Leu Leu Phe Leu Lys Val Pro
           1               5                   10

gca caa aat gct ata agc aca act ttc cct tat act gga gac cct cct    277
Ala Gln Asn Ala Ile Ser Thr Thr Phe Pro Tyr Thr Gly Asp Pro Pro
    15                  20                  25

tac agc cat ggg aca gga aca gga tac acc atg gat act gtc aac agg    325
Tyr Ser His Gly Thr Gly Thr Gly Tyr Thr Met Asp Thr Val Asn Arg
30                  35                  40                  45

aca cat cag tac tca gaa agg gga aga tgg aca aca aac acc gaa act    373
Thr His Gln Tyr Ser Glu Arg Gly Arg Trp Thr Thr Asn Thr Glu Thr
                50                  55                  60

gga gca ccg caa ctc aac ccg att gat ggg cca ctg cca gaa gac aat    421
Gly Ala Pro Gln Leu Asn Pro Ile Asp Gly Pro Leu Pro Glu Asp Asn
            65                  70                  75

gaa cca agt ggt tat gcc caa aca gat tgt gta ttg gaa gca atg gcc    469
Glu Pro Ser Gly Tyr Ala Gln Thr Asp Cys Val Leu Glu Ala Met Ala
        80                  85                  90

ttc ctt gag gaa tcc cat cct ggt atc ttt gag acc tcg tgt ctt gaa    517
Phe Leu Glu Glu Ser His Pro Gly Ile Phe Glu Thr Ser Cys Leu Glu
    95                  100                 105

acg atg gag gtt gtt cag caa aca cga gtg gac aag ctg aca caa ggc    565
Thr Met Glu Val Val Gln Gln Thr Arg Val Asp Lys Leu Thr Gln Gly
110                 115                 120                 125

cga cag acc tat gac tgg act cta aat agg aac cag cct gct gca aca    613
Arg Gln Thr Tyr Asp Trp Thr Leu Asn Arg Asn Gln Pro Ala Ala Thr
                130                 135                 140

gca ttg gcc aac aca ata gaa gtg ttc aga tca aat ggc ctc acg gcc    661
Ala Leu Ala Asn Thr Ile Glu Val Phe Arg Ser Asn Gly Leu Thr Ala
            145                 150                 155

aat gaa tct gga agg ctc ata gac ttc ctt aag gat gta atg gag tca    709
Asn Glu Ser Gly Arg Leu Ile Asp Phe Leu Lys Asp Val Met Glu Ser
        160                 165                 170
```

```
atg aac aaa gaa gaa atg gag atc aca act cat ttt cag aga aag aga      757
Met Asn Lys Glu Glu Met Glu Ile Thr Thr His Phe Gln Arg Lys Arg
    175                 180                 185

cga gtg aga gac aat atg act aag aaa atg gtg aca cag aga aca ata      805
Arg Val Arg Asp Asn Met Thr Lys Lys Met Val Thr Gln Arg Thr Ile
190                 195                 200                 205

ggt aaa agg aag cag aga ttg aac aaa agg agt tat cta att agg gca      853
Gly Lys Arg Lys Gln Arg Leu Asn Lys Arg Ser Tyr Leu Ile Arg Ala
                210                 215                 220

tta acc ctg aac aca atg acc aaa gat gct gag aga ggg aag cta aaa      901
Leu Thr Leu Asn Thr Met Thr Lys Asp Ala Glu Arg Gly Lys Leu Lys
            225                 230                 235

cgg aga gca att gca aca cca ggg atg cag atc aga ggg ttt gtg tac      949
Arg Arg Ala Ile Ala Thr Pro Gly Met Gln Ile Arg Gly Phe Val Tyr
        240                 245                 250

ttt gtt gag aca ctg gcg aga agc att tgc gag aag ctt gaa cag tct      997
Phe Val Glu Thr Leu Ala Arg Ser Ile Cys Glu Lys Leu Glu Gln Ser
    255                 260                 265

ggg cta cca gtt gga ggg aat gag aag aaa gct aaa ttg gca aat gtc      1045
Gly Leu Pro Val Gly Gly Asn Glu Lys Lys Ala Lys Leu Ala Asn Val
270                 275                 280                 285

gtg agg aag atg atg acg aac tca caa gac act gag ctc tct ttc aca      1093
Val Arg Lys Met Met Thr Asn Ser Gln Asp Thr Glu Leu Ser Phe Thr
                290                 295                 300

atc acc gga gac aat acc aaa tgg aat gag aac caa aac ccc cga atg      1141
Ile Thr Gly Asp Asn Thr Lys Trp Asn Glu Asn Gln Asn Pro Arg Met
            305                 310                 315

ttc ctg gca atg ata aca tac atc aca aga aac caa cct gag tgg ttt      1189
Phe Leu Ala Met Ile Thr Tyr Ile Thr Arg Asn Gln Pro Glu Trp Phe
        320                 325                 330

aga aat gtc ttg agc atc gcg ccg ata atg ttt tcg aac aaa atg gcg      1237
Arg Asn Val Leu Ser Ile Ala Pro Ile Met Phe Ser Asn Lys Met Ala
    335                 340                 345

agg cta ggg aaa ggg tac atg ttc gaa agc aaa agc atg aag ctc cga      1285
Arg Leu Gly Lys Gly Tyr Met Phe Glu Ser Lys Ser Met Lys Leu Arg
350                 355                 360                 365

aca caa ata cca gca gaa atg cta gca agt att gat cta aaa tat ttc      1333
Thr Gln Ile Pro Ala Glu Met Leu Ala Ser Ile Asp Leu Lys Tyr Phe
                370                 375                 380

aat gaa cca aca agg aag aaa atc gag aaa ata agg cct ctc ata ata      1381
Asn Glu Pro Thr Arg Lys Lys Ile Glu Lys Ile Arg Pro Leu Ile Ile
            385                 390                 395

gac ggc aca gcc tca tta agc ccg gga atg atg atg ggt atg ttc aac      1429
Asp Gly Thr Ala Ser Leu Ser Pro Gly Met Met Met Gly Met Phe Asn
        400                 405                 410

atg ctg agt aca gtg ttg gga gtc tca atc ctg aat ctt ggg caa aag      1477
Met Leu Ser Thr Val Leu Gly Val Ser Ile Leu Asn Leu Gly Gln Lys
    415                 420                 425

aga tac acc aaa acc aca tac tgg tgg gat gga ctt cag tcc tct gat      1525
Arg Tyr Thr Lys Thr Thr Tyr Trp Trp Asp Gly Leu Gln Ser Ser Asp
430                 435                 440                 445
```

```
gat ttt gct ctc atc gtg aat gca cca aat cat gag gga ata caa gcg    1573
Asp Phe Ala Leu Ile Val Asn Ala Pro Asn His Glu Gly Ile Gln Ala
                450                 455                 460

gga gtg gat aga ttc tac aga acc tgc aag cta gtt ggg atc aat atg    1621
Gly Val Asp Arg Phe Tyr Arg Thr Cys Lys Leu Val Gly Ile Asn Met
            465                 470                 475

agc aag aaa aag tcc tat ata aat agg aca gga aca ttc gaa ttc aca    1669
Ser Lys Lys Lys Ser Tyr Ile Asn Arg Thr Gly Thr Phe Glu Phe Thr
        480                 485                 490

agc ttt ttc tac cgc tat gga ttt gta gcc aat ttt agt atg gag ttg    1717
Ser Phe Phe Tyr Arg Tyr Gly Phe Val Ala Asn Phe Ser Met Glu Leu
    495                 500                 505

ccc agc ttt gga gta tca gga att aat gaa tcg gct gat atg agc att    1765
Pro Ser Phe Gly Val Ser Gly Ile Asn Glu Ser Ala Asp Met Ser Ile
510                 515                 520                 525

gga gta aca gtg ata aag aat aac atg ata aac aat gat ctt gga ccg    1813
Gly Val Thr Val Ile Lys Asn Asn Met Ile Asn Asn Asp Leu Gly Pro
                530                 535                 540

gca aca gcc caa atg gct ctc caa tta ttc atc aag gac tac aga tat    1861
Ala Thr Ala Gln Met Ala Leu Gln Leu Phe Ile Lys Asp Tyr Arg Tyr
            545                 550                 555

aca tac agg tgt cac agg gga gac aca caa atc caa acg agg agg tca    1909
Thr Tyr Arg Cys His Arg Gly Asp Thr Gln Ile Gln Thr Arg Arg Ser
        560                 565                 570

ttc gag cta aag aag ctg tgg gag cag acc cgc tca aag gca gga ctg    1957
Phe Glu Leu Lys Lys Leu Trp Glu Gln Thr Arg Ser Lys Ala Gly Leu
    575                 580                 585

ttg gtt tca gat ggc gga cca aac ctg tac aac att cgg aat ctc cac    2005
Leu Val Ser Asp Gly Gly Pro Asn Leu Tyr Asn Ile Arg Asn Leu His
590                 595                 600                 605

atc ccg gaa gtt tgc ctg aaa tgg gaa cta atg gat gaa gac tat cag    2053
Ile Pro Glu Val Cys Leu Lys Trp Glu Leu Met Asp Glu Asp Tyr Gln
                610                 615                 620

gga aga ctt tgt aat ccc atg aac ccg ttt gtc agt cat aag gaa att    2101
Gly Arg Leu Cys Asn Pro Met Asn Pro Phe Val Ser His Lys Glu Ile
            625                 630                 635

gaa tct gta aac aat gct gcg gta atg cca gcc cat ggt cca gcc aaa    2149
Glu Ser Val Asn Asn Ala Ala Val Met Pro Ala His Gly Pro Ala Lys
        640                 645                 650

agc atg gaa tat gat gct gtg gca act aca cac tct tgg atc cct aag    2197
Ser Met Glu Tyr Asp Ala Val Ala Thr Thr His Ser Trp Ile Pro Lys
    655                 660                 665

aga aac cgt tcc att ctc aat acg agt caa agg gga atc ctt gag gat    2245
Arg Asn Arg Ser Ile Leu Asn Thr Ser Gln Arg Gly Ile Leu Glu Asp
670                 675                 680                 685

gaa caa atg tac cag aag tgt tgc aac cta ttc gag aaa ttc ttc cct    2293
Glu Gln Met Tyr Gln Lys Cys Cys Asn Leu Phe Glu Lys Phe Phe Pro
                690                 695                 700
```

```
agc agc tca tac aga aga cca gtt gga att tcc agt atg gtg gag gcc    2341
Ser Ser Ser Tyr Arg Arg Pro Val Gly Ile Ser Ser Met Val Glu Ala
            705                 710                 715

atg gtg tct agg gcc cgg att gat gca cga att gac ttc gag tct gga    2389
Met Val Ser Arg Ala Arg Ile Asp Ala Arg Ile Asp Phe Glu Ser Gly
        720                 725                 730

agg att aag aag gaa gag ttt gct gag atc atg aag atc tgt tcc acc    2437
Arg Ile Lys Lys Glu Glu Phe Ala Glu Ile Met Lys Ile Cys Ser Thr
    735                 740                 745

att gaa gag ctc aga cgg caa aaa tagtgaattt agcttgtcct tcatgaaaaa  2491
Ile Glu Glu Leu Arg Arg Gln Lys
750                 755

atgccttgtt tctactaata acccggcggc ccaaaatgcc gactcggagc gaaagatata 2551

cctcccccgg ggccgggagg tcgcgtcacc gaccacgccg ccggcccagg cgacgcgcga 2611

cacggacacc tgtccccaaa aacgccacca tcgcagccac acacggagcg cccggggccc 2671

tctggtcaac cccaggacac acgcgggagc agcgccgggc cggggacgcc ctcccggccg 2731

cccgtgccac acgcaggggg ccggcccgtg tctccagagc gggagccgga agcattttcg 2791

gccggcccct cctacgaccg ggacacacga gggaccgaag gccggccagg cgcgacctct 2851

cgggccgcac gcgcgctcag ggagcgctct ccgactccgc acggggactc gccagaaagg 2911

atcgtgacct gcattaatga atcaggggat aacgcaggaa agaacatgtg agcaaaaggc 2971

cagcaaaagg ccaggaaccg taaaaaggcc gcgttgctgg cgtttttcca taggctccgc 3031

cccccctgacg agcatcacaa aaatcgacgc tcaagtcaga ggtggcgaaa cccgacagga 3091

ctataaagat accaggcgtt tccccctgga agctccctcg tgcgctctcc tgttccgacc 3151

ctgccgctta ccggatacct gtccgccttt ctcccttcgg gaagcgtggc gctttctcat 3211

agctcacgct gtaggtatct cagttcggtg taggtcgttc gctccaagct gggctgtgtg 3271

cacgaacccc ccgttcagcc cgaccgctgc gccttatccg gtaactatcg tcttgagtcc 3331

aacccggtaa gacacgactt atcgccactg gcagcagcca ctggtaacag gattagcaga 3391

gcgaggtatg taggcggtgc tacagagttc ttgaagtggt ggcctaacta cggctacact 3451

agaaggacag tatttggtat ctgcgctctg ctgaagccag ttaccttcgg aaaaagagtt 3511

ggtagctctt gatccggcaa acaaaccacc gctggtagcg gtggtttttt tgtttgcaag 3571

cagcagatta cgcgcagaaa aaaaggatct caagaagatc ctttgatctt ttctacgggg 3631

tctgacgctc agtggaacga aaactcacgt taagggattt tggtcatgag attatcaaaa 3691

aggatcttca cctagatcct tttaaattaa aaatgaagtt ttaaatcaat ctaaagtata 3751

tatgagtaaa cttggtctga cagttaccaa tgcttaatca gtgaggcacc tatctcagcg 3811

atctgtctat ttcgttcatc catagttgcc tgactccccg tcgtgtagat aactacgata 3871

cgggagggct taccatctgg ccccagtgct gcaatgatac cgcgagaccc acgctcaccg 3931

gctccagatt tatcagcaat aaaccagcca gccggaaggg ccgagcgcag aagtggtcct 3991

gcaactttat ccgcctccat ccagtctatt aattgttgcc gggaagctag agtaagtagt 4051
```

tcgccagtta atagtttgcg caacgttgtt gccattgcta caggcatcgt ggtgtcacgc 4111
tcgtcgtttg gtatggcttc attcagctcc ggttcccaac gatcaaggcg agttacatga 4171
tcccccatgt tgtgcaaaaa agcggttagc tccttcggtc ctccgatcgt tgtcagaagt 4231
aagttggccg cagtgttatc actcatggtt atggcagcac tgcataattc tcttactgtc 4291
atgccatccg taagatgctt ttctgtgact ggtgagtact caaccaagtc attctgagaa 4351
tagtgtatgc ggcgaccgag ttgctcttgc ccggcgtcaa cacgggataa taccgcgcca 4411
catagcagaa ctttaaaagt gctcatcatt ggaaaacgtt cttcggggcg aaaactctca 4471
aggatcttac cgctgttgag atccagttcg atgtaaccca ctcgtgcacc caactgatct 4531
tcagcatctt ttactttcac cagcgtttct gggtgagcaa aaacaggaag gcaaaatgcc 4591
gcaaaaaagg gaataagggc gacacggaaa tgttgaatac tcatactctt cctttttcaa 4651
tattattgaa gcatttatca gggttattgt ctcatgagcg gatacatatt tgaatgtatt 4711
tagaaaaata aacaaaagag tttgtagaaa cgcaaaaagg ccatccgtca ggatggcctt 4771
ctgcttaatt tgatgcctgg cagtttatgg cgggcgtcct gcccgccacc ctccgggccg 4831
ttgcttcgca acgttcaaat ccgctcccgg cggatttgtc ctactcagga gagcgttcac 4891
cgacaaacaa cagataaaac gaaaggccca gtctttcgac tgagcctttc gttttatttg 4951
atgcctggca gttccctact ctcgcatggg gagaccccac actaccatcg gcgctacggc 5011
gtttcacttc tgagttcggc atggggtcag gtgggaccac cgcgctactg ccgccaggca 5071
aattctgttt tatcagaccg cttctgcgtt ctgatttaat ctgtatcagg ctgaaaatct 5131
tctctcatcc gccaaaacag ccaagctagc ggccgatc 5169

<210> 31
<211> 757
<212> PRT
<213> Artificial Sequence
<223> Description of Artificial Sequence: pHL3130

<400> 31
Met Asp Val Asn Pro Thr Leu Leu Phe Leu Lys Val Pro Ala Gln Asn
1 5 10 15

Ala Ile Ser Thr Thr Phe Pro Tyr Thr Gly Asp Pro Pro Tyr Ser His
20 25 30

Gly Thr Gly Thr Gly Tyr Thr Met Asp Thr Val Asn Arg Thr His Gln
35 40 45

Tyr Ser Glu Arg Gly Arg Trp Thr Thr Asn Thr Glu Thr Gly Ala Pro
50 55 60

Gln Leu Asn Pro Ile Asp Gly Pro Leu Pro Glu Asp Asn Glu Pro Ser
65 70 75 80

Gly Tyr Ala Gln Thr Asp Cys Val Leu Glu Ala Met Ala Phe Leu Glu
85 90 95

Glu Ser His Pro Gly Ile Phe Glu Thr Ser Cys Leu Glu Thr Met Glu
100 105 110

```
Val Val Gln Gln Thr Arg Val Asp Lys Leu Thr Gln Gly Arg Gln Thr
        115                 120                 125

Tyr Asp Trp Thr Leu Asn Arg Asn Gln Pro Ala Ala Thr Ala Leu Ala
    130                 135                 140

Asn Thr Ile Glu Val Phe Arg Ser Asn Gly Leu Thr Ala Asn Glu Ser
145                 150                 155                 160

Gly Arg Leu Ile Asp Phe Leu Lys Asp Val Met Glu Ser Met Asn Lys
                165                 170                 175

Glu Glu Met Glu Ile Thr Thr His Phe Gln Arg Lys Arg Arg Val Arg
            180                 185                 190

Asp Asn Met Thr Lys Lys Met Val Thr Gln Arg Thr Ile Gly Lys Arg
        195                 200                 205

Lys Gln Arg Leu Asn Lys Arg Ser Tyr Leu Ile Arg Ala Leu Thr Leu
    210                 215                 220

Asn Thr Met Thr Lys Asp Ala Glu Arg Gly Lys Leu Lys Arg Arg Ala
225                 230                 235                 240

Ile Ala Thr Pro Gly Met Gln Ile Arg Gly Phe Val Tyr Phe Val Glu
                245                 250                 255

Thr Leu Ala Arg Ser Ile Cys Glu Lys Leu Glu Gln Ser Gly Leu Pro
            260                 265                 270

Val Gly Gly Asn Glu Lys Lys Ala Lys Leu Ala Asn Val Val Arg Lys
        275                 280                 285

Met Met Thr Asn Ser Gln Asp Thr Glu Leu Ser Phe Thr Ile Thr Gly
    290                 295                 300

Asp Asn Thr Lys Trp Asn Glu Asn Gln Asn Pro Arg Met Phe Leu Ala
305                 310                 315                 320

Met Ile Thr Tyr Ile Thr Arg Asn Gln Pro Glu Trp Phe Arg Asn Val
                325                 330                 335

Leu Ser Ile Ala Pro Ile Met Phe Ser Asn Lys Met Ala Arg Leu Gly
            340                 345                 350

Lys Gly Tyr Met Phe Glu Ser Lys Ser Met Lys Leu Arg Thr Gln Ile
        355                 360                 365

Pro Ala Glu Met Leu Ala Ser Ile Asp Leu Lys Tyr Phe Asn Glu Pro
    370                 375                 380

Thr Arg Lys Lys Ile Glu Lys Ile Arg Pro Leu Ile Ile Asp Gly Thr
385                 390                 395                 400

Ala Ser Leu Ser Pro Gly Met Met Met Gly Met Phe Asn Met Leu Ser
                405                 410                 415

Thr Val Leu Gly Val Ser Ile Leu Asn Leu Gly Gln Lys Arg Tyr Thr
            420                 425                 430

Lys Thr Thr Tyr Trp Trp Asp Gly Leu Gln Ser Ser Asp Asp Phe Ala
        435                 440                 445

Leu Ile Val Asn Ala Pro Asn His Glu Gly Ile Gln Ala Gly Val Asp
    450                 455                 460
```

```
Arg Phe Tyr Arg Thr Cys Lys Leu Val Gly Ile Asn Met Ser Lys Lys
465                 470                 475                 480

Lys Ser Tyr Ile Asn Arg Thr Gly Thr Phe Glu Phe Thr Ser Phe Phe
                485                 490                 495

Tyr Arg Tyr Gly Phe Val Ala Asn Phe Ser Met Glu Leu Pro Ser Phe
            500                 505                 510

Gly Val Ser Gly Ile Asn Glu Ser Ala Asp Met Ser Ile Gly Val Thr
        515                 520                 525

Val Ile Lys Asn Asn Met Ile Asn Asn Asp Leu Gly Pro Ala Thr Ala
    530                 535                 540

Gln Met Ala Leu Gln Leu Phe Ile Lys Asp Tyr Arg Tyr Thr Tyr Arg
545                 550                 555                 560

Cys His Arg Gly Asp Thr Gln Ile Gln Thr Arg Arg Ser Phe Glu Leu
                565                 570                 575

Lys Lys Leu Trp Glu Gln Thr Arg Ser Lys Ala Gly Leu Leu Val Ser
            580                 585                 590

Asp Gly Gly Pro Asn Leu Tyr Asn Ile Arg Asn Leu His Ile Pro Glu
        595                 600                 605

Val Cys Leu Lys Trp Glu Leu Met Asp Glu Asp Tyr Gln Gly Arg Leu
    610                 615                 620

Cys Asn Pro Met Asn Pro Phe Val Ser His Lys Glu Ile Glu Ser Val
625                 630                 635                 640

Asn Asn Ala Ala Val Met Pro Ala His Gly Pro Ala Lys Ser Met Glu
                645                 650                 655

Tyr Asp Ala Val Ala Thr Thr His Ser Trp Ile Pro Lys Arg Asn Arg
            660                 665                 670

Ser Ile Leu Asn Thr Ser Gln Arg Gly Ile Leu Glu Asp Glu Gln Met
        675                 680                 685

Tyr Gln Lys Cys Cys Asn Leu Phe Glu Lys Phe Phe Pro Ser Ser Ser
    690                 695                 700

Tyr Arg Arg Pro Val Gly Ile Ser Ser Met Val Glu Ala Met Val Ser
705                 710                 715                 720

Arg Ala Arg Ile Asp Ala Arg Ile Asp Phe Glu Ser Gly Arg Ile Lys
                725                 730                 735

Lys Glu Glu Phe Ala Glu Ile Met Lys Ile Cys Ser Thr Ile Glu Glu
            740                 745                 750

Leu Arg Arg Gln Lys
        755
```

<210> 32
<211> 5169
<212> DNA
<213> Artificial Sequence

<220>
<223> Description of Artificial Sequence: pHL3131

<220>

<221> CDS
<222> (191)..(2461)

<400> 32
```
cccaaaaaaa aaaaaaaaaa aagagtccag agtggccccg ccgctccgcg ccgggggggg 60

gggggggggg ggacactttc ggacatctgg tcgacctcca gcatcggggg aaaaaaaaaa 120

acaaagtgtc gcccggagta ctggtcgacc tccgaagttg ggggggagcg aaagcaggca 180

aaccatttga atg gat gtc aat ccg act tta ctt ttc tta aaa gtg cca     229
           Met Asp Val Asn Pro Thr Leu Leu Phe Leu Lys Val Pro
           1               5                   10

gca caa aat gct ata agc aca act ttc cct tat act gga gac cct cct     277
Ala Gln Asn Ala Ile Ser Thr Thr Phe Pro Tyr Thr Gly Asp Pro Pro
    15                  20                  25

tac agc cat ggg aca gga aca gga tac acc atg gat act gtc aac agg     325
Tyr Ser His Gly Thr Gly Thr Gly Tyr Thr Met Asp Thr Val Asn Arg
30                  35                  40                  45

aca cat cag tac tca gaa agg gga aga tgg aca aca aac acc gaa act     373
Thr His Gln Tyr Ser Glu Arg Gly Arg Trp Thr Thr Asn Thr Glu Thr
                50                  55                  60

gga gca ccg caa ctc aac ccg att gat ggg cca ctg cca gaa gac aat     421
Gly Ala Pro Gln Leu Asn Pro Ile Asp Gly Pro Leu Pro Glu Asp Asn
            65                  70                  75

gaa cca agt ggt tat gcc caa aca gat tgt gta ttg gaa gca atg gcc     469
Glu Pro Ser Gly Tyr Ala Gln Thr Asp Cys Val Leu Glu Ala Met Ala
        80                  85                  90

ttc ctt gag gaa tcc cat cct ggt atc ttt gag acc tcg tgt ctt gaa     517
Phe Leu Glu Glu Ser His Pro Gly Ile Phe Glu Thr Ser Cys Leu Glu
    95                  100                 105

acg atg gag gtt gtt cag caa aca cga gtg gac aag ctg aca caa ggc     565
Thr Met Glu Val Val Gln Gln Thr Arg Val Asp Lys Leu Thr Gln Gly
110                 115                 120                 125

cga cag acc tat gac tgg act cta aat agg aac cag cct gct gca aca     613
Arg Gln Thr Tyr Asp Trp Thr Leu Asn Arg Asn Gln Pro Ala Ala Thr
                130                 135                 140

gca ttg gcc aac aca ata gaa gtg ttc aga tca aat ggc ctc acg gcc     661
Ala Leu Ala Asn Thr Ile Glu Val Phe Arg Ser Asn Gly Leu Thr Ala
            145                 150                 155

aat gaa tct gga agg ctc ata gac ttc ctt aag gat gta atg gag tca     709
Asn Glu Ser Gly Arg Leu Ile Asp Phe Leu Lys Asp Val Met Glu Ser
        160                 165                 170

atg aac aaa gaa gaa atg gag atc aca act cat ttt cag aga aag aga     757
Met Asn Lys Glu Glu Met Glu Ile Thr Thr His Phe Gln Arg Lys Arg
    175                 180                 185

cga gtg aga gac aat atg act aag aaa atg gtg aca cag aga aca ata     805
Arg Val Arg Asp Asn Met Thr Lys Lys Met Val Thr Gln Arg Thr Ile
190                 195                 200                 205

ggt aaa agg aag cag aga ttg aac aaa agg agt tat cta att agg gca     853
Gly Lys Arg Lys Gln Arg Leu Asn Lys Arg Ser Tyr Leu Ile Arg Ala
                210                 215                 220

tta acc ctg aac aca atg acc aaa gat gct gag aga ggg aag cta aaa     901
```

```
Leu Thr Leu Asn Thr Met Thr Lys Asp Ala Glu Arg Gly Lys Leu Lys
            225                 230                 235

cgg aga gca att gca aca cca ggg atg cag atc aga ggg ttt gtg tac     949
Arg Arg Ala Ile Ala Thr Pro Gly Met Gln Ile Arg Gly Phe Val Tyr
        240                 245                 250

ttt gtt gag aca ctg gcg aga agc att tgc gag aag ctt gaa cag tct     997
Phe Val Glu Thr Leu Ala Arg Ser Ile Cys Glu Lys Leu Glu Gln Ser
    255                 260                 265

ggg cta cca gtt gga ggg aat gag aag aaa gct aaa ttg gca aat gtc    1045
Gly Leu Pro Val Gly Gly Asn Glu Lys Lys Ala Lys Leu Ala Asn Val
270                 275                 280                 285

gtg agg aag atg atg acg aac tca caa gac act gag ctc tct ttc aca    1093
Val Arg Lys Met Met Thr Asn Ser Gln Asp Thr Glu Leu Ser Phe Thr
                290                 295                 300

atc acc gga gac aat acc aaa tgg aat gag aac caa aac ccc cga atg    1141
Ile Thr Gly Asp Asn Thr Lys Trp Asn Glu Asn Gln Asn Pro Arg Met
            305                 310                 315

ttc ctg gca atg ata aca tac atc aca aga aac caa cct gag tgg ttt    1189
Phe Leu Ala Met Ile Thr Tyr Ile Thr Arg Asn Gln Pro Glu Trp Phe
        320                 325                 330

aga aat gtc ttg agc atc gcg ccg ata atg ttt tcg aac aaa atg gcg    1237
Arg Asn Val Leu Ser Ile Ala Pro Ile Met Phe Ser Asn Lys Met Ala
    335                 340                 345

agg cta ggg aaa ggg tac atg ttc gaa agc aaa agc atg aag ctc cga    1285
Arg Leu Gly Lys Gly Tyr Met Phe Glu Ser Lys Ser Met Lys Leu Arg
350                 355                 360                 365

aca caa ata cca gca gaa atg cta gca agt att gat cta aaa tat ttc    1333
Thr Gln Ile Pro Ala Glu Met Leu Ala Ser Ile Asp Leu Lys Tyr Phe
                370                 375                 380

aat gaa cca aca agg aag aaa atc gag aaa ata agg cct ctc ata ata    1381
Asn Glu Pro Thr Arg Lys Lys Ile Glu Lys Ile Arg Pro Leu Ile Ile
            385                 390                 395

gac ggc aca gcc tca tta agc ccg gga atg atg atg ggt atg ttc aac    1429
Asp Gly Thr Ala Ser Leu Ser Pro Gly Met Met Met Gly Met Phe Asn
        400                 405                 410

atg ctg agt aca gtg ttg gga gtc tca atc ctg aat ctt ggg caa aag    1477
Met Leu Ser Thr Val Leu Gly Val Ser Ile Leu Asn Leu Gly Gln Lys
    415                 420                 425

aga tac acc aaa acc aca tac tgg tgg gat gga ctt cag tcc tct gat    1525
Arg Tyr Thr Lys Thr Thr Tyr Trp Trp Asp Gly Leu Gln Ser Ser Asp
430                 435                 440                 445

gat ttt gct ctc atc gtg aat gca cca aat cat gag gga ata caa gcg    1573
Asp Phe Ala Leu Ile Val Asn Ala Pro Asn His Glu Gly Ile Gln Ala
                450                 455                 460

gga gtg gat aga ttc tac aga acc tgc aag cta gtt ggg atc aat atg    1621
Gly Val Asp Arg Phe Tyr Arg Thr Cys Lys Leu Val Gly Ile Asn Met
            465                 470                 475

agc aag aaa aag tcc tat ata aat agg aca gga aca ttc gaa ttc aca    1669
Ser Lys Lys Lys Ser Tyr Ile Asn Arg Thr Gly Thr Phe Glu Phe Thr
        480                 485                 490
```

```
agc ttt ttc tat cgt tat ggg ttt gtt gcc aat ttc agc atg gag ctt   1717
Ser Phe Phe Tyr Arg Tyr Gly Phe Val Ala Asn Phe Ser Met Glu Leu
    495                 500                 505

ccc agc ttt ggg gtg tct ggg atc aac gag tct gcg gac atg agt att   1765
Pro Ser Phe Gly Val Ser Gly Ile Asn Glu Ser Ala Asp Met Ser Ile
510                 515                 520                 525

gga gtt act gtc atc aaa aac aat atg ata aac aat gat ctt ggt cca   1813
Gly Val Thr Val Ile Lys Asn Asn Met Ile Asn Asn Asp Leu Gly Pro
                530                 535                 540

gca acc gct caa atg gcc ctt cag ctg ttc atc aaa gat tac agg tac   1861
Ala Thr Ala Gln Met Ala Leu Gln Leu Phe Ile Lys Asp Tyr Arg Tyr
            545                 550                 555

acg tac cgg tgc cat aga ggt gac aca caa ata caa acc cga aga tca   1909
Thr Tyr Arg Cys His Arg Gly Asp Thr Gln Ile Gln Thr Arg Arg Ser
        560                 565                 570

ttt gaa ata aag aaa ctg tgg gag caa acc cat tcc aaa gct gga ctg   1957
Phe Glu Ile Lys Lys Leu Trp Glu Gln Thr His Ser Lys Ala Gly Leu
    575                 580                 585

ctg gtc tcc gac gga ggc cca aat tta tac aac att aga aat ctc cac   2005
Leu Val Ser Asp Gly Gly Pro Asn Leu Tyr Asn Ile Arg Asn Leu His
590                 595                 600                 605

att cct gaa gtc tgc ttg aaa tgg gaa tta atg gat gag gat tac cag   2053
Ile Pro Glu Val Cys Leu Lys Trp Glu Leu Met Asp Glu Asp Tyr Gln
                610                 615                 620

ggg cgt tta tgc aac cca ctg aac cca ttt gtc aac cat aaa gac att   2101
Gly Arg Leu Cys Asn Pro Leu Asn Pro Phe Val Asn His Lys Asp Ile
            625                 630                 635

gaa tca gtg aac aat gca gtg ata atg cca gca cat ggt cca gcc aaa   2149
Glu Ser Val Asn Asn Ala Val Ile Met Pro Ala His Gly Pro Ala Lys
        640                 645                 650

aac atg gag tat gat gct gtt gca aca aca cac tcc tgg atc ccc aaa   2197
Asn Met Glu Tyr Asp Ala Val Ala Thr Thr His Ser Trp Ile Pro Lys
    655                 660                 665

aga aat cga tcc atc ttg aat aca agc caa aga gga ata ctt gaa gat   2245
Arg Asn Arg Ser Ile Leu Asn Thr Ser Gln Arg Gly Ile Leu Glu Asp
670                 675                 680                 685

gaa caa atg tac caa aag tgc tgc aac tta ttt gaa aaa ttc ttc ccc   2293
Glu Gln Met Tyr Gln Lys Cys Cys Asn Leu Phe Glu Lys Phe Phe Pro
                690                 695                 700

agc agt tca tac aga aga cca gtc ggg ata tcc agt atg gtg gag gct   2341
Ser Ser Ser Tyr Arg Arg Pro Val Gly Ile Ser Ser Met Val Glu Ala
            705                 710                 715

atg gtt tcc aga gcc cga att gat gca cga att gat ttc gaa tct gga   2389
Met Val Ser Arg Ala Arg Ile Asp Ala Arg Ile Asp Phe Glu Ser Gly
        720                 725                 730

agg ata aag aaa gag gag ttc act gag atc atg aag atc tgt tcc acc   2437
Arg Ile Lys Lys Glu Glu Phe Thr Glu Ile Met Lys Ile Cys Ser Thr
    735                 740                 745

att gaa gag ctc aga cgg caa aaa tagtgaattt agcttgtcct tcatgaaaaa  2491
Ile Glu Glu Leu Arg Arg Gln Lys
750                 755
```

atgccttgtt tctactaata acccggcggc ccaaaatgcc gactcggagc gaaagatata 2551
cctcccccgg ggccgggagg tcgcgtcacc gaccacgccg ccggcccagg cgacgcgcga 2611
cacggacacc tgtccccaaa aacgccacca tcgcagccac acacggagcg cccggggccc 2671
tctggtcaac cccaggacac acgcgggagc agcgccgggc cggggacgcc ctcccggccg 2731
cccgtgccac acgcaggggg ccggcccgtg tctccagagc gggagccgga agcattttcg 2791
gccggcccct cctacgaccg ggacacacga gggaccgaag gccggccagg cgcgacctct 2851
cgggccgcac gcgcgctcag ggagcgctct ccgactccgc acggggactc gccagaaagg 2911
atcgtgacct gcattaatga atcaggggat aacgcaggaa agaacatgtg agcaaaaggc 2971
cagcaaaagg ccaggaaccg taaaaaggcc gcgttgctgg cgtttttcca taggctccgc 3031
cccccctgacg agcatcacaa aaatcgacgc tcaagtcaga ggtggcgaaa cccgacagga 3091
ctataaagat accaggcgtt tccccctgga agctccctcg tgcgctctcc tgttccgacc 3151
ctgccgctta ccggatacct gtccgccttt ctcccttcgg gaagcgtggc gctttctcat 3211
agctcacgct gtaggtatct cagttcggtg taggtcgttc gctccaagct gggctgtgtg 3271
cacgaacccc ccgttcagcc cgaccgctgc gccttatccg gtaactatcg tcttgagtcc 3331
aacccggtaa gacacgactt atcgccactg gcagcagcca ctggtaacag gattagcaga 3391
gcgaggtatg taggcggtgc tacagagttc ttgaagtggt ggcctaacta cggctacact 3451
agaaggacag tatttggtat ctgcgctctg ctgaagccag ttaccttcgg aaaaagagtt 3511
ggtagctctt gatccggcaa acaaaccacc gctggtagcg gtggtttttt tgtttgcaag 3571
cagcagatta cgcgcagaaa aaaaggatct caagaagatc ctttgatctt ttctacgggg 3631
tctgacgctc agtggaacga aaactcacgt taagggattt tggtcatgag attatcaaaa 3691
aggatcttca cctagatcct tttaaattaa aaatgaagtt ttaaatcaat ctaaagtata 3751
tatgagtaaa cttggtctga cagttaccaa tgcttaatca gtgaggcacc tatctcagcg 3811
atctgtctat ttcgttcatc catagttgcc tgactccccg tcgtgtagat aactacgata 3871
cgggagggct taccatctgg ccccagtgct gcaatgatac cgcgagaccc acgctcaccg 3931
gctccagatt tatcagcaat aaaccagcca gccggaaggg ccgagcgcag aagtggtcct 3991
gcaactttat ccgcctccat ccagtctatt aattgttgcc gggaagctag agtaagtagt 4051
tcgccagtta atagtttgcg caacgttgtt gccattgcta caggcatcgt ggtgtcacgc 4111
tcgtcgtttg gtatggcttc attcagctcc ggttcccaac gatcaaggcg agttacatga 4171
tcccccatgt tgtgcaaaaa agcggttagc tccttcggtc ctccgatcgt tgtcagaagt 4231
aagttggccg cagtgttatc actcatggtt atggcagcac tgcataattc tcttactgtc 4291
atgccatccg taagatgctt ttctgtgact ggtgagtact caaccaagtc attctgagaa 4351
tagtgtatgc ggcgaccgag ttgctcttgc ccggcgtcaa cacgggataa taccgcgcca 4411
catagcagaa ctttaaaagt gctcatcatt ggaaaacgtt cttcggggcg aaaactctca 4471

aggatcttac cgctgttgag atccagttcg atgtaaccca ctcgtgcacc caactgatct 4531

tcagcatctt ttactttcac cagcgtttct gggtgagcaa aaacaggaag gcaaaatgcc 4591

gcaaaaaagg gaataagggc gacacggaaa tgttgaatac tcatactctt cctttttcaa 4651

tattattgaa gcatttatca gggttattgt ctcatgagcg gatacatatt tgaatgtatt 4711

tagaaaaata aacaaaagag tttgtagaaa cgcaaaaagg ccatccgtca ggatggcctt 4771

ctgcttaatt tgatgcctgg cagtttatgg cgggcgtcct gcccgccacc ctccgggccg 4831

ttgcttcgca acgttcaaat ccgctcccgg cggatttgtc ctactcagga gagcgttcac 4891

cgacaaacaa cagataaaac gaaaggccca gtctttcgac tgagcctttc gttttatttg 4951

atgcctggca gttccctact ctcgcatggg gagaccccac actaccatcg gcgctacggc 5011

gtttcacttc tgagttcggc atggggtcag gtgggaccac cgcgctactg ccgccaggca 5071

aattctgttt tatcagaccg cttctgcgtt ctgatttaat ctgtatcagg ctgaaaatct 5131

tctctcatcc gccaaaacag ccaagctagc ggccgatc 5169

<210> 33
<211> 757
<212> PRT
<213> Artificial Sequence
<223> Description of Artificial Sequence: pHL3131

<400> 33
Met Asp Val Asn Pro Thr Leu Leu Phe Leu Lys Val Pro Ala Gln Asn
1 5 10 15

Ala Ile Ser Thr Thr Phe Pro Tyr Thr Gly Asp Pro Pro Tyr Ser His
20 25 30

Gly Thr Gly Thr Gly Tyr Thr Met Asp Thr Val Asn Arg Thr His Gln
35 40 45

Tyr Ser Glu Arg Gly Arg Trp Thr Thr Asn Thr Glu Thr Gly Ala Pro
50 55 60

Gln Leu Asn Pro Ile Asp Gly Pro Leu Pro Glu Asp Asn Glu Pro Ser
65 70 75 80

Gly Tyr Ala Gln Thr Asp Cys Val Leu Glu Ala Met Ala Phe Leu Glu
85 90 95

Glu Ser His Pro Gly Ile Phe Glu Thr Ser Cys Leu Glu Thr Met Glu
100 105 110

Val Val Gln Gln Thr Arg Val Asp Lys Leu Thr Gln Gly Arg Gln Thr
115 120 125

Tyr Asp Trp Thr Leu Asn Arg Asn Gln Pro Ala Ala Thr Ala Leu Ala
130 135 140

Asn Thr Ile Glu Val Phe Arg Ser Asn Gly Leu Thr Ala Asn Glu Ser
145 150 155 160

Gly Arg Leu Ile Asp Phe Leu Lys Asp Val Met Glu Ser Met Asn Lys
165 170 175

Glu Glu Met Glu Ile Thr Thr His Phe Gln Arg Lys Arg Arg Val Arg
180 185 190

```
Asp Asn Met Thr Lys Lys Met Val Thr Gln Arg Thr Ile Gly Lys Arg
        195                 200                 205

Lys Gln Arg Leu Asn Lys Arg Ser Tyr Leu Ile Arg Ala Leu Thr Leu
    210                 215                 220

Asn Thr Met Thr Lys Asp Ala Glu Arg Gly Lys Leu Lys Arg Arg Ala
225                 230                 235                 240

Ile Ala Thr Pro Gly Met Gln Ile Arg Gly Phe Val Tyr Phe Val Glu
                245                 250                 255

Thr Leu Ala Arg Ser Ile Cys Glu Lys Leu Glu Gln Ser Gly Leu Pro
            260                 265                 270

Val Gly Gly Asn Glu Lys Lys Ala Lys Leu Ala Asn Val Val Arg Lys
        275                 280                 285

Met Met Thr Asn Ser Gln Asp Thr Glu Leu Ser Phe Thr Ile Thr Gly
    290                 295                 300

Asp Asn Thr Lys Trp Asn Glu Asn Gln Asn Pro Arg Met Phe Leu Ala
305                 310                 315                 320

Met Ile Thr Tyr Ile Thr Arg Asn Gln Pro Glu Trp Phe Arg Asn Val
                325                 330                 335

Leu Ser Ile Ala Pro Ile Met Phe Ser Asn Lys Met Ala Arg Leu Gly
            340                 345                 350

Lys Gly Tyr Met Phe Glu Ser Lys Ser Met Lys Leu Arg Thr Gln Ile
        355                 360                 365

Pro Ala Glu Met Leu Ala Ser Ile Asp Leu Lys Tyr Phe Asn Glu Pro
    370                 375                 380

Thr Arg Lys Lys Ile Glu Lys Ile Arg Pro Leu Ile Ile Asp Gly Thr
385                 390                 395                 400

Ala Ser Leu Ser Pro Gly Met Met Met Gly Met Phe Asn Met Leu Ser
                405                 410                 415

Thr Val Leu Gly Val Ser Ile Leu Asn Leu Gly Gln Lys Arg Tyr Thr
            420                 425                 430

Lys Thr Thr Tyr Trp Trp Asp Gly Leu Gln Ser Ser Asp Asp Phe Ala
        435                 440                 445

Leu Ile Val Asn Ala Pro Asn His Glu Gly Ile Gln Ala Gly Val Asp
    450                 455                 460

Arg Phe Tyr Arg Thr Cys Lys Leu Val Gly Ile Asn Met Ser Lys Lys
465                 470                 475                 480

Lys Ser Tyr Ile Asn Arg Thr Gly Thr Phe Glu Phe Thr Ser Phe Phe
                485                 490                 495

Tyr Arg Tyr Gly Phe Val Ala Asn Phe Ser Met Glu Leu Pro Ser Phe
            500                 505                 510

Gly Val Ser Gly Ile Asn Glu Ser Ala Asp Met Ser Ile Gly Val Thr
        515                 520                 525

Val Ile Lys Asn Asn Met Ile Asn Asn Asp Leu Gly Pro Ala Thr Ala
    530                 535                 540
```

```
Gln Met Ala Leu Gln Leu Phe Ile Lys Asp Tyr Arg Tyr Thr Tyr Arg
545                 550                 555                 560

Cys His Arg Gly Asp Thr Gln Ile Gln Thr Arg Arg Ser Phe Glu Ile
                565                 570                 575

Lys Lys Leu Trp Glu Gln Thr His Ser Lys Ala Gly Leu Leu Val Ser
            580                 585                 590

Asp Gly Gly Pro Asn Leu Tyr Asn Ile Arg Asn Leu His Ile Pro Glu
        595                 600                 605

Val Cys Leu Lys Trp Glu Leu Met Asp Glu Asp Tyr Gln Gly Arg Leu
    610                 615                 620

Cys Asn Pro Leu Asn Pro Phe Val Asn His Lys Asp Ile Glu Ser Val
625                 630                 635                 640

Asn Asn Ala Val Ile Met Pro Ala His Gly Pro Ala Lys Asn Met Glu
                645                 650                 655

Tyr Asp Ala Val Ala Thr Thr His Ser Trp Ile Pro Lys Arg Asn Arg
            660                 665                 670

Ser Ile Leu Asn Thr Ser Gln Arg Gly Ile Leu Glu Asp Glu Gln Met
        675                 680                 685

Tyr Gln Lys Cys Cys Asn Leu Phe Glu Lys Phe Phe Pro Ser Ser Ser
    690                 695                 700

Tyr Arg Arg Pro Val Gly Ile Ser Ser Met Val Glu Ala Met Val Ser
705                 710                 715                 720

Arg Ala Arg Ile Asp Ala Arg Ile Asp Phe Glu Ser Gly Arg Ile Lys
                725                 730                 735

Lys Glu Glu Phe Thr Glu Ile Met Lys Ile Cys Ser Thr Ile Glu Glu
            740                 745                 750

Leu Arg Arg Gln Lys
        755


<210> 34
<211> 5169
<212> DNA
<213> Artificial Sequence

<220>
<223> Description of Artificial Sequence: pHL3203

<220>
<221> CDS
<222> (191)..(2461)

<400> 34
cccaaaaaaa aaaaaaaaaa aagagtccag agtggccccg ccgctccgcg ccgggggggg 60

gggggggggg ggacactttc ggacatctgg tcgacctcca gcatcggggg aaaaaaaaaa 120

acaaagtgtc gcccggagta ctggtcgacc tccgaagttg gggggagcg aaagcaggca 180

aaccatttga atg gat gtc aat ccg act tta ctt ttc tta aaa gtg cca    229
           Met Asp Val Asn Pro Thr Leu Leu Phe Leu Lys Val Pro
           1               5                   10
```

```
gca caa aat gct ata agc aca act ttc cct tat act gga gac cct cct    277
Ala Gln Asn Ala Ile Ser Thr Thr Phe Pro Tyr Thr Gly Asp Pro Pro
    15                  20                  25

tac agc cat ggg aca gga aca gga tac acc atg gat act gtc aac agg    325
Tyr Ser His Gly Thr Gly Thr Gly Tyr Thr Met Asp Thr Val Asn Arg
30                  35                  40                  45

aca cat cag tac tca gaa agg gga aga tgg aca aca aac acc gaa act    373
Thr His Gln Tyr Ser Glu Arg Gly Arg Trp Thr Thr Asn Thr Glu Thr
                50                  55                  60

gga gca ccg caa ctc aac ccg att gat ggg cca ctg cca gaa gac aat    421
Gly Ala Pro Gln Leu Asn Pro Ile Asp Gly Pro Leu Pro Glu Asp Asn
            65                  70                  75

gaa cca agt ggt tat gcc caa aca gat tgt gta ttg gaa gca atg gcc    469
Glu Pro Ser Gly Tyr Ala Gln Thr Asp Cys Val Leu Glu Ala Met Ala
        80                  85                  90

ttc ctt gag gaa tcc cat cct ggt atc ttt gag acc tcg tgt ctt gaa    517
Phe Leu Glu Glu Ser His Pro Gly Ile Phe Glu Thr Ser Cys Leu Glu
    95                  100                 105

acg atg gag gtt gtt cag caa aca cga gtg gac aag ctg aca caa ggc    565
Thr Met Glu Val Val Gln Gln Thr Arg Val Asp Lys Leu Thr Gln Gly
110                 115                 120                 125

cga cag acc tat gac tgg act cta aat agg aac cag cct gct gca aca    613
Arg Gln Thr Tyr Asp Trp Thr Leu Asn Arg Asn Gln Pro Ala Ala Thr
                130                 135                 140

gca ttg gcc aac aca ata gaa gtg ttc aga tca aat ggc ctc acg gcc    661
Ala Leu Ala Asn Thr Ile Glu Val Phe Arg Ser Asn Gly Leu Thr Ala
            145                 150                 155

aat gaa tct gga agg ctc ata gac ttc ctt aag gat gta atg gag tca    709
Asn Glu Ser Gly Arg Leu Ile Asp Phe Leu Lys Asp Val Met Glu Ser
        160                 165                 170

atg aac aaa gaa gaa atg gag atc aca act cat ttt cag aga aag aga    757
Met Asn Lys Glu Glu Met Glu Ile Thr Thr His Phe Gln Arg Lys Arg
    175                 180                 185

cga gtg aga gac aat atg act aag aaa atg gtg aca cag aga aca ata    805
Arg Val Arg Asp Asn Met Thr Lys Lys Met Val Thr Gln Arg Thr Ile
190                 195                 200                 205

ggt aaa agg aag cag aga ttg aac aaa agg agt tat cta att agg gca    853
Gly Lys Arg Lys Gln Arg Leu Asn Lys Arg Ser Tyr Leu Ile Arg Ala
                210                 215                 220

tta acc ctg aac aca atg acc aaa gat gct gag aga ggg aag cta aaa    901
Leu Thr Leu Asn Thr Met Thr Lys Asp Ala Glu Arg Gly Lys Leu Lys
            225                 230                 235

cgg aga gca att gca acc cca ggg atg caa ata agg ggg ttt gta tac    949
Arg Arg Ala Ile Ala Thr Pro Gly Met Gln Ile Arg Gly Phe Val Tyr
        240                 245                 250

ttt gtt gag aca cta gca agg agt ata tgt gag aaa ctt gaa caa tca    997
Phe Val Glu Thr Leu Ala Arg Ser Ile Cys Glu Lys Leu Glu Gln Ser
    255                 260                 265

gga ttg cca gtt gga ggc aat gag aag aaa gca aag ttg gca aat gtt    1045
Gly Leu Pro Val Gly Gly Asn Glu Lys Lys Ala Lys Leu Ala Asn Val
270                 275                 280                 285
```

```
gta agg aag atg atg acc aat tct cag gac act gaa att tct ttc acc     1093
Val Arg Lys Met Met Thr Asn Ser Gln Asp Thr Glu Ile Ser Phe Thr
                290                 295                 300

atc act gga gat aac acc aaa tgg aac gaa aat cag aac cct cgg atg     1141
Ile Thr Gly Asp Asn Thr Lys Trp Asn Glu Asn Gln Asn Pro Arg Met
            305                 310                 315

ttt ttg gcc atg atc aca tat ata acc aga aat cag ccc gaa tgg ttc     1189
Phe Leu Ala Met Ile Thr Tyr Ile Thr Arg Asn Gln Pro Glu Trp Phe
        320                 325                 330

aga aat gtt cta agt att gct cca ata atg ttc tca aac aaa atg gcg     1237
Arg Asn Val Leu Ser Ile Ala Pro Ile Met Phe Ser Asn Lys Met Ala
    335                 340                 345

aga ctg gga aag ggg tac atg ttt gag agc aag agt atg aaa att aga     1285
Arg Leu Gly Lys Gly Tyr Met Phe Glu Ser Lys Ser Met Lys Ile Arg
350                 355                 360                 365

act caa ata cct gca gaa atg cta gca agc atc gat ttg aaa tac ttc     1333
Thr Gln Ile Pro Ala Glu Met Leu Ala Ser Ile Asp Leu Lys Tyr Phe
                370                 375                 380

aat gat tca act aga aag aag att gaa aaa atc cgg ccg ctc tta ata     1381
Asn Asp Ser Thr Arg Lys Lys Ile Glu Lys Ile Arg Pro Leu Leu Ile
            385                 390                 395

gat ggg act gca tca ttg agc cct gga atg atg atg ggc atg ttc aat     1429
Asp Gly Thr Ala Ser Leu Ser Pro Gly Met Met Met Gly Met Phe Asn
        400                 405                 410

atg tta agt act gta tta ggc gtc tcc atc ctg aat ctt gga caa aag     1477
Met Leu Ser Thr Val Leu Gly Val Ser Ile Leu Asn Leu Gly Gln Lys
    415                 420                 425

aga cac acc aag act act tac tgg tgg gat ggt ctt caa tct tct gat     1525
Arg His Thr Lys Thr Thr Tyr Trp Trp Asp Gly Leu Gln Ser Ser Asp
430                 435                 440                 445

gat ttt gct ctg att gtg aat gca ccc aat cat gaa ggg att caa gcc     1573
Asp Phe Ala Leu Ile Val Asn Ala Pro Asn His Glu Gly Ile Gln Ala
                450                 455                 460

gga gtc aac agg ttt tat cga acc tgt aag cta ctt gga att aat atg     1621
Gly Val Asn Arg Phe Tyr Arg Thr Cys Lys Leu Leu Gly Ile Asn Met
            465                 470                 475

agc aag aaa aag tct tac ata aac aga aca ggt aca ttt gaa ttc aca     1669
Ser Lys Lys Lys Ser Tyr Ile Asn Arg Thr Gly Thr Phe Glu Phe Thr
        480                 485                 490

agc ttt ttc tat cgt tat ggg ttt gtt gcc aat ttc agc atg gag ctt     1717
Ser Phe Phe Tyr Arg Tyr Gly Phe Val Ala Asn Phe Ser Met Glu Leu
    495                 500                 505

ccc agc ttt ggg gtg tct ggg atc aac gag tct gcg gac atg agt att     1765
Pro Ser Phe Gly Val Ser Gly Ile Asn Glu Ser Ala Asp Met Ser Ile
510                 515                 520                 525

gga gtt act gtc atc aaa aac aat atg ata aac aat gat ctt ggt cca     1813
Gly Val Thr Val Ile Lys Asn Asn Met Ile Asn Asn Asp Leu Gly Pro
                530                 535                 540
```

```
gca acc gct caa atg gcc ctt cag ctg ttc atc aaa gat tac agg tac   1861
Ala Thr Ala Gln Met Ala Leu Gln Leu Phe Ile Lys Asp Tyr Arg Tyr
            545                 550                 555

acg tac cgg tgc cat aga ggt gac aca caa ata caa acc cga aga tca   1909
Thr Tyr Arg Cys His Arg Gly Asp Thr Gln Ile Gln Thr Arg Arg Ser
        560                 565                 570

ttt gaa ata aag aaa ctg tgg gag caa acc cat tcc aaa gct gga ctg   1957
Phe Glu Ile Lys Lys Leu Trp Glu Gln Thr His Ser Lys Ala Gly Leu
    575                 580                 585

ctg gtc tcc gac gga ggc cca aat ttg tac aac att cgg aat ctc cac   2005
Leu Val Ser Asp Gly Gly Pro Asn Leu Tyr Asn Ile Arg Asn Leu His
590                 595                 600                 605

atc ccg gaa gtt tgc ctg aaa tgg gaa cta atg gat gaa gac tat cag   2053
Ile Pro Glu Val Cys Leu Lys Trp Glu Leu Met Asp Glu Asp Tyr Gln
                610                 615                 620

gga aga ctt tgt aat ccc atg aac ccg ttt gtc agt cat aag gaa att   2101
Gly Arg Leu Cys Asn Pro Met Asn Pro Phe Val Ser His Lys Glu Ile
            625                 630                 635

gaa tct gta aac aat gct gcg gta atg cca gcc cat ggt cca gcc aaa   2149
Glu Ser Val Asn Asn Ala Ala Val Met Pro Ala His Gly Pro Ala Lys
        640                 645                 650

agc atg gaa tat gat gct gtg gca act aca cac tct tgg atc cct aag   2197
Ser Met Glu Tyr Asp Ala Val Ala Thr Thr His Ser Trp Ile Pro Lys
    655                 660                 665

aga aac cgt tcc att ctc aat acg agt caa agg gga atc ctt gag gat   2245
Arg Asn Arg Ser Ile Leu Asn Thr Ser Gln Arg Gly Ile Leu Glu Asp
670                 675                 680                 685

gaa caa atg tac cag aag tgt tgc aac cta ttc gag aaa ttc ttc cct   2293
Glu Gln Met Tyr Gln Lys Cys Cys Asn Leu Phe Glu Lys Phe Phe Pro
                690                 695                 700

agc agc tca tac aga aga cca gtt gga att tcc agt atg gtg gag gcc   2341
Ser Ser Ser Tyr Arg Arg Pro Val Gly Ile Ser Ser Met Val Glu Ala
            705                 710                 715

atg gtg tct agg gcc cgg att gat gca cga att gac ttc gag tct gga   2389
Met Val Ser Arg Ala Arg Ile Asp Ala Arg Ile Asp Phe Glu Ser Gly
        720                 725                 730

agg att aag aag gaa gag ttt gct gag atc atg aag atc tgt tcc acc   2437
Arg Ile Lys Lys Glu Glu Phe Ala Glu Ile Met Lys Ile Cys Ser Thr
    735                 740                 745

att gaa gag ctc aga cgg caa aaa tagtgaattt agcttgtcct tcatgaaaaa  2491
Ile Glu Glu Leu Arg Arg Gln Lys
750                 755

atgccttgtt tctactaata acccggcggc ccaaaatgcc gactcggagc gaaagatata 2551

cctcccccgg ggccgggagg tcgcgtcacc gaccacgccg ccggcccagg cgacgcgcga 2611

cacggacacc tgtccccaaa aacgccacca tcgcagccac acacggagcg cccggggccc 2671

tctggtcaac cccaggacac acgcgggagc agcgccgggc cggggacgcc ctcccggccg 2731

cccgtgccac acgcaggggg ccggcccgtg tctccagagc gggagccgga agcattttcg 2791

gccggcccct cctacgaccg ggacacacga gggaccgaag gccggccagg cgcgacctct 2851
```

cgggccgcac gcgcgctcag ggagcgctct ccgactccgc acggggactc gccagaaagg 2911
atcgtgacct gcattaatga atcaggggat aacgcaggaa agaacatgtg agcaaaaggc 2971
cagcaaaagg ccaggaaccg taaaaaggcc gcgttgctgg cgtttttcca taggctccgc 3031
cccccctgacg agcatcacaa aaatcgacgc tcaagtcaga ggtggcgaaa cccgacagga 3091
ctataaagat accaggcgtt tccccctgga agctccctcg tgcgctctcc tgttccgacc 3151
ctgccgctta ccggatacct gtccgccttt ctcccttcgg gaagcgtggc gctttctcat 3211
agctcacgct gtaggtatct cagttcggtg taggtcgttc gctccaagct gggctgtgtg 3271
cacgaacccc ccgttcagcc cgaccgctgc gccttatccg gtaactatcg tcttgagtcc 3331
aacccggtaa gacacgactt atcgccactg gcagcagcca ctggtaacag gattagcaga 3391
gcgaggtatg taggcggtgc tacagagttc ttgaagtggt ggcctaacta cggctacact 3451
agaaggacag tatttggtat ctgcgctctg ctgaagccag ttaccttcgg aaaaagagtt 3511
ggtagctctt gatccggcaa acaaaccacc gctggtagcg gtggtttttt tgtttgcaag 3571
cagcagatta cgcgcagaaa aaaaggatct caagaagatc ctttgatctt ttctacgggg 3631
tctgacgctc agtggaacga aaactcacgt taagggattt tggtcatgag attatcaaaa 3691
aggatcttca cctagatcct tttaaattaa aaatgaagtt ttaaatcaat ctaaagtata 3751
tatgagtaaa cttggtctga cagttaccaa tgcttaatca gtgaggcacc tatctcagcg 3811
atctgtctat ttcgttcatc catagttgcc tgactccccg tcgtgtagat aactacgata 3871
cgggagggct taccatctgg ccccagtgct gcaatgatac cgcgagaccc acgctcaccg 3931
gctccagatt tatcagcaat aaaccagcca gccggaaggg ccgagcgcag aagtggtcct 3991
gcaactttat ccgcctccat ccagtctatt aattgttgcc gggaagctag agtaagtagt 4051
tcgccagtta atagtttgcg caacgttgtt gccattgcta caggcatcgt ggtgtcacgc 4111
tcgtcgtttg gtatggcttc attcagctcc ggttcccaac gatcaaggcg agttacatga 4171
tcccccatgt tgtgcaaaaa agcggttagc tccttcggtc ctccgatcgt tgtcagaagt 4231
aagttggccg cagtgttatc actcatggtt atggcagcac tgcataattc tcttactgtc 4291
atgccatccg taagatgctt ttctgtgact ggtgagtact caaccaagtc attctgagaa 4351
tagtgtatgc ggcgaccgag ttgctcttgc ccggcgtcaa cacgggataa taccgcgcca 4411
catagcagaa ctttaaaagt gctcatcatt ggaaaacgtt cttcggggcg aaaactctca 4471
aggatcttac cgctgttgag atccagttcg atgtaaccca ctcgtgcacc caactgatct 4531
tcagcatctt ttactttcac cagcgtttct gggtgagcaa aaacaggaag gcaaaatgcc 4591
gcaaaaaagg gaataagggc gacacggaaa tgttgaatac tcatactctt cctttttcaa 4651
tattattgaa gcatttatca gggttattgt ctcatgagcg gatacatatt tgaatgtatt 4711
tagaaaaata aacaaaagag tttgtagaaa cgcaaaaagg ccatccgtca ggatggcctt 4771
ctgcttaatt tgatgcctgg cagtttatgg cgggcgtcct gcccgccacc ctccgggccg 4831

```
ttgcttcgca acgttcaaat ccgctcccgg cggatttgtc ctactcagga gagcgttcac 4891
cgacaaacaa cagataaaac gaaaggccca gtctttcgac tgagcctttc gttttatttg 4951
atgcctggca gttccctact ctcgcatggg gagaccccac actaccatcg gcgctacggc 5011
gtttcacttc tgagttcggc atggggtcag gtgggaccac cgcgctactg ccgccaggca 5071
aattctgttt tatcagaccg cttctgcgtt ctgatttaat ctgtatcagg ctgaaaatct 5131
tctctcatcc gccaaaacag ccaagctagc ggccgatc                         5169
```

```
<210> 35
<211> 757
<212> PRT
<213> Artificial Sequence
<223> Description of Artificial Sequence: pHL3203

<400> 35
Met Asp Val Asn Pro Thr Leu Leu Phe Leu Lys Val Pro Ala Gln Asn
  1               5                  10                  15

Ala Ile Ser Thr Thr Phe Pro Tyr Thr Gly Asp Pro Pro Tyr Ser His
             20                  25                  30

Gly Thr Gly Thr Gly Tyr Thr Met Asp Thr Val Asn Arg Thr His Gln
         35                  40                  45

Tyr Ser Glu Arg Gly Arg Trp Thr Thr Asn Thr Glu Thr Gly Ala Pro
     50                  55                  60

Gln Leu Asn Pro Ile Asp Gly Pro Leu Pro Glu Asp Asn Glu Pro Ser
 65                  70                  75                  80

Gly Tyr Ala Gln Thr Asp Cys Val Leu Glu Ala Met Ala Phe Leu Glu
                 85                  90                  95

Glu Ser His Pro Gly Ile Phe Glu Thr Ser Cys Leu Glu Thr Met Glu
            100                 105                 110

Val Val Gln Gln Thr Arg Val Asp Lys Leu Thr Gln Gly Arg Gln Thr
        115                 120                 125

Tyr Asp Trp Thr Leu Asn Arg Asn Gln Pro Ala Ala Thr Ala Leu Ala
    130                 135                 140

Asn Thr Ile Glu Val Phe Arg Ser Asn Gly Leu Thr Ala Asn Glu Ser
145                 150                 155                 160

Gly Arg Leu Ile Asp Phe Leu Lys Asp Val Met Glu Ser Met Asn Lys
                165                 170                 175

Glu Glu Met Glu Ile Thr Thr His Phe Gln Arg Lys Arg Arg Val Arg
            180                 185                 190

Asp Asn Met Thr Lys Lys Met Val Thr Gln Arg Thr Ile Gly Lys Arg
        195                 200                 205

Lys Gln Arg Leu Asn Lys Arg Ser Tyr Leu Ile Arg Ala Leu Thr Leu
    210                 215                 220

Asn Thr Met Thr Lys Asp Ala Glu Arg Gly Lys Leu Lys Arg Arg Ala
225                 230                 235                 240

Ile Ala Thr Pro Gly Met Gln Ile Arg Gly Phe Val Tyr Phe Val Glu
                245                 250                 255
```

```
Thr Leu Ala Arg Ser Ile Cys Glu Lys Leu Glu Gln Ser Gly Leu Pro
            260                 265                 270

Val Gly Gly Asn Glu Lys Lys Ala Lys Leu Ala Asn Val Val Arg Lys
        275                 280                 285

Met Met Thr Asn Ser Gln Asp Thr Glu Ile Ser Phe Thr Ile Thr Gly
    290                 295                 300

Asp Asn Thr Lys Trp Asn Glu Asn Gln Asn Pro Arg Met Phe Leu Ala
305                 310                 315                 320

Met Ile Thr Tyr Ile Thr Arg Asn Gln Pro Glu Trp Phe Arg Asn Val
                325                 330                 335

Leu Ser Ile Ala Pro Ile Met Phe Ser Asn Lys Met Ala Arg Leu Gly
            340                 345                 350

Lys Gly Tyr Met Phe Glu Ser Lys Ser Met Lys Ile Arg Thr Gln Ile
        355                 360                 365

Pro Ala Glu Met Leu Ala Ser Ile Asp Leu Lys Tyr Phe Asn Asp Ser
    370                 375                 380

Thr Arg Lys Lys Ile Glu Lys Ile Arg Pro Leu Leu Ile Asp Gly Thr
385                 390                 395                 400

Ala Ser Leu Ser Pro Gly Met Met Met Gly Met Phe Asn Met Leu Ser
                405                 410                 415

Thr Val Leu Gly Val Ser Ile Leu Asn Leu Gly Gln Lys Arg His Thr
            420                 425                 430

Lys Thr Thr Tyr Trp Trp Asp Gly Leu Gln Ser Ser Asp Asp Phe Ala
        435                 440                 445

Leu Ile Val Asn Ala Pro Asn His Glu Gly Ile Gln Ala Gly Val Asn
    450                 455                 460

Arg Phe Tyr Arg Thr Cys Lys Leu Leu Gly Ile Asn Met Ser Lys Lys
465                 470                 475                 480

Lys Ser Tyr Ile Asn Arg Thr Gly Thr Phe Glu Phe Thr Ser Phe Phe
                485                 490                 495

Tyr Arg Tyr Gly Phe Val Ala Asn Phe Ser Met Glu Leu Pro Ser Phe
            500                 505                 510

Gly Val Ser Gly Ile Asn Glu Ser Ala Asp Met Ser Ile Gly Val Thr
        515                 520                 525

Val Ile Lys Asn Asn Met Ile Asn Asn Asp Leu Gly Pro Ala Thr Ala
    530                 535                 540

Gln Met Ala Leu Gln Leu Phe Ile Lys Asp Tyr Arg Tyr Thr Tyr Arg
545                 550                 555                 560

Cys His Arg Gly Asp Thr Gln Ile Gln Thr Arg Arg Ser Phe Glu Ile
                565                 570                 575

Lys Lys Leu Trp Glu Gln Thr His Ser Lys Ala Gly Leu Leu Val Ser
            580                 585                 590

Asp Gly Gly Pro Asn Leu Tyr Asn Ile Arg Asn Leu His Ile Pro Glu
        595                 600                 605
```

```
Val Cys Leu Lys Trp Glu Leu Met Asp Glu Asp Tyr Gln Gly Arg Leu
    610                 615                 620

Cys Asn Pro Met Asn Pro Phe Val Ser His Lys Glu Ile Glu Ser Val
625                 630                 635                 640

Asn Asn Ala Ala Val Met Pro Ala His Gly Pro Ala Lys Ser Met Glu
                645                 650                 655

Tyr Asp Ala Val Ala Thr Thr His Ser Trp Ile Pro Lys Arg Asn Arg
            660                 665                 670

Ser Ile Leu Asn Thr Ser Gln Arg Gly Ile Leu Glu Asp Glu Gln Met
        675                 680                 685

Tyr Gln Lys Cys Cys Asn Leu Phe Glu Lys Phe Phe Pro Ser Ser Ser
    690                 695                 700

Tyr Arg Arg Pro Val Gly Ile Ser Ser Met Val Glu Ala Met Val Ser
705                 710                 715                 720

Arg Ala Arg Ile Asp Ala Arg Ile Asp Phe Glu Ser Gly Arg Ile Lys
                725                 730                 735

Lys Glu Glu Phe Ala Glu Ile Met Lys Ile Cys Ser Thr Ile Glu Glu
            740                 745                 750

Leu Arg Arg Gln Lys
        755


<210> 36
<211> 5169
<212> DNA
<213> Artificial Sequence

<220>
<223> Description of Artificial Sequence: pHL3204

<220>
<221> CDS
<222> (191)..(2461)

<400> 36
cccaaaaaaa aaaaaaaaaa aagagtccag agtggccccg ccgctccgcg ccgggggggg 60

gggggggggg ggacactttc ggacatctgg tcgacctcca gcatcggggg aaaaaaaaaa 120

acaaagtgtc gcccggagta ctggtcgacc tccgaagttg gggggagcg aaagcaggca 180

aaccatttga atg gat gtc aat ccg act tta ctt ttc tta aaa gtg cca    229
           Met Asp Val Asn Pro Thr Leu Leu Phe Leu Lys Val Pro
             1               5                  10

gca caa aat gct ata agc aca act ttc cct tat act gga gac cct cct    277
Ala Gln Asn Ala Ile Ser Thr Thr Phe Pro Tyr Thr Gly Asp Pro Pro
     15                  20                  25

tac agc cat ggg aca gga aca gga tac acc atg gat act gtc aac agg    325
Tyr Ser His Gly Thr Gly Thr Gly Tyr Thr Met Asp Thr Val Asn Arg
 30                  35                  40                  45

aca cat cag tac tca gaa agg gga aga tgg aca aca aac acc gaa act    373
Thr His Gln Tyr Ser Glu Arg Gly Arg Trp Thr Thr Asn Thr Glu Thr
                 50                  55                  60
```

```
gga gca ccg caa ctc aac ccg att gat ggg cca ctg cca gaa gac aat    421
Gly Ala Pro Gln Leu Asn Pro Ile Asp Gly Pro Leu Pro Glu Asp Asn
            65                  70                  75

gaa cca agt ggt tat gcc caa aca gat tgt gta ttg gaa gca atg gcc    469
Glu Pro Ser Gly Tyr Ala Gln Thr Asp Cys Val Leu Glu Ala Met Ala
        80                  85                  90

ttc ctt gag gaa tcc cat cct ggt atc ttt gag acc tcg tgt ctt gaa    517
Phe Leu Glu Glu Ser His Pro Gly Ile Phe Glu Thr Ser Cys Leu Glu
    95                  100                 105

acg atg gag gtt gtt cag caa aca cga gtg gac aag ctg aca caa ggc    565
Thr Met Glu Val Val Gln Gln Thr Arg Val Asp Lys Leu Thr Gln Gly
110                 115                 120                 125

cga cag acc tat gac tgg act cta aat agg aac cag cct gct gca aca    613
Arg Gln Thr Tyr Asp Trp Thr Leu Asn Arg Asn Gln Pro Ala Ala Thr
                130                 135                 140

gca ttg gcc aac aca ata gaa gtg ttc aga tca aat ggc ctc acg gcc    661
Ala Leu Ala Asn Thr Ile Glu Val Phe Arg Ser Asn Gly Leu Thr Ala
            145                 150                 155

aat gaa tct gga agg ctc ata gac ttc ctt aag gat gta atg gag tca    709
Asn Glu Ser Gly Arg Leu Ile Asp Phe Leu Lys Asp Val Met Glu Ser
        160                 165                 170

atg aac aaa gaa gaa atg gag atc aca act cat ttt cag aga aag aga    757
Met Asn Lys Glu Glu Met Glu Ile Thr Thr His Phe Gln Arg Lys Arg
    175                 180                 185

cga gtg aga gac aat atg act aag aaa atg gtg aca cag aga aca ata    805
Arg Val Arg Asp Asn Met Thr Lys Lys Met Val Thr Gln Arg Thr Ile
190                 195                 200                 205

ggt aaa agg aag cag aga ttg aac aaa agg agt tat cta att agg gca    853
Gly Lys Arg Lys Gln Arg Leu Asn Lys Arg Ser Tyr Leu Ile Arg Ala
                210                 215                 220

tta acc ctg aac aca atg acc aaa gat gct gag aga ggg aag cta aaa    901
Leu Thr Leu Asn Thr Met Thr Lys Asp Ala Glu Arg Gly Lys Leu Lys
            225                 230                 235

cgg aga gca att gca acc cca ggg atg caa ata agg ggg ttt gta tac    949
Arg Arg Ala Ile Ala Thr Pro Gly Met Gln Ile Arg Gly Phe Val Tyr
        240                 245                 250

ttt gtt gag aca cta gca agg agt ata tgt gag aaa ctt gaa caa tca    997
Phe Val Glu Thr Leu Ala Arg Ser Ile Cys Glu Lys Leu Glu Gln Ser
    255                 260                 265

gga ttg cca gtt gga ggc aat gag aag aaa gca aag ttg gca aat gtt    1045
Gly Leu Pro Val Gly Gly Asn Glu Lys Lys Ala Lys Leu Ala Asn Val
270                 275                 280                 285

gta agg aag atg atg acc aat tct cag gac act gaa att tct ttc acc    1093
Val Arg Lys Met Met Thr Asn Ser Gln Asp Thr Glu Ile Ser Phe Thr
                290                 295                 300

atc act gga gat aac acc aaa tgg aac gaa aat cag aac cct cgg atg    1141
Ile Thr Gly Asp Asn Thr Lys Trp Asn Glu Asn Gln Asn Pro Arg Met
            305                 310                 315

ttt ttg gcc atg atc aca tat ata acc aga aat cag ccc gaa tgg ttc    1189
Phe Leu Ala Met Ile Thr Tyr Ile Thr Arg Asn Gln Pro Glu Trp Phe
        320                 325                 330
```

```
aga aat gtt cta agt att gct cca ata atg ttc tca aac aaa atg gcg    1237
Arg Asn Val Leu Ser Ile Ala Pro Ile Met Phe Ser Asn Lys Met Ala
    335                 340                 345

aga ctg gga aag ggg tac atg ttt gag agc aag agt atg aaa att aga    1285
Arg Leu Gly Lys Gly Tyr Met Phe Glu Ser Lys Ser Met Lys Ile Arg
350                 355                 360                 365

act caa ata cct gca gaa atg cta gca agc atc gat ttg aaa tac ttc    1333
Thr Gln Ile Pro Ala Glu Met Leu Ala Ser Ile Asp Leu Lys Tyr Phe
                370                 375                 380

aat gat tca act aga aag aag att gaa aaa atc cgg ccg ctc tta ata    1381
Asn Asp Ser Thr Arg Lys Lys Ile Glu Lys Ile Arg Pro Leu Leu Ile
            385                 390                 395

gat ggg act gca tca ttg agc cct gga atg atg atg ggc atg ttc aat    1429
Asp Gly Thr Ala Ser Leu Ser Pro Gly Met Met Met Gly Met Phe Asn
        400                 405                 410

atg tta agt act gta tta ggc gtc tcc atc ctg aat ctt gga caa aag    1477
Met Leu Ser Thr Val Leu Gly Val Ser Ile Leu Asn Leu Gly Gln Lys
    415                 420                 425

aga cac acc aag act act tac tgg tgg gat ggt ctt caa tct tct gat    1525
Arg His Thr Lys Thr Thr Tyr Trp Trp Asp Gly Leu Gln Ser Ser Asp
430                 435                 440                 445

gat ttt gct ctg att gtg aat gca ccc aat cat gaa ggg att caa gcc    1573
Asp Phe Ala Leu Ile Val Asn Ala Pro Asn His Glu Gly Ile Gln Ala
                450                 455                 460

gga gtc aac agg ttt tat cga acc tgt aag cta ctt gga att aat atg    1621
Gly Val Asn Arg Phe Tyr Arg Thr Cys Lys Leu Leu Gly Ile Asn Met
            465                 470                 475

agc aag aaa aag tct tac ata aac aga aca ggt aca ttt gaa ttc aca    1669
Ser Lys Lys Lys Ser Tyr Ile Asn Arg Thr Gly Thr Phe Glu Phe Thr
        480                 485                 490

agc ttt ttc tac cgc tat gga ttt gta gcc aat ttt agt atg gag ttg    1717
Ser Phe Phe Tyr Arg Tyr Gly Phe Val Ala Asn Phe Ser Met Glu Leu
    495                 500                 505

ccc agc ttt gga gta tca gga att aat gaa tcg gct gat atg agc att    1765
Pro Ser Phe Gly Val Ser Gly Ile Asn Glu Ser Ala Asp Met Ser Ile
510                 515                 520                 525

gga gta aca gtg ata aag aat aac atg ata aac aat gat ctt gga ccg    1813
Gly Val Thr Val Ile Lys Asn Asn Met Ile Asn Asn Asp Leu Gly Pro
                530                 535                 540

gca aca gcc caa atg gct ctc caa tta ttc atc aag gac tac aga tat    1861
Ala Thr Ala Gln Met Ala Leu Gln Leu Phe Ile Lys Asp Tyr Arg Tyr
            545                 550                 555

aca tac agg tgt cac agg gga gac aca caa atc caa acg agg agg tca    1909
Thr Tyr Arg Cys His Arg Gly Asp Thr Gln Ile Gln Thr Arg Arg Ser
        560                 565                 570

ttc gag cta aag aag ctg tgg gag cag acc cgc tca aag gca gga ctg    1957
Phe Glu Leu Lys Lys Leu Trp Glu Gln Thr Arg Ser Lys Ala Gly Leu
    575                 580                 585
```

```
ttg gtt tca gat ggc gga cca aac ctg tac aac att aga aat ctc cac   2005
Leu Val Ser Asp Gly Gly Pro Asn Leu Tyr Asn Ile Arg Asn Leu His
590                 595                 600                 605

att cct gaa gtc tgc ttg aaa tgg gaa tta atg gat gag gat tac cag   2053
Ile Pro Glu Val Cys Leu Lys Trp Glu Leu Met Asp Glu Asp Tyr Gln
                610                 615                 620

ggg cgt tta tgc aac cca ctg aac cca ttt gtc aac cat aaa gac att   2101
Gly Arg Leu Cys Asn Pro Leu Asn Pro Phe Val Asn His Lys Asp Ile
            625                 630                 635

gaa tca gtg aac aat gca gtg ata atg cca gca cat ggt cca gcc aaa   2149
Glu Ser Val Asn Asn Ala Val Ile Met Pro Ala His Gly Pro Ala Lys
        640                 645                 650

aac atg gag tat gat gct gtt gca aca aca cac tcc tgg atc ccc aaa   2197
Asn Met Glu Tyr Asp Ala Val Ala Thr Thr His Ser Trp Ile Pro Lys
    655                 660                 665

aga aat cga tcc atc ttg aat aca agc caa aga gga ata ctt gaa gat   2245
Arg Asn Arg Ser Ile Leu Asn Thr Ser Gln Arg Gly Ile Leu Glu Asp
670                 675                 680                 685

gaa caa atg tac caa aag tgc tgc aac tta ttt gaa aaa ttc ttc ccc   2293
Glu Gln Met Tyr Gln Lys Cys Cys Asn Leu Phe Glu Lys Phe Phe Pro
                690                 695                 700

agc agt tca tac aga aga cca gtc ggg ata tcc agt atg gtg gag gct   2341
Ser Ser Ser Tyr Arg Arg Pro Val Gly Ile Ser Ser Met Val Glu Ala
            705                 710                 715

atg gtt tcc aga gcc cga att gat gca cga att gat ttc gaa tct gga   2389
Met Val Ser Arg Ala Arg Ile Asp Ala Arg Ile Asp Phe Glu Ser Gly
        720                 725                 730

agg ata aag aaa gag gag ttc act gag atc atg aag atc tgt tcc acc   2437
Arg Ile Lys Lys Glu Glu Phe Thr Glu Ile Met Lys Ile Cys Ser Thr
    735                 740                 745

att gaa gag ctc aga cgg caa aaa tagtgaattt agcttgtcct tcatgaaaaa  2491
Ile Glu Glu Leu Arg Arg Gln Lys
750                 755

atgccttgtt tctactaata acccggcggc ccaaaatgcc gactcggagc gaaagatata 2551

cctcccccgg ggccgggagg tcgcgtcacc gaccacgccg ccggcccagg cgacgcgcga 2611

cacggacacc tgtccccaaa aacgccacca tcgcagccac acacggagcg cccggggccc 2671

tctggtcaac cccaggacac acgcgggagc agcgccgggc cggggacgcc ctcccggccg 2731

cccgtgccac acgcaggggg ccggcccgtg tctccagagc gggagccgga agcattttcg 2791

gccggcccct cctacgaccg ggacacacga gggaccgaag gccggccagg cgcgacctct 2851

cgggccgcac gcgcgctcag ggagcgctct ccgactccgc acggggactc gccagaaagg 2911

atcgtgacct gcattaatga atcaggggat aacgcaggaa agaacatgtg agcaaaaggc 2971

cagcaaaagg ccaggaaccg taaaaaggcc gcgttgctgg cgtttttcca taggctccgc 3031

ccccctgacg agcatcacaa aaatcgacgc tcaagtcaga ggtggcgaaa cccgacagga 3091

ctataaagat accaggcgtt tccccctgga agctccctcg tgcgctctcc tgttccgacc 3151

ctgccgctta ccggatacct gtccgccttt ctcccttcgg gaagcgtggc gctttctcat 3211
```

agctcacgct gtaggtatct cagttcggtg taggtcgttc gctccaagct gggctgtgtg 3271
cacgaacccc ccgttcagcc cgaccgctgc gccttatccg gtaactatcg tcttgagtcc 3331
aacccggtaa gacacgactt atcgccactg gcagcagcca ctggtaacag gattagcaga 3391
gcgaggtatg taggcggtgc tacagagttc ttgaagtggt ggcctaacta cggctacact 3451
agaaggacag tatttggtat ctgcgctctg ctgaagccag ttaccttcgg aaaaagagtt 3511
ggtagctctt gatccggcaa acaaaccacc gctggtagcg gtggtttttt tgtttgcaag 3571
cagcagatta cgcgcagaaa aaaaggatct caagaagatc ctttgatctt ttctacgggg 3631
tctgacgctc agtggaacga aaactcacgt taagggattt tggtcatgag attatcaaaa 3691
aggatcttca cctagatcct tttaaattaa aaatgaagtt ttaaatcaat ctaaagtata 3751
tatgagtaaa cttggtctga cagttaccaa tgcttaatca gtgaggcacc tatctcagcg 3811
atctgtctat ttcgttcatc catagttgcc tgactccccg tcgtgtagat aactacgata 3871
cgggagggct taccatctgg ccccagtgct gcaatgatac cgcgagaccc acgctcaccg 3931
gctccagatt tatcagcaat aaaccagcca gccggaaggg ccgagcgcag aagtggtcct 3991
gcaactttat ccgcctccat ccagtctatt aattgttgcc gggaagctag agtaagtagt 4051
tcgccagtta atagtttgcg caacgttgtt gccattgcta caggcatcgt ggtgtcacgc 4111
tcgtcgtttg gtatggcttc attcagctcc ggttcccaac gatcaaggcg agttacatga 4171
tcccccatgt tgtgcaaaaa agcggttagc tccttcggtc ctccgatcgt tgtcagaagt 4231
aagttggccg cagtgttatc actcatggtt atggcagcac tgcataattc tcttactgtc 4291
atgccatccg taagatgctt ttctgtgact ggtgagtact caaccaagtc attctgagaa 4351
tagtgtatgc ggcgaccgag ttgctcttgc ccggcgtcaa cacgggataa taccgcgcca 4411
catagcagaa ctttaaaagt gctcatcatt ggaaaacgtt cttcggggcg aaaactctca 4471
aggatcttac cgctgttgag atccagttcg atgtaaccca ctcgtgcacc caactgatct 4531
tcagcatctt ttactttcac cagcgtttct gggtgagcaa aaacaggaag gcaaaatgcc 4591
gcaaaaaagg gaataagggc gacacggaaa tgttgaatac tcatactctt cctttttcaa 4651
tattattgaa gcatttatca gggttattgt ctcatgagcg gatacatatt tgaatgtatt 4711
tagaaaaata aacaaaagag tttgtagaaa cgcaaaaagg ccatccgtca ggatggcctt 4771
ctgcttaatt tgatgcctgg cagtttatgg cgggcgtcct gcccgccacc ctccgggccg 4831
ttgcttcgca acgttcaaat ccgctcccgg cggatttgtc ctactcagga gagcgttcac 4891
cgacaaacaa cagataaaac gaaaggccca gtctttcgac tgagcctttc gttttatttg 4951
atgcctggca gttccctact ctcgcatggg gagaccccac actaccatcg gcgctacggc 5011
gtttcacttc tgagttcggc atggggtcag gtgggaccac cgcgctactg ccgccaggca 5071
aattctgttt tatcagaccg cttctgcgtt ctgatttaat ctgtatcagg ctgaaaatct 5131
tctctcatcc gccaaaacag ccaagctagc ggccgatc 5169

<210> 37
<211> 757
<212> PRT
<213> Artificial Sequence
<223> Description of Artificial Sequence: pHL3204

<400> 37
Met Asp Val Asn Pro Thr Leu Leu Phe Leu Lys Val Pro Ala Gln Asn
1 5 10 15

Ala Ile Ser Thr Thr Phe Pro Tyr Thr Gly Asp Pro Pro Tyr Ser His
20 25 30

Gly Thr Gly Thr Gly Tyr Thr Met Asp Thr Val Asn Arg Thr His Gln
35 40 45

Tyr Ser Glu Arg Gly Arg Trp Thr Thr Asn Thr Glu Thr Gly Ala Pro
50 55 60

Gln Leu Asn Pro Ile Asp Gly Pro Leu Pro Glu Asp Asn Glu Pro Ser
65 70 75 80

Gly Tyr Ala Gln Thr Asp Cys Val Leu Glu Ala Met Ala Phe Leu Glu
85 90 95

Glu Ser His Pro Gly Ile Phe Glu Thr Ser Cys Leu Glu Thr Met Glu
100 105 110

Val Val Gln Gln Thr Arg Val Asp Lys Leu Thr Gln Gly Arg Gln Thr
115 120 125

Tyr Asp Trp Thr Leu Asn Arg Asn Gln Pro Ala Ala Thr Ala Leu Ala
130 135 140

Asn Thr Ile Glu Val Phe Arg Ser Asn Gly Leu Thr Ala Asn Glu Ser
145 150 155 160

Gly Arg Leu Ile Asp Phe Leu Lys Asp Val Met Glu Ser Met Asn Lys
165 170 175

Glu Glu Met Glu Ile Thr Thr His Phe Gln Arg Lys Arg Arg Val Arg
180 185 190

Asp Asn Met Thr Lys Lys Met Val Thr Gln Arg Thr Ile Gly Lys Arg
195 200 205

Lys Gln Arg Leu Asn Lys Arg Ser Tyr Leu Ile Arg Ala Leu Thr Leu
210 215 220

Asn Thr Met Thr Lys Asp Ala Glu Arg Gly Lys Leu Lys Arg Arg Ala
225 230 235 240

Ile Ala Thr Pro Gly Met Gln Ile Arg Gly Phe Val Tyr Phe Val Glu
245 250 255

Thr Leu Ala Arg Ser Ile Cys Glu Lys Leu Glu Gln Ser Gly Leu Pro
260 265 270

Val Gly Gly Asn Glu Lys Lys Ala Lys Leu Ala Asn Val Val Arg Lys
275 280 285

Met Met Thr Asn Ser Gln Asp Thr Glu Ile Ser Phe Thr Ile Thr Gly
290 295 300

Asp Asn Thr Lys Trp Asn Glu Asn Gln Asn Pro Arg Met Phe Leu Ala
305 310 315 320

```
Met Ile Thr Tyr Ile Thr Arg Asn Gln Pro Glu Trp Phe Arg Asn Val
                325                 330                 335

Leu Ser Ile Ala Pro Ile Met Phe Ser Asn Lys Met Ala Arg Leu Gly
            340                 345                 350

Lys Gly Tyr Met Phe Glu Ser Lys Ser Met Lys Ile Arg Thr Gln Ile
        355                 360                 365

Pro Ala Glu Met Leu Ala Ser Ile Asp Leu Lys Tyr Phe Asn Asp Ser
    370                 375                 380

Thr Arg Lys Lys Ile Glu Lys Ile Arg Pro Leu Leu Ile Asp Gly Thr
385                 390                 395                 400

Ala Ser Leu Ser Pro Gly Met Met Met Gly Met Phe Asn Met Leu Ser
                405                 410                 415

Thr Val Leu Gly Val Ser Ile Leu Asn Leu Gly Gln Lys Arg His Thr
            420                 425                 430

Lys Thr Thr Tyr Trp Trp Asp Gly Leu Gln Ser Ser Asp Asp Phe Ala
        435                 440                 445

Leu Ile Val Asn Ala Pro Asn His Glu Gly Ile Gln Ala Gly Val Asn
    450                 455                 460

Arg Phe Tyr Arg Thr Cys Lys Leu Leu Gly Ile Asn Met Ser Lys Lys
465                 470                 475                 480

Lys Ser Tyr Ile Asn Arg Thr Gly Thr Phe Glu Phe Thr Ser Phe Phe
                485                 490                 495

Tyr Arg Tyr Gly Phe Val Ala Asn Phe Ser Met Glu Leu Pro Ser Phe
            500                 505                 510

Gly Val Ser Gly Ile Asn Glu Ser Ala Asp Met Ser Ile Gly Val Thr
        515                 520                 525

Val Ile Lys Asn Asn Met Ile Asn Asn Asp Leu Gly Pro Ala Thr Ala
    530                 535                 540

Gln Met Ala Leu Gln Leu Phe Ile Lys Asp Tyr Arg Tyr Thr Tyr Arg
545                 550                 555                 560

Cys His Arg Gly Asp Thr Gln Ile Gln Thr Arg Arg Ser Phe Glu Leu
                565                 570                 575

Lys Lys Leu Trp Glu Gln Thr Arg Ser Lys Ala Gly Leu Leu Val Ser
            580                 585                 590

Asp Gly Gly Pro Asn Leu Tyr Asn Ile Arg Asn Leu His Ile Pro Glu
        595                 600                 605

Val Cys Leu Lys Trp Glu Leu Met Asp Glu Asp Tyr Gln Gly Arg Leu
    610                 615                 620

Cys Asn Pro Leu Asn Pro Phe Val Asn His Lys Asp Ile Glu Ser Val
625                 630                 635                 640

Asn Asn Ala Val Ile Met Pro Ala His Gly Pro Ala Lys Asn Met Glu
                645                 650                 655

Tyr Asp Ala Val Ala Thr Thr His Ser Trp Ile Pro Lys Arg Asn Arg
            660                 665                 670
```

```
Ser Ile Leu Asn Thr Ser Gln Arg Gly Ile Leu Glu Asp Glu Gln Met
        675                 680                 685

Tyr Gln Lys Cys Cys Asn Leu Phe Glu Lys Phe Phe Pro Ser Ser Ser
    690                 695                 700

Tyr Arg Arg Pro Val Gly Ile Ser Ser Met Val Glu Ala Met Val Ser
705                 710                 715                 720

Arg Ala Arg Ile Asp Ala Arg Ile Asp Phe Glu Ser Gly Arg Ile Lys
                725                 730                 735

Lys Glu Glu Phe Thr Glu Ile Met Lys Ile Cys Ser Thr Ile Glu Glu
            740                 745                 750

Leu Arg Arg Gln Lys
        755


<210> 38
<211> 5169
<212> DNA
<213> Artificial Sequence

<220>
<223> Description of Artificial Sequence: pHL3246

<220>
<221> CDS
<222> (191)..(2461)

<400> 38
cccaaaaaaa aaaaaaaaaa aagagtccag agtggccccg ccgctccgcg ccgggggggg 60

gggggggggg ggacactttc ggacatctgg tcgacctcca gcatcggggg aaaaaaaaaa 120

acaaagtgtc gcccggagta ctggtcgacc tccgaagttg ggggggagcg aaagcaggca 180

aaccatttga atg gat gtc aat ccg act tta ctt ttc tta aaa gtg cca     229
           Met Asp Val Asn Pro Thr Leu Leu Phe Leu Lys Val Pro
           1               5                   10

gca caa aat gct ata agc aca act ttc cct tat act gga gac cct cct     277
Ala Gln Asn Ala Ile Ser Thr Thr Phe Pro Tyr Thr Gly Asp Pro Pro
    15                  20                  25

tac agc cat ggg aca gga aca gga tac acc atg gat act gtc aac agg     325
Tyr Ser His Gly Thr Gly Thr Gly Tyr Thr Met Asp Thr Val Asn Arg
30                  35                  40                  45

aca cat cag tac tca gaa agg gga aga tgg aca aca aac acc gaa act     373
Thr His Gln Tyr Ser Glu Arg Gly Arg Trp Thr Thr Asn Thr Glu Thr
                50                  55                  60

gga gca ccg caa ctc aac ccg att gat ggg cca ctg cca gaa gac aat     421
Gly Ala Pro Gln Leu Asn Pro Ile Asp Gly Pro Leu Pro Glu Asp Asn
            65                  70                  75

gaa cca agt ggt tat gcc caa aca gat tgt gta ttg gaa gca atg gcc     469
Glu Pro Ser Gly Tyr Ala Gln Thr Asp Cys Val Leu Glu Ala Met Ala
        80                  85                  90

ttc ctt gag gaa tcc cat cct ggt atc ttt gag acc tcg tgt ctt gaa     517
Phe Leu Glu Glu Ser His Pro Gly Ile Phe Glu Thr Ser Cys Leu Glu
    95                  100                 105
```

```
acg atg gag gtt gtt cag caa aca cga gtg gac aag ctg aca caa ggc    565
Thr Met Glu Val Val Gln Gln Thr Arg Val Asp Lys Leu Thr Gln Gly
110                 115                 120                 125

cga cag acc tat gac tgg act cta aat agg aac cag cct gct gca aca    613
Arg Gln Thr Tyr Asp Trp Thr Leu Asn Arg Asn Gln Pro Ala Ala Thr
                130                 135                 140

gca ttg gcc aac aca ata gaa gtg ttc aga tca aat ggc ctc acg gcc    661
Ala Leu Ala Asn Thr Ile Glu Val Phe Arg Ser Asn Gly Leu Thr Ala
            145                 150                 155

aat gaa tct gga agg ctc ata gac ttc ctt aag gat gta atg gag tca    709
Asn Glu Ser Gly Arg Leu Ile Asp Phe Leu Lys Asp Val Met Glu Ser
        160                 165                 170

atg aac aaa gaa gaa atg gag atc aca act cat ttt cag aga aag aga    757
Met Asn Lys Glu Glu Met Glu Ile Thr Thr His Phe Gln Arg Lys Arg
    175                 180                 185

cga gtg aga gac aat atg act aag aaa atg gtg aca cag aga aca ata    805
Arg Val Arg Asp Asn Met Thr Lys Lys Met Val Thr Gln Arg Thr Ile
190                 195                 200                 205

ggt aaa agg aag cag aga ttg aac aaa agg agt tat cta att agg gca    853
Gly Lys Arg Lys Gln Arg Leu Asn Lys Arg Ser Tyr Leu Ile Arg Ala
                210                 215                 220

tta acc ctg aac aca atg acc aaa gat gct gag aga ggg aag cta aaa    901
Leu Thr Leu Asn Thr Met Thr Lys Asp Ala Glu Arg Gly Lys Leu Lys
            225                 230                 235

cgg aga gca att gca aca cca ggg atg cag atc aga ggg ttt gtg tac    949
Arg Arg Ala Ile Ala Thr Pro Gly Met Gln Ile Arg Gly Phe Val Tyr
        240                 245                 250

ttt gtt gag aca ctg gcg aga agc att tgc gag aag ctt gaa cag tct    997
Phe Val Glu Thr Leu Ala Arg Ser Ile Cys Glu Lys Leu Glu Gln Ser
    255                 260                 265

ggg cta cca gtt gga ggg aat gag aag aaa gct aaa ttg gca aat gtc    1045
Gly Leu Pro Val Gly Gly Asn Glu Lys Lys Ala Lys Leu Ala Asn Val
270                 275                 280                 285

gtg agg aag atg atg acg aac tca caa gac act gag ctc tct ttc aca    1093
Val Arg Lys Met Met Thr Asn Ser Gln Asp Thr Glu Leu Ser Phe Thr
                290                 295                 300

atc acc gga gac aat acc aaa tgg aat gag aac caa aac ccc cga atg    1141
Ile Thr Gly Asp Asn Thr Lys Trp Asn Glu Asn Gln Asn Pro Arg Met
            305                 310                 315

ttc ctg gca atg ata aca tac atc aca aga aac caa cct gag tgg ttt    1189
Phe Leu Ala Met Ile Thr Tyr Ile Thr Arg Asn Gln Pro Glu Trp Phe
        320                 325                 330

aga aat gtc ttg agc atc gcg ccg ata atg ttt tcg aac aaa atg gcg    1237
Arg Asn Val Leu Ser Ile Ala Pro Ile Met Phe Ser Asn Lys Met Ala
    335                 340                 345

agg cta ggg aaa ggg tac atg ttc gaa agc aaa agc atg aag ctc cga    1285
Arg Leu Gly Lys Gly Tyr Met Phe Glu Ser Lys Ser Met Lys Leu Arg
350                 355                 360                 365

aca caa ata cca gca gaa atg cta gca agc atc gat ttg aaa tac ttc    1333
Thr Gln Ile Pro Ala Glu Met Leu Ala Ser Ile Asp Leu Lys Tyr Phe
                370                 375                 380
```

```
aat gat tca act aga aag aag att gaa aaa atc cgg ccg ctc tta ata     1381
Asn Asp Ser Thr Arg Lys Lys Ile Glu Lys Ile Arg Pro Leu Leu Ile
            385                 390                 395

gat ggg act gca tca ttg agc cct gga atg atg atg ggc atg ttc aat     1429
Asp Gly Thr Ala Ser Leu Ser Pro Gly Met Met Met Gly Met Phe Asn
        400                 405                 410

atg tta agt act gta tta ggc gtc tcc atc ctg aat ctt gga caa aag     1477
Met Leu Ser Thr Val Leu Gly Val Ser Ile Leu Asn Leu Gly Gln Lys
    415                 420                 425

aga cac acc aag act act tac tgg tgg gat ggt ctt caa tct tct gat     1525
Arg His Thr Lys Thr Thr Tyr Trp Trp Asp Gly Leu Gln Ser Ser Asp
430                 435                 440                 445

gat ttt gct ctg att gtg aat gca ccc aat cat gaa ggg att caa gcc     1573
Asp Phe Ala Leu Ile Val Asn Ala Pro Asn His Glu Gly Ile Gln Ala
                450                 455                 460

gga gtc aac agg ttt tat cga acc tgt aag cta ctt gga att aat atg     1621
Gly Val Asn Arg Phe Tyr Arg Thr Cys Lys Leu Leu Gly Ile Asn Met
            465                 470                 475

agc aag aaa aag tct tac ata aac aga aca ggt aca ttt gaa ttc aca     1669
Ser Lys Lys Lys Ser Tyr Ile Asn Arg Thr Gly Thr Phe Glu Phe Thr
        480                 485                 490

agc ttt ttc tat cgt tat ggg ttt gtt gcc aat ttc agc atg gag ctt     1717
Ser Phe Phe Tyr Arg Tyr Gly Phe Val Ala Asn Phe Ser Met Glu Leu
    495                 500                 505

ccc agc ttt ggg gtg tct ggg atc aac gag tct gcg gac atg agt att     1765
Pro Ser Phe Gly Val Ser Gly Ile Asn Glu Ser Ala Asp Met Ser Ile
510                 515                 520                 525

gga gtt act gtc atc aaa aac aat atg ata aac aat gat ctt ggt cca     1813
Gly Val Thr Val Ile Lys Asn Asn Met Ile Asn Asn Asp Leu Gly Pro
                530                 535                 540

gca acc gct caa atg gcc ctt cag ctg ttc atc aaa gat tac agg tac     1861
Ala Thr Ala Gln Met Ala Leu Gln Leu Phe Ile Lys Asp Tyr Arg Tyr
            545                 550                 555

acg tac cgg tgc cat aga ggt gac aca caa ata caa acc cga aga tca     1909
Thr Tyr Arg Cys His Arg Gly Asp Thr Gln Ile Gln Thr Arg Arg Ser
        560                 565                 570

ttt gaa ata aag aaa ctg tgg gag caa acc cat tcc aaa gct gga ctg     1957
Phe Glu Ile Lys Lys Leu Trp Glu Gln Thr His Ser Lys Ala Gly Leu
    575                 580                 585

ctg gtc tcc gac gga ggc cca aat tta tac aac att aga aat ctc cac     2005
Leu Val Ser Asp Gly Gly Pro Asn Leu Tyr Asn Ile Arg Asn Leu His
590                 595                 600                 605

att cct gaa gtc tgc ttg aaa tgg gaa tta atg gat gag gat tac cag     2053
Ile Pro Glu Val Cys Leu Lys Trp Glu Leu Met Asp Glu Asp Tyr Gln
                610                 615                 620

ggg cgt tta tgc aac cca ctg aac cca ttt gtc aac cat aaa gac att     2101
Gly Arg Leu Cys Asn Pro Leu Asn Pro Phe Val Asn His Lys Asp Ile
            625                 630                 635
```

```
gaa tca gtg aac aat gca gtg ata atg cca gca cat ggt cca gcc aaa   2149
Glu Ser Val Asn Asn Ala Val Ile Met Pro Ala His Gly Pro Ala Lys
        640                 645                 650

aac atg gag tat gat gct gtt gca aca aca cac tcc tgg atc ccc aaa   2197
Asn Met Glu Tyr Asp Ala Val Ala Thr Thr His Ser Trp Ile Pro Lys
    655                 660                 665

aga aat cga tcc atc ttg aat aca agc caa aga gga ata ctt gaa gat   2245
Arg Asn Arg Ser Ile Leu Asn Thr Ser Gln Arg Gly Ile Leu Glu Asp
670                 675                 680                 685

gaa caa atg tac caa aag tgc tgc aac tta ttt gaa aaa ttc ttc ccc   2293
Glu Gln Met Tyr Gln Lys Cys Cys Asn Leu Phe Glu Lys Phe Phe Pro
                690                 695                 700

agc agt tca tac aga aga cca gtc ggg ata tcc agt atg gtg gag gct   2341
Ser Ser Ser Tyr Arg Arg Pro Val Gly Ile Ser Ser Met Val Glu Ala
            705                 710                 715

atg gtt tcc aga gcc cga att gat gca cga att gat ttc gaa tct gga   2389
Met Val Ser Arg Ala Arg Ile Asp Ala Arg Ile Asp Phe Glu Ser Gly
        720                 725                 730

agg ata aag aaa gag gag ttc act gag atc atg aag atc tgt tcc acc   2437
Arg Ile Lys Lys Glu Glu Phe Thr Glu Ile Met Lys Ile Cys Ser Thr
    735                 740                 745

att gaa gag ctc aga cgg caa aaa tagtgaattt agcttgtcct tcatgaaaaa  2491
Ile Glu Glu Leu Arg Arg Gln Lys
750                 755

atgccttgtt tctactaata acccggcggc ccaaaatgcc gactcggagc gaaagatata 2551

cctcccccgg ggccgggagg tcgcgtcacc gaccacgccg ccggcccagg cgacgcgcga 2611

cacggacacc tgtccccaaa aacgccacca tcgcagccac acacggagcg cccggggccc 2671

tctggtcaac cccaggacac acgcgggagc agcgccgggc cggggacgcc ctcccggccg 2731

cccgtgccac acgcaggggg ccggcccgtg tctccagagc gggagccgga agcattttcg 2791

gccggcccct cctacgaccg ggacacacga gggaccgaag gccggccagg cgcgacctct 2851

cgggccgcac gcgcgctcag ggagcgctct ccgactccgc acggggactc gccagaaagg 2911

atcgtgacct gcattaatga atcaggggat aacgcaggaa agaacatgtg agcaaaaggc 2971

cagcaaaagg ccaggaaccg taaaaaggcc gcgttgctgg cgtttttcca taggctccgc 3031

cccccctgacg agcatcacaa aaatcgacgc tcaagtcaga ggtggcgaaa cccgacagga 3091

ctataaagat accaggcgtt tccccctgga agctccctcg tgcgctctcc tgttccgacc 3151

ctgccgctta ccggatacct gtccgccttt ctcccttcgg gaagcgtggc gctttctcat 3211

agctcacgct gtaggtatct cagttcggtg taggtcgttc gctccaagct gggctgtgtg 3271

cacgaacccc ccgttcagcc cgaccgctgc gccttatccg gtaactatcg tcttgagtcc 3331

aacccggtaa gacacgactt atcgccactg gcagcagcca ctggtaacag gattagcaga 3391

gcgaggtatg taggcggtgc tacagagttc ttgaagtggt ggcctaacta cggctacact 3451

agaaggacag tatttggtat ctgcgctctg ctgaagccag ttaccttcgg aaaaagagtt 3511

ggtagctctt gatccggcaa acaaaccacc gctggtagcg gtggtttttt tgtttgcaag 3571
```

```
cagcagatta cgcgcagaaa aaaaggatct caagaagatc ctttgatctt ttctacgggg 3631
tctgacgctc agtggaacga aaactcacgt taagggattt tggtcatgag attatcaaaa 3691
aggatcttca cctagatcct tttaaattaa aaatgaagtt ttaaatcaat ctaaagtata 3751
tatgagtaaa cttggtctga cagttaccaa tgcttaatca gtgaggcacc tatctcagcg 3811
atctgtctat ttcgttcatc catagttgcc tgactccccg tcgtgtagat aactacgata 3871
cgggagggct taccatctgg ccccagtgct gcaatgatac cgcgagaccc acgctcaccg 3931
gctccagatt tatcagcaat aaaccagcca gccggaaggg ccgagcgcag aagtggtcct 3991
gcaactttat ccgcctccat ccagtctatt aattgttgcc gggaagctag agtaagtagt 4051
tcgccagtta atagtttgcg caacgttgtt gccattgcta caggcatcgt ggtgtcacgc 4111
tcgtcgtttg gtatggcttc attcagctcc ggttcccaac gatcaaggcg agttacatga 4171
tcccccatgt tgtgcaaaaa agcggttagc tccttcggtc ctccgatcgt tgtcagaagt 4231
aagttggccg cagtgttatc actcatggtt atggcagcac tgcataattc tcttactgtc 4291
atgccatccg taagatgctt ttctgtgact ggtgagtact caaccaagtc attctgagaa 4351
tagtgtatgc ggcgaccgag ttgctcttgc ccggcgtcaa cacgggataa taccgcgcca 4411
catagcagaa ctttaaaagt gctcatcatt ggaaaacgtt cttcggggcg aaaactctca 4471
aggatcttac cgctgttgag atccagttcg atgtaaccca ctcgtgcacc caactgatct 4531
tcagcatctt ttactttcac cagcgtttct gggtgagcaa aaacaggaag gcaaaatgcc 4591
gcaaaaaagg gaataagggc gacacggaaa tgttgaatac tcatactctt cctttttcaa 4651
tattattgaa gcatttatca gggttattgt ctcatgagcg gatacatatt tgaatgtatt 4711
tagaaaaata aacaaaagag tttgtagaaa cgcaaaaagg ccatccgtca ggatggcctt 4771
ctgcttaatt tgatgcctgg cagtttatgg cgggcgtcct gcccgccacc ctccgggccg 4831
ttgcttcgca acgttcaaat ccgctcccgg cggatttgtc ctactcagga gagcgttcac 4891
cgacaaacaa cagataaaac gaaaggccca gtctttcgac tgagcctttc gttttatttg 4951
atgcctggca gttccctact ctcgcatggg gagaccccac actaccatcg gcgctacggc 5011
gtttcacttc tgagttcggc atggggtcag gtgggaccac cgcgctactg ccgccaggca 5071
aattctgttt tatcagaccg cttctgcgtt ctgatttaat ctgtatcagg ctgaaaatct 5131
tctctcatcc gccaaaacag ccaagctagc ggccgatc                         5169
```

<210> 39
<211> 757
<212> PRT
<213> Artificial Sequence
<223> Description of Artificial Sequence: pHL3246

<400> 39

```
Met Asp Val Asn Pro Thr Leu Leu Phe Leu Lys Val Pro Ala Gln Asn
  1               5                  10                  15
```

```
Ala Ile Ser Thr Thr Phe Pro Tyr Thr Gly Asp Pro Pro Tyr Ser His
            20                  25                  30

Gly Thr Gly Thr Gly Tyr Thr Met Asp Thr Val Asn Arg Thr His Gln
        35                  40                  45

Tyr Ser Glu Arg Gly Arg Trp Thr Thr Asn Thr Glu Thr Gly Ala Pro
    50                  55                  60

Gln Leu Asn Pro Ile Asp Gly Pro Leu Pro Glu Asp Asn Glu Pro Ser
65                  70                  75                  80

Gly Tyr Ala Gln Thr Asp Cys Val Leu Glu Ala Met Ala Phe Leu Glu
                85                  90                  95

Glu Ser His Pro Gly Ile Phe Glu Thr Ser Cys Leu Glu Thr Met Glu
            100                 105                 110

Val Val Gln Gln Thr Arg Val Asp Lys Leu Thr Gln Gly Arg Gln Thr
        115                 120                 125

Tyr Asp Trp Thr Leu Asn Arg Asn Gln Pro Ala Ala Thr Ala Leu Ala
    130                 135                 140

Asn Thr Ile Glu Val Phe Arg Ser Asn Gly Leu Thr Ala Asn Glu Ser
145                 150                 155                 160

Gly Arg Leu Ile Asp Phe Leu Lys Asp Val Met Glu Ser Met Asn Lys
                165                 170                 175

Glu Glu Met Glu Ile Thr Thr His Phe Gln Arg Lys Arg Arg Val Arg
            180                 185                 190

Asp Asn Met Thr Lys Lys Met Val Thr Gln Arg Thr Ile Gly Lys Arg
        195                 200                 205

Lys Gln Arg Leu Asn Lys Arg Ser Tyr Leu Ile Arg Ala Leu Thr Leu
    210                 215                 220

Asn Thr Met Thr Lys Asp Ala Glu Arg Gly Lys Leu Lys Arg Arg Ala
225                 230                 235                 240

Ile Ala Thr Pro Gly Met Gln Ile Arg Gly Phe Val Tyr Phe Val Glu
                245                 250                 255

Thr Leu Ala Arg Ser Ile Cys Glu Lys Leu Glu Gln Ser Gly Leu Pro
            260                 265                 270

Val Gly Gly Asn Glu Lys Lys Ala Lys Leu Ala Asn Val Val Arg Lys
        275                 280                 285

Met Met Thr Asn Ser Gln Asp Thr Glu Leu Ser Phe Thr Ile Thr Gly
    290                 295                 300

Asp Asn Thr Lys Trp Asn Glu Asn Gln Asn Pro Arg Met Phe Leu Ala
305                 310                 315                 320

Met Ile Thr Tyr Ile Thr Arg Asn Gln Pro Glu Trp Phe Arg Asn Val
                325                 330                 335

Leu Ser Ile Ala Pro Ile Met Phe Ser Asn Lys Met Ala Arg Leu Gly
            340                 345                 350

Lys Gly Tyr Met Phe Glu Ser Lys Ser Met Lys Leu Arg Thr Gln Ile
        355                 360                 365
```

```
Pro Ala Glu Met Leu Ala Ser Ile Asp Leu Lys Tyr Phe Asn Asp Ser
    370                 375                 380

Thr Arg Lys Lys Ile Glu Lys Ile Arg Pro Leu Leu Ile Asp Gly Thr
385                 390                 395                 400

Ala Ser Leu Ser Pro Gly Met Met Met Gly Met Phe Asn Met Leu Ser
                405                 410                 415

Thr Val Leu Gly Val Ser Ile Leu Asn Leu Gly Gln Lys Arg His Thr
            420                 425                 430

Lys Thr Thr Tyr Trp Trp Asp Gly Leu Gln Ser Ser Asp Asp Phe Ala
        435                 440                 445

Leu Ile Val Asn Ala Pro Asn His Glu Gly Ile Gln Ala Gly Val Asn
    450                 455                 460

Arg Phe Tyr Arg Thr Cys Lys Leu Leu Gly Ile Asn Met Ser Lys Lys
465                 470                 475                 480

Lys Ser Tyr Ile Asn Arg Thr Gly Thr Phe Glu Phe Thr Ser Phe Phe
                485                 490                 495

Tyr Arg Tyr Gly Phe Val Ala Asn Phe Ser Met Glu Leu Pro Ser Phe
            500                 505                 510

Gly Val Ser Gly Ile Asn Glu Ser Ala Asp Met Ser Ile Gly Val Thr
        515                 520                 525

Val Ile Lys Asn Asn Met Ile Asn Asn Asp Leu Gly Pro Ala Thr Ala
    530                 535                 540

Gln Met Ala Leu Gln Leu Phe Ile Lys Asp Tyr Arg Tyr Thr Tyr Arg
545                 550                 555                 560

Cys His Arg Gly Asp Thr Gln Ile Gln Thr Arg Arg Ser Phe Glu Ile
                565                 570                 575

Lys Lys Leu Trp Glu Gln Thr His Ser Lys Ala Gly Leu Leu Val Ser
            580                 585                 590

Asp Gly Gly Pro Asn Leu Tyr Asn Ile Arg Asn Leu His Ile Pro Glu
        595                 600                 605

Val Cys Leu Lys Trp Glu Leu Met Asp Glu Asp Tyr Gln Gly Arg Leu
    610                 615                 620

Cys Asn Pro Leu Asn Pro Phe Val Asn His Lys Asp Ile Glu Ser Val
625                 630                 635                 640

Asn Asn Ala Val Ile Met Pro Ala His Gly Pro Ala Lys Asn Met Glu
                645                 650                 655

Tyr Asp Ala Val Ala Thr Thr His Ser Trp Ile Pro Lys Arg Asn Arg
            660                 665                 670

Ser Ile Leu Asn Thr Ser Gln Arg Gly Ile Leu Glu Asp Glu Gln Met
        675                 680                 685

Tyr Gln Lys Cys Cys Asn Leu Phe Glu Lys Phe Phe Pro Ser Ser Ser
    690                 695                 700

Tyr Arg Arg Pro Val Gly Ile Ser Ser Met Val Glu Ala Met Val Ser
705                 710                 715                 720
```

```
Arg Ala Arg Ile Asp Ala Arg Ile Asp Phe Glu Ser Gly Arg Ile Lys
                725                 730                 735

Lys Glu Glu Phe Thr Glu Ile Met Lys Ile Cys Ser Thr Ile Glu Glu
            740                 745                 750

Leu Arg Arg Gln Lys
        755


<210> 40
<211> 5169
<212> DNA
<213> Artificial Sequence

<220>
<223> Description of Artificial Sequence: pHL3247

<220>
<221> CDS
<222> (191)..(2461)

<400> 40
cccaaaaaaa aaaaaaaaaa aagagtccag agtggccccg ccgctccgcg ccgggggggg 60

gggggggggg ggacactttc ggacatctgg tcgacctcca gcatcggggg aaaaaaaaaa 120

acaaagtgtc gcccggagta ctggtcgacc tccgaagttg ggggggagcg aaagcaggca 180

aaccatttga atg gat gtc aat ccg act tta ctt ttc tta aaa gtg cca    229
           Met Asp Val Asn Pro Thr Leu Leu Phe Leu Lys Val Pro
           1               5                   10

gca caa aat gct ata agc aca act ttc cct tat act gga gac cct cct    277
Ala Gln Asn Ala Ile Ser Thr Thr Phe Pro Tyr Thr Gly Asp Pro Pro
    15                  20                  25

tac agc cat ggg aca gga aca gga tac acc atg gat act gtc aac agg    325
Tyr Ser His Gly Thr Gly Thr Gly Tyr Thr Met Asp Thr Val Asn Arg
30                  35                  40                  45

aca cat cag tac tca gaa agg gga aga tgg aca aca aac acc gaa act    373
Thr His Gln Tyr Ser Glu Arg Gly Arg Trp Thr Thr Asn Thr Glu Thr
                50                  55                  60

gga gca ccg caa ctc aac ccg att gat ggg cca ctg cca gaa gac aat    421
Gly Ala Pro Gln Leu Asn Pro Ile Asp Gly Pro Leu Pro Glu Asp Asn
            65                  70                  75

gaa cca agt ggt tat gcc caa aca gat tgt gta ttg gaa gca atg gcc    469
Glu Pro Ser Gly Tyr Ala Gln Thr Asp Cys Val Leu Glu Ala Met Ala
        80                  85                  90

ttc ctt gag gaa tcc cat cct ggt atc ttt gag acc tcg tgt ctt gaa    517
Phe Leu Glu Glu Ser His Pro Gly Ile Phe Glu Thr Ser Cys Leu Glu
    95                  100                 105

acg atg gag gtt gtt cag caa aca cga gtg gac aag ctg aca caa ggc    565
Thr Met Glu Val Val Gln Gln Thr Arg Val Asp Lys Leu Thr Gln Gly
110                 115                 120                 125

cga cag acc tat gac tgg act cta aat agg aac cag cct gct gca aca    613
Arg Gln Thr Tyr Asp Trp Thr Leu Asn Arg Asn Gln Pro Ala Ala Thr
                130                 135                 140
```

```
gca ttg gcc aac aca ata gaa gtg ttc aga tca aat ggc ctc acg gcc    661
Ala Leu Ala Asn Thr Ile Glu Val Phe Arg Ser Asn Gly Leu Thr Ala
            145                 150                 155

aat gaa tct gga agg ctc ata gac ttc ctt aag gat gta atg gag tca    709
Asn Glu Ser Gly Arg Leu Ile Asp Phe Leu Lys Asp Val Met Glu Ser
        160                 165                 170

atg aac aaa gaa gaa atg gag atc aca act cat ttt cag aga aag aga    757
Met Asn Lys Glu Glu Met Glu Ile Thr Thr His Phe Gln Arg Lys Arg
    175                 180                 185

cga gtg aga gac aat atg act aag aaa atg gtg aca cag aga aca ata    805
Arg Val Arg Asp Asn Met Thr Lys Lys Met Val Thr Gln Arg Thr Ile
190                 195                 200                 205

ggt aaa agg aag cag aga ttg aac aaa agg agt tat cta att agg gca    853
Gly Lys Arg Lys Gln Arg Leu Asn Lys Arg Ser Tyr Leu Ile Arg Ala
                210                 215                 220

tta acc ctg aac aca atg acc aaa gat gct gag aga ggg aag cta aaa    901
Leu Thr Leu Asn Thr Met Thr Lys Asp Ala Glu Arg Gly Lys Leu Lys
            225                 230                 235

cgg aga gca att gca acc cca ggg atg caa ata agg ggg ttt gta tac    949
Arg Arg Ala Ile Ala Thr Pro Gly Met Gln Ile Arg Gly Phe Val Tyr
        240                 245                 250

ttt gtt gag aca cta gca agg agt ata tgt gag aaa ctt gaa caa tca    997
Phe Val Glu Thr Leu Ala Arg Ser Ile Cys Glu Lys Leu Glu Gln Ser
    255                 260                 265

gga ttg cca gtt gga ggc aat gag aag aaa gca aag ttg gca aat gtt    1045
Gly Leu Pro Val Gly Gly Asn Glu Lys Lys Ala Lys Leu Ala Asn Val
270                 275                 280                 285

gta agg aag atg atg acc aat tct cag gac act gaa att tct ttc acc    1093
Val Arg Lys Met Met Thr Asn Ser Gln Asp Thr Glu Ile Ser Phe Thr
                290                 295                 300

atc act gga gat aac acc aaa tgg aac gaa aat cag aac cct cgg atg    1141
Ile Thr Gly Asp Asn Thr Lys Trp Asn Glu Asn Gln Asn Pro Arg Met
            305                 310                 315

ttt ttg gcc atg atc aca tat ata acc aga aat cag ccc gaa tgg ttc    1189
Phe Leu Ala Met Ile Thr Tyr Ile Thr Arg Asn Gln Pro Glu Trp Phe
        320                 325                 330

aga aat gtt cta agt att gct cca ata atg ttc tca aac aaa atg gcg    1237
Arg Asn Val Leu Ser Ile Ala Pro Ile Met Phe Ser Asn Lys Met Ala
    335                 340                 345

aga ctg gga aag ggg tac atg ttt gag agc aag agt atg aaa att aga    1285
Arg Leu Gly Lys Gly Tyr Met Phe Glu Ser Lys Ser Met Lys Ile Arg
350                 355                 360                 365

act caa ata cct gca gaa atg cta gca agt att gat cta aaa tat ttc    1333
Thr Gln Ile Pro Ala Glu Met Leu Ala Ser Ile Asp Leu Lys Tyr Phe
                370                 375                 380

aat gaa cca aca agg aag aaa atc gag aaa ata agg cct ctc ata ata    1381
Asn Glu Pro Thr Arg Lys Lys Ile Glu Lys Ile Arg Pro Leu Ile Ile
            385                 390                 395

gac ggc aca gcc tca tta agc ccg gga atg atg atg ggt atg ttc aac    1429
Asp Gly Thr Ala Ser Leu Ser Pro Gly Met Met Met Gly Met Phe Asn
        400                 405                 410
```

```
atg ctg agt aca gtg ttg gga gtc tca atc ctg aat ctt ggg caa aag      1477
Met Leu Ser Thr Val Leu Gly Val Ser Ile Leu Asn Leu Gly Gln Lys
    415                 420                 425

aga tac acc aaa acc aca tac tgg tgg gat gga ctt cag tcc tct gat      1525
Arg Tyr Thr Lys Thr Thr Tyr Trp Trp Asp Gly Leu Gln Ser Ser Asp
430                 435                 440                 445

gat ttt gct ctc atc gtg aat gca cca aat cat gag gga ata caa gcg      1573
Asp Phe Ala Leu Ile Val Asn Ala Pro Asn His Glu Gly Ile Gln Ala
                450                 455                 460

gga gtg gat aga ttc tac aga acc tgc aag cta gtt ggg atc aat atg      1621
Gly Val Asp Arg Phe Tyr Arg Thr Cys Lys Leu Val Gly Ile Asn Met
            465                 470                 475

agc aag aaa aag tcc tat ata aat agg aca gga aca ttc gaa ttc aca      1669
Ser Lys Lys Lys Ser Tyr Ile Asn Arg Thr Gly Thr Phe Glu Phe Thr
        480                 485                 490

agc ttt ttc tat cgt tat ggg ttt gtt gcc aat ttc agc atg gag ctt      1717
Ser Phe Phe Tyr Arg Tyr Gly Phe Val Ala Asn Phe Ser Met Glu Leu
    495                 500                 505

ccc agc ttt ggg gtg tct ggg atc aac gag tct gcg gac atg agt att      1765
Pro Ser Phe Gly Val Ser Gly Ile Asn Glu Ser Ala Asp Met Ser Ile
510                 515                 520                 525

gga gtt act gtc atc aaa aac aat atg ata aac aat gat ctt ggt cca      1813
Gly Val Thr Val Ile Lys Asn Asn Met Ile Asn Asn Asp Leu Gly Pro
                530                 535                 540

gca acc gct caa atg gcc ctt cag ctg ttc atc aaa gat tac agg tac      1861
Ala Thr Ala Gln Met Ala Leu Gln Leu Phe Ile Lys Asp Tyr Arg Tyr
            545                 550                 555

acg tac cgg tgc cat aga ggt gac aca caa ata caa acc cga aga tca      1909
Thr Tyr Arg Cys His Arg Gly Asp Thr Gln Ile Gln Thr Arg Arg Ser
        560                 565                 570

ttt gaa ata aag aaa ctg tgg gag caa acc cat tcc aaa gct gga ctg      1957
Phe Glu Ile Lys Lys Leu Trp Glu Gln Thr His Ser Lys Ala Gly Leu
    575                 580                 585

ctg gtc tcc gac gga ggc cca aat tta tac aac att aga aat ctc cac      2005
Leu Val Ser Asp Gly Gly Pro Asn Leu Tyr Asn Ile Arg Asn Leu His
590                 595                 600                 605

att cct gaa gtc tgc ttg aaa tgg gaa tta atg gat gag gat tac cag      2053
Ile Pro Glu Val Cys Leu Lys Trp Glu Leu Met Asp Glu Asp Tyr Gln
                610                 615                 620

ggg cgt tta tgc aac cca ctg aac cca ttt gtc aac cat aaa gac att      2101
Gly Arg Leu Cys Asn Pro Leu Asn Pro Phe Val Asn His Lys Asp Ile
            625                 630                 635

gaa tca gtg aac aat gca gtg ata atg cca gca cat ggt cca gcc aaa      2149
Glu Ser Val Asn Asn Ala Val Ile Met Pro Ala His Gly Pro Ala Lys
        640                 645                 650

aac atg gag tat gat gct gtt gca aca aca cac tcc tgg atc ccc aaa      2197
Asn Met Glu Tyr Asp Ala Val Ala Thr Thr His Ser Trp Ile Pro Lys
    655                 660                 665
```

```
aga aat cga tcc atc ttg aat aca agc caa aga gga ata ctt gaa gat   2245
Arg Asn Arg Ser Ile Leu Asn Thr Ser Gln Arg Gly Ile Leu Glu Asp
670                 675                 680                 685

gaa caa atg tac caa aag tgc tgc aac tta ttt gaa aaa ttc ttc ccc   2293
Glu Gln Met Tyr Gln Lys Cys Cys Asn Leu Phe Glu Lys Phe Phe Pro
                690                 695                 700

agc agt tca tac aga aga cca gtc ggg ata tcc agt atg gtg gag gct   2341
Ser Ser Ser Tyr Arg Arg Pro Val Gly Ile Ser Ser Met Val Glu Ala
            705                 710                 715

atg gtt tcc aga gcc cga att gat gca cga att gat ttc gaa tct gga   2389
Met Val Ser Arg Ala Arg Ile Asp Ala Arg Ile Asp Phe Glu Ser Gly
        720                 725                 730

agg ata aag aaa gag gag ttc act gag atc atg aag atc tgt tcc acc   2437
Arg Ile Lys Lys Glu Glu Phe Thr Glu Ile Met Lys Ile Cys Ser Thr
    735                 740                 745

att gaa gag ctc aga cgg caa aaa tagtgaattt agcttgtcct tcatgaaaaa  2491
Ile Glu Glu Leu Arg Arg Gln Lys
750                 755

atgccttgtt tctactaata acccggcggc ccaaaatgcc gactcggagc gaaagatata 2551

cctcccccgg ggccgggagg tcgcgtcacc gaccacgccg ccggcccagg cgacgcgcga 2611

cacggacacc tgtccccaaa aacgccacca tcgcagccac acacggagcg cccggggccc 2671

tctggtcaac cccaggacac acgcgggagc agcgccgggc cggggacgcc ctcccggccg 2731

cccgtgccac acgcaggggg ccggcccgtg tctccagagc gggagccgga agcattttcg 2791

gccggcccct cctacgaccg ggacacacga gggaccgaag gccggccagg cgcgacctct 2851

cgggccgcac gcgcgctcag ggagcgctct ccgactccgc acggggactc gccagaaagg 2911

atcgtgacct gcattaatga atcaggggat aacgcaggaa agaacatgtg agcaaaaggc 2971

cagcaaaagg ccaggaaccg taaaaaggcc gcgttgctgg cgtttttcca taggctccgc 3031

cccccctgacg agcatcacaa aaatcgacgc tcaagtcaga ggtggcgaaa cccgacagga 3091

ctataaagat accaggcgtt tccccctgga agctccctcg tgcgctctcc tgttccgacc 3151

ctgccgctta ccggatacct gtccgccttt ctcccttcgg gaagcgtggc gctttctcat 3211

agctcacgct gtaggtatct cagttcggtg taggtcgttc gctccaagct gggctgtgtg 3271

cacgaacccc ccgttcagcc cgaccgctgc gccttatccg gtaactatcg tcttgagtcc 3331

aacccggtaa gacacgactt atcgccactg gcagcagcca ctggtaacag gattagcaga 3391

gcgaggtatg taggcggtgc tacagagttc ttgaagtggt ggcctaacta cggctacact 3451

agaaggacag tatttggtat ctgcgctctg ctgaagccag ttaccttcgg aaaaagagtt 3511

ggtagctctt gatccggcaa acaaaccacc gctggtagcg gtggtttttt tgtttgcaag 3571

cagcagatta cgcgcagaaa aaaaggatct caagaagatc ctttgatctt ttctacgggg 3631

tctgacgctc agtggaacga aaactcacgt taagggattt tggtcatgag attatcaaaa 3691

aggatcttca cctagatcct tttaaattaa aaatgaagtt ttaaatcaat ctaaagtata 3751

tatgagtaaa cttggtctga cagttaccaa tgcttaatca gtgaggcacc tatctcagcg 3811
```

```
atctgtctat ttcgttcatc catagttgcc tgactccccg tcgtgtagat aactacgata 3871
cgggagggct taccatctgg ccccagtgct gcaatgatac cgcgagaccc acgctcaccg 3931
gctccagatt tatcagcaat aaaccagcca gccggaaggg ccgagcgcag aagtggtcct 3991
gcaactttat ccgcctccat ccagtctatt aattgttgcc gggaagctag agtaagtagt 4051
tcgccagtta atagtttgcg caacgttgtt gccattgcta caggcatcgt ggtgtcacgc 4111
tcgtcgtttg gtatggcttc attcagctcc ggttcccaac gatcaaggcg agttacatga 4171
tcccccatgt tgtgcaaaaa agcggttagc tccttcggtc ctccgatcgt tgtcagaagt 4231
aagttggccg cagtgttatc actcatggtt atggcagcac tgcataattc tcttactgtc 4291
atgccatccg taagatgctt ttctgtgact ggtgagtact caaccaagtc attctgagaa 4351
tagtgtatgc ggcgaccgag ttgctcttgc ccggcgtcaa cacgggataa taccgcgcca 4411
catagcagaa ctttaaaagt gctcatcatt ggaaaacgtt cttcggggcg aaaactctca 4471
aggatcttac cgctgttgag atccagttcg atgtaaccca ctcgtgcacc caactgatct 4531
tcagcatctt ttactttcac cagcgtttct gggtgagcaa aaacaggaag gcaaaatgcc 4591
gcaaaaaagg gaataagggc gacacggaaa tgttgaatac tcatactctt cctttttcaa 4651
tattattgaa gcatttatca gggttattgt ctcatgagcg gatacatatt tgaatgtatt 4711
tagaaaaata aacaaaagag tttgtagaaa cgcaaaaagg ccatccgtca ggatggcctt 4771
ctgcttaatt tgatgcctgg cagtttatgg cgggcgtcct gcccgccacc ctccgggccg 4831
ttgcttcgca acgttcaaat ccgctcccgg cggatttgtc ctactcagga gagcgttcac 4891
cgacaaacaa cagataaaac gaaaggccca gtctttcgac tgagcctttc gttttatttg 4951
atgcctggca gttccctact ctcgcatggg gagaccccac actaccatcg gcgctacggc 5011
gtttcacttc tgagttcggc atggggtcag gtgggaccac cgcgctactg ccgccaggca 5071
aattctgttt tatcagaccg cttctgcgtt ctgatttaat ctgtatcagg ctgaaaatct 5131
tctctcatcc gccaaaacag ccaagctagc ggccgatc                         5169
```

<210> 41
<211> 757
<212> PRT
<213> Artificial Sequence
<223> Description of Artificial Sequence: pHL3247

<400> 41

```
Met Asp Val Asn Pro Thr Leu Leu Phe Leu Lys Val Pro Ala Gln Asn
 1               5                  10                  15

Ala Ile Ser Thr Thr Phe Pro Tyr Thr Gly Asp Pro Pro Tyr Ser His
             20                  25                  30

Gly Thr Gly Thr Gly Tyr Thr Met Asp Thr Val Asn Arg Thr His Gln
         35                  40                  45

Tyr Ser Glu Arg Gly Arg Trp Thr Thr Asn Thr Glu Thr Gly Ala Pro
     50                  55                  60
```

```
Gln Leu Asn Pro Ile Asp Gly Pro Leu Pro Glu Asp Asn Glu Pro Ser
 65                  70                  75                  80

Gly Tyr Ala Gln Thr Asp Cys Val Leu Glu Ala Met Ala Phe Leu Glu
                 85                  90                  95

Glu Ser His Pro Gly Ile Phe Glu Thr Ser Cys Leu Glu Thr Met Glu
            100                 105                 110

Val Val Gln Gln Thr Arg Val Asp Lys Leu Thr Gln Gly Arg Gln Thr
        115                 120                 125

Tyr Asp Trp Thr Leu Asn Arg Asn Gln Pro Ala Ala Thr Ala Leu Ala
    130                 135                 140

Asn Thr Ile Glu Val Phe Arg Ser Asn Gly Leu Thr Ala Asn Glu Ser
145                 150                 155                 160

Gly Arg Leu Ile Asp Phe Leu Lys Asp Val Met Glu Ser Met Asn Lys
                165                 170                 175

Glu Glu Met Glu Ile Thr Thr His Phe Gln Arg Lys Arg Arg Val Arg
            180                 185                 190

Asp Asn Met Thr Lys Lys Met Val Thr Gln Arg Thr Ile Gly Lys Arg
        195                 200                 205

Lys Gln Arg Leu Asn Lys Arg Ser Tyr Leu Ile Arg Ala Leu Thr Leu
    210                 215                 220

Asn Thr Met Thr Lys Asp Ala Glu Arg Gly Lys Leu Lys Arg Arg Ala
225                 230                 235                 240

Ile Ala Thr Pro Gly Met Gln Ile Arg Gly Phe Val Tyr Phe Val Glu
                245                 250                 255

Thr Leu Ala Arg Ser Ile Cys Glu Lys Leu Glu Gln Ser Gly Leu Pro
            260                 265                 270

Val Gly Gly Asn Glu Lys Lys Ala Lys Leu Ala Asn Val Val Arg Lys
        275                 280                 285

Met Met Thr Asn Ser Gln Asp Thr Glu Ile Ser Phe Thr Ile Thr Gly
    290                 295                 300

Asp Asn Thr Lys Trp Asn Glu Asn Gln Asn Pro Arg Met Phe Leu Ala
305                 310                 315                 320

Met Ile Thr Tyr Ile Thr Arg Asn Gln Pro Glu Trp Phe Arg Asn Val
                325                 330                 335

Leu Ser Ile Ala Pro Ile Met Phe Ser Asn Lys Met Ala Arg Leu Gly
            340                 345                 350

Lys Gly Tyr Met Phe Glu Ser Lys Ser Met Lys Ile Arg Thr Gln Ile
        355                 360                 365

Pro Ala Glu Met Leu Ala Ser Ile Asp Leu Lys Tyr Phe Asn Glu Pro
    370                 375                 380

Thr Arg Lys Lys Ile Glu Lys Ile Arg Pro Leu Ile Ile Asp Gly Thr
385                 390                 395                 400

Ala Ser Leu Ser Pro Gly Met Met Met Gly Met Phe Asn Met Leu Ser
                405                 410                 415
```

```
Thr Val Leu Gly Val Ser Ile Leu Asn Leu Gly Gln Lys Arg Tyr Thr
            420                 425                 430

Lys Thr Thr Tyr Trp Trp Asp Gly Leu Gln Ser Ser Asp Asp Phe Ala
        435                 440                 445

Leu Ile Val Asn Ala Pro Asn His Glu Gly Ile Gln Ala Gly Val Asp
    450                 455                 460

Arg Phe Tyr Arg Thr Cys Lys Leu Val Gly Ile Asn Met Ser Lys Lys
465                 470                 475                 480

Lys Ser Tyr Ile Asn Arg Thr Gly Thr Phe Glu Phe Thr Ser Phe Phe
                485                 490                 495

Tyr Arg Tyr Gly Phe Val Ala Asn Phe Ser Met Glu Leu Pro Ser Phe
            500                 505                 510

Gly Val Ser Gly Ile Asn Glu Ser Ala Asp Met Ser Ile Gly Val Thr
        515                 520                 525

Val Ile Lys Asn Asn Met Ile Asn Asn Asp Leu Gly Pro Ala Thr Ala
    530                 535                 540

Gln Met Ala Leu Gln Leu Phe Ile Lys Asp Tyr Arg Tyr Thr Tyr Arg
545                 550                 555                 560

Cys His Arg Gly Asp Thr Gln Ile Gln Thr Arg Arg Ser Phe Glu Ile
                565                 570                 575

Lys Lys Leu Trp Glu Gln Thr His Ser Lys Ala Gly Leu Leu Val Ser
            580                 585                 590

Asp Gly Gly Pro Asn Leu Tyr Asn Ile Arg Asn Leu His Ile Pro Glu
        595                 600                 605

Val Cys Leu Lys Trp Glu Leu Met Asp Glu Asp Tyr Gln Gly Arg Leu
    610                 615                 620

Cys Asn Pro Leu Asn Pro Phe Val Asn His Lys Asp Ile Glu Ser Val
625                 630                 635                 640

Asn Asn Ala Val Ile Met Pro Ala His Gly Pro Ala Lys Asn Met Glu
                645                 650                 655

Tyr Asp Ala Val Ala Thr Thr His Ser Trp Ile Pro Lys Arg Asn Arg
            660                 665                 670

Ser Ile Leu Asn Thr Ser Gln Arg Gly Ile Leu Glu Asp Glu Gln Met
        675                 680                 685

Tyr Gln Lys Cys Cys Asn Leu Phe Glu Lys Phe Phe Pro Ser Ser Ser
    690                 695                 700

Tyr Arg Arg Pro Val Gly Ile Ser Ser Met Val Glu Ala Met Val Ser
705                 710                 715                 720

Arg Ala Arg Ile Asp Ala Arg Ile Asp Phe Glu Ser Gly Arg Ile Lys
                725                 730                 735

Lys Glu Glu Phe Thr Glu Ile Met Lys Ile Cys Ser Thr Ile Glu Glu
            740                 745                 750

Leu Arg Arg Gln Lys
        755
```

```
<210> 42
<211> 5169
<212> DNA
<213> Artificial Sequence

<220>
<223> Description of Artificial Sequence: pHL3258

<220>
<221> CDS
<222> (191)..(2461)

<400> 42
cccaaaaaaa aaaaaaaaaa aagagtccag agtggccccg ccgctccgcg ccgggggggg 60

gggggggggg ggacactttc ggacatctgg tcgacctcca gcatcggggg aaaaaaaaaa 120

acaaagtgtc gcccggagta ctggtcgacc tccgaagttg ggggggagcg aaagcaggca 180

aaccatttga atg gat gtc aat ccg act tta ctt ttc tta aaa gtg cca    229
           Met Asp Val Asn Pro Thr Leu Leu Phe Leu Lys Val Pro
             1               5                  10

gca caa aat gct ata agc aca act ttc cct tat act gga gac cct cct    277
Ala Gln Asn Ala Ile Ser Thr Thr Phe Pro Tyr Thr Gly Asp Pro Pro
     15                  20                  25

tac agc cat ggg aca gga aca gga tac acc atg gat act gtc aac agg    325
Tyr Ser His Gly Thr Gly Thr Gly Tyr Thr Met Asp Thr Val Asn Arg
 30                  35                  40                  45

aca cat cag tac tca gaa agg gga aga tgg aca aca aac acc gaa act    373
Thr His Gln Tyr Ser Glu Arg Gly Arg Trp Thr Thr Asn Thr Glu Thr
                 50                  55                  60

gga gca ccg caa ctc aac ccg att gat ggg cca ctg cca gaa gac aat    421
Gly Ala Pro Gln Leu Asn Pro Ile Asp Gly Pro Leu Pro Glu Asp Asn
             65                  70                  75

gaa cca agt ggt tat gcc caa aca gat tgt gta ttg gaa gca atg gcc    469
Glu Pro Ser Gly Tyr Ala Gln Thr Asp Cys Val Leu Glu Ala Met Ala
         80                  85                  90

ttc ctt gag gaa tcc cat cct ggt atc ttt gag acc tcg tgt ctt gaa    517
Phe Leu Glu Glu Ser His Pro Gly Ile Phe Glu Thr Ser Cys Leu Glu
     95                 100                 105

acg atg gag gtt gtt cag caa aca cga gtg gac aag ctg aca caa ggc    565
Thr Met Glu Val Val Gln Gln Thr Arg Val Asp Lys Leu Thr Gln Gly
110                 115                 120                 125

cga cag acc tat gac tgg act cta aat agg aac cag cct gct gca aca    613
Arg Gln Thr Tyr Asp Trp Thr Leu Asn Arg Asn Gln Pro Ala Ala Thr
                130                 135                 140

gca ttg gcc aac aca ata gaa gtg ttc aga tca aat ggc ctc acg gcc    661
Ala Leu Ala Asn Thr Ile Glu Val Phe Arg Ser Asn Gly Leu Thr Ala
            145                 150                 155

aat gaa tct gga agg ctc ata gac ttc ctt aag gat gta atg gag tca    709
Asn Glu Ser Gly Arg Leu Ile Asp Phe Leu Lys Asp Val Met Glu Ser
        160                 165                 170

atg aac aaa gaa gaa atg gag atc aca act cat ttt cag aga aag aga    757
Met Asn Lys Glu Glu Met Glu Ile Thr Thr His Phe Gln Arg Lys Arg
    175                 180                 185
```

```
cga gtg aga gac aat atg act aag aaa atg gtg aca cag aga aca ata    805
Arg Val Arg Asp Asn Met Thr Lys Lys Met Val Thr Gln Arg Thr Ile
190                 195                 200                 205

ggt aaa agg aag cag aga ttg aac aaa agg agt tat cta att agg gca    853
Gly Lys Arg Lys Gln Arg Leu Asn Lys Arg Ser Tyr Leu Ile Arg Ala
                210                 215                 220

tta acc ctg aac aca atg acc aaa gat gct gag aga ggg aag cta aaa    901
Leu Thr Leu Asn Thr Met Thr Lys Asp Ala Glu Arg Gly Lys Leu Lys
            225                 230                 235

cgg aga gca att gca acc cca ggg atg caa ata agg ggg ttt gta tac    949
Arg Arg Ala Ile Ala Thr Pro Gly Met Gln Ile Arg Gly Phe Val Tyr
        240                 245                 250

ttt gtt gag aca cta gca agg agt ata tgt gag aaa ctt gaa caa tca    997
Phe Val Glu Thr Leu Ala Arg Ser Ile Cys Glu Lys Leu Glu Gln Ser
    255                 260                 265

gga ttg cca gtt gga ggc aat gag aag aaa gca aag ttg gca aat gtt    1045
Gly Leu Pro Val Gly Gly Asn Glu Lys Lys Ala Lys Leu Ala Asn Val
270                 275                 280                 285

gta agg aag atg atg acc aat tct cag gac act gaa att tct ttc acc    1093
Val Arg Lys Met Met Thr Asn Ser Gln Asp Thr Glu Ile Ser Phe Thr
                290                 295                 300

atc act gga gat aac acc aaa tgg aac gaa aat cag aac cct cgg atg    1141
Ile Thr Gly Asp Asn Thr Lys Trp Asn Glu Asn Gln Asn Pro Arg Met
            305                 310                 315

ttt ttg gcc atg atc aca tat ata acc aga aat cag ccc gaa tgg ttc    1189
Phe Leu Ala Met Ile Thr Tyr Ile Thr Arg Asn Gln Pro Glu Trp Phe
        320                 325                 330

aga aat gtt cta agt att gct cca ata atg ttc tca aac aaa atg gcg    1237
Arg Asn Val Leu Ser Ile Ala Pro Ile Met Phe Ser Asn Lys Met Ala
    335                 340                 345

aga ctg gga aag ggg tac atg ttt gag agc aag agt atg aaa att aga    1285
Arg Leu Gly Lys Gly Tyr Met Phe Glu Ser Lys Ser Met Lys Ile Arg
350                 355                 360                 365

act caa ata cct gca gaa atg cta gca agt att gat cta aaa tat ttc    1333
Thr Gln Ile Pro Ala Glu Met Leu Ala Ser Ile Asp Leu Lys Tyr Phe
                370                 375                 380

aat gaa cca aca agg aag aaa atc gag aaa ata agg cct ctc tta ata    1381
Asn Glu Pro Thr Arg Lys Lys Ile Glu Lys Ile Arg Pro Leu Leu Ile
            385                 390                 395

gat ggg act gca tca ttg agc cct gga atg atg atg ggc atg ttc aat    1429
Asp Gly Thr Ala Ser Leu Ser Pro Gly Met Met Met Gly Met Phe Asn
        400                 405                 410

atg tta agt act gta tta ggc gtc tcc atc ctg aat ctt gga caa aag    1477
Met Leu Ser Thr Val Leu Gly Val Ser Ile Leu Asn Leu Gly Gln Lys
    415                 420                 425

aga cac acc aag act act tac tgg tgg gat ggt ctt caa tct tct gat    1525
Arg His Thr Lys Thr Thr Tyr Trp Trp Asp Gly Leu Gln Ser Ser Asp
430                 435                 440                 445

gat ttt gct ctg att gtg aat gca ccc aat cat gaa ggg att caa gcc    1573
Asp Phe Ala Leu Ile Val Asn Ala Pro Asn His Glu Gly Ile Gln Ala
                450                 455                 460
```

```
gga gtc aac agg ttt tat cga acc tgt aag cta ctt gga att aat atg    1621
Gly Val Asn Arg Phe Tyr Arg Thr Cys Lys Leu Leu Gly Ile Asn Met
            465                 470                 475

agc aag aaa aag tct tac ata aac aga aca ggt aca ttt gaa ttc aca    1669
Ser Lys Lys Lys Ser Tyr Ile Asn Arg Thr Gly Thr Phe Glu Phe Thr
        480                 485                 490

agc ttt ttc tat cgt tat ggg ttt gtt gcc aat ttc agc atg gag ctt    1717
Ser Phe Phe Tyr Arg Tyr Gly Phe Val Ala Asn Phe Ser Met Glu Leu
    495                 500                 505

ccc agc ttt ggg gtg tct ggg atc aac gag tct gcg gac atg agt att    1765
Pro Ser Phe Gly Val Ser Gly Ile Asn Glu Ser Ala Asp Met Ser Ile
510                 515                 520                 525

gga gtt act gtc atc aaa aac aat atg ata aac aat gat ctt ggt cca    1813
Gly Val Thr Val Ile Lys Asn Asn Met Ile Asn Asn Asp Leu Gly Pro
                530                 535                 540

gca acc gct caa atg gcc ctt cag ctg ttc atc aaa gat tac agg tac    1861
Ala Thr Ala Gln Met Ala Leu Gln Leu Phe Ile Lys Asp Tyr Arg Tyr
            545                 550                 555

acg tac cgg tgc cat aga ggt gac aca caa ata caa acc cga aga tca    1909
Thr Tyr Arg Cys His Arg Gly Asp Thr Gln Ile Gln Thr Arg Arg Ser
        560                 565                 570

ttt gaa ata aag aaa ctg tgg gag caa acc cat tcc aaa gct gga ctg    1957
Phe Glu Ile Lys Lys Leu Trp Glu Gln Thr His Ser Lys Ala Gly Leu
    575                 580                 585

ctg gtc tcc gac gga ggc cca aat tta tac aac att aga aat ctc cac    2005
Leu Val Ser Asp Gly Gly Pro Asn Leu Tyr Asn Ile Arg Asn Leu His
590                 595                 600                 605

att cct gaa gtc tgc ttg aaa tgg gaa tta atg gat gag gat tac cag    2053
Ile Pro Glu Val Cys Leu Lys Trp Glu Leu Met Asp Glu Asp Tyr Gln
                610                 615                 620

ggg cgt tta tgc aac cca ctg aac cca ttt gtc aac cat aaa gac att    2101
Gly Arg Leu Cys Asn Pro Leu Asn Pro Phe Val Asn His Lys Asp Ile
            625                 630                 635

gaa tca gtg aac aat gca gtg ata atg cca gca cat ggt cca gcc aaa    2149
Glu Ser Val Asn Asn Ala Val Ile Met Pro Ala His Gly Pro Ala Lys
        640                 645                 650

aac atg gag tat gat gct gtt gca aca aca cac tcc tgg atc ccc aaa    2197
Asn Met Glu Tyr Asp Ala Val Ala Thr Thr His Ser Trp Ile Pro Lys
    655                 660                 665

aga aat cga tcc atc ttg aat aca agc caa aga gga ata ctt gaa gat    2245
Arg Asn Arg Ser Ile Leu Asn Thr Ser Gln Arg Gly Ile Leu Glu Asp
670                 675                 680                 685

gaa caa atg tac caa aag tgc tgc aac tta ttt gaa aaa ttc ttc ccc    2293
Glu Gln Met Tyr Gln Lys Cys Cys Asn Leu Phe Glu Lys Phe Phe Pro
                690                 695                 700

agc agt tca tac aga aga cca gtc ggg ata tcc agt atg gtg gag gct    2341
Ser Ser Ser Tyr Arg Arg Pro Val Gly Ile Ser Ser Met Val Glu Ala
            705                 710                 715
```

```
atg gtt tcc aga gcc cga att gat gca cga att gat ttc gaa tct gga   2389
Met Val Ser Arg Ala Arg Ile Asp Ala Arg Ile Asp Phe Glu Ser Gly
        720                 725                 730

agg ata aag aaa gag gag ttc act gag atc atg aag atc tgt tcc acc   2437
Arg Ile Lys Lys Glu Glu Phe Thr Glu Ile Met Lys Ile Cys Ser Thr
    735                 740                 745

att gaa gag ctc aga cgg caa aaa tagtgaattt agcttgtcct tcatgaaaaa  2491
Ile Glu Glu Leu Arg Arg Gln Lys
750                 755

atgccttgtt tctactaata acccggcggc ccaaaatgcc gactcggagc gaaagatata 2551

cctcccccgg ggccgggagg tcgcgtcacc gaccacgccg ccggcccagg cgacgcgcga 2611

cacggacacc tgtccccaaa aacgccacca tcgcagccac acacggagcg cccggggccc 2671

tctggtcaac cccaggacac acgcgggagc agcgccgggc cggggacgcc ctcccggccg 2731

cccgtgccac acgcaggggg ccggcccgtg tctccagagc gggagccgga agcattttcg 2791

gccggcccct cctacgaccg ggacacacga gggaccgaag gccggccagg cgcgacctct 2851

cgggccgcac gcgcgctcag ggagcgctct ccgactccgc acggggactc gccagaaagg 2911

atcgtgacct gcattaatga atcaggggat aacgcaggaa agaacatgtg agcaaaaggc 2971

cagcaaaagg ccaggaaccg taaaaaggcc gcgttgctgg cgtttttcca taggctccgc 3031

cccccctgacg agcatcacaa aaatcgacgc tcaagtcaga ggtggcgaaa cccgacagga 3091

ctataaagat accaggcgtt tccccctgga agctccctcg tgcgctctcc tgttccgacc 3151

ctgccgctta ccggatacct gtccgccttt ctcccttcgg gaagcgtggc gctttctcat 3211

agctcacgct gtaggtatct cagttcggtg taggtcgttc gctccaagct gggctgtgtg 3271

cacgaacccc ccgttcagcc cgaccgctgc gccttatccg gtaactatcg tcttgagtcc 3331

aacccggtaa gacacgactt atcgccactg gcagcagcca ctggtaacag gattagcaga 3391

gcgaggtatg taggcggtgc tacagagttc ttgaagtggt ggcctaacta cggctacact 3451

agaaggacag tatttggtat ctgcgctctg ctgaagccag ttaccttcgg aaaaagagtt 3511

ggtagctctt gatccggcaa acaaaccacc gctggtagcg gtggtttttt tgtttgcaag 3571

cagcagatta cgcgcagaaa aaaaggatct caagaagatc ctttgatctt ttctacgggg 3631

tctgacgctc agtggaacga aaactcacgt taagggattt tggtcatgag attatcaaaa 3691

aggatcttca cctagatcct tttaaattaa aaatgaagtt ttaaatcaat ctaaagtata 3751

tatgagtaaa cttggtctga cagttaccaa tgcttaatca gtgaggcacc tatctcagcg 3811

atctgtctat ttcgttcatc catagttgcc tgactccccg tcgtgtagat aactacgata 3871

cgggagggct taccatctgg ccccagtgct gcaatgatac cgcgagaccc acgctcaccg 3931

gctccagatt tatcagcaat aaaccagcca gccggaaggg ccgagcgcag aagtggtcct 3991

gcaactttat ccgcctccat ccagtctatt aattgttgcc gggaagctag agtaagtagt 4051

tcgccagtta atagtttgcg caacgttgtt gccattgcta caggcatcgt ggtgtcacgc 4111

tcgtcgtttg gtatggcttc attcagctcc ggttcccaac gatcaaggcg agttacatga 4171
```

tcccccatgt tgtgcaaaaa agcggttagc tccttcggtc ctccgatcgt tgtcagaagt 4231

aagttggccg cagtgttatc actcatggtt atggcagcac tgcataattc tcttactgtc 4291

atgccatccg taagatgctt ttctgtgact ggtgagtact caaccaagtc attctgagaa 4351

tagtgtatgc ggcgaccgag ttgctcttgc ccggcgtcaa cacgggataa taccgcgcca 4411

catagcagaa ctttaaaagt gctcatcatt ggaaaacgtt cttcggggcg aaaactctca 4471

aggatcttac cgctgttgag atccagttcg atgtaaccca ctcgtgcacc caactgatct 4531

tcagcatctt ttactttcac cagcgtttct gggtgagcaa aaacaggaag gcaaaatgcc 4591

gcaaaaaagg gaataagggc gacacggaaa tgttgaatac tcatactctt cctttttcaa 4651

tattattgaa gcatttatca gggttattgt ctcatgagcg gatacatatt tgaatgtatt 4711

tagaaaaata aacaaaagag tttgtagaaa cgcaaaaagg ccatccgtca ggatggcctt 4771

ctgcttaatt tgatgcctgg cagtttatgg cgggcgtcct gcccgccacc ctccgggccg 4831

ttgcttcgca acgttcaaat ccgctcccgg cggatttgtc ctactcagga gagcgttcac 4891

cgacaaacaa cagataaaac gaaaggccca gtctttcgac tgagcctttc gttttatttg 4951

atgcctggca gttccctact ctcgcatggg gagaccccac actaccatcg gcgctacggc 5011

gtttcacttc tgagttcggc atggggtcag gtgggaccac cgcgctactg ccgccaggca 5071

aattctgttt tatcagaccg cttctgcgtt ctgatttaat ctgtatcagg ctgaaaatct 5131

tctctcatcc gccaaaacag ccaagctagc ggccgatc 5169

<210> 43
<211> 757
<212> PRT
<213> Artificial Sequence
<223> Description of Artificial Sequence: pHL3258

<400> 43

```
Met Asp Val Asn Pro Thr Leu Leu Phe Leu Lys Val Pro Ala Gln Asn
 1               5                  10                  15

Ala Ile Ser Thr Thr Phe Pro Tyr Thr Gly Asp Pro Pro Tyr Ser His
            20                  25                  30

Gly Thr Gly Thr Gly Tyr Thr Met Asp Thr Val Asn Arg Thr His Gln
        35                  40                  45

Tyr Ser Glu Arg Gly Arg Trp Thr Thr Asn Thr Glu Thr Gly Ala Pro
    50                  55                  60

Gln Leu Asn Pro Ile Asp Gly Pro Leu Pro Glu Asp Asn Glu Pro Ser
65                  70                  75                  80

Gly Tyr Ala Gln Thr Asp Cys Val Leu Glu Ala Met Ala Phe Leu Glu
                85                  90                  95

Glu Ser His Pro Gly Ile Phe Glu Thr Ser Cys Leu Glu Thr Met Glu
            100                 105                 110

Val Val Gln Gln Thr Arg Val Asp Lys Leu Thr Gln Gly Arg Gln Thr
        115                 120                 125
```

```
Tyr Asp Trp Thr Leu Asn Arg Asn Gln Pro Ala Ala Thr Ala Leu Ala
    130                 135                 140

Asn Thr Ile Glu Val Phe Arg Ser Asn Gly Leu Thr Ala Asn Glu Ser
145                 150                 155                 160

Gly Arg Leu Ile Asp Phe Leu Lys Asp Val Met Glu Ser Met Asn Lys
                165                 170                 175

Glu Glu Met Glu Ile Thr Thr His Phe Gln Arg Lys Arg Arg Val Arg
            180                 185                 190

Asp Asn Met Thr Lys Lys Met Val Thr Gln Arg Thr Ile Gly Lys Arg
        195                 200                 205

Lys Gln Arg Leu Asn Lys Arg Ser Tyr Leu Ile Arg Ala Leu Thr Leu
    210                 215                 220

Asn Thr Met Thr Lys Asp Ala Glu Arg Gly Lys Leu Lys Arg Arg Ala
225                 230                 235                 240

Ile Ala Thr Pro Gly Met Gln Ile Arg Gly Phe Val Tyr Phe Val Glu
                245                 250                 255

Thr Leu Ala Arg Ser Ile Cys Glu Lys Leu Glu Gln Ser Gly Leu Pro
            260                 265                 270

Val Gly Gly Asn Glu Lys Lys Ala Lys Leu Ala Asn Val Val Arg Lys
        275                 280                 285

Met Met Thr Asn Ser Gln Asp Thr Glu Ile Ser Phe Thr Ile Thr Gly
    290                 295                 300

Asp Asn Thr Lys Trp Asn Glu Asn Gln Asn Pro Arg Met Phe Leu Ala
305                 310                 315                 320

Met Ile Thr Tyr Ile Thr Arg Asn Gln Pro Glu Trp Phe Arg Asn Val
                325                 330                 335

Leu Ser Ile Ala Pro Ile Met Phe Ser Asn Lys Met Ala Arg Leu Gly
            340                 345                 350

Lys Gly Tyr Met Phe Glu Ser Lys Ser Met Lys Ile Arg Thr Gln Ile
        355                 360                 365

Pro Ala Glu Met Leu Ala Ser Ile Asp Leu Lys Tyr Phe Asn Glu Pro
    370                 375                 380

Thr Arg Lys Lys Ile Glu Lys Ile Arg Pro Leu Leu Ile Asp Gly Thr
385                 390                 395                 400

Ala Ser Leu Ser Pro Gly Met Met Met Gly Met Phe Asn Met Leu Ser
                405                 410                 415

Thr Val Leu Gly Val Ser Ile Leu Asn Leu Gly Gln Lys Arg His Thr
            420                 425                 430

Lys Thr Thr Tyr Trp Trp Asp Gly Leu Gln Ser Ser Asp Asp Phe Ala
        435                 440                 445

Leu Ile Val Asn Ala Pro Asn His Glu Gly Ile Gln Ala Gly Val Asn
    450                 455                 460

Arg Phe Tyr Arg Thr Cys Lys Leu Leu Gly Ile Asn Met Ser Lys Lys
465                 470                 475                 480
```

```
Lys Ser Tyr Ile Asn Arg Thr Gly Thr Phe Glu Phe Thr Ser Phe Phe
                485                 490                 495

Tyr Arg Tyr Gly Phe Val Ala Asn Phe Ser Met Glu Leu Pro Ser Phe
            500                 505                 510

Gly Val Ser Gly Ile Asn Glu Ser Ala Asp Met Ser Ile Gly Val Thr
        515                 520                 525

Val Ile Lys Asn Asn Met Ile Asn Asn Asp Leu Gly Pro Ala Thr Ala
    530                 535                 540

Gln Met Ala Leu Gln Leu Phe Ile Lys Asp Tyr Arg Tyr Thr Tyr Arg
545                 550                 555                 560

Cys His Arg Gly Asp Thr Gln Ile Gln Thr Arg Arg Ser Phe Glu Ile
                565                 570                 575

Lys Lys Leu Trp Glu Gln Thr His Ser Lys Ala Gly Leu Leu Val Ser
            580                 585                 590

Asp Gly Gly Pro Asn Leu Tyr Asn Ile Arg Asn Leu His Ile Pro Glu
        595                 600                 605

Val Cys Leu Lys Trp Glu Leu Met Asp Glu Asp Tyr Gln Gly Arg Leu
    610                 615                 620

Cys Asn Pro Leu Asn Pro Phe Val Asn His Lys Asp Ile Glu Ser Val
625                 630                 635                 640

Asn Asn Ala Val Ile Met Pro Ala His Gly Pro Ala Lys Asn Met Glu
                645                 650                 655

Tyr Asp Ala Val Ala Thr Thr His Ser Trp Ile Pro Lys Arg Asn Arg
            660                 665                 670

Ser Ile Leu Asn Thr Ser Gln Arg Gly Ile Leu Glu Asp Glu Gln Met
        675                 680                 685

Tyr Gln Lys Cys Cys Asn Leu Phe Glu Lys Phe Phe Pro Ser Ser Ser
    690                 695                 700

Tyr Arg Arg Pro Val Gly Ile Ser Ser Met Val Glu Ala Met Val Ser
705                 710                 715                 720

Arg Ala Arg Ile Asp Ala Arg Ile Asp Phe Glu Ser Gly Arg Ile Lys
                725                 730                 735

Lys Glu Glu Phe Thr Glu Ile Met Lys Ile Cys Ser Thr Ile Glu Glu
            740                 745                 750

Leu Arg Arg Gln Lys
        755


<210> 44
<211> 5169
<212> DNA
<213> Artificial Sequence

<220>
<223> Description of Artificial Sequence: pHL3259

<220>
<221> CDS
<222> (191)..(2461)
```

```
<400> 44
cccaaaaaaa aaaaaaaaaa aagagtccag agtggccccg ccgctccgcg ccgggggggg 60

gggggggggg ggacactttc ggacatctgg tcgacctcca gcatcggggg aaaaaaaaaa 120

acaaagtgtc gcccggagta ctggtcgacc tccgaagttg gggggagcg aaagcaggca 180

aaccatttga atg gat gtc aat ccg act tta ctt ttc tta aaa gtg cca     229
           Met Asp Val Asn Pro Thr Leu Leu Phe Leu Lys Val Pro
            1               5                   10

gca caa aat gct ata agc aca act ttc cct tat act gga gac cct cct    277
Ala Gln Asn Ala Ile Ser Thr Thr Phe Pro Tyr Thr Gly Asp Pro Pro
    15                  20                  25

tac agc cat ggg aca gga aca gga tac acc atg gat act gtc aac agg    325
Tyr Ser His Gly Thr Gly Thr Gly Tyr Thr Met Asp Thr Val Asn Arg
30                  35                  40                  45

aca cat cag tac tca gaa agg gga aga tgg aca aca aac acc gaa act    373
Thr His Gln Tyr Ser Glu Arg Gly Arg Trp Thr Thr Asn Thr Glu Thr
                50                  55                  60

gga gca ccg caa ctc aac ccg att gat ggg cca ctg cca gaa gac aat    421
Gly Ala Pro Gln Leu Asn Pro Ile Asp Gly Pro Leu Pro Glu Asp Asn
            65                  70                  75

gaa cca agt ggt tat gcc caa aca gat tgt gta ttg gaa gca atg gcc    469
Glu Pro Ser Gly Tyr Ala Gln Thr Asp Cys Val Leu Glu Ala Met Ala
        80                  85                  90

ttc ctt gag gaa tcc cat cct ggt atc ttt gag acc tcg tgt ctt gaa    517
Phe Leu Glu Glu Ser His Pro Gly Ile Phe Glu Thr Ser Cys Leu Glu
    95                  100                 105

acg atg gag gtt gtt cag caa aca cga gtg gac aag ctg aca caa ggc    565
Thr Met Glu Val Val Gln Gln Thr Arg Val Asp Lys Leu Thr Gln Gly
110                 115                 120                 125

cga cag acc tat gac tgg act cta aat agg aac cag cct gct gca aca    613
Arg Gln Thr Tyr Asp Trp Thr Leu Asn Arg Asn Gln Pro Ala Ala Thr
                130                 135                 140

gca ttg gcc aac aca ata gaa gtg ttc aga tca aat ggc ctc acg gcc    661
Ala Leu Ala Asn Thr Ile Glu Val Phe Arg Ser Asn Gly Leu Thr Ala
            145                 150                 155

aat gaa tct gga agg ctc ata gac ttc ctt aag gat gta atg gag tca    709
Asn Glu Ser Gly Arg Leu Ile Asp Phe Leu Lys Asp Val Met Glu Ser
        160                 165                 170

atg aac aaa gaa gaa atg gag atc aca act cat ttt cag aga aag aga    757
Met Asn Lys Glu Glu Met Glu Ile Thr Thr His Phe Gln Arg Lys Arg
    175                 180                 185

cga gtg aga gac aat atg act aag aaa atg gtg aca cag aga aca ata    805
Arg Val Arg Asp Asn Met Thr Lys Lys Met Val Thr Gln Arg Thr Ile
190                 195                 200                 205

ggt aaa agg aag cag aga ttg aac aaa agg agt tat cta att agg gca    853
Gly Lys Arg Lys Gln Arg Leu Asn Lys Arg Ser Tyr Leu Ile Arg Ala
                210                 215                 220

tta acc ctg aac aca atg acc aaa gat gct gag aga ggg aag cta aaa    901
Leu Thr Leu Asn Thr Met Thr Lys Asp Ala Glu Arg Gly Lys Leu Lys
            225                 230                 235
```

```
cgg aga gca att gca acc cca ggg atg caa ata agg ggg ttt gta tac     949
Arg Arg Ala Ile Ala Thr Pro Gly Met Gln Ile Arg Gly Phe Val Tyr
        240                 245                 250

ttt gtt gag aca cta gca agg agt ata tgt gag aaa ctt gaa caa tca     997
Phe Val Glu Thr Leu Ala Arg Ser Ile Cys Glu Lys Leu Glu Gln Ser
    255                 260                 265

gga ttg cca gtt gga ggc aat gag aag aaa gca aag ttg gca aat gtt    1045
Gly Leu Pro Val Gly Gly Asn Glu Lys Lys Ala Lys Leu Ala Asn Val
270                 275                 280                 285

gta agg aag atg atg acc aat tct cag gac act gaa att tct ttc acc    1093
Val Arg Lys Met Met Thr Asn Ser Gln Asp Thr Glu Ile Ser Phe Thr
                290                 295                 300

atc act gga gat aac acc aaa tgg aac gaa aat cag aac cct cgg atg    1141
Ile Thr Gly Asp Asn Thr Lys Trp Asn Glu Asn Gln Asn Pro Arg Met
            305                 310                 315

ttt ttg gcc atg atc aca tat ata acc aga aat cag ccc gaa tgg ttc    1189
Phe Leu Ala Met Ile Thr Tyr Ile Thr Arg Asn Gln Pro Glu Trp Phe
        320                 325                 330

aga aat gtt cta agt att gct cca ata atg ttc tca aac aaa atg gcg    1237
Arg Asn Val Leu Ser Ile Ala Pro Ile Met Phe Ser Asn Lys Met Ala
    335                 340                 345

aga ctg gga aag ggg tac atg ttt gag agc aag agt atg aaa att aga    1285
Arg Leu Gly Lys Gly Tyr Met Phe Glu Ser Lys Ser Met Lys Ile Arg
350                 355                 360                 365

act caa ata cct gca gaa atg cta gca agt att gat cta aaa tat ttc    1333
Thr Gln Ile Pro Ala Glu Met Leu Ala Ser Ile Asp Leu Lys Tyr Phe
                370                 375                 380

aat gaa cca aca agg aag aaa atc gag aaa ata agg cct ctc tta ata    1381
Asn Glu Pro Thr Arg Lys Lys Ile Glu Lys Ile Arg Pro Leu Leu Ile
            385                 390                 395

gat ggg act gca tca ttg agc cct gga atg atg atg ggc atg ttc aat    1429
Asp Gly Thr Ala Ser Leu Ser Pro Gly Met Met Met Gly Met Phe Asn
        400                 405                 410

atg tta agt act gta tta ggc gtc tcc atc ctg aat ctt gga caa aag    1477
Met Leu Ser Thr Val Leu Gly Val Ser Ile Leu Asn Leu Gly Gln Lys
    415                 420                 425

aga cac acc aag act act tac tgg tgg gat ggt ctt caa tct tct gat    1525
Arg His Thr Lys Thr Thr Tyr Trp Trp Asp Gly Leu Gln Ser Ser Asp
430                 435                 440                 445

gat ttt gct ctg att gtg aat gca ccc aat cat gaa ggg att caa gcc    1573
Asp Phe Ala Leu Ile Val Asn Ala Pro Asn His Glu Gly Ile Gln Ala
                450                 455                 460

gga gtc aac agg ttt tat cga acc tgt aag cta ctt gga att aat atg    1621
Gly Val Asn Arg Phe Tyr Arg Thr Cys Lys Leu Leu Gly Ile Asn Met
            465                 470                 475

agc aag aaa aag tct tac ata aac aga aca ggt aca ttt gaa ttc aca    1669
Ser Lys Lys Lys Ser Tyr Ile Asn Arg Thr Gly Thr Phe Glu Phe Thr
        480                 485                 490

agc ttt ttc tac cgc tat gga ttt gta gcc aat ttt agt atg gag ttg    1717
Ser Phe Phe Tyr Arg Tyr Gly Phe Val Ala Asn Phe Ser Met Glu Leu
    495                 500                 505
```

```
ccc agc ttt gga gta tca gga att aat gaa tcg gct gat atg agc att     1765
Pro Ser Phe Gly Val Ser Gly Ile Asn Glu Ser Ala Asp Met Ser Ile
510                 515                 520                 525

gga gta aca gtg ata aag aat aac atg ata aac aat gat ctt gga ccg     1813
Gly Val Thr Val Ile Lys Asn Asn Met Ile Asn Asn Asp Leu Gly Pro
                530                 535                 540

gca aca gcc caa atg gct ctc caa tta ttc atc aag gac tac aga tat     1861
Ala Thr Ala Gln Met Ala Leu Gln Leu Phe Ile Lys Asp Tyr Arg Tyr
            545                 550                 555

aca tac agg tgt cac agg gga gac aca caa atc caa acg agg agg tca     1909
Thr Tyr Arg Cys His Arg Gly Asp Thr Gln Ile Gln Thr Arg Arg Ser
        560                 565                 570

ttc gag cta aag aag ctg tgg gag cag acc cgc tca aag gca gga ctg     1957
Phe Glu Leu Lys Lys Leu Trp Glu Gln Thr Arg Ser Lys Ala Gly Leu
    575                 580                 585

ttg gtt tca gat ggc gga cca aac ctg tac aac att cga aat ctc cac     2005
Leu Val Ser Asp Gly Gly Pro Asn Leu Tyr Asn Ile Arg Asn Leu His
590                 595                 600                 605

att cct gaa gtc tgc ttg aaa tgg gaa tta atg gat gag gat tac cag     2053
Ile Pro Glu Val Cys Leu Lys Trp Glu Leu Met Asp Glu Asp Tyr Gln
                610                 615                 620

ggg cgt tta tgc aac cca ctg aac cca ttt gtc aac cat aaa gac att     2101
Gly Arg Leu Cys Asn Pro Leu Asn Pro Phe Val Asn His Lys Asp Ile
            625                 630                 635

gaa tca gtg aac aat gca gtg ata atg cca gca cat ggt cca gcc aaa     2149
Glu Ser Val Asn Asn Ala Val Ile Met Pro Ala His Gly Pro Ala Lys
        640                 645                 650

aac atg gag tat gat gct gtt gca aca aca cac tcc tgg atc ccc aaa     2197
Asn Met Glu Tyr Asp Ala Val Ala Thr Thr His Ser Trp Ile Pro Lys
    655                 660                 665

aga aat cga tcc atc ttg aat aca agc caa aga gga ata ctt gaa gat     2245
Arg Asn Arg Ser Ile Leu Asn Thr Ser Gln Arg Gly Ile Leu Glu Asp
670                 675                 680                 685

gaa caa atg tac caa aag tgc tgc aac tta ttt gaa aaa ttc ttc ccc     2293
Glu Gln Met Tyr Gln Lys Cys Cys Asn Leu Phe Glu Lys Phe Phe Pro
                690                 695                 700

agc agt tca tac aga aga cca gtc ggg ata tcc agt atg gtg gag gct     2341
Ser Ser Ser Tyr Arg Arg Pro Val Gly Ile Ser Ser Met Val Glu Ala
            705                 710                 715

atg gtt tcc aga gcc cga att gat gca cga att gat ttc gaa tct gga     2389
Met Val Ser Arg Ala Arg Ile Asp Ala Arg Ile Asp Phe Glu Ser Gly
        720                 725                 730

agg ata aag aaa gag gag ttc act gag atc atg aag atc tgt tcc acc     2437
Arg Ile Lys Lys Glu Glu Phe Thr Glu Ile Met Lys Ile Cys Ser Thr
    735                 740                 745

att gaa gag ctc aga cgg caa aaa tagtgaattt agcttgtcct tcatgaaaaa   2491
Ile Glu Glu Leu Arg Arg Gln Lys
750                 755

atgccttgtt tctactaata acccggcggc ccaaaatgcc gactcggagc gaaagatata 2551
```

cctcccccgg ggccgggagg tcgcgtcacc gaccacgccg ccggcccagg cgacgcgcga 2611
cacggacacc tgtccccaaa aacgccacca tcgcagccac acacggagcg cccggggccc 2671
tctggtcaac cccaggacac acgcgggagc agcgccgggc cggggacgcc ctcccggccg 2731
cccgtgccac acgcaggggg ccggcccgtg tctccagagc gggagccgga agcattttcg 2791
gccggcccct cctacgaccg ggacacacga gggaccgaag gccggccagg cgcgacctct 2851
cgggccgcac gcgcgctcag ggagcgctct ccgactccgc acggggactc gccagaaagg 2911
atcgtgacct gcattaatga atcaggggat aacgcaggaa agaacatgtg agcaaaaggc 2971
cagcaaaagg ccaggaaccg taaaaaggcc gcgttgctgg cgtttttcca taggctccgc 3031
cccccctgacg agcatcacaa aaatcgacgc tcaagtcaga ggtggcgaaa cccgacagga 3091
ctataaagat accaggcgtt tccccctgga agctccctcg tgcgctctcc tgttccgacc 3151
ctgccgctta ccggatacct gtccgccttt ctcccttcgg gaagcgtggc gctttctcat 3211
agctcacgct gtaggtatct cagttcggtg taggtcgttc gctccaagct gggctgtgtg 3271
cacgaacccc ccgttcagcc cgaccgctgc gccttatccg gtaactatcg tcttgagtcc 3331
aacccggtaa gacacgactt atcgccactg gcagcagcca ctggtaacag gattagcaga 3391
gcgaggtatg taggcggtgc tacagagttc ttgaagtggt ggcctaacta cggctacact 3451
agaaggacag tatttggtat ctgcgctctg ctgaagccag ttaccttcgg aaaaagagtt 3511
ggtagctctt gatccggcaa acaaaccacc gctggtagcg gtggtttttt tgtttgcaag 3571
cagcagatta cgcgcagaaa aaaaggatct caagaagatc ctttgatctt ttctacgggg 3631
tctgacgctc agtggaacga aaactcacgt taagggattt tggtcatgag attatcaaaa 3691
aggatcttca cctagatcct tttaaattaa aaatgaagtt ttaaatcaat ctaaagtata 3751
tatgagtaaa cttggtctga cagttaccaa tgcttaatca gtgaggcacc tatctcagcg 3811
atctgtctat ttcgttcatc catagttgcc tgactccccg tcgtgtagat aactacgata 3871
cgggagggct taccatctgg ccccagtgct gcaatgatac cgcgagaccc acgctcaccg 3931
gctccagatt tatcagcaat aaaccagcca gccggaaggg ccgagcgcag aagtggtcct 3991
gcaactttat ccgcctccat ccagtctatt aattgttgcc gggaagctag agtaagtagt 4051
tcgccagtta atagtttgcg caacgttgtt gccattgcta caggcatcgt ggtgtcacgc 4111
tcgtcgtttg gtatggcttc attcagctcc ggttcccaac gatcaaggcg agttacatga 4171
tcccccatgt tgtgcaaaaa agcggttagc tccttcggtc ctccgatcgt tgtcagaagt 4231
aagttggccg cagtgttatc actcatggtt atggcagcac tgcataattc tcttactgtc 4291
atgccatccg taagatgctt ttctgtgact ggtgagtact caaccaagtc attctgagaa 4351
tagtgtatgc ggcgaccgag ttgctcttgc ccggcgtcaa cacgggataa taccgcgcca 4411
catagcagaa ctttaaaagt gctcatcatt ggaaaacgtt cttcggggcg aaaactctca 4471
aggatcttac cgctgttgag atccagttcg atgtaaccca ctcgtgcacc caactgatct 4531
tcagcatctt ttactttcac cagcgtttct gggtgagcaa aaacaggaag gcaaaatgcc 4591

gcaaaaaagg gaataagggc gacacggaaa tgttgaatac tcatactctt cctttttcaa 4651

tattattgaa gcatttatca gggttattgt ctcatgagcg gatacatatt tgaatgtatt 4711

tagaaaaata aacaaaagag tttgtagaaa cgcaaaaagg ccatccgtca ggatggcctt 4771

ctgcttaatt tgatgcctgg cagtttatgg cgggcgtcct gcccgccacc ctccgggccg 4831

ttgcttcgca acgttcaaat ccgctcccgg cggatttgtc ctactcagga gagcgttcac 4891

cgacaaacaa cagataaaac gaaaggccca gtctttcgac tgagcctttc gttttatttg 4951

atgcctggca gttccctact ctcgcatggg gagaccccac actaccatcg gcgctacggc 5011

gtttcacttc tgagttcggc atggggtcag gtgggaccac cgcgctactg ccgccaggca 5071

aattctgttt tatcagaccg cttctgcgtt ctgatttaat ctgtatcagg ctgaaaatct 5131

tctctcatcc gccaaaacag ccaagctagc ggccgatc 5169

<210> 45
<211> 757
<212> PRT
<213> Artificial Sequence
<223> Description of Artificial Sequence: pHL3259

<400> 45

```
Met Asp Val Asn Pro Thr Leu Leu Phe Leu Lys Val Pro Ala Gln Asn
 1               5                  10                  15

Ala Ile Ser Thr Thr Phe Pro Tyr Thr Gly Asp Pro Pro Tyr Ser His
            20                  25                  30

Gly Thr Gly Thr Gly Tyr Thr Met Asp Thr Val Asn Arg Thr His Gln
        35                  40                  45

Tyr Ser Glu Arg Gly Arg Trp Thr Thr Asn Thr Glu Thr Gly Ala Pro
    50                  55                  60

Gln Leu Asn Pro Ile Asp Gly Pro Leu Pro Glu Asp Asn Glu Pro Ser
65                  70                  75                  80

Gly Tyr Ala Gln Thr Asp Cys Val Leu Glu Ala Met Ala Phe Leu Glu
                85                  90                  95

Glu Ser His Pro Gly Ile Phe Glu Thr Ser Cys Leu Glu Thr Met Glu
            100                 105                 110

Val Val Gln Gln Thr Arg Val Asp Lys Leu Thr Gln Gly Arg Gln Thr
        115                 120                 125

Tyr Asp Trp Thr Leu Asn Arg Asn Gln Pro Ala Ala Thr Ala Leu Ala
    130                 135                 140

Asn Thr Ile Glu Val Phe Arg Ser Asn Gly Leu Thr Ala Asn Glu Ser
145                 150                 155                 160

Gly Arg Leu Ile Asp Phe Leu Lys Asp Val Met Glu Ser Met Asn Lys
                165                 170                 175

Glu Glu Met Glu Ile Thr Thr His Phe Gln Arg Lys Arg Arg Val Arg
            180                 185                 190

Asp Asn Met Thr Lys Lys Met Val Thr Gln Arg Thr Ile Gly Lys Arg
        195                 200                 205
```

```
Lys Gln Arg Leu Asn Lys Arg Ser Tyr Leu Ile Arg Ala Leu Thr Leu
    210                 215                 220

Asn Thr Met Thr Lys Asp Ala Glu Arg Gly Lys Leu Lys Arg Arg Ala
225                 230                 235                 240

Ile Ala Thr Pro Gly Met Gln Ile Arg Gly Phe Val Tyr Phe Val Glu
                245                 250                 255

Thr Leu Ala Arg Ser Ile Cys Glu Lys Leu Glu Gln Ser Gly Leu Pro
            260                 265                 270

Val Gly Gly Asn Glu Lys Lys Ala Lys Leu Ala Asn Val Val Arg Lys
        275                 280                 285

Met Met Thr Asn Ser Gln Asp Thr Glu Ile Ser Phe Thr Ile Thr Gly
    290                 295                 300

Asp Asn Thr Lys Trp Asn Glu Asn Gln Asn Pro Arg Met Phe Leu Ala
305                 310                 315                 320

Met Ile Thr Tyr Ile Thr Arg Asn Gln Pro Glu Trp Phe Arg Asn Val
                325                 330                 335

Leu Ser Ile Ala Pro Ile Met Phe Ser Asn Lys Met Ala Arg Leu Gly
            340                 345                 350

Lys Gly Tyr Met Phe Glu Ser Lys Ser Met Lys Ile Arg Thr Gln Ile
        355                 360                 365

Pro Ala Glu Met Leu Ala Ser Ile Asp Leu Lys Tyr Phe Asn Glu Pro
    370                 375                 380

Thr Arg Lys Lys Ile Glu Lys Ile Arg Pro Leu Leu Ile Asp Gly Thr
385                 390                 395                 400

Ala Ser Leu Ser Pro Gly Met Met Met Gly Met Phe Asn Met Leu Ser
                405                 410                 415

Thr Val Leu Gly Val Ser Ile Leu Asn Leu Gly Gln Lys Arg His Thr
            420                 425                 430

Lys Thr Thr Tyr Trp Trp Asp Gly Leu Gln Ser Ser Asp Asp Phe Ala
        435                 440                 445

Leu Ile Val Asn Ala Pro Asn His Glu Gly Ile Gln Ala Gly Val Asn
    450                 455                 460

Arg Phe Tyr Arg Thr Cys Lys Leu Leu Gly Ile Asn Met Ser Lys Lys
465                 470                 475                 480

Lys Ser Tyr Ile Asn Arg Thr Gly Thr Phe Glu Phe Thr Ser Phe Phe
                485                 490                 495

Tyr Arg Tyr Gly Phe Val Ala Asn Phe Ser Met Glu Leu Pro Ser Phe
            500                 505                 510

Gly Val Ser Gly Ile Asn Glu Ser Ala Asp Met Ser Ile Gly Val Thr
        515                 520                 525

Val Ile Lys Asn Asn Met Ile Asn Asn Asp Leu Gly Pro Ala Thr Ala
    530                 535                 540

Gln Met Ala Leu Gln Leu Phe Ile Lys Asp Tyr Arg Tyr Thr Tyr Arg
545                 550                 555                 560
```

```
Cys His Arg Gly Asp Thr Gln Ile Gln Thr Arg Arg Ser Phe Glu Leu
                565                 570                 575

Lys Lys Leu Trp Glu Gln Thr Arg Ser Lys Ala Gly Leu Leu Val Ser
            580                 585                 590

Asp Gly Gly Pro Asn Leu Tyr Asn Ile Arg Asn Leu His Ile Pro Glu
        595                 600                 605

Val Cys Leu Lys Trp Glu Leu Met Asp Glu Asp Tyr Gln Gly Arg Leu
    610                 615                 620

Cys Asn Pro Leu Asn Pro Phe Val Asn His Lys Asp Ile Glu Ser Val
625                 630                 635                 640

Asn Asn Ala Val Ile Met Pro Ala His Gly Pro Ala Lys Asn Met Glu
                645                 650                 655

Tyr Asp Ala Val Ala Thr Thr His Ser Trp Ile Pro Lys Arg Asn Arg
            660                 665                 670

Ser Ile Leu Asn Thr Ser Gln Arg Gly Ile Leu Glu Asp Glu Gln Met
        675                 680                 685

Tyr Gln Lys Cys Cys Asn Leu Phe Glu Lys Phe Phe Pro Ser Ser Ser
    690                 695                 700

Tyr Arg Arg Pro Val Gly Ile Ser Ser Met Val Glu Ala Met Val Ser
705                 710                 715                 720

Arg Ala Arg Ile Asp Ala Arg Ile Asp Phe Glu Ser Gly Arg Ile Lys
                725                 730                 735

Lys Glu Glu Phe Thr Glu Ile Met Lys Ile Cys Ser Thr Ile Glu Glu
            740                 745                 750

Leu Arg Arg Gln Lys
        755


<210> 46
<211> 5169
<212> DNA
<213> Artificial Sequence

<220>
<223> Description of Artificial Sequence: pHL3268

<220>
<221> CDS
<222> (191)..(2461)

<400> 46
cccaaaaaaa aaaaaaaaaa aagagtccag agtggccccg ccgctccgcg ccgggggggg 60

gggggggggg ggacactttc ggacatctgg tcgacctcca gcatcggggg aaaaaaaaaa 120

acaaagtgtc gcccggagta ctggtcgacc tccgaagttg ggggggagcg aaagcaggca 180

aaccatttga atg gat gtc aat ccg act tta ctt ttc tta aaa gtg cca    229
           Met Asp Val Asn Pro Thr Leu Leu Phe Leu Lys Val Pro
             1               5                  10

gca caa aat gct ata agc aca act ttc cct tat act gga gac cct cct    277
Ala Gln Asn Ala Ile Ser Thr Thr Phe Pro Tyr Thr Gly Asp Pro Pro
     15                  20                  25
```

```
tac agc cat ggg aca gga aca gga tac acc atg gat act gtc aac agg     325
Tyr Ser His Gly Thr Gly Thr Gly Tyr Thr Met Asp Thr Val Asn Arg
 30                 35                  40                  45

aca cat cag tac tca gaa agg gga aga tgg aca aca aac acc gaa act     373
Thr His Gln Tyr Ser Glu Arg Gly Arg Trp Thr Thr Asn Thr Glu Thr
                50                  55                  60

gga gca ccg caa ctc aac ccg att gat ggg cca ctg cca gaa gac aat     421
Gly Ala Pro Gln Leu Asn Pro Ile Asp Gly Pro Leu Pro Glu Asp Asn
            65                  70                  75

gaa cca agt ggt tat gcc caa aca gat tgt gta ttg gaa gca atg gcc     469
Glu Pro Ser Gly Tyr Ala Gln Thr Asp Cys Val Leu Glu Ala Met Ala
        80                  85                  90

ttc ctt gag gaa tcc cat cct ggt atc ttt gag acc tcg tgt ctt gaa     517
Phe Leu Glu Glu Ser His Pro Gly Ile Phe Glu Thr Ser Cys Leu Glu
    95                  100                 105

acg atg gag gtt gtt cag caa aca cga gtg gac aag ctg aca caa ggc     565
Thr Met Glu Val Val Gln Gln Thr Arg Val Asp Lys Leu Thr Gln Gly
110                 115                 120                 125

cga cag acc tat gac tgg act cta aat agg aac cag cct gct gca aca     613
Arg Gln Thr Tyr Asp Trp Thr Leu Asn Arg Asn Gln Pro Ala Ala Thr
                130                 135                 140

gca ttg gcc aac aca ata gaa gtg ttc aga tca aat ggc ctc acg gcc     661
Ala Leu Ala Asn Thr Ile Glu Val Phe Arg Ser Asn Gly Leu Thr Ala
            145                 150                 155

aat gaa tct gga agg ctc ata gac ttc ctt aag gat gta atg gag tca     709
Asn Glu Ser Gly Arg Leu Ile Asp Phe Leu Lys Asp Val Met Glu Ser
        160                 165                 170

atg aac aaa gaa gaa atg gag atc aca act cat ttt cag aga aag aga     757
Met Asn Lys Glu Glu Met Glu Ile Thr Thr His Phe Gln Arg Lys Arg
    175                 180                 185

cga gtg aga gac aat atg act aag aaa atg gtg aca cag aga aca ata     805
Arg Val Arg Asp Asn Met Thr Lys Lys Met Val Thr Gln Arg Thr Ile
190                 195                 200                 205

ggt aaa agg aag cag aga ttg aac aaa agg agt tat cta att agg gca     853
Gly Lys Arg Lys Gln Arg Leu Asn Lys Arg Ser Tyr Leu Ile Arg Ala
                210                 215                 220

tta acc ctg aac aca atg acc aaa gat gct gag aga ggg aag cta aaa     901
Leu Thr Leu Asn Thr Met Thr Lys Asp Ala Glu Arg Gly Lys Leu Lys
            225                 230                 235

cgg aga gca att gca acc cca ggg atg caa ata agg ggg ttt gta tac     949
Arg Arg Ala Ile Ala Thr Pro Gly Met Gln Ile Arg Gly Phe Val Tyr
        240                 245                 250

ttt gtt gag aca cta gca agg agt ata tgt gag aaa ctt gaa caa tca     997
Phe Val Glu Thr Leu Ala Arg Ser Ile Cys Glu Lys Leu Glu Gln Ser
    255                 260                 265

gga ttg cca gtt gga ggc aat gag aag aaa gca aag ttg gca aat gtt     1045
Gly Leu Pro Val Gly Gly Asn Glu Lys Lys Ala Lys Leu Ala Asn Val
270                 275                 280                 285

gta agg aag atg atg acc aat tct cag gac act gaa att tct ttc acc     1093
Val Arg Lys Met Met Thr Asn Ser Gln Asp Thr Glu Ile Ser Phe Thr
                290                 295                 300
```

```
atc act gga gat aac acc aaa tgg aac gaa aat cag aac cct cgg atg    1141
Ile Thr Gly Asp Asn Thr Lys Trp Asn Glu Asn Gln Asn Pro Arg Met
            305                 310                 315

ttt ttg gcc atg atc aca tat ata acc aga aat cag ccc gaa tgg ttc    1189
Phe Leu Ala Met Ile Thr Tyr Ile Thr Arg Asn Gln Pro Glu Trp Phe
        320                 325                 330

aga aat gtt cta agt att gct cca ata atg ttc tca aac aaa atg gcg    1237
Arg Asn Val Leu Ser Ile Ala Pro Ile Met Phe Ser Asn Lys Met Ala
    335                 340                 345

aga ctg gga aag ggg tac atg ttt gag agc aag agt atg aaa att aga    1285
Arg Leu Gly Lys Gly Tyr Met Phe Glu Ser Lys Ser Met Lys Ile Arg
350                 355                 360                 365

act caa ata cct gca gaa atg cta gca agt att gat cta aaa tat ttc    1333
Thr Gln Ile Pro Ala Glu Met Leu Ala Ser Ile Asp Leu Lys Tyr Phe
                370                 375                 380

aat gaa cca aca agg aag aaa atc gag aaa ata agg cct ctc tta ata    1381
Asn Glu Pro Thr Arg Lys Lys Ile Glu Lys Ile Arg Pro Leu Leu Ile
            385                 390                 395

gat ggg act gca tca ttg agc cct gga atg atg atg ggc atg ttc aat    1429
Asp Gly Thr Ala Ser Leu Ser Pro Gly Met Met Met Gly Met Phe Asn
        400                 405                 410

atg tta agt act gta tta ggc gtc tcc atc ctg aat ctt gga caa aag    1477
Met Leu Ser Thr Val Leu Gly Val Ser Ile Leu Asn Leu Gly Gln Lys
    415                 420                 425

aga cac acc aag act act tac tgg tgg gat ggt ctt caa tct tct gat    1525
Arg His Thr Lys Thr Thr Tyr Trp Trp Asp Gly Leu Gln Ser Ser Asp
430                 435                 440                 445

gat ttt gct ctg att gtg aat gca ccc aat cat gaa ggg att caa gcc    1573
Asp Phe Ala Leu Ile Val Asn Ala Pro Asn His Glu Gly Ile Gln Ala
                450                 455                 460

gga gtc aac agg ttt tat cga acc tgt aag cta ctt gga att aat atg    1621
Gly Val Asn Arg Phe Tyr Arg Thr Cys Lys Leu Leu Gly Ile Asn Met
            465                 470                 475

agc aag aaa aag tct tac ata aac aga aca ggt aca ttt gaa ttc aca    1669
Ser Lys Lys Lys Ser Tyr Ile Asn Arg Thr Gly Thr Phe Glu Phe Thr
        480                 485                 490

agc ttt ttc tat cgt tat ggg ttt gtt gcc aat ttc agc atg gag ctt    1717
Ser Phe Phe Tyr Arg Tyr Gly Phe Val Ala Asn Phe Ser Met Glu Leu
    495                 500                 505

ccc agc ttt ggg gtg tct ggg atc aac gag tct gcg gac atg agt att    1765
Pro Ser Phe Gly Val Ser Gly Ile Asn Glu Ser Ala Asp Met Ser Ile
510                 515                 520                 525

gga gtt act gtc atc aaa aac aat atg ata aac aat gat ctt ggt cca    1813
Gly Val Thr Val Ile Lys Asn Asn Met Ile Asn Asn Asp Leu Gly Pro
                530                 535                 540

gca acc gct caa atg gcc ctt cag ctg ttc atc aaa gat tac agg tac    1861
Ala Thr Ala Gln Met Ala Leu Gln Leu Phe Ile Lys Asp Tyr Arg Tyr
            545                 550                 555

acg tac cgg tgc cat aga ggt gac aca caa ata caa acc cga aga tca    1909
Thr Tyr Arg Cys His Arg Gly Asp Thr Gln Ile Gln Thr Arg Arg Ser
```

```
        560                 565                 570

ttt gaa ata aag aaa ctg tgg gag caa acc cat tcc aaa gct gga ctg    1957
Phe Glu Ile Lys Lys Leu Trp Glu Gln Thr His Ser Lys Ala Gly Leu
    575                 580                 585

ctg gtc tcc gac gga ggc cca aat tta tac aac att cgg aat ctc cac    2005
Leu Val Ser Asp Gly Gly Pro Asn Leu Tyr Asn Ile Arg Asn Leu His
590                 595                 600                 605

atc ccg gaa gtt tgc ctg aaa tgg gaa cta atg gat gaa gac tat cag    2053
Ile Pro Glu Val Cys Leu Lys Trp Glu Leu Met Asp Glu Asp Tyr Gln
                610                 615                 620

gga aga ctt tgt aat ccc atg aac ccg ttt gtc agt cat aag gaa att    2101
Gly Arg Leu Cys Asn Pro Met Asn Pro Phe Val Ser His Lys Glu Ile
            625                 630                 635

gaa tct gta aac aat gct gcg gta atg cca gcc cat ggt cca gcc aaa    2149
Glu Ser Val Asn Asn Ala Ala Val Met Pro Ala His Gly Pro Ala Lys
        640                 645                 650

agc atg gaa tat gat gct gtg gca act aca cac tct tgg atc cct aag    2197
Ser Met Glu Tyr Asp Ala Val Ala Thr Thr His Ser Trp Ile Pro Lys
    655                 660                 665

aga aac cgt tcc att ctc aat acg agt caa agg gga atc ctt gag gat    2245
Arg Asn Arg Ser Ile Leu Asn Thr Ser Gln Arg Gly Ile Leu Glu Asp
670                 675                 680                 685

gaa caa atg tac cag aag tgt tgc aac cta ttc gag aaa ttc ttc cct    2293
Glu Gln Met Tyr Gln Lys Cys Cys Asn Leu Phe Glu Lys Phe Phe Pro
                690                 695                 700

agc agc tca tac aga aga cca gtt gga att tcc agt atg gtg gag gcc    2341
Ser Ser Ser Tyr Arg Arg Pro Val Gly Ile Ser Ser Met Val Glu Ala
            705                 710                 715

atg gtg tct agg gcc cgg att gat gca cga att gac ttc gag tct gga    2389
Met Val Ser Arg Ala Arg Ile Asp Ala Arg Ile Asp Phe Glu Ser Gly
        720                 725                 730

agg att aag aag gaa gag ttt gct gag atc atg aag atc tgt tcc acc    2437
Arg Ile Lys Lys Glu Glu Phe Ala Glu Ile Met Lys Ile Cys Ser Thr
    735                 740                 745

att gaa gag ctc aga cgg caa aaa tagtgaattt agcttgtcct tcatgaaaaa   2491
Ile Glu Glu Leu Arg Arg Gln Lys
750                 755

atgccttgtt tctactaata acccggcggc ccaaaatgcc gactcggagc gaaagatata 2551

cctcccccgg ggccgggagg tcgcgtcacc gaccacgccg ccggcccagg cgacgcgcga 2611

cacggacacc tgtccccaaa aacgccacca tcgcagccac acacggagcg cccggggccc 2671

tctggtcaac cccaggacac acgcgggagc agcgccgggc cggggacgcc ctcccggccg 2731

cccgtgccac acgcaggggg ccggcccgtg tctccagagc gggagccgga agcattttcg 2791

gccggcccct cctacgaccg ggacacacga gggaccgaag gccggccagg cgcgacctct 2851

cgggccgcac gcgcgctcag ggagcgctct ccgactccgc acggggactc gccagaaagg 2911

atcgtgacct gcattaatga atcaggggat aacgcaggaa agaacatgtg agcaaaaggc 2971

cagcaaaagg ccaggaaccg taaaaaggcc gcgttgctgg cgtttttcca taggctccgc 3031
```

```
ccccctgacg agcatcacaa aaatcgacgc tcaagtcaga ggtggcgaaa cccgacagga 3091
ctataaagat accaggcgtt tccccctgga agctccctcg tgcgctctcc tgttccgacc 3151
ctgccgctta ccggatacct gtccgccttt ctcccttcgg gaagcgtggc gctttctcat 3211
agctcacgct gtaggtatct cagttcggtg taggtcgttc gctccaagct gggctgtgtg 3271
cacgaacccc ccgttcagcc cgaccgctgc gccttatccg gtaactatcg tcttgagtcc 3331
aacccggtaa gacacgactt atcgccactg gcagcagcca ctggtaacag gattagcaga 3391
gcgaggtatg taggcggtgc tacagagttc ttgaagtggt ggcctaacta cggctacact 3451
agaaggacag tatttggtat ctgcgctctg ctgaagccag ttaccttcgg aaaaagagtt 3511
ggtagctctt gatccggcaa acaaaccacc gctggtagcg gtggtttttt tgtttgcaag 3571
cagcagatta cgcgcagaaa aaaaggatct caagaagatc ctttgatctt ttctacgggg 3631
tctgacgctc agtggaacga aaactcacgt taagggattt tggtcatgag attatcaaaa 3691
aggatcttca cctagatcct tttaaattaa aaatgaagtt ttaaatcaat ctaaagtata 3751
tatgagtaaa cttggtctga cagttaccaa tgcttaatca gtgaggcacc tatctcagcg 3811
atctgtctat ttcgttcatc catagttgcc tgactccccg tcgtgtagat aactacgata 3871
cgggagggct taccatctgg ccccagtgct gcaatgatac cgcgagaccc acgctcaccg 3931
gctccagatt tatcagcaat aaaccagcca gccggaaggg ccgagcgcag aagtggtcct 3991
gcaactttat ccgcctccat ccagtctatt aattgttgcc gggaagctag agtaagtagt 4051
tcgccagtta atagtttgcg caacgttgtt gccattgcta caggcatcgt ggtgtcacgc 4111
tcgtcgtttg gtatggcttc attcagctcc ggttcccaac gatcaaggcg agttacatga 4171
tcccccatgt tgtgcaaaaa agcggttagc tccttcggtc ctccgatcgt tgtcagaagt 4231
aagttggccg cagtgttatc actcatggtt atggcagcac tgcataattc tcttactgtc 4291
atgccatccg taagatgctt ttctgtgact ggtgagtact caaccaagtc attctgagaa 4351
tagtgtatgc ggcgaccgag ttgctcttgc ccggcgtcaa cacgggataa taccgcgcca 4411
catagcagaa ctttaaaagt gctcatcatt ggaaaacgtt cttcggggcg aaaactctca 4471
aggatcttac cgctgttgag atccagttcg atgtaaccca ctcgtgcacc caactgatct 4531
tcagcatctt ttactttcac cagcgtttct gggtgagcaa aaacaggaag gcaaaatgcc 4591
gcaaaaaagg gaataagggc gacacggaaa tgttgaatac tcatactctt cctttttcaa 4651
tattattgaa gcatttatca gggttattgt ctcatgagcg gatacatatt tgaatgtatt 4711
tagaaaaata aacaaaagag tttgtagaaa cgcaaaaagg ccatccgtca ggatggcctt 4771
ctgcttaatt tgatgcctgg cagtttatgg cgggcgtcct gcccgccacc ctccgggccg 4831
ttgcttcgca acgttcaaat ccgctcccgg cggatttgtc ctactcagga gagcgttcac 4891
cgacaaacaa cagataaaac gaaaggccca gtctttcgac tgagcctttc gttttatttg 4951
atgcctggca gttccctact ctcgcatggg gagaccccac actaccatcg gcgctacggc 5011
```

gtttcacttc tgagttcggc atggggtcag gtgggaccac cgcgctactg ccgccaggca 5071

aattctgttt tatcagaccg cttctgcgtt ctgatttaat ctgtatcagg ctgaaatct 5131

tctctcatcc gccaaaacag ccaagctagc ggccgatc 5169

<210> 47
<211> 757
<212> PRT
<213> Artificial Sequence
<223> Description of Artificial Sequence: pHL3268

<400> 47
Met Asp Val Asn Pro Thr Leu Leu Phe Leu Lys Val Pro Ala Gln Asn
1               5                   10                  15

Ala Ile Ser Thr Thr Phe Pro Tyr Thr Gly Asp Pro Pro Tyr Ser His
            20                  25                  30

Gly Thr Gly Thr Gly Tyr Thr Met Asp Thr Val Asn Arg Thr His Gln
        35                  40                  45

Tyr Ser Glu Arg Gly Arg Trp Thr Thr Asn Thr Glu Thr Gly Ala Pro
    50                  55                  60

Gln Leu Asn Pro Ile Asp Gly Pro Leu Pro Glu Asp Asn Glu Pro Ser
65                  70                  75                  80

Gly Tyr Ala Gln Thr Asp Cys Val Leu Glu Ala Met Ala Phe Leu Glu
                85                  90                  95

Glu Ser His Pro Gly Ile Phe Glu Thr Ser Cys Leu Glu Thr Met Glu
            100                 105                 110

Val Val Gln Gln Thr Arg Val Asp Lys Leu Thr Gln Gly Arg Gln Thr
        115                 120                 125

Tyr Asp Trp Thr Leu Asn Arg Asn Gln Pro Ala Ala Thr Ala Leu Ala
    130                 135                 140

Asn Thr Ile Glu Val Phe Arg Ser Asn Gly Leu Thr Ala Asn Glu Ser
145                 150                 155                 160

Gly Arg Leu Ile Asp Phe Leu Lys Asp Val Met Glu Ser Met Asn Lys
                165                 170                 175

Glu Glu Met Glu Ile Thr Thr His Phe Gln Arg Lys Arg Arg Val Arg
            180                 185                 190

Asp Asn Met Thr Lys Lys Met Val Thr Gln Arg Thr Ile Gly Lys Arg
        195                 200                 205

Lys Gln Arg Leu Asn Lys Arg Ser Tyr Leu Ile Arg Ala Leu Thr Leu
    210                 215                 220

Asn Thr Met Thr Lys Asp Ala Glu Arg Gly Lys Leu Lys Arg Arg Ala
225                 230                 235                 240

Ile Ala Thr Pro Gly Met Gln Ile Arg Gly Phe Val Tyr Phe Val Glu
                245                 250                 255

Thr Leu Ala Arg Ser Ile Cys Glu Lys Leu Glu Gln Ser Gly Leu Pro
            260                 265                 270

Val Gly Gly Asn Glu Lys Lys Ala Lys Leu Ala Asn Val Val Arg Lys
        275                 280                 285

```
Met Met Thr Asn Ser Gln Asp Thr Glu Ile Ser Phe Thr Ile Thr Gly
    290                 295                 300

Asp Asn Thr Lys Trp Asn Glu Asn Gln Asn Pro Arg Met Phe Leu Ala
305                 310                 315                 320

Met Ile Thr Tyr Ile Thr Arg Asn Gln Pro Glu Trp Phe Arg Asn Val
                325                 330                 335

Leu Ser Ile Ala Pro Ile Met Phe Ser Asn Lys Met Ala Arg Leu Gly
            340                 345                 350

Lys Gly Tyr Met Phe Glu Ser Lys Ser Met Lys Ile Arg Thr Gln Ile
        355                 360                 365

Pro Ala Glu Met Leu Ala Ser Ile Asp Leu Lys Tyr Phe Asn Glu Pro
    370                 375                 380

Thr Arg Lys Lys Ile Glu Lys Ile Arg Pro Leu Leu Ile Asp Gly Thr
385                 390                 395                 400

Ala Ser Leu Ser Pro Gly Met Met Met Gly Met Phe Asn Met Leu Ser
                405                 410                 415

Thr Val Leu Gly Val Ser Ile Leu Asn Leu Gly Gln Lys Arg His Thr
            420                 425                 430

Lys Thr Thr Tyr Trp Trp Asp Gly Leu Gln Ser Ser Asp Asp Phe Ala
        435                 440                 445

Leu Ile Val Asn Ala Pro Asn His Glu Gly Ile Gln Ala Gly Val Asn
    450                 455                 460

Arg Phe Tyr Arg Thr Cys Lys Leu Leu Gly Ile Asn Met Ser Lys Lys
465                 470                 475                 480

Lys Ser Tyr Ile Asn Arg Thr Gly Thr Phe Glu Phe Thr Ser Phe Phe
                485                 490                 495

Tyr Arg Tyr Gly Phe Val Ala Asn Phe Ser Met Glu Leu Pro Ser Phe
            500                 505                 510

Gly Val Ser Gly Ile Asn Glu Ser Ala Asp Met Ser Ile Gly Val Thr
        515                 520                 525

Val Ile Lys Asn Asn Met Ile Asn Asn Asp Leu Gly Pro Ala Thr Ala
    530                 535                 540

Gln Met Ala Leu Gln Leu Phe Ile Lys Asp Tyr Arg Tyr Thr Tyr Arg
545                 550                 555                 560

Cys His Arg Gly Asp Thr Gln Ile Gln Thr Arg Arg Ser Phe Glu Ile
                565                 570                 575

Lys Lys Leu Trp Glu Gln Thr His Ser Lys Ala Gly Leu Leu Val Ser
            580                 585                 590

Asp Gly Gly Pro Asn Leu Tyr Asn Ile Arg Asn Leu His Ile Pro Glu
        595                 600                 605

Val Cys Leu Lys Trp Glu Leu Met Asp Glu Asp Tyr Gln Gly Arg Leu
    610                 615                 620

Cys Asn Pro Met Asn Pro Phe Val Ser His Lys Glu Ile Glu Ser Val
625                 630                 635                 640
```

```
Asn Asn Ala Ala Val Met Pro Ala His Gly Pro Ala Lys Ser Met Glu
                645                 650                 655

Tyr Asp Ala Val Ala Thr Thr His Ser Trp Ile Pro Lys Arg Asn Arg
            660                 665                 670

Ser Ile Leu Asn Thr Ser Gln Arg Gly Ile Leu Glu Asp Glu Gln Met
        675                 680                 685

Tyr Gln Lys Cys Cys Asn Leu Phe Glu Lys Phe Phe Pro Ser Ser Ser
    690                 695                 700

Tyr Arg Arg Pro Val Gly Ile Ser Ser Met Val Glu Ala Met Val Ser
705                 710                 715                 720

Arg Ala Arg Ile Asp Ala Arg Ile Asp Phe Glu Ser Gly Arg Ile Lys
                725                 730                 735

Lys Glu Glu Phe Ala Glu Ile Met Lys Ile Cys Ser Thr Ile Glu Glu
            740                 745                 750

Leu Arg Arg Gln Lys
        755
```